(19) Europäisches Patentamt
European Patent Office
Office européen des brevets

(11) EP 1 469 065 A1

(12) # EUROPEAN PATENT APPLICATION
published in accordance with Art. 158(3) EPC

(43) Date of publication:
**20.10.2004 Bulletin 2004/43**

(21) Application number: **02793391.0**

(22) Date of filing: **25.12.2002**

(51) Int Cl.[7]: **C12N 5/10**, C12N 15/00, A01K 67/027, A01H 5/00, C07K 16/28, A61K 39/395, A61P 35/00, A61P 37/00

(86) International application number:
**PCT/JP2002/013534**

(87) International publication number:
**WO 2003/055993 (10.07.2003 Gazette 2003/28)**

(84) Designated Contracting States:
**AT BE BG CH CY CZ DE DK EE ES FI FR GB GR IE IT LI LU MC NL PT SE SI SK TR**
Designated Extension States:
**AL LT LV MK RO**

(30) Priority: **25.12.2001 JP 2001392753**
**09.04.2002 JP 2002106948**
**01.11.2002 JP 2002319975**

(71) Applicant: **KYOWA HAKKO KOGYO CO., LTD.**
**Chiyoda-ku, Tokyo 100-8185 (JP)**

(72) Inventors:
- **SHITARA, Kenya, Kyowa Hakko Kogyo Co., Ltd.**
**Machida-shi, Tokyo 194-8533 (JP)**
- **SAKURADA, Mikiko,**
**Kyowa Hakko Kogyo Co., Ltd.**
**Machida-shi, Tokyo 194-8533 (JP)**
- **UCHIDA, Kazuhisa,**
**Kyowa Hakko Kogyo Co., Ltd.**
**Machida-shi, Tokyo 194-8533 (JP)**
- **SHINKAWA, Toyohide,**
**Kyowa Hakko Kogyo Co., Ltd.**
**Machida-shi, Tokyo 194-8533 (JP)**
- **SATOH, Mitsuo, Kyowa Hakko Kogyo Co., Ltd.**
**Machida-shi, Tokyo 194-8533 (JP)**
- **NAKANO, Ryosuke,**
**Kyowa Hakko Kogyo Co., Ltd.**
**Machida-shi, Tokyo 194-8533 (JP)**

(74) Representative: **VOSSIUS & PARTNER**
**Siebertstrasse 4**
**81675 München (DE)**

(54) **COMPOSITION OF ANTIBODY SPECIFICALLY BINDING TO CD20**

(57) The present invention provides an antibody composition which specifically binds to CD20 and comprises an antibody molecule which has complex N-glycoside-linked sugar chains bound to the Fc region; a process for producing the antibody composition; and a medicament comprising the antibody composition.

EP 1 469 065 A1

SEQUENCE LISTING

<110> KYOWA HAKKO KOGYO CO., LTD.

<120> ANTI-CD20 ANTIBODY COMPOSITION

<130> K 2085 EP

<140> JP 2001-392753
<141> 2001-12-25

<140> JP 2002-106948
<141> 2001-04-09

<140> JP 2002-319975
<141> 2001-11-01

<160> 63

<170> PatentIn Ver. 2.1

<210> 1
<211> 2008
<212> DNA
<213> Cricetulus griseus

<400> 1
aacagaaact tattttcctg tgtggctaac tagaaccaga gtacaatgtt tccaattctt 60

tgagctccga gaagacagaa gggagttgaa actctgaaaa tgcgggcatg gactggttcc 120

tggcgttgga ttatgctcat tctttttgcc tgggggacct tattgtttta tataggtggt 180

catttggttc gagataatga ccaccctgac cattctagca gagaactctc caagattctt 240

gcaaagctgg agcgcttaaa acaacaaaat gaagacttga ggagaatggc tgagtctctc 300

cgaataccag aaggccctat tgatcagggg acagctacag gaagagtccg tgttttagaa 360

gaacagcttg ttaaggccaa agaacagatt gaaaattaca agaaacaagc taggaatgat 420

ctgggaaagg atcatgaaat cttaaggagg aggattgaaa atggagctaa agagctctgg 480

ttttttctac aaagtgaatt gaagaaatta aagaaattag aaggaaacga actccaaaga 540

catgcagatg aaattctttt ggatttagga catcatgaaa ggtctatcat gacagatcta 600

tactacctca gtcaaacaga tggagcaggt gagtggcggg aaaaagaagc caaagatctg 660

acagagctgg tccagcggag aataacatat ctgcagaatc ccaaggactg cagcaaagcc 720

agaaagctgg tatgtaatat caacaaaggc tgtggctatg gatgtcaact ccatcatgtg 780

gtttactgct tcatgattgc ttatggcacc cagcgaacac tcatcttgga atctcagaat 840

tggcgctatg ctactggagg atgggagact gtgtttagac ctgtaagtga gacatgcaca 900

gacaggtctg gcctctccac tggacactgg tcaggtgaag tgaaggacaa aaatgttcaa 960

gtggtcgagc tccccattgt agacagcctc catcctcgtc ctccttactt acccttggct 1020

gtaccagaag accttgcaga tcgactcctg agagtccatg gtgatcctgc agtgtggtgg 1080

gtatcccagt ttgtcaaata cttgatccgt ccacaacctt ggctggaaag ggaaatagaa 1140

gaaaccacca agaagcttgg cttcaaacat ccagttattg gagtccatgt cagacgcact 1200

gacaaagtgg gaacagaagc agccttccat cccattgagg aatacatggt acacgttgaa 1260

gaacattttc agcttctcga acgcagaatg aaagtggata aaaaaagagt gtatctggcc 1320

actgatgacc cttctttgtt aaaggaggca aagacaaagt actccaatta tgaatttatt 1380

agtgataact ctatttcttg gtcagctgga ctacacaacc gatacacaga aaattcactt 1440

cggggcgtga tcctggatat acactttctc tcccaggctg acttccttgt gtgtactttt 1500

tcatcccagg tctgtagggt tgcttatgaa atcatgcaaa cactgcatcc tgatgcctct 1560

gcaaacttcc attctttaga tgacatctac tattttggag gccaaaatgc ccacaaccag 1620

attgcagttt atcctcacca acctcgaact aaagaggaaa tccccatgga acctggagat 1680

atcattggtg tggctggaaa ccattggaat ggttactcta aaggtgtcaa cagaaaacta 1740

ggaaaaacag gcctgtaccc ttcctacaaa gtccgagaga agatagaaac agtcaaatac 1800

cctacatatc ctgaagctga aaaatagaga tggagtgtaa gagattaaca acagaattta 1860

gttcagacca tctcagccaa gcagaagacc cagactaaca tatggttcat tgacagacat 1920

gctccgcacc aagagcaagt gggaaccctc agatgctgca ctggtggaac gcctctttgt 1980

gaagggctgc tgtgccctca agcccatg 2008

<210> 2
<211> 1728
<212> DNA
<213> Mus musculus

<400> 2
atgcgggcat ggactggttc ctggcgttgg attatgctca ttctttttgc ctgggggacc 60

ttgttatttt atataggtgg tcatttggtt cgagataatg accaccctga tcactccagc 120

agagaactct ccaagattct tgcaaagctt gaacgcttaa aacagcaaaa tgaagacttg 180

aggcgaatgg ctgagtctct ccgaatacca gaaggcccca ttgaccaggg gacagctaca 240

ggaagagtcc gtgttttaga agaacagctt gttaaggcca aagaacagat tgaaaattac 300

aagaaacaag ctagaaatgg tctggggaag gatcatgaaa tcttaagaag gaggattgaa 360

aatggagcta aagagctctg gtttttcta caaagcgaac tgaagaaatt aaagcattta 420

gaaggaaatg aactccaaag acatgcagat gaaattcttt tggatttagg acaccatgaa 480

aggtctatca tgacagatct atactacctc agtcaaacag atggagcagg ggattggcgt 540

gaaaaagagg ccaaagatct gacagagctg gtccagcgga gaataacata tctccagaat 600

cctaaggact gcagcaaagc caggaagctg gtgtgtaaca tcaataaagg ctgtggctat 660

ggttgtcaac tccatcacgt ggtctactgt ttcatgattg cttatggcac ccagcgaaca 720

ctcatcttgg aatctcagaa ttggcgctat gctactggtg gatgggagac tgtgtttaga 780

cctgtaagtg agacatgtac agacagatct ggcctctcca ctggacactg gtcaggtgaa 840

gtaaatgaca aaaacattca agtggtcgag ctccccattg tagacagcct ccatcctcgg 900

cctccttact taccactggc tgttccagaa gaccttgcag accgactcct aagagtccat 960

ggtgaccctg cagtgtggtg ggtgtcccag tttgtcaaat acttgattcg tccacaacct 1020

tggctggaaa aggaaataga agaagccacc aagaagcttg gcttcaaaca tccagttatt 1080

ggagtccatg tcagacgcac agacaaagtg ggaacagaag cagccttcca ccccatcgag 1140

gagtacatgg tacacgttga agaacatttt cagcttctcg cacgcagaat gcaagtggat 1200

aaaaaaagag tatatctggc tactgatgat cctactttgt taaaggaggc aaagacaaag 1260

tactccaatt atgaatttat tagtgataac tctatttctt ggtcagctgg actacacaat 1320

cggtacacag aaaattcact tcggggtgtg atcctggata tacactttct ctcacaggct 1380

gactttctag tgtgtacttt ttcatcccag gtctgtcggg ttgcttatga aatcatgcaa 1440

accctgcatc ctgatgcctc tgcgaacttc cattctttgg atgacatcta ctattttgga 1500

ggccaaaatg cccacaatca gattgctgtt tatcctcaca aacctcgaac tgaagaggaa 1560

attccaatgg aacctggaga tatcattggt gtggctggaa accattggga tggttattct 1620

aaaggtatca acagaaaact tggaaaaaca ggcttatatc cctcctacaa agtccgagag 1680

aagatagaaa cagtcaagta tcccacatat cctgaagctg aaaaatag 1728

<210> 3
<211> 9196
<212> DNA
<213> Cricetulus griseus

<400> 3
tctagaccag gctggtctcg aactcacaga gaaccacctg cctctgccac ctgagtgctg 60

ggattaaagg tgtgcaccac caccgcccgg cgtaaaatca tatttttgaa tattgtgata 120

atttacatta taattgtaag taaaaatttt cagcctattt tgttatacat ttttgcgtaa 180

attattcttt tttgaaagtt ttgttgtcca taatagtcta gggaaacata aagttataat 240

ttttgtctat gtatttgcat atatatctat ttaatctcct aatgtccagg aaataaatag 300

ggtatgtaat agcttcaaca tgtggtatga tagaattttt cagtgctata taagttgtta 360

cagcaaagtg ttattaattc atatgtccat atttcaattt tttatgaatt attaaattga 420

atccttaagc tgccagaact agaattttat tttaatcagg aagccccaaa tctgttcatt 480

ctttctatat atgtggaaag gtaggcctca ctaactgatt cttcacctgt tttagaacat 540

ggtccaagaa tggagttatg taaggggaat tacaagtgtg agaaaactcc tagaaaacaa 600

gatgagtctt gtgaccttag tttctttaaa aacacaaaat tcttggaatg tgttttcatg 660

ttcctcccag gtggatagga gtgagtttat ttcagattat ttattacaac tggctgttgt 720

tacttgtttc tatgtcttta tagaaaaaca tatttttttt gccacatgca gcttgtcctt 780

atgattttat acttgtgtga ctcttaactc tcagagtata aattgtctga tgctatgaat 840

aaagttggct attgtatgag acttcagccc acttcaatta ttggcttcat tctctcagat 900

cccaccacct ccagagtggt aaacaacttg aaccattaaa cagactttag tctttatttg 960

aatgatagat ggggatatca gatttatagg cacagggttt tgagaaaggg agaaggtaaa 1020

cagtagagtt taacaacaac aaaaagtata ctttgtaaac gtaaaactat ttattaaagt 1080

agtagacaag acattaaata ttccttggga ttagtgcttt ttgaattttg ctttcaaata 1140

atagtcagtg agtatacccc tcccccattc tatattttag cagaaatcag aataaatggt 1200

gtttctggta cattcttttg tagagaattt attttctttg ggtttttgtg catttaaagt 1260

caataaaaat taaggttcag taatagaaaa aaaactctga tttttggaat ccccttcctt 1320

cagcttttct atttaatctc ttaatgataa tttaatttgt ggccatgtgg tcaaagtata 1380

tagccttgta tatgtaaatg ttttaaccaa cctgccttta cagtaactat ataattttat 1440

tctataatat atgacttttc ttccatagct ttagagttgc ccagtcactt taagttacat 1500

tttcatatat gttctttgtg ggaggagata attttatttc taagagaatc ctaagcatac 1560

tgattgagaa atggcaaaca aaacacataa ttaaagctga taaagaacga acatttggag 1620

tttaaaatac atagccaccc taagggttta actgttgtta gccttctttt ggaattttta 1680

ttagttcata tagaaaaatg gattttatcg tgacatttcc atatatgtat ataatatatt 1740

tacatcatat ccacctgtaa ttattagtgt ttttaaatat atttgaaaaa ataatggtct 1800

ggtttgatcc atttgaacct tttgatgttt ggtgtggttg ccaattggtt gatggttatg 1860

ataacctttg cttctctaag gttcaagtca gtttgagaat atgtcctcta aaaatgacag 1920

gttgcaagtt aagtagtgag atgacagcga gatggagtga tgagaatttg tagaaatgaa 1980

ttcacttata ctgagaactt gttttgcttt tagataatga acatattagc ctgaagtaca 2040

tagccgaatt gattaattat tcaaagatat aatcttttaa tccctataaa agaggtatta 2100

cacaacaatt caagaaagat agaattagac ttccagtatt ggagtgaacc atttgttatc 2160

aggtagaacc ctaacgtgtg tggttgactt aaagtgttta ctttttacct gatactgggt 2220

agctaattgt ctttcagcct cctggccaaa gataccatga aagtcaactt acgttgtatt 2280

ctatatctca aacaactcag ggtgtttctt actctttcca cagcatgtag agcccaggaa 2340

gcacaggaca agaaagctgc ctccttgtat caccaggaag atctttttgt aagagtcatc 2400

acagtatacc agagagacta attttgtctg aagcatcatg tgttgaaaca acagaaactt 2460

attttcctgt gtggctaact agaaccagag tacaatgttt ccaattcttt gagctccgag 2520

aagacagaag ggagttgaaa ctctgaaaat gcgggcatgg actggttcct ggcgttggat 2580

tatgctcatt ctttttgcct gggggacctt attgttttat ataggtggtc atttggttcg 2640

agataatgac caccctgacc attctagcag agaactctcc aagattcttg caaagctgga 2700

gcgcttaaaa caacaaaatg aagacttgag gagaatggct gagtctctcc ggtaggtttg 2760

aaatactcaa ggatttgatg aaatactgtg cttgaccttt aggtataggg tctcagtctg 2820

ctgttgaaaa atataatttc tacaaaccgt ctttgtaaaa ttttaagtat tgtagcagac 2880

tttttaaaag tcagtgatac atctatatag tcaatatagg tttacatagt tgcaatctta 2940

ttttgcatat gaatcagtat atagaagcag tggcatttat atgcttatgt tgcatttaca 3000

attatgttta gacgaacaca aactttatgt gatttggatt agtgctcatt aaattttttt 3060

attctatgga ctacaacaga gacataaatt ttgaaaggct tagttactct taaattctta 3120

tgatgaaaag caaaaattca ttgttaaata gaacagtgca tccggaatgt gggtaattat 3180

tgccatattt ctagtctact aaaaattgtg gcataactgt tcaaagtcat cagttgtttg 3240

gaaagccaaa gtctgattta aatggaaaac ataaacaatg atatctattt ctagatacct 3300

ttaacttgca gttactgagt ttacaagttg tctgacaact ttggattctc ttacttcata 3360

tctaagaatg atcatgtgta cagtgcttac tgtcacttta aaaaactgca gggctagaca 3420

tgcagatatg aagactttga cattagatgt ggtaattggc actaccagca agtggtatta 3480

agatacagct gaatatatta ctttttgagg aacataattc atgaatggaa agtggagcat 3540

tagagaggat gccttctggc tctcccacac cactgtttgc atccattgca tttcacactg 3600

cttttagaac tcagatgttt catatggtat attgtgtaac tcaccatcag ttttatcttt 3660

aaatgtctat ggatgataat gttgtatgtt aacactttta caaaaacaaa tgaagccata 3720

tcctcggtgt gagttgtgat ggtggtaatt gtcacaatag gattattcag caaggaacta 3780

agtcagggac aagaagtggg cgatactttg ttggattaaa tcattttact ggaagttcat 3840

cagggagggt tatgaaagtt gtggtctttg aactgaaatt atatgtgatt cattattctt 3900

gatttaggcc ttgctaatag taactatcat ttattgggaa tttgtcatat gtgccaattt 3960

gtcatgggcc agacagcgtg ttttactgaa tttctagata tctttatgag attctagtac 4020

tgttttcagc cattttacag atgaagaatc ttaaaaaatg ttaaataatt tagtttgccc 4080

aagattatac gttaacaaat ggtagaacct tctttgaatt ctggcagtat ggctacacag 4140

tccgaactct tatcttccta agctgaaaac agaaaaagca atgacccaga aaattttatt 4200

taaaagtctc aggagagact tcccatcctg agaagatctc ttttcccttt tataatttag 4260

gctcctgaat aatcactgaa ttttctccat gttccatcta tagtactgtt atttctgttt 4320

tcctttttc ttaccacaaa gtatcttgtt tttgctgtat gaaagaaaat gtgttattgt 4380

aatgtgaaat tctctgtccc tgcagggtcc cacatccgcc tcaatcccaa ataaacacac 4440

agaggctgta ttaattatga aactgttggt cagttggcta gggcttctta ttggctagct 4500

ctgtcttaat tattaaacca taactactat tgtaagtatt tccatgtggt cttatcttac 4560

caaggaaagg gtccagggac ctcttactcc tctggcgtgt tggcagtgaa gaggagagag 4620

cgatttccta tttgtctctg cttattttct gattctgctc agctatgtca cttcctgcct 4680

ggccaatcag ccaatcagtg ttttattcat tagccaataa aagaaacatt tacacagaag 4740

gacttccccc atcatgttat ttgtatgagt tcttcagaaa atcatagtat cttttaatac 4800

taatttttat aaaaaattaa ttgtattgaa aattatgtgt atatgtgtct gtgtgtcgat 4860

ttgtgctcat aagtagcatg gagtgcagaa gagggaatca gatcttttt taagggacaa 4920

agagtttatt cagattacat tttaaggtga taatgtatga ttgcaaggtt atcaacatgg 4980

cagaaatgtg aagaagctgg tcacattaca tccagagtca agagtagaga gcaatgaatt 5040

gatgcatgca ttcctgtgct cagctcactt ttcctggagc tgagctgatt gtaagccatc 5100

tgatgtcttt gctgggaact aactcaaagg caagttcaaa acctgttctt aagtataagc 5160

catctctcca gtccctcata tggtctctta agacactttc tttatattct tgtacataga 5220

aattgaattc ctaacaactg cattcaaatt acaaaatagt ttttaaaagc tgatataata 5280

aatgtaaata caatctagaa catttttata aataagcata ttaactcagt aaaaataaat 5340

gcatggttat tttccttcat tagggaagta tgtctcccca ggctgttctc tagattctac 5400

tagtaatgct gtttgtacac catccacagg ggttttattt taaagctaag acatgaatga 5460

tggacatgct tgttagcatt tagacttttt tccttactat aattgagcta gtatttttgt 5520

gctcagtttg atatctgtta attcagataa atgtaatagt aggtaatttc tttgtgataa 5580

aggcatataa attgaagttg gaaaacaaaa gcctgaaatg acagtttta agattcagaa 5640

caataatttt caaaagcagt tacccaactt tccaaataca atctgcagtt ttcttgatat 5700

gtgataaatt tagacaaaga aatagcacat tttaaaatag ctatttactc ttgatttttt 5760

tttcaaattt aggctagttc actagttgtg tgtaaggtta tggctgcaaa catctttgac 5820

tcttggttag ggaatccagg atgatttacg tgtttggcca aaatcttgtt ccattctggg 5880

tttcttctct atctaggtag ctagcacaag ttaaaggtgt ggtagtattg gaaggctctc 5940

aggtatatat ttctatattc tgtatttttt tcctctgtca tatatttgct ttctgtttta 6000

ttgatttcta ctgttagttt gatacttact ttcttacact ttctttggga tttattttgc 6060

tgttctaaga tttcttagca agttcatatc actgatttta acagttgctt cttttgtaat 6120

atagactgaa tgccccttat ttgaaatgct tgggatcaga aactcagatt tgaacttttc 6180

ttttttaata tttccatcaa gtttaccagc tgaatgtcct gatccaagaa tatgaaatct 6240

gaaatgcttt gaaatctgaa acttttagag tgataaagct tccctttaaa ttaatttgtg 6300

ttctatattt tttgacaatg tcaacctttc attgttatcc aatgagtgaa catattttca 6360

atttttttgt ttgatctgtt atattttgat ctgaccatat ttataaaatt ttatttaatt 6420

tgaatgttgt gctgttactt atctttatta ttatttttgc ttattttcta gccaaatgaa 6480

attatattct gtattatttt agtttgaatt ttactttgtg gcttagtaac tgccttttgt 6540

tggtgaatgc ttaagaaaaa cgtgtggtct actgatattg gttctaatct tatatagcat 6600

gttgtttgtt aggtagttga ttatgctggt cagattgtct tgagtttatg caaatgtaaa 6660

atatttagat gcttgttttg ttgtctaaga acaaagtatg cttgctgtct cctatcggtt 6720

ctggtttttc cattcatctc ttcaagctgt tttgtgtgtt gaatactaac tccgtactat 6780

cttgttttct gtgaattaac cccttttcaa aggtttcttt tctttttttt tttaagggac 6840

aacaagttta ttcagattac attttaagct gataatgtat gattgcaagg ttatcaacat 6900

ggcagaaatg tgaagaagct aggcacatta catccacatg gagtcaagag cagagagcag 6960

tgaattaatg catgcattcc tgtggtcagc tcacttttcc tattcttaga tagtctagga 7020

tcataaacct ggggaatagt gctaccacaa tgggcatatc cacttacttc agttcatgca 7080

atcaaccaag gcacatccac aggaaaaact gatttagaca acctctcatt gagactcttc 7140

ccagatgatt agactgtgtc aagttgacaa ttaaaactat cacacctgaa gccatcacta 7200

gtaaatataa tgaaaatgtt gattatcacc ataattcatc tgtatccctt tgttattgta 7260

gattttgtga agttcctatt caagtccctg ttccttcctt aaaaacctgt tttttagtta 7320

aataggtttt ttagtgttcc tgtctgtaaa tactttttta aagttagata ttattttcaa 7380

gtatgttctc ccagtctttg gcttgtattt tcatcccttc aatacatata tttttgtaat 7440

ttattttttt tatttaaatt agaaacaaag ctgcttttac atgtcagtct cagttccctc 7500

tccctcccct cctcccctgc tccccaccta agccccaatt ccaactcctt tcttctcccc 7560

aggaagggtg aggccctcca tgggggaaat cttcaatgtc tgtcatatca tttggagcag 7620

ggcctagacc ctccccagtg tgtctaggct gagagagtat ccctctatgt ggagagggct 7680

cccaaagttc atttgtgtac taggggtaaa tactgatcca ctatcagtgg ccccatagat 7740

tgtccggacc tccaaactga cttcctcctt cagggagtct ggaacagttc tatgctggtt 7800

tcccagatat cagtctgggg tccatgagca accccttgtt caggtcagtt gtttctgtag 7860

gtttccccag cccggtcttg accccttttgc tcatcacttc tccctctctg caactggatt 7920

ccagagttca gctcagtgtt tagctgtggg tgtctgcatc tgcttccatc agctactgga 7980

tgagggctct aggatggcat ataaggtagt catcagtctc attatcagag aagggctttt 8040

aaggtagcct cttgattatt gcttagattg ttagttgggg tcaaccttgt aggtctctgg 8100

acagtgacag aattctcttt aaacctataa tggctccctc tgtggtggta tccctttct 8160

tgctctcatc cgttcctccc ctgactagat cttcctgctc cctcatgtcc tcctctcccc 8220

tccccttctc cccttctctt tcttctaact ccctctcccc tccacccacg atccccatta 8280

gcttatgaga tcttgtcctt attttagcaa aaccttttg gctataaaat taattaattt 8340

aatatgctta tatcaggttt attttggcta gtatttgtat gtgtttggtt agtgttttta 8400

accttaattg acatgtatcc ttatatttag acacagattt aaatatttga agtttttttt 8460

tttttttttt ttaaagattt atttattttt tatgtcttct gcctgcatgc cagaagaggg 8520

caccagatct cattcaaggt ggttgtgagc caccatgtgg ttgctgggaa ttgaactcag 8580

gacctctgga agaacagtca gtgctcttaa ccgctgagcc atctctccag cccctgaagt 8640

gtttctttta aagaggatag cagtgcatca tttttccctt tgaccaatga ctcctacctt 8700

actgaattgt tttagccatt tatatgtaat gctgttacca ggtttacatt ttcttttatc 8760

ttgctaaatt tcttccctgt ttgtctcatc tcttattttt gtctgttgga ttatataggc 8820

ttttattttt ctgtttttac agtaagttat atcaaattaa aattatttta tggaatgggt 8880

gtgttgacta catgtatgtc tgtgcaccat gtgctgacct ggtcttggcc agaagaaggt 8940

gtcatattct ctgaaactgg tattgtggat gttacgaact gccatagggt gctaggaatc 9000

aaaccccagc tcctctggaa aagcagccac tgctctgagc cactgagtcc tctcttcaag 9060

caggtgatgc caacttttaa tggttaccag tggataagag tgcttgtatc tctagcaccc 9120

atgaaaattt atgcattgct atatgggctt gtcacttcag cattgtgtga cagagacagg 9180

aggatcccaa gagctc 9196

<210> 4
<211> 297
<212> PRT
<213> Homo sapiens

<400> 4
Met Thr Thr Pro Arg Asn Ser Val Asn Gly Thr Phe Pro Ala Glu Pro
1 5 10 15

Met Lys Gly Pro Ile Ala Met Gln Ser Gly Pro Lys Pro Leu Phe Arg
20 25 30

Arg Met Ser Ser Leu Val Gly Pro Thr Gln Ser Phe Phe Met Arg Glu
35 40 45

Ser Lys Thr Leu Gly Ala Val Gln Ile Met Asn Gly Leu Phe His Ile
50 55 60

Ala Leu Gly Gly Leu Leu Met Ile Pro Ala Gly Ile Tyr Ala Pro Ile
65 70 75 80

Cys Val Thr Val Trp Tyr Pro Leu Trp Gly Gly Ile Met Tyr Ile Ile
85 90 95

Ser Gly Ser Leu Leu Ala Ala Thr Glu Lys Asn Ser Arg Lys Cys Leu
100 105 110

Val Lys Gly Lys Met Ile Met Asn Ser Leu Ser Leu Phe Ala Ala Ile
115 120 125

Ser Gly Met Ile Leu Ser Ile Met Asp Ile Leu Asn Ile Lys Ile Ser
130 135 140

His Phe Leu Lys Met Glu Ser Leu Asn Phe Ile Arg Ala His Thr Pro
145 150 155 160

Tyr Ile Asn Ile Tyr Asn Cys Glu Pro Ala Asn Pro Ser Glu Lys Asn
165 170 175

Ser Pro Ser Thr Gln Tyr Cys Tyr Ser Ile Gln Ser Leu Phe Leu Gly
180 185 190

Ile Leu Ser Val Met Leu Ile Phe Ala Phe Phe Gln Glu Leu Val Ile
195 200 205

Ala Gly Ile Val Glu Asn Glu Trp Lys Arg Thr Cys Ser Arg Pro Lys
210 215 220

Ser Asn Ile Val Leu Leu Ser Ala Glu Glu Lys Lys Glu Gln Thr Ile
225 230 235 240

Glu Ile Lys Glu Glu Val Val Gly Leu Thr Glu Thr Ser Ser Gln Pro
245 250 255

Lys Asn Glu Glu Asp Ile Glu Ile Ile Pro Ile Gln Glu Glu Glu Glu
260 265 270

Glu Glu Thr Glu Thr Asn Phe Pro Glu Pro Pro Gln Asp Gln Glu Ser
275 280 285

Ser Pro Ile Glu Asn Asp Ser Ser Pro
290 295

<210> 5
<211> 10
<212> PRT
<213> Mus musculus

<400> 5

Arg Ala Ser Ser Ser Val Ser Tyr Ile His
1 5

<210> 6
<211> 7
<212> PRT
<213> Mus musculus

<400> 6
Ala Thr Ser Asn Leu Ala Ser
1 5

<210> 7
<211> 9
<212> PRT
<213> Mus musculus

<400> 7
Gln Gln Trp Thr Ser Asn Pro Pro Thr
1 5

<210> 8
<211> 5
<212> PRT
<213> Mus musculus

<400> 8
Ser Tyr Asn Met His
1

<210> 9
<211> 17

<212> PRT
<213> Mus musculus

<400> 9
Ala Ile Tyr Pro Gly Asn Gly Asp Thr Ser Tyr Asn Gln Lys Phe Lys
1 5 10 15

Gly

<210> 10
<211> 12
<212> PRT
<213> Mus musculus

<400> 10
Ser Thr Tyr Tyr Gly Gly Asp Trp Tyr Phe Asn Val
1 5 10
<210> 11
<211> 384
<212> DNA
<213> Mus musculus

<400> 11
atg gat ttt cag gtg cag att atc agc ttc ctg cta atc agt gct tca 48
Met Asp Phe Gln Val Gln Ile Ile Ser Phe Leu Leu Ile Ser Ala Ser
1 5 10 15

gtc ata atg tcc aga gga caa att gtt ctc tcc cag tct cca gca atc 96
Val Ile Met Ser Arg Gly Gln Ile Val Leu Ser Gln Ser Pro Ala Ile
20 25 30

ctg tct gca tct cca ggg gag aag gtc aca atg act tgc agg gcc agc 144
Leu Ser Ala Ser Pro Gly Glu Lys Val Thr Met Thr Cys Arg Ala Ser
35 40 45

tca agt gta agt tac atc cac tgg ttc cag cag aag cca gga tcc tcc 192

Ser Ser Val Ser Tyr Ile His Trp Phe Gln Gln Lys Pro Gly Ser Ser
50 55 60

ccc aaa ccc tgg att tat gcc aca tcc aac ctg gct tct gga gtc cct 240
Pro Lys Pro Trp Ile Tyr Ala Thr Ser Asn Leu Ala Ser Gly Val Pro
65 70 75 80
gtt cgc ttc agt ggc agt ggg tct ggg act tct tac tct ctc acc atc 288
Val Arg Phe Ser Gly Ser Gly Ser Gly Thr Ser Tyr Ser Leu Thr Ile
85 90 95

agc aga gtg gag gct gaa gat gct gcc act tat tac tgc cag cag tgg 336
Ser Arg Val Glu Ala Glu Asp Ala Ala Thr Tyr Tyr Cys Gln Gln Trp
100 105 110

act agt aac cca ccc acg ttc gga ggg ggg acc aag ctg gaa atc aaa 384
Thr Ser Asn Pro Pro Thr Phe Gly Gly Gly Thr Lys Leu Glu Ile Lys
115 120 125

<210> 12
<211> 128
<212> PRT
<213> Mus musculus

<400> 12
Met Asp Phe Gln Val Gln Ile Ile Ser Phe Leu Leu Ile Ser Ala Ser
1 5 10 15

Val Ile Met Ser Arg Gly Gln Ile Val Leu Ser Gln Ser Pro Ala Ile
20 25 30

Leu Ser Ala Ser Pro Gly Glu Lys Val Thr Met Thr Cys Arg Ala Ser
35 40 45

Ser Ser Val Ser Tyr Ile His Trp Phe Gln Gln Lys Pro Gly Ser Ser
50 55 60

Pro Lys Pro Trp Ile Tyr Ala Thr Ser Asn Leu Ala Ser Gly Val Pro
65 70 75 80

Val Arg Phe Ser Gly Ser Gly Ser Gly Thr Ser Tyr Ser Leu Thr Ile
85 90 95

Ser Arg Val Glu Ala Glu Asp Ala Ala Thr Tyr Tyr Cys Gln Gln Trp
100 105 110

Thr Ser Asn Pro Pro Thr Phe Gly Gly Gly Thr Lys Leu Glu Ile Lys
115 120 125

<210> 13
<211> 420
<212> DNA
<213> Mus musculus

<400> 13
atg ggt tgg agc ctc atc ttg ctc ttc ctt gtc gct gtt gct acg cgt 48
Met Gly Trp Ser Leu Ile Leu Leu Phe Leu Val Ala Val Ala Thr Arg
1 5 10 15

gtc ctg tcc cag gta caa ctg cag cag cct ggg gct gag ctg gtg aag 96
Val Leu Ser Gln Val Gln Leu Gln Gln Pro Gly Ala Glu Leu Val Lys
20 25 30

cct ggg gcc tca gtg aag atg tcc tgc aag gct tct ggc tac aca ttt 144
Pro Gly Ala Ser Val Lys Met Ser Cys Lys Ala Ser Gly Tyr Thr Phe
35 40 45

acc agt tac aat atg cac tgg gta aaa cag aca cct ggt cgg ggc ctg 192
Thr Ser Tyr Asn Met His Trp Val Lys Gln Thr Pro Gly Arg Gly Leu
50 55 60

gaa tgg att gga gct att tat ccc gga aat ggt gat act tcc tac aat 240

Glu Trp Ile Gly Ala Ile Tyr Pro Gly Asn Gly Asp Thr Ser Tyr Asn
65 70 75 80

cag aag ttc aaa ggc aag gcc aca ttg act gca gac aaa tcc tcc agc 288
Gln Lys Phe Lys Gly Lys Ala Thr Leu Thr Ala Asp Lys Ser Ser Ser
85 90 95

aca gcc tac atg cag ctc agc agc ctg aca tct gag gac tct gcg gtc 336
Thr Ala Tyr Met Gln Leu Ser Ser Leu Thr Ser Glu Asp Ser Ala Val
100 105 110

tat tac tgt gca aga tcg act tac tac ggc ggt gac tgg tac ttc aat 384
Tyr Tyr Cys Ala Arg Ser Thr Tyr Tyr Gly Gly Asp Trp Tyr Phe Asn
115 120 125

gtc tgg ggc gca ggg acc acg gtc acc gtc tct gca 420
Val Trp Gly Ala Gly Thr Thr Val Thr Val Ser Ala
130 135 140

<210> 14
<211> 140
<212> PRT
<213> Mus musculus

<400> 14
Met Gly Trp Ser Leu Ile Leu Leu Phe Leu Val Ala Val Ala Thr Arg
1 5 10 15

Val Leu Ser Gln Val Gln Leu Gln Gln Pro Gly Ala Glu Leu Val Lys
20 25 30

Pro Gly Ala Ser Val Lys Met Ser Cys Lys Ala Ser Gly Tyr Thr Phe
35 40 45

Thr Ser Tyr Asn Met His Trp Val Lys Gln Thr Pro Gly Arg Gly Leu
50 55 60

Glu Trp Ile Gly Ala Ile Tyr Pro Gly Asn Gly Asp Thr Ser Tyr Asn
65 70 75 80

Gln Lys Phe Lys Gly Lys Ala Thr Leu Thr Ala Asp Lys Ser Ser Ser
85 90 95

Thr Ala Tyr Met Gln Leu Ser Ser Leu Thr Ser Glu Asp Ser Ala Val
100 105 110

Tyr Tyr Cys Ala Arg Ser Thr Tyr Tyr Gly Gly Asp Trp Tyr Phe Asn
115 120 125

Val Trp Gly Ala Gly Thr Thr Val Thr Val Ser Ala
130 135

<210> 15
<211> 91
<212> DNA
<213> Artificial Sequence

<220>
<223> Description of Artificial Sequense: Synthetic DNA

<400> 15
caggaaacag ctatgacgaa ttcgcctcct caaaatggat tttcaggtgc agattatcag 60

cttcctgcta atcagtgctt cagtcataat g 91

<210> 16
<211> 91
<212> DNA
<213> Artificial Sequence

<220>
<223> Description of Artificial Sequense: Synthetic DNA

<400> 16
gtgaccttct cccctggaga tgcagacagg attgctggag actgggagag aacaatttgt 60

cctctggaca ttatgactga agcactgatt a 91

<210> 17
<211> 90
<212> DNA
<213> Artificial Sequence

<220>
<223> Description of Artificial Sequense: Synthetic DNA

<400> 17
ctccagggga gaaggtcaca atgacttgca gggccagctc aagtgtaagt tacatccact 60

ggttccagca gaagccagga tcctccccca 90

<210> 18
<211> 89
<212> DNA
<213> Artificial Sequence

<220>
<223> Description of Artificial Sequense: Synthetic DNA

<400> 18
ccagacccac tgccactgaa gcgaacaggg actccagaag ccaggttgga tgtggcataa 60

atccagggtt tgggggagga tcctggctt 89

<210> 19
<211> 91
<212> DNA
<213> Artificial Sequence

<220>
<223> Description of Artificial Sequense: Synthetic DNA

<400> 19
tcagtggcag tgggtctggg acttcttact ctctcaccat cagcagagtg gaggctgaag 60

atgctgccac ttattactgc cagcagtgga c 91

<210> 20
<211> 90
<212> DNA
<213> Artificial Sequence

<220>
<223> Description of Artificial Sequense: Synthetic DNA

<400> 20
gttttcccag tcacgaccgt acgtttgatt tccagcttgg tcccccctcc gaacgtgggt 60

gggttactag tccactgctg gcagtaataa 90

<210> 21
<211> 24
<212> DNA
<213> Artificial Sequence

<220>
<223> Description of Artificial Sequense: Synthetic DNA

<400> 21
gtctgaagca ttatgtgttg aagc 24

<210> 22
<211> 23
<212> DNA
<213> Artificial Sequence
<220>
<223> Description of Artificial Sequense: Synthetic DNA

<400> 22
gtgagtacat tcattgtact gtg 23

<210> 23
<211> 575
<212> PRT
<213> Cricetulus griseus

<400> 23
Met Arg Ala Trp Thr Gly Ser Trp Arg Trp Ile Met Leu Ile Leu Phe
1 5 10 15

Ala Trp Gly Thr Leu Leu Phe Tyr Ile Gly Gly His Leu Val Arg Asp
20 25 30

Asn Asp His Pro Asp His Ser Ser Arg Glu Leu Ser Lys Ile Leu Ala
35 40 45

Lys Leu Glu Arg Leu Lys Gln Gln Asn Glu Asp Leu Arg Arg Met Ala
50 55 60

Glu Ser Leu Arg Ile Pro Glu Gly Pro Ile Asp Gln Gly Thr Ala Thr
65 70 75 80

Gly Arg Val Arg Val Leu Glu Glu Gln Leu Val Lys Ala Lys Glu Gln
85 90 95

Ile Glu Asn Tyr Lys Lys Gln Ala Arg Asn Asp Leu Gly Lys Asp His
100 105 110

Glu Ile Leu Arg Arg Arg Ile Glu Asn Gly Ala Lys Glu Leu Trp Phe
115 120 125

Phe Leu Gln Ser Glu Leu Lys Lys Leu Lys Lys Leu Glu Gly Asn Glu
130 135 140

Leu Gln Arg His Ala Asp Glu Ile Leu Leu Asp Leu Gly His His Glu
145 150 155 160

Arg Ser Ile Met Thr Asp Leu Tyr Tyr Leu Ser Gln Thr Asp Gly Ala
165 170 175

Gly Glu Trp Arg Glu Lys Glu Ala Lys Asp Leu Thr Glu Leu Val Gln
180 185 190

Arg Arg Ile Thr Tyr Leu Gln Asn Pro Lys Asp Cys Ser Lys Ala Arg
195 200 205

Lys Leu Val Cys Asn Ile Asn Lys Gly Cys Gly Tyr Gly Cys Gln Leu
210 215 220

His His Val Val Tyr Cys Phe Met Ile Ala Tyr Gly Thr Gln Arg Thr
225 230 235 240

Leu Ile Leu Glu Ser Gln Asn Trp Arg Tyr Ala Thr Gly Gly Trp Glu
245 250 255

Thr Val Phe Arg Pro Val Ser Glu Thr Cys Thr Asp Arg Ser Gly Leu
260 265 270

Ser Thr Gly His Trp Ser Gly Glu Val Lys Asp Lys Asn Val Gln Val
275 280 285

Val Glu Leu Pro Ile Val Asp Ser Leu His Pro Arg Pro Pro Tyr Leu
290 295 300

Pro Leu Ala Val Pro Glu Asp Leu Ala Asp Arg Leu Leu Arg Val His
305 310 315 320

Gly Asp Pro Ala Val Trp Trp Val Ser Gln Phe Val Lys Tyr Leu Ile
325 330 335

Arg Pro Gln Pro Trp Leu Glu Arg Glu Ile Glu Glu Thr Thr Lys Lys
340 345 350

Leu Gly Phe Lys His Pro Val Ile Gly Val His Val Arg Arg Thr Asp
355 360 365

Lys Val Gly Thr Glu Ala Ala Phe His Pro Ile Glu Glu Tyr Met Val
370 375 380

His Val Glu Glu His Phe Gln Leu Leu Glu Arg Arg Met Lys Val Asp
385 390 395 400

Lys Lys Arg Val Tyr Leu Ala Thr Asp Asp Pro Ser Leu Leu Lys Glu
405 410 415

Ala Lys Thr Lys Tyr Ser Asn Tyr Glu Phe Ile Ser Asp Asn Ser Ile
420 425 430

Ser Trp Ser Ala Gly Leu His Asn Arg Tyr Thr Glu Asn Ser Leu Arg
435 440 445

Gly Val Ile Leu Asp Ile His Phe Leu Ser Gln Ala Asp Phe Leu Val
450 455 460

Cys Thr Phe Ser Ser Gln Val Cys Arg Val Ala Tyr Glu Ile Met Gln
465 470 475 480

Thr Leu His Pro Asp Ala Ser Ala Asn Phe His Ser Leu Asp Asp Ile
485 490 495

Tyr Tyr Phe Gly Gly Gln Asn Ala His Asn Gln Ile Ala Val Tyr Pro
500 505 510

His Gln Pro Arg Thr Lys Glu Glu Ile Pro Met Glu Pro Gly Asp Ile
515 520 525

Ile Gly Val Ala Gly Asn His Trp Asn Gly Tyr Ser Lys Gly Val Asn
530 535 540

Arg Lys Leu Gly Lys Thr Gly Leu Tyr Pro Ser Tyr Lys Val Arg Glu
545 550 555 560

Lys Ile Glu Thr Val Lys Tyr Pro Thr Tyr Pro Glu Ala Glu Lys

565 570 575

<210> 24
<211> 575
<212> PRT
<213> Mus musculus

<400> 24
Met Arg Ala Trp Thr Gly Ser Trp Arg Trp Ile Met Leu Ile Leu Phe
1 5 10 15

Ala Trp Gly Thr Leu Leu Phe Tyr Ile Gly Gly His Leu Val Arg Asp
20 25 30

Asn Asp His Pro Asp His Ser Ser Arg Glu Leu Ser Lys Ile Leu Ala

35 40 45

Lys Leu Glu Arg Leu Lys Gln Gln Asn Glu Asp Leu Arg Arg Met Ala
50 55 60

Glu Ser Leu Arg Ile Pro Glu Gly Pro Ile Asp Gln Gly Thr Ala Thr
65 70 75 80

Gly Arg Val Arg Val Leu Glu Glu Gln Leu Val Lys Ala Lys Glu Gln
85 90 95

Ile Glu Asn Tyr Lys Lys Gln Ala Arg Asn Gly Leu Gly Lys Asp His
100 105 110

Glu Ile Leu Arg Arg Arg Ile Glu Asn Gly Ala Lys Glu Leu Trp Phe
115 120 125

Phe Leu Gln Ser Glu Leu Lys Lys Leu Lys His Leu Glu Gly Asn Glu
130 135 140

Leu Gln Arg His Ala Asp Glu Ile Leu Leu Asp Leu Gly His His Glu
145 150 155 160

Arg Ser Ile Met Thr Asp Leu Tyr Tyr Leu Ser Gln Thr Asp Gly Ala
165 170 175

Gly Asp Trp Arg Glu Lys Glu Ala Lys Asp Leu Thr Glu Leu Val Gln
180 185 190

Arg Arg Ile Thr Tyr Leu Gln Asn Pro Lys Asp Cys Ser Lys Ala Arg
195 200 205

Lys Leu Val Cys Asn Ile Asn Lys Gly Cys Gly Tyr Gly Cys Gln Leu
210 215 220

His His Val Val Tyr Cys Phe Met Ile Ala Tyr Gly Thr Gln Arg Thr

225 230 235 240

Leu Ile Leu Glu Ser Gln Asn Trp Arg Tyr Ala Thr Gly Gly Trp Glu
245 250 255

Thr Val Phe Arg Pro Val Ser Glu Thr Cys Thr Asp Arg Ser Gly Leu
260 265 270

Ser Thr Gly His Trp Ser Gly Glu Val Asn Asp Lys Asn Ile Gln Val
275 280 285

Val Glu Leu Pro Ile Val Asp Ser Leu His Pro Arg Pro Pro Tyr Leu
290 295 300

Pro Leu Ala Val Pro Glu Asp Leu Ala Asp Arg Leu Leu Arg Val His
305 310 315 320

Gly Asp Pro Ala Val Trp Trp Val Ser Gln Phe Val Lys Tyr Leu Ile
325 330 335

Arg Pro Gln Pro Trp Leu Glu Lys Glu Ile Glu Glu Ala Thr Lys Lys
340 345 350

Leu Gly Phe Lys His Pro Val Ile Gly Val His Val Arg Arg Thr Asp
355 360 365

Lys Val Gly Thr Glu Ala Ala Phe His Pro Ile Glu Glu Tyr Met Val
370 375 380

His Val Glu Glu His Phe Gln Leu Leu Ala Arg Arg Met Gln Val Asp
385 390 395 400

Lys Lys Arg Val Tyr Leu Ala Thr Asp Asp Pro Thr Leu Leu Lys Glu
405 410 415

Ala Lys Thr Lys Tyr Ser Asn Tyr Glu Phe Ile Ser Asp Asn Ser Ile
420 425 430

Ser Trp Ser Ala Gly Leu His Asn Arg Tyr Thr Glu Asn Ser Leu Arg
435 440 445

Gly Val Ile Leu Asp Ile His Phe Leu Ser Gln Ala Asp Phe Leu Val
450 455 460

Cys Thr Phe Ser Ser Gln Val Cys Arg Val Ala Tyr Glu Ile Met Gln
465 470 475 480

Thr Leu His Pro Asp Ala Ser Ala Asn Phe His Ser Leu Asp Asp Ile
485 490 495

Tyr Tyr Phe Gly Gly Gln Asn Ala His Asn Gln Ile Ala Val Tyr Pro
500 505 510

His Lys Pro Arg Thr Glu Glu Glu Ile Pro Met Glu Pro Gly Asp Ile
515 520 525

Ile Gly Val Ala Gly Asn His Trp Asp Gly Tyr Ser Lys Gly Ile Asn
530 535 540

Arg Lys Leu Gly Lys Thr Gly Leu Tyr Pro Ser Tyr Lys Val Arg Glu
545 550 555 560

Lys Ile Glu Thr Val Lys Tyr Pro Thr Tyr Pro Glu Ala Glu Lys
565 570 575

<210> 25
<211> 99
<212> DNA
<213> Artificial Sequence

<220>
<223> Description of Artificial Sequense: Synthetic DNA

<400> 25
caggaaacag ctatgacgcg gccgcgaccc ctcaccatgg gttggagcct catcttgctc 60

ttccttgtcg ctgttgctac gcgtgtcctg tcccaggta 99

<210> 26
<211> 98
<212> DNA
<213> Artificial Sequence

<220>
<223> Description of Artificial Sequense: Synthetic DNA

<400> 26
atgtgtagcc agaagccttg caggacatct tcactgaggc cccagccttc accagctcag 60

ccccaggctg ctgcagttgt acctgggaca ggacacgc 98

<210> 27
<211> 97
<212> DNA
<213> Artificial Sequence

<220>
<223> Description of Artificial Sequense: Synthetic DNA

<400> 27
caaggcttct ggctacacat ttaccagtta caatatgcac tgggtaaaac agacacctgg 60

tcggggcctg gaatggattg gagctattta tcccgga 97

<210> 28
<211> 99
<212> DNA
<213> Artificial Sequence

<220>
<223> Description of Artificial Sequense: Synthetic DNA

<400> 28
gtaggctgtg ctggaggatt tgtctgcagt caatgtggcc ttgcctttga acttctgatt 60

gtaggaagta tcaccatttc cgggataaat agctccaat 99

<210> 29
<211> 99
<212> DNA
<213> Artificial Sequence

<220>
<223> Description of Artificial Sequense: Synthetic DNA

<400> 29
aatcctccag cacagcctac atgcagctca gcagcctgac atctgaggac tctgcggtct 60

attactgtgc aagatcgact tactacggcg gtgactggt 99

<210> 30
<211> 98
<212> DNA
<213> Artificial Sequence

<220>
<223> Description of Artificial Sequense: Synthetic DNA

<400> 30
gttttcccag tcacgacggg cccttggtgg aggctgcaga gacggtgacc gtggtccctg 60

cgccccagac attgaagtac cagtcaccgc cgtagtaa 98

<210> 31
<211> 25
<212> DNA
<213> Artificial Sequence

<220>
<223> Description of Artificial Sequense: Synthetic DNA

<400> 31
gagctggtga agcctggggc ctcag 25

<210> 32
<211> 28
<212> DNA
<213> Artificial Sequence

<220>
<223> Description of Artificial Sequence: Synthetic DNA

<400> 32
atggctcaag ctcccgctaa gtgcccga 28

<210> 33
<211> 27
<212> DNA

<213> Artificial Sequence

<220>

<223> Description of Artificial Sequence: Synthetic DNA

<400> 33
tcaagcgttt gggttggtcc tcatgag 27

<210> 34
<211> 25
<212> DNA
<213> Artificial Sequence

<220>
<223> Description of Artificial Sequence: Synthetic DNA

<400> 34
tccggggatg gcgagatggg caagc 25

<210> 35
<211> 24
<212> DNA
<213> Artificial Sequence

<220>
<223> Description of Artificial Sequence: Synthetic DNA

<400> 35
cttgacatgg ctctgggctc caag 24

<210> 36

<211> 25
<212> DNA
<213> Artificial Sequence

<220>
<223> Description of Artificial Sequence: Synthetic DNA

<400> 36
ccacttcagt cggtcggtag tattt 25

<210> 37
<211> 24
<212> DNA
<213> Artificial Sequence

<220>
<223> Description of Artificial Sequence: Synthetic DNA

<400> 37
cgctcacccg cctgaggcga catg 24

<210> 38
<211> 32
<212> DNA
<213> Artificial Sequence

<220>
<223> Description of Artificial Sequence: Synthetic DNA

<400> 38
ggcaggtgct gtcggtgagg tcaccatagt gc 32

<210> 39

<211> 24
<212> DNA
<213> Artificial Sequence

<220>
<223> Description of Artificial Sequence: Synthetic DNA

<400> 39
ggggccatgc caaggactat gtcg 24

<210> 40
<211> 25
<212> DNA
<213> Artificial Sequence

<220>
<223> Description of Artificial Sequence: Synthetic DNA

<400> 40
atgtggctga tgttacaaaa tgatg 25

<210> 41
<211> 1504
<212> DNA
<213> Cricetulus griseus

<220>
<221> CDS
<222> (1)..(1119)

<400> 41
atg gct cac gct ccc gct agc tgc ccg agc tcc agg aac tct ggg gac 48
Met Ala His Ala Pro Ala Ser Cys Pro Ser Ser Arg Asn Ser Gly Asp

1 5 10 15

ggc gat aag ggc aag ccc agg aag gtg gcg ctc atc acg ggc atc acc 96
Gly Asp Lys Gly Lys Pro Arg Lys Val Ala Leu Ile Thr Gly Ile Thr
20 25 30

ggc cag gat ggc tca tac ttg gca gaa ttc ctg ctg gag aaa gga tac 144
Gly Gln Asp Gly Ser Tyr Leu Ala Glu Phe Leu Leu Glu Lys Gly Tyr
35 40 45

gag gtt cat gga att gta cgg cga tcc agt tca ttt aat aca ggt cga 192
Glu Val His Gly Ile Val Arg Arg Ser Ser Ser Phe Asn Thr Gly Arg
50 55 60

att gaa cat tta tat aag aat cca cag gct cat att gaa gga aac atg 240
Ile Glu His Leu Tyr Lys Asn Pro Gln Ala His Ile Glu Gly Asn Met
65 70 75 80

aag ttg cac tat ggt gac ctc acc gac agc acc tgc cta gta aaa atc 288
Lys Leu His Tyr Gly Asp Leu Thr Asp Ser Thr Cys Leu Val Lys Ile
85 90 95

atc aat gaa gtc aaa cct aca gag atc tac aat ctt ggt gcc cag agc 336
Ile Asn Glu Val Lys Pro Thr Glu Ile Tyr Asn Leu Gly Ala Gln Ser
100 105 110

cat gtc aag att tcc ttt gac tta gca gag tac act gca gat gtt gat 384
His Val Lys Ile Ser Phe Asp Leu Ala Glu Tyr Thr Ala Asp Val Asp
115 120 125

gga gtt ggc acc ttg cgg ctt ctg gat gca att aag act tgt ggc ctt 432
Gly Val Gly Thr Leu Arg Leu Leu Asp Ala Ile Lys Thr Cys Gly Leu
130 135 140

ata aat tct gtg aag ttc tac cag gcc tca act agt gaa ctg tat gga 480
Ile Asn Ser Val Lys Phe Tyr Gln Ala Ser Thr Ser Glu Leu Tyr Gly

145 150 155 160

aaa gtg caa gaa ata ccc cag aaa gag acc acc cct ttc tat cca agg 528
Lys Val Gln Glu Ile Pro Gln Lys Glu Thr Thr Pro Phe Tyr Pro Arg
165 170 175

tcg ccc tat gga gca gcc aaa ctt tat gcc tat tgg att gta gtg aac 576
Ser Pro Tyr Gly Ala Ala Lys Leu Tyr Ala Tyr Trp Ile Val Val Asn
180 185 190

ttt cga gag gct tat aat ctc ttt gcg gtg aac ggc att ctc ttc aat 624
Phe Arg Glu Ala Tyr Asn Leu Phe Ala Val Asn Gly Ile Leu Phe Asn
195 200 205

cat gag agt cct aga aga gga gct aat ttt gtt act cga aaa att agc 672
His Glu Ser Pro Arg Arg Gly Ala Asn Phe Val Thr Arg Lys Ile Ser
210 215 220

cgg tca gta gct aag att tac ctt gga caa ctg gaa tgt ttc agt ttg 720
Arg Ser Val Ala Lys Ile Tyr Leu Gly Gln Leu Glu Cys Phe Ser Leu
225 230 235 240

gga aat ctg gac gcc aaa cga gac tgg ggc cat gcc aag gac tat gtc 768
Gly Asn Leu Asp Ala Lys Arg Asp Trp Gly His Ala Lys Asp Tyr Val
245 250 255

gag gct atg tgg ctg atg tta caa aat gat gaa cca gag gac ttt gtc 816
Glu Ala Met Trp Leu Met Leu Gln Asn Asp Glu Pro Glu Asp Phe Val
260 265 270

ata gct act ggg gaa gtt cat agt gtc cgt gaa ttt gtt gag aaa tca 864
Ile Ala Thr Gly Glu Val His Ser Val Arg Glu Phe Val Glu Lys Ser
275 280 285

ttc atg cac att gga aag acc att gtg tgg gaa gga aag aat gaa aat 912
Phe Met His Ile Gly Lys Thr Ile Val Trp Glu Gly Lys Asn Glu Asn

290 295 300

gaa gtg ggc aga tgt aaa gag acc ggc aaa att cat gtg act gtg gat 960
Glu Val Gly Arg Cys Lys Glu Thr Gly Lys Ile His Val Thr Val Asp
305 310 315 320

ctg aaa tac tac cga cca act gaa gtg gac ttc ctg cag gga gac tgc 1008
Leu Lys Tyr Tyr Arg Pro Thr Glu Val Asp Phe Leu Gln Gly Asp Cys
325 330 335

tcc aag gcg cag cag aaa ctg aac tgg aag ccc cgc gtt gcc ttt gac 1056
Ser Lys Ala Gln Gln Lys Leu Asn Trp Lys Pro Arg Val Ala Phe Asp
340 345 350

gag ctg gtg agg gag atg gtg caa gcc gat gtg gag ctc atg aga acc 1104
Glu Leu Val Arg Glu Met Val Gln Ala Asp Val Glu Leu Met Arg Thr
355 360 365

aac ccc aac gcc tga gcacctctac aaaaaaattc gcgagacatg gactatggtg 1159
Asn Pro Asn Ala
370

cagagccagc caaccagagt ccagccactc ctgagaccat cgaccataaa ccctcgactg 1219
cctgtgtcgt ccccacagct aagagctggg ccacaggttt gtgggcacca ggacggggac 1279
actccagagc taaggccact tcgcttttgt caaaggctcc tctcaatgat tttgggaaat 1339
caagaagttt aaaatcacat actcatttta cttgaaatta tgtcactaga caacttaaat 1399
ttttgagtct tgagattgtt tttctctttt cttattaaat gatctttcta tgacccagca 1459
aaaaaaaaaa aaaaaaggga tataaaaaaa aaaaaaaaaa aaaaa 1504

<210> 42
<211> 17
<212> DNA
<213> Artificial Sequence

<220>

<223> Description of Artificial Sequence: Synthetic DNA

<400> 42
gccatccaga aggtggt 17

<210> 43
<211> 17
<212> DNA
<213> Artificial Sequence

<220>
<223> Description of Artificial Sequence: Synthetic DNA

<400> 43
gtcttgtcag ggaagat 17

<210> 44
<211> 28
<212> DNA
<213> Artificial Sequence

<220>
<223> Description of Artificial Sequence: Synthetic DNA

<400> 44
ggcaggagac caccttgcga gtgcccac 28

<210> 45
<211> 28
<212> DNA
<213> Artificial Sequence

<220>

<223> Description of Artificial Sequence: Synthetic DNA

<400> 45
gggtgggctg taccttctgg aacagggc 28

<210> 46
<211> 28
<212> DNA
<213> Artificial Sequence

<220>
<223> Description of Artificial Sequence: Synthetic DNA

<400> 46
ggcgctggct tacccggaga ggaatggg 28

<210> 47
<211> 28
<212> DNA
<213> Artificial Sequence

<220>
<223> Description of Artificial Sequence: Synthetic DNA

<400> 47
ggaatgggtg tttgtctcctc caaagatgc 28

<210> 48
<211> 1316
<212> DNA
<213> Cricetulus griseus

<400> 48
gccccgcccc ctccacctgg accgagagta gctggagaat tgtgcaccgg aagtagctct 60

tggactggtg gaaccctgcg caggtgcagc aacaatgggt gagccccagg gatccaggag 120

gatcctagtg acagggggct ctggactggt gggcagagct atccagaagg tggtcgcaga 180

tggcgctggc ttacccggag aggaatgggt gtttgtctcc tccaaagatg cagatctgac 240

ggatgcagca caaacccaag ccctgttcca gaaggtacag cccacccatg tcatccatct 300

tgctgcaatg gtaggaggcc ttttccggaa tatcaaatac aacttggatt tctggaggaa 360

gaatgtgcac atcaatgaca acgtcctgca ctcagctttc gaggtgggca ctcgcaaggt 420

ggtctcctgc ctgtccacct gtatcttccc tgacaagacc acctatccta ttgatgaaac 480

aatgatccac aatggtccac cccacagcag caattttggg tactcgtatg ccaagaggat 540

gattgacgtg cagaacaggg cctacttcca gcagcatggc tgcaccttca ctgctgtcat 600

ccctaccaat gtctttggac ctcatgacaa cttcaacatt gaagatggcc atgtgctgcc 660

tggcctcatc cataaggtgc atctggccaa gagtaatggt tcagccttga ctgtttgggg 720

tacagggaaa ccacggaggc agttcatcta ctcactggac ctagcccggc tcttcatctg 780

ggtcctgcgg gagtacaatg aagttgagcc catcatcctc tcagtgggcg aggaagatga 840

agtctccatt aaggaggcag ctgaggctgt agtggaggcc atggacttct gtggggaagt 900

cacttttgat tcaacaaagt cagatgggca gtataagaag acagccagca atggcaagct 960

tcgggcctac ttgcctgatt tccgtttcac acccttcaag caggctgtga aggagacctg 1020

tgcctggttc accgacaact atgagcaggc ccggaagtga agcatgggac aagcgggtgc 1080

tcagctggca atgcccagtc agtaggctgc agtctcatca tttgcttgtc aagaactgag 1140

gacagtatcc agcaacctga gccacatgct ggtctctctg ccagggggct tcatgcagcc 1200

atccagtagg gcccatgttt gtccatcctc gggggaaggc cagaccaaca ccttgtttgt 1260

ctgcttctgc cccaacctca gtgcatccat gctggtcctg ctgtcccttg tctaga 1316

<210> 49
<211> 23
<212> DNA
<213> Artificial Sequence

<220>
<223> Description of Artificial Sequence: Synthetic DNA

<400> 49
gatcctgctg ggaccaaaat tgg 23

<210> 50
<211> 22
<212> DNA
<213> Artificial Sequence

<220>
<223> Description of Artificial Sequence: Synthetic DNA

<400> 50
cttaacatcc caagggatgc tg 22

<210> 51
<211> 1965
<212> DNA
<213> Cricetulus griseus

<400> 51
acgggggct cccggaagcg gggaccatgg cgtctctgcg cgaagcgagc ctgcggaagc 60

tgcggcgctt ttccgagatg agaggcaaac ctgtggcaac tgggaaattc tgggatgtag 120

ttgtaataac agcagctgac gaaaagcagg agcttgctta caagcaacag ttgtcggaga 180

agctgaagag aaaggaattg ccccttggag ttaactacca tgttttcact gatcctcctg 240

gaaccaaaat tggaaatgga ggatcaacac tttgttctct tcagtgcctg gaaagcctct 300

atggagacaa gtggaattcc ttcacagtcc tgttaattca ctctggtggc tacagtcaac 360

gacttcccaa tgcaagcgct ttaggaaaaa tcttcacggc tttaccactt ggtgagccca 420

tttatcagat gttggactta aaactagcca tgtacatgga tttcccctca cgcatgaagc 480

ctggagtttt ggtcacctgt gcagatgata ttgaactata cagcattggg gactctgagt 540

ccattgcatt tgagcagcct ggctttactg ccctagccca tccatctagt ctggctgtag 600

gcaccacaca tggagtattt gtattggact ctgccggttc tttgcaacat ggtgacctag 660

agtacaggca atgccaccgt ttcctccata agcccagcat tgaaaacatg caccacttta 720

atgccgtgca tagactagga agctttggtc aacaggactt gagtgggggt gacaccacct 780

gtcatccatt gcactctgag tatgtctaca cagatagcct attttacatg gatcataaat 840

cagccaaaaa gctacttgat ttctatgaaa gtgtaggccc actgaactgt gaaatagatg 900

cctatggtga ctttctgcag gcactgggac ctggagcaac tgcagagtac accaagaaca 960

cctcacacgt cactaaagag gaatcacact tgttggacat gaggcagaaa atattccacc 1020

tcctcaaggg aacacccctg aatgttgttg tccttaataa ctccaggttt tatcacattg 1080

gaacaacgga ggagtatctg ctacatttca cttccaatgg ttcgttacag gcagagctgg 1140

gcttgcaatc catagctttc agtgtctttc caaatgtgcc tgaagactcc catgagaaac 1200

cctgtgtcat tcacagcatc ctgaattcag gatgctgtgt ggcccctggc tcagtggtag 1260

aatattccag attaggacct gaggtgtcca tctcggaaaa ctgcattatc agcggttctg 1320

tcatagaaaa agctgttctg cccccatgtt ctttcgtgtg ctctttaagt gtggagataa 1380

atggacactt agaatattca actatggtgt ttggcatgga agacaacttg aagaacagtg 1440

ttaaaaccat atcagatata aagatgcttc agttctttgg agtctgtttc ctgacttgtt 1500

tagatatttg gaaccttaaa gctatggaag aactattttc aggaagtaag acgcagctga 1560

gcctgtggac tgctcgaatt ttccctgtct gttcttctct gagtgagtcg gttgcagcat 1620

cccttgggat gttaaatgcc attcgaaacc attcgccatt cagcctgagc aacttcaagc 1680

tgctgtccat ccaggaaatg cttctctgca aagatgtagg agacatgctt gcttacaggg 1740

agcaactctt tctagaaatc agttcaaaga gaaaacagtc tgattcggag aaatcttaaa 1800

tacaatggat tttgcctgga aacaggattg caaatgcagg catattctat agatctctgg 1860

gttcttcttt ctttctcccc tctctccttt cctttccctt tgatgtaatg acaaaggtaa 1920

aaatggccac ttctgatgga aaaaaaaaaa aaaaaaaaaa aaaaa 1965

<210> 52
<211> 27
<212> DNA
<213> Artificial Sequence

<220>
<223> Description of Artificial Sequence: Synthetic DNA

<400> 52
caggggtgtt cccttgagga ggtggaa 27

<210> 53
<211> 27
<212> DNA
<213> Artificial Sequence

<220>
<223> Description of Artificial Sequence: Synthetic DNA

<400> 53
cactgagcca ggggccacac agcatcc 27

<210> 54
<211> 23
<212> DNA
<213> Artificial Sequence

<220>
<223> Description of Artificial Sequence: Synthetic DNA

<400> 54
cccctcacgc atgaagcctg gag 23

<210> 55
<211> 27
<212> DNA
<213> Artificial Sequence

<220>
<223> Description of Artificial Sequence: Synthetic DNA

<400> 55
tgccaccgtt tcctccataa gcccagc 27

<210> 56
<211> 25
<212> DNA
<213> Artificial Sequence

<220>
<223> Description of Artificial Sequence: Synthetic DNA

<400> 56
atgaagttgc actatggtga cctca 25

<210> 57
<211> 59
<212> DNA
<213> Cricetulus griseus

<400> 57
ccgacagcac ctgcctagta aaaatcatca atgaagtcaa acctacagag atctacaat 59

<210> 58
<211> 25
<212> DNA
<213> Artificial Sequence

<220>
<223> Description of Artificial Sequence: Synthetic DNA

<400> 58
gacttagcag agtacactgc agatg 25

<210> 59
<211> 25
<212> DNA
<213> Artificial Sequence

<220>
<223> Description of Artificial Sequence: Synthetic DNA

<400> 59
accttggata gaaaggggtg gtctc 25

<210> 60
<211> 125
<212> DNA
<213> Cricetulus griseus

<400> 60
ttgatggagt tggcaccttg cggcttctgg atgcaattaa gacttgtggc cttataaatt 60
ctgtgaagtt ctaccaggcc tcaactagtg aactgtatgg aaaagtgcaa gaaatacccc 120
agaaa 125

<210> 61
<211> 372
<212> PRT
<213> Cricetulus griseus

<400> 61
Met Ala His Ala Pro Ala Ser Cys Pro Ser Ser Arg Asn Ser Gly Asp
1 5 10 15

Gly Asp Lys Gly Lys Pro Arg Lys Val Ala Leu Ile Thr Gly Ile Thr
20 25 30

Gly Gln Asp Gly Ser Tyr Leu Ala Glu Phe Leu Leu Glu Lys Gly Tyr
35 40 45

Glu Val His Gly Ile Val Arg Arg Ser Ser Ser Phe Asn Thr Gly Arg
50 55 60

Ile Glu His Leu Tyr Lys Asn Pro Gln Ala His Ile Glu Gly Asn Met
65 70 75 80

Lys Leu His Tyr Gly Asp Leu Thr Asp Ser Thr Cys Leu Val Lys Ile
85 90 95

Ile Asn Glu Val Lys Pro Thr Glu Ile Tyr Asn Leu Gly Ala Gln Ser
100 105 110

His Val Lys Ile Ser Phe Asp Leu Ala Glu Tyr Thr Ala Asp Val Asp
115 120 125

Gly Val Gly Thr Leu Arg Leu Leu Asp Ala Ile Lys Thr Cys Gly Leu
130 135 140

Ile Asn Ser Val Lys Phe Tyr Gln Ala Ser Thr Ser Glu Leu Tyr Gly
145 150 155 160

Lys Val Gln Glu Ile Pro Gln Lys Glu Thr Thr Pro Phe Tyr Pro Arg
165 170 175

Ser Pro Tyr Gly Ala Ala Lys Leu Tyr Ala Tyr Trp Ile Val Val Asn
180 185 190

Phe Arg Glu Ala Tyr Asn Leu Phe Ala Val Asn Gly Ile Leu Phe Asn
195 200 205

His Glu Ser Pro Arg Arg Gly Ala Asn Phe Val Thr Arg Lys Ile Ser
210 215 220

Arg Ser Val Ala Lys Ile Tyr Leu Gly Gln Leu Glu Cys Phe Ser Leu
225 230 235 240

Gly Asn Leu Asp Ala Lys Arg Asp Trp Gly His Ala Lys Asp Tyr Val
245 250 255

Glu Ala Met Trp Leu Met Leu Gln Asn Asp Glu Pro Glu Asp Phe Val
260 265 270

Ile Ala Thr Gly Glu Val His Ser Val Arg Glu Phe Val Glu Lys Ser
275 280 285

Phe Met His Ile Gly Lys Thr Ile Val Trp Glu Gly Lys Asn Glu Asn
290 295 300

Glu Val Gly Arg Cys Lys Glu Thr Gly Lys Ile His Val Thr Val Asp
305 310 315 320

Leu Lys Tyr Tyr Arg Pro Thr Glu Val Asp Phe Leu Gln Gly Asp Cys
325 330 335

Ser Lys Ala Gln Gln Lys Leu Asn Trp Lys Pro Arg Val Ala Phe Asp
340 345 350

Glu Leu Val Arg Glu Met Val Gln Ala Asp Val Glu Leu Met Arg Thr
355 360 365

Asn Pro Asn Ala
370

<210> 62
<211> 321
<212> PRT

<213> Cricetulus griseus

<400> 62

Met Gly Glu Pro Gln Gly Ser Arg Arg Ile Leu Val Thr Gly Gly Ser
1 5 10 15

Gly Leu Val Gly Arg Ala Ile Gln Lys Val Val Ala Asp Gly Ala Gly
20 25 30

Leu Pro Gly Glu Glu Trp Val Phe Val Ser Ser Lys Asp Ala Asp Leu
35 40 45

Thr Asp Ala Ala Gln Thr Gln Ala Leu Phe Gln Lys Val Gln Pro Thr
50 55 60

His Val Ile His Leu Ala Ala Met Val Gly Gly Leu Phe Arg Asn Ile
65 70 75 80

Lys Tyr Asn Leu Asp Phe Trp Arg Lys Asn Val His Ile Asn Asp Asn
85 90 95

Val Leu His Ser Ala Phe Glu Val Gly Thr Arg Lys Val Val Ser Cys
100 105 110

Leu Ser Thr Cys Ile Phe Pro Asp Lys Thr Thr Tyr Pro Ile Asp Glu
115 120 125

Thr Met Ile His Asn Gly Pro Pro His Ser Ser Asn Phe Gly Tyr Ser
130 135 140

Tyr Ala Lys Arg Met Ile Asp Val Gln Asn Arg Ala Tyr Phe Gln Gln
145 150 155 160

His Gly Cys Thr Phe Thr Ala Val Ile Pro Thr Asn Val Phe Gly Pro
165 170 175

His Asp Asn Phe Asn Ile Glu Asp Gly His Val Leu Pro Gly Leu Ile
180 185 190

His Lys Val His Leu Ala Lys Ser Asn Gly Ser Ala Leu Thr Val Trp
195 200 205

Gly Thr Gly Lys Pro Arg Arg Gln Phe Ile Tyr Ser Leu Asp Leu Ala
210 215 220

Arg Leu Phe Ile Trp Val Leu Arg Glu Tyr Asn Glu Val Glu Pro Ile
225 230 235 240

Ile Leu Ser Val Gly Glu Glu Asp Glu Val Ser Ile Lys Glu Ala Ala
245 250 255

Glu Ala Val Val Glu Ala Met Asp Phe Cys Gly Glu Val Thr Phe Asp
260 265 270

Ser Thr Lys Ser Asp Gly Gln Tyr Lys Lys Thr Ala Ser Asn Gly Lys
275 280 285

Leu Arg Ala Tyr Leu Pro Asp Phe Arg Phe Thr Pro Phe Lys Gln Ala
290 295 300

Val Lys Glu Thr Cys Ala Trp Phe Thr Asp Asn Tyr Glu Gln Ala Arg
305 310 315 320

Lys

<210> 63
<211> 590
<212> PRT
<213> Cricetulus griseus

<400> 63

Met Ala Ser Leu Arg Glu Ala Ser Leu Arg Lys Leu Arg Arg Phe Ser
1 5 10 15

Glu Met Arg Gly Lys Pro Val Ala Thr Gly Lys Phe Trp Asp Val Val
20 25 30

Val Ile Thr Ala Ala Asp Glu Lys Gln Glu Leu Ala Tyr Lys Gln Gln
35 40 45

Leu Ser Glu Lys Leu Lys Arg Lys Glu Leu Pro Leu Gly Val Asn Tyr
50 55 60

His Val Phe Thr Asp Pro Pro Gly Thr Lys Ile Gly Asn Gly Gly Ser
65 70 75 80

Thr Leu Cys Ser Leu Gln Cys Leu Glu Ser Leu Tyr Gly Asp Lys Trp
85 90 95

Asn Ser Phe Thr Val Leu Leu Ile His Ser Gly Gly Tyr Ser Gln Arg
100 105 110

Leu Pro Asn Ala Ser Ala Leu Gly Lys Ile Phe Thr Ala Leu Pro Leu
115 120 125

Gly Glu Pro Ile Tyr Gln Met Leu Asp Leu Lys Leu Ala Met Tyr Met
130 135 140

Asp Phe Pro Ser Arg Met Lys Pro Gly Val Leu Val Thr Cys Ala Asp
145 150 155 160

Asp Ile Glu Leu Tyr Ser Ile Gly Asp Ser Glu Ser Ile Ala Phe Glu
165 170 175

Gln Pro Gly Phe Thr Ala Leu Ala His Pro Ser Ser Leu Ala Val Gly
180 185 190

Thr Thr His Gly Val Phe Val Leu Asp Ser Ala Gly Ser Leu Gln His
195 200 205

Gly Asp Leu Glu Tyr Arg Gln Cys His Arg Phe Leu His Lys Pro Ser
210 215 220

Ile Glu Asn Met His His Phe Asn Ala Val His Arg Leu Gly Ser Phe
225 230 235 240

Gly Gln Gln Asp Leu Ser Gly Gly Asp Thr Thr Cys His Pro Leu His
245 250 255

Ser Glu Tyr Val Tyr Thr Asp Ser Leu Phe Tyr Met Asp His Lys Ser
260 265 270

Ala Lys Lys Leu Leu Asp Phe Tyr Glu Ser Val Gly Pro Leu Asn Cys
275 280 285

Glu Ile Asp Ala Tyr Gly Asp Phe Leu Gln Ala Leu Gly Pro Gly Ala
290 295 300

Thr Ala Glu Tyr Thr Lys Asn Thr Ser His Val Thr Lys Glu Glu Ser
305 310 315 320

His Leu Leu Asp Met Arg Gln Lys Ile Phe His Leu Leu Lys Gly Thr
325 330 335

Pro Leu Asn Val Val Val Leu Asn Asn Ser Arg Phe Tyr His Ile Gly
340 345 350

Thr Thr Glu Glu Tyr Leu Leu His Phe Thr Ser Asn Gly Ser Leu Gln
355 360 365

Ala Glu Leu Gly Leu Gln Ser Ile Ala Phe Ser Val Phe Pro Asn Val
370 375 380

Pro Glu Asp Ser His Glu Lys Pro Cys Val Ile His Ser Ile Leu Asn
385 390 395 400

Ser Gly Cys Cys Val Ala Pro Gly Ser Val Val Glu Tyr Ser Arg Leu
405 410 415

Gly Pro Glu Val Ser Ile Ser Glu Asn Cys Ile Ile Ser Gly Ser Val
420 425 430

Ile Glu Lys Ala Val Leu Pro Pro Cys Ser Phe Val Cys Ser Leu Ser
435 440 445

Val Glu Ile Asn Gly His Leu Glu Tyr Ser Thr Met Val Phe Gly Met
450 455 460

Glu Asp Asn Leu Lys Asn Ser Val Lys Thr Ile Ser Asp Ile Lys Met
465 470 475 480

Leu Gln Phe Phe Gly Val Cys Phe Leu Thr Cys Leu Asp Ile Trp Asn
485 490 495

Leu Lys Ala Met Glu Glu Leu Phe Ser Gly Ser Lys Thr Gln Leu Ser
500 505 510

Leu Trp Thr Ala Arg Ile Phe Pro Val Cys Ser Ser Leu Ser Glu Ser
515 520 525

Val Ala Ala Ser Leu Gly Met Leu Asn Ala Ile Arg Asn His Ser Pro
530 535 540

Phe Ser Leu Ser Asn Phe Lys Leu Leu Ser Ile Gln Glu Met Leu Leu
545 550 555 560

Cys Lys Asp Val Gly Asp Met Leu Ala Tyr Arg Glu Gln Leu Phe Leu
565 570 575

```
Glu Ile Ser Ser Lys Arg Lys Gln Ser Asp Ser Glu Lys Ser
            580                 585                 590
```

## Description

### TECHNICAL FIELD

The present invention relates to an antibody composition which is useful for treating diseases relating to CD20-positive cells such as B cell lymphoma and the like, a cell for producing the antibody composition, and a process for producing the antibody composition using the cell.

### BACKGROUND ART

Since antibodies have high binding activity, binding specificity and high stability in blood, their applications to diagnosis, prevention and treatment of various human diseases have been attempted [*Monoclonal Antibodies: Principles and Applications*, Wiley-Liss, Inc., Chapter 2.1 (1995)]. Also, production of a humanized antibody such as a human chimeric antibody or a human complementarity determining region (hereinafter referred to as "CDR")-grafted antibody from an antibody derived from a non-human animal have been attempted by using genetic recombination techniques. The human chimeric antibody is an antibody in which its antibody variable region (hereinafter referred to as "V region") is an antibody derived from a non-human animal and its constant region (hereinafter referred to as "C region") is derived from a human antibody. The human CDR-grafted antibody is an antibody in which the CDR of a human antibody is replaced by CDR of an antibody derived from a non-human animal.

It has been found that five classes, namely IgM, IgD, IgG, IgA and IgE, are present in antibodies derived from mammals. Antibodies of a human IgG class are mainly used for diagnosis, prevention and treatment of various human diseases because they have functional characteristics such as long half-life in blood, various effector functions and the like [*Monoclonal Antibodies: Principles and Applications*, Wiley-Liss, Inc., Chapter 1 (1995)]. The human IgG class antibody is further classified into the following 4 subclasses: IgG1, IgG2, IgG3 and IgG4. A large number of studies have so far been conducted for antibody-dependent cell-mediated cytotoxic activity (hereinafter referred to as "ADCC activity") and complement-dependent cytotoxic activity (hereinafter referred to as "CDC activity") as effector functions of the IgG class antibody, and it has been reported that among antibodies of the human IgG class, the IgG1 subclass has the highest ADCC activity and CDC activity [*Chemical Immunology,* 65, 88 (1997)]. Actually, it has been reported that, although depletion of CD20-positive B cells is detected when an anti-CD20-chimeric antibody of the IgG1 subclass is administered to a monkey, the depletion is not detected when the antibody of the IgG4 class is used. In view of the above, among commercially available antibodies for treatments, most of the anti-tumor humanized antibodies which require high effector functions for the expression of their effects are antibodies of the human IgGl subclass.

CD20, also called Bp35, is a polypeptide of about 35 kDa, and was identified as a human B lymphocyte-specific antigen B1 using a monoclonal antibody [*J. Immunol*., 125, 1678 (1980)]. It is considered that CD20 is a four-transmembrane molecule, functions as a calcium channel, and relates to activation, proliferation and differentiation of B cells [*Immunology Today*, 15, 450 (1994)]. Expression of CD20 is limited to the stage from pre-B cells to mature B cells, and CD20 is not expressed in undifferentiated cells and plasma cells. Also, since CD20 has such characteristics that CD20 expresses in 90% or more of B cell non-Hodgkin lymphoma and does not internalize into cells even when an antibody is bound thereto, treatment of B cell lymphoma by an anti-CD20 antibody has been attempted for a long time [*Blood,* 69, 584 (1987)]. However, since a mouse monoclonal antibody was used in the early stage, a human antibody for the mouse antibody (HAMA; Human Anti Mouse Antibody) was induced in the human body and it lacked in the effector function, so that its therapeutic effect was limited. Accordingly, an attempt was made to prepare a chimeric antibody of a mouse antibody with a human IgG1 subclass using genetic recombination techniques [*J. Immunol*., 139, 3521 (1987), WO 88/04936]. In addition, it has been confirmed by tests using monkeys that a chimeric antibody IDEC-C2B8, a human IgG1 subclass prepared using a mouse monoclonal antibody 2B8 has an activity to deplete CD20-positive cells even in the living body [*Blood*, 83, 435 (1994), WO 94/11026], and this antibody was put on the market in November, 1997, in the United States as Rituxan™ (manufactured by IDEC/Genentech, also called Rituximab, and hereinafter referred to as "Rituxan™") via clinical tests.

The phase III study of Rituxan™ in the United States was carried out by administration to 166 cases of relapsed low grade and follicular lymphomas at a dose of 375 mg/m$^2$/week for 4 weeks, and the efficacy was 48% (complete remission: 6%, partial remission: 42%) [*J, Clin. Oncol*., 16, 2825 (1998)]. As the action mechanism of Rituxan™, activity to induce apoptosis in cells by crosslinking CD20 in addition to its ADCC activity and CDC activity are considered [*Current Opinion in Immunology,* 11, 541 (1999)]. Regarding the CDC activity, since the sensitivity varies depending on the target B lymphoma cell, discussions have been made on a possibility of increasing therapeutic effect of Rituxan™ by inhibiting the function of complement inhibitory molecules CD55 and CD59 considered to relate its control [*Current Opinion in Immunology,* 11, 541 (1999)]. However, it has been also reported that the expression of these inhibitory molecules in tumor cells of patients and *in vitro* sensitivity of the CDC activity are not always correlative with clinical results [*Blood,* 98, 1352 (2001)]. In addition, it has been shown by an examination using a model mouse transplanted

with a human B lymphoma cell line Raji cell that the ADCC activity via an antibody receptor (hereinafter, the antibody receptor is called FcγR) is important for the antitumor effect [*Nature Medicine,* 6, 443 (2000)].

Combined use of Rituxan™ and chemotherapy (CHOP; Cyclophosphamide, Doxorubicin, Vincristine, Prednisone) has been examined, and it has been reported that the efficacy in the phase II study was 95% (complete remission: 55%, partial remission: 45%) in 40 cases of low-grade and follicular lymphomas, but with side effects caused by CHOP [*J. Clin. Oncol.,* 17, 268 (1999)]. In addition, radioisotope-labeled antibodies such as Zevalin (manufactured by IDEC) and Bexxar (manufactured by Corixa) have been developed as other anti-CD20 antibodies for treatments, but since both of them are mouse antibodies and a radioactive isotope is used therein, there is a possibility of causing side effects due to their strong toxicity.

Expression of ADCC activity and CDC activity of the human IgG1 subclass antibodies requires binding of the Fc region of the antibody to an antibody receptor existing on the surface of an effector cell, such as a killer cell, a natural killer cell, an activated macrophage or the like and various complement components. Regarding the binding, it has been suggested that several amino acid residues in the hinge region and the second domain of C region (hereinafter referred to as "Cγ2 domain") of the antibody are important [*Eur. J. Immumol.*, 23, 1098 (1993); *Immunology,* 86, 319 (1995); *Chemical Immunology,* 65, 88 (1997); *Chemical Immunology,* 65, 88 (1997)]. Regarding Rituxan™, as a result of the study using the antibody in which an amino acid of the Cγ2 domain was substituted, amino acids which are mainly important for CDC activity have been identified [*J. Immunol.*, 164, 4178 (2000); *J. Imunol.*, 166, 2571 (2001)].

Furthermore, importance of a sugar chain bound to the Cγ2 domain is suggested [*J. Immunology,* 65, 88 (1997)]. Regarding the sugar chain, Boyd *et al.* have examined effects of a sugar chain on the ADCC activity and CDC activity by treating a human CDR-grafted antibody CAMPATH-1H (human IgG1 subclass) produced by a Chinese hamster ovary cell (hereinafter referred to as "CHO cell") or a mouse myeloma NS0 cell (hereinafter referred to as "NS0 cell") with various sugar hydrolyzing enzymes, and reported that elimination of the non-reducing end sialic acid did not have influence upon both activities, but the CDC activity alone was affected by further elimination of galactose residue and about 50% of the activity was decreased, and that complete elimination of the sugar chain caused disappearance of both activities [*Molecular Immunol.*, 32, 1311 (1995)]. Also, Lifely *et al.* have analyzed the sugar chain bound to a human CDR-grafted antibody CAMPATH-1H (human IgG1 subclass) which was produced by CHO cell, NS0 cell or rat myeloma YO cell, measured its ADCC activity, and reported that the CAMPATH-1H derived from YO cell showed the highest ADCC activity, suggesting that *N*-acetylglucosamine (hereinafter referred also to as "GlcNAc") at the bisecting position is important for the activity [*Glycobiology*, 5, 813 (1995); WO 99/54342]. These reports indicate that the structure of the sugar chain plays an important role in the effector functions of human antibodies of IgG1 subclass and that it is possible to prepare an antibody having higher effector function by changing the structure of the sugar chain. However, actually, structures of sugar chains are various and complex, and it cannot be said that an actual important structure for the effector function was identified.

As an example of the modification of the sugar chain structure of a product by introducing a gene of an enzyme relating to the modification of sugar chains into a host cell, it has been reported that a protein in which sialic acid is added in a large number to the non-reducing end of a sugar chain can be produced by introducing rat β-galactoside-α2,6-sialyltransferase into CHO cell [*J. Biol. Chem.,* 261, 13848 (1989)].

Also, expression of an H antigen in which fucose (hereinafter referred also to as "Fuc") is bound to the non-reducing end of a sugar chain (Fucα1-2Galβ1-) has been confirmed by introducing human β-galactoside-2-α-fucosyltransferase into a mouse L cell [*Science,* 252, 668 (1991)]. Furthermore, based on the knowledge that binding of the bisecting N-acetylglucosamine of N-glycoside-linked sugar chains is important for the ADCC activity of antibodies, Umana *et al*. have prepared a β-1,4-N-acetylglucosamine transferase III (GnTIII)-expressing CHO cell and compared with the parent strain. No expression of GnTIII was observed in the parent CHO cell [*J. Biol. Chem.*, 259, 13370 (1984)], and it has been confirmed that the antibody expressed using the prepared GnTIII-expressing CHO cell has higher ADCC activity than the antibody expressed in the parent cell [*Nature Biotechnol.*, 17, 176 (1999); WO 99/54342]. In this case, Umana *et al*. have also prepared a β-1,4-N-acetylglucosamine transferase V (GnTV) gene-introduced CHO cell and reported that over-expression of GnTIII or GnTV shows toxicity upon CHO cell. Regarding Rituxan™, it has been reported that the antibody prepared using the GnTIII-introduced CHO cell shows higher ADCC activity than the antibody expressed in the parent cell, and difference in the activity is approximately 10 to 20 times [*Biotechnol. Bioeng.,* 74, 288 (2001)].

## DISCLOSURE OF THE INVENTION

Since the effector function-enhanced anti-CD20 antibody shows increased therapeutic effects, alleviation of patient's burden can be expected by its reduced dose. In addition, other effects such as reduction of side effects can also be expected, because combined use with chemotherapy, a radioactive isotope or the like becomes unnecessary. An object of the present invention is to provide an anti-CD20 antibody-producing cell in which an effector function is enhanced, an anti-CD-20 antibody composition in which an effector function is enhanced, a process for producing the

antibody composition, a medicament comprising the antibody composition, and the like.

The present invention relates to the following (1) to (48).

(1) A cell which produces an antibody composition comprising an antibody molecule which specifically binds to CD20 and has complex *N*-glycoside-linked sugar chains bound to the Fc region, wherein among the total complex *N*-glycoside-linked sugar chains bound to the Fc region in the composition, the ratio of a sugar chain in which fucose is not bound to *N*-acetylglucosamine in the reducing end in the sugar chain is 20% or more.

(2) The cell according to (1), wherein the sugar chain to which fucose is not bound is a complex *N*-glycoside-linked sugar chain in which 1-position of fucose is not bound to 6-position of *N*-acetylglucosamine in the reducing end through $\alpha$-bond.

(3) The cell according to (1) or (2), wherein the activity of an enzyme relating to the synthesis of an intracellular sugar nucleotide, GDP-fucose, and/or the activity of an enzyme relating to the modification of a sugar chain in which 1-position of fucose is bound to 6-position of *N*-acetylglucosamine in the reducing end through $\alpha$-bond in the complex *N*-glycoside-linked sugar chain is decreased or deleted.

(4) The cell according to (3), wherein the enzyme relating to the synthesis of an intracellular sugar nucleotide, GDP-fucose is an enzyme selected from the group consisting of the following (a), (b) and (c):

(a) GMD (GDP-mannose 4,6-dehydratase);
(b) Fx (GDP-keto-6-deoxymannose 3,5-epimerase, 4-reductase);
(c) GFPP (GDP-beta-L-fucose pyrophosphorylase).

(5) The cell according to (4), wherein the GMD is a protein encoded by a DNA of the following (a) or (b):

(a) a DNA comprising the nucleotide sequence represented by SEQ ID NO:41;
(b) a DNA which hybridizes with the DNA consisting of the nucleotide sequence represented by SEQ ID NO: 41 under stringent conditions and encodes a protein having GMD activity.

(6) The cell according to (4), wherein the GMD is a protein selected from the group consisting of the following (a), (b) and (c):

(a) a protein comprising the amino acid sequence represented by SEQ ID NO:61;
(b) a protein which consists of an amino acid sequence in which at least one amino acid is deleted, substituted, inserted and/or added in the amino acid sequence represented by SEQ ID NO:61 and has GMD activity;
(c) a protein which consists of an amino acid sequence having a homology of at least 80% with the amino acid sequence represented by SEQ ID NO:61 and has GMD activity.

(7) The cell according to (4), wherein the Fx is a protein encoded by a DNA of the following (a) or (b):

(a) a DNA comprising the nucleotide sequence represented by SEQ ID NO:48;
(b) a DNA which hybridizes with the DNA consisting of the nucleotide sequence represented by SEQ ID NO: 48 under stringent conditions and encodes a protein having Fx activity.

(8) The cell according to (4), wherein the Fx is a protein selected from the group consisting of the following (a), (b) and (c):

(a) a protein comprising the amino acid sequence represented by SEQ ID NO:62;
(b) a protein which consists of an amino acid sequence in which at least one amino acid is deleted, substituted, inserted and/or added in the amino acid sequence represented by SEQ ID NO:62 and has Fx activity;
(c) a protein which consists of an amino acid sequence having a homology of at least 80% with the amino acid sequence represented by SEQ ID NO:62 and has Fx activity.

(9) The cell according to (4), wherein the GFPP is a protein encoded by a DNA of the following (a) or (b):

(a) a DNA comprising the nucleotide sequence represented by SEQ ID NO:51;
(b) a DNA which hybridizes with the DNA consisting of the nucleotide sequence represented by SEQ ID NO: 51 under stringent conditions and encodes a protein having GFPP activity.

(10) The cell according to (4), wherein the GFPP is a protein selected from the group consisting of the following

(a), (b) and (c):

(a) a protein comprising the amino acid sequence represented by SEQ ID NO:63;
(b) a protein which consists of an amino acid sequence in which at least one amino acid is deleted, substituted, inserted and/or added in the amino acid sequence represented by SEQ ID NO:63 and has GFPP activity;
(c) a protein which consists of an amino acid sequence having a homology of at least 80% with the amino acid sequence represented by SEQ ID NO:63 and has GFPP activity.

(11) The cell according to (3), wherein the enzyme relating to the modification of a sugar chain in which 1-position of fucose is bound to 6-position of the *N*-acetylglucosamine in the reducing end through α-bond in the complex *N*-glycoside-linked sugar chain is α1,6-fucosyltransferase.
(12) The cell according to (11), wherein the α1,6-fucosyltransferase is a protein encoded by a DNA of the following (a), (b), (c) and (d):

(a) a DNA comprising the nucleotide sequence represented by SEQ ID NO:1;
(b) a DNA comprising the nucleotide sequence represented by SEQ ID NO:2;
(c) a DNA which hybridizes with the DNA consisting of the nucleotide sequence represented by SEQ ID NO: 1 under stringent conditions and encodes a protein having α1,6-fucosyltransferase activity;
(d) a DNA which hybridizes with the DNA consisting of the nucleotide sequence represented by SEQ ID NO: 2 under stringent conditions and encodes a protein having α1,6-fucosyltransferase activity.

(13) The cell according to (11), wherein the α1,6-fucosyltransferase is a protein selected from the group consisting of the following (a), (b), (c), (d), (e) and (f):

(a) a protein comprising the amino acid sequence represented by SEQ ID NO:23;
(b) a protein comprising the amino acid sequence represented by SEQ ID NO:24;
(c) a protein which consists of an amino acid sequence in which at least one amino acid is deleted, substituted, inserted and/or added in the amino acid sequence represented by SEQ ID NO:23 and has α1,6-fucosyltransferase activity;
(d) a protein which consists of an amino acid sequence in which at least one amino acid is deleted, substituted, inserted and/or added in the amino acid sequence represented by SEQ ID NO:24 and has α1,6-fucosyltransferase activity;
(e) a protein which consists of an amino acid sequence having a homology of at least 80% with the amino acid sequence represented by SEQ ID NO:23 and has α1,6-fucosyltransferase activity;
(f) a protein which consists of an amino acid sequence having a homology of at least 80% with the amino acid sequence represented by SEQ ID NO:24 and has α1,6-fucosyltransferase activity.

(14) The cell according to any one of (3) to (13), wherein the enzyme activity is decreased or deleted by a technique selected from the group consisting of the following (a), (b), (c), (d) and (e):

(a) a gene disruption technique targeting a gene encoding the enzyme;
(b) a technique for introducing a dominant negative mutant of a gene encoding the enzyme;
(c) a technique for introducing mutation into the enzyme;
(d) a technique for inhibiting transcription or translation of a gene encoding the enzyme;
(e) a technique for selecting a cell line resistant to a lectin which recognizes a sugar chain in which 1-position of fucose is bound to 6-position of *N*-acetylglucosamine in the reducing end through α-bond in the complex *N*-glycoside-linked sugar chain.

(15) The cell according to any one of (1) to (14), which is resistant to at least a lectin which recognizes a sugar chain in which 1-position of fucose is bound to 6-position of *N*-acetylglucosamine in the reducing end through α-bond in the complex *N*-glycoside-linked sugar chain.
(16) The cell according to any one of (1) to (15), which is a cell selected from the group consisting of the following (a) to (j):

(a) a CHO cell derived from a Chinese hamster ovary tissue;
(b) a rat myeloma cell line, YB2/3HL.P2.G11.16Ag.20 cell;
(c) a mouse myeloma cell line, NS0 cell;
(d) a mouse myeloma cell line, SP2/0-Ag14 cell;

(e) a BHK cell derived from a syrian hamster kidney tissue;
(f) a monkey COS cell;
(g) an antibody-producing hybridoma cell;
(h) a human leukemia cell line, Namalwa cell;
(i) an embryonic stem cell;
(j) a fertilized egg cell.

(17) A transgenic non-human animal or plant or the progenies thereof into which an antibody molecule which specifically binds to CD20 and has complex *N*-glycoside-linked sugar chains bound to the Fc region is introduced, which produces an antibody composition comprising the antibody molecule, wherein among the total complex *N*-glycoside-linked sugar chains bound to the Fc region in the composition, the ratio of a sugar chain in which fucose is not bound to *N*-acetylglucosamine in the reducing end in the sugar chain is 20% or more.
(18) The transgenic non-human animal or plant or the progenies thereof according to (17), wherein the sugar chain in which fucose is not bound to *N*-acetylglucosamine is a sugar chain in which 1-position of the fucose is not bound to 6-position of *N*-acetylglucosamine in the reducing end through α-bond in the *N*-glycoside-linked sugar chain.
(19) The transgenic non-human animal or plant or the progenies thereof according to (17) or (18), wherein a genome is modified such that the activity of an enzyme relating to the synthesis of an intracellular sugar nucleotide, GDP-fucose and/or the activity of an enzyme relating to the modification of a sugar chain in which 1-position of fucose is bound to 6-position of *N*-acetylglucosamine in the reducing end through α-bond in the *N*-glycoside-linked sugar chain is decreased.
(20) The transgenic non-human animal or plant or the progenies thereof according to (17) or (18), wherein a gene encoding the enzyme relating to the synthesis of an intracellular sugar nucleotide, GDP-fucose, and/or a gene encoding the enzyme relating to the modification of a sugar chain in which 1-position of fucose is bound to 6-position of *N*-acetylglucosamine in the reducing end through α-bond in the *N*-glycoside-linked sugar chain is knocked out.
(21) The transgenic non-human animal or plant or the progenies thereof according to (19) or (20), wherein the enzyme relating to the synthesis of an intracellular sugar nucleotide, GDP-fucose is an enzyme selected from the group consisting of the following (a), (b) and (c):

(a) GMD (GDP-mannose 4,6-dehydratase);
(b) Fx (GDP-keto-6-deoxymannose 3,5-epimerase, 4-reductase);
(c) GFPP (GDP-beta-L-fucose pyrophosphorylase).

(22) The transgenic non-human animal or plant or the progenies thereof according to (21), wherein the GMD is a protein encoded by a DNA of the following (a) or (b):

(a) a DNA comprising the nucleotide sequence represented by SEQ ID NO:41;
(b) a DNA which hybridizes with the DNA consisting of the nucleotide sequence represented by SEQ ID NO: 41 under stringent conditions and encodes a protein having GMD activity.

(23) The transgenic non-human animal or plant or the progenies thereof according to (21), wherein the Fx is a protein encoded by a DNA of the following (a) or (b):

(a) a DNA comprising the nucleotide sequence represented by SEQ ID NO:48;
(b) a DNA which hybridizes with the DNA consisting of the nucleotide sequence represented by SEQ ID NO: 48 under stringent conditions and encodes a protein having Fx activity.

(24) The transgenic non-human animal or plant or the progenies thereof according to (21), wherein the GFPP is a protein encoded by a DNA of the following (a) or (b):

(a) a DNA comprising the nucleotide sequence represented by SEQ ID NO:51;
(b) a DNA which hybridizes with the DNA consisting of the nucleotide sequence represented by SEQ ID NO: 51 under stringent conditions and encodes a protein having GFPP activity.

(25) The transgenic non-human animal or plant or the progenies thereof according to (19) or (20), wherein the enzyme relating to the modification of a sugar chain in which 1-position of fucose is bound to 6-position of *N*-acetylglucosamine in the reducing end through α-bond in the *N*-glycoside-linked sugar chain is α1,6-fucosyltransferase.
(26) The transgenic non-human animal or plant or the progenies thereof according to (25), wherein the α1,6-fucosyltransferase is a protein encoded by a DNA selected from the group consisting of the following (a), (b), (c) and (d):

(a) a DNA comprising the nucleotide sequence represented by SEQ ID NO:1;
(b) a DNA comprising the nucleotide sequence represented by SEQ ID NO:2;
(c) a DNA which hybridizes with the DNA consisting of the nucleotide sequence represented by SEQ ID NO: 1 under stringent conditions and encodes a protein having α1,6-fucosyltransferase activity;
(d) a DNA which hybridizes with the DNA consisting of the nucleotide sequence represented by SEQ ID NO: 2 under stringent conditions and encodes a protein having α1,6-fucosyltransferase activity.

(27) The transgenic non-human animal or plant or the progenies thereof according to any one of (17) to (26), wherein the transgenic non-human animal is an animal selected from the group consisting of cattle, sheep, goat, pig, horse, mouse, rat, fowl, monkey and rabbit.
(28) The cell according to any one of (1) to (16), wherein the antibody molecule is a molecule selected from the group consisting of (a), (b), (c) and (d):

(a) a human antibody;
(b) a humanized antibody;
(c) an antibody fragment comprising an Fc region of (a) or (b);
(d) a fusion protein comprising an Fc region of (a) or (b).

(29) The cell according to any one of (1) to (16) and (28), wherein the antibody molecule belongs to an IgG class.
(30) The cell according to any one of (1) to (16), (28) and (29), wherein the antibody molecule comprises complementarity determining regions 1, 2 and 3 of an antibody light chain variable region comprising the amino acid sequences represented by SEQ ID NOs:5, 6 and 7, respectively, and/or complementarity determining regions 1, 2 and 3 of an antibody heavy chain comprising the amino acid sequences represented by SEQ ID NOs:8, 9 and 10, respectively.
(31) The cell according to any one of (1) to (16), (28), (29) and (30), wherein the antibody molecule comprises a light chain variable region comprising the amino acid sequence represented by SEQ ID NO:12 and/or a heavy chain variable region comprising the amino acid sequence represented by SEQ ID NO:14.
(32) The transgenic non-human animal or plant or the progenies thereof according to any one of (17) to (27), wherein the antibody molecule is a molecule selected from the group consisting of (a), (b), (c) and (d):

(a) a human antibody;
(b) a humanized antibody;
(c) an antibody fragment comprising an Fc region of (a) or (b);
(d) a fusion protein comprising an Fc region of (a) or (b).

(33) The transgenic non-human animal or plant or the progenies thereof according to any one of (17) to (27) and (32), wherein the antibody molecule belongs to an IgG class.
(34) The transgenic non-human animal or plant or the progenies thereof according to any one of (17) to (27), (32) and (33), wherein the antibody molecule comprises complementarity determining regions 1, 2 and 3 of an antibody light chain variable region comprising the amino acid sequences represented by SEQ ID NOs:5, 6 and 7, respectively, and/or complementarity determining regions 1, 2 and 3 of an antibody heavy chain comprising the amino acid sequences represented by SEQ ID NOs:8, 9 and 10, respectively.
(35) The transgenic non-human animal or plant or the progenies thereof according to any one of (17) to (27), (32), (33) and (34), wherein the antibody molecule comprises a light chain variable region comprising the amino acid sequence represented by SEQ ID NO: 12 and/or a heavy chain variable region comprising the amino acid sequence represented by SEQ ID NO: 14.
(36) An antibody composition which is produced by the cell according to any one of (1) to (16) and (28) to (31).
(37) An antibody composition which is obtainable by rearing the transgenic non-human animal or plant or the progenies thereof according to any one of (17) to (27) and (32) to (35).
(38) An antibody composition comprising an antibody molecule which specifically binds to CD20 and has complex *N*-glycoside-linked sugar chains bound to the Fc region, wherein among the total complex *N*-glycoside-linked sugar chains bound to the Fc region in the composition, the ratio of a sugar chain in which fucose is not bound to *N*-acetylglucosamine in the reducing end in the sugar chain is 20% or more.
(39) The antibody composition according to (38), wherein the sugar chain to which fucose is not bound is a complex *N*-glycoside-linked sugar chain in which 1-position of fucose is not bound to 6-position of *N*-acetylglucosamine in

the reducing end through α-bond.

(40) The antibody composition according to (38), wherein the antibody molecule is a molecule selected from the group consisting of (a), (b), (c) and (d):

(a) a human antibody;
(b) a humanized antibody;
(c) an antibody fragment comprising an Fc region of (a) or (b);
(d) a fusion protein comprising an Fc region of (a) or (b).

(41) The antibody composition according to any one of (38) to (40), wherein the antibody molecule belongs to an IgG class.

(42) The antibody composition according to any one of (38) to (41), wherein the antibody molecule comprises complementarity determining regions 1, 2 and 3 of an antibody light chain variable region comprising the amino acid sequences represented by SEQ ID NOs:5, 6 and 7, respectively, and/or complementarity determining regions 1, 2 and 3 of an antibody heavy chain comprising the amino acid sequences represented by SEQ ID NOs:8, 9 and 10, respectively.

(43) The antibody composition according to any one of (38) to (42), wherein the antibody molecule comprises a light chain variable region comprising the amino acid sequence represented by SEQ ID NO:12 and/or a heavy chain variable region comprising the amino acid sequence represented by SEQ ID NO:14.

(44) A process for producing the antibody composition according to any one of (36) and (38) to (43), which comprises culturing the cell according to any one of (1) to (16) and (28) to (31) to form and accumulate the antibody composition in the culture; and recovering the antibody composition from the culture.

(45) A process for producing the antibody composition according to any one of (36) and (38) to (43), which comprises rearing the transgenic non-human animal or plant or the progenies thereof according to any one of (17) to (27) and (32) to (35); isolating tissue or body fluid from the reared animal or plant; and recovering the antibody composition from the isolated tissue or body fluid.

(46) A medicament which comprises the antibody composition according to any one of (36) to (43) as an active ingredient.

(47) An agent for treating diseases relating to CD20, which comprises the antibody composition according to any one of (36) to (43) as an active ingredient.

(48) The agent according to (47), wherein the disease relating to CD20 is a cancer or an immunological disease.

The cell of the present invention may be any cell, so long as the cell produces an antibody composition comprising an antibody molecule which specifically binds to CD20 and has complex *N*-glycoside-linked sugar chains bound to the Fc region, wherein among the total complex *N*-glycoside-linked sugar chains bound to the Fc region in the composition, the ratio of a sugar chain in which fucose is not bound to *N*-acetylglucosamine in the reducing end in the sugar chain is 20% or more.

In the present invention, CD20 is a cell surface membrane protein of about 35 kDa which is also called B1 or Bp35, and includes a protein represented by the amino acid sequence represented by SEQ ID NO:4, and a protein which comprises an amino acid sequence in which one or several amino acids are substituted, deleted, inserted and/or added in the amino acid sequence represented by SEQ ID NO:4 and has properties which are substantially similar to those of CD20.

The protein which comprises an amino acid sequence in which one or several amino acids are substituted, deleted, inserted and/or added in the amino acid sequence represented by SEQ ID NO:4 and has substantially similar activities to CD20 can be obtained e.g., by introducing a site-directed mutation into a DNA encoding a protein having the amino acid sequence represented by SEQ ID NO:4, using the site-directed mutagenesis described in, e.g., *Molecular Cloning*, *A Laboratory Manual*, Second Edition, Cold Spring Harbor Laboratory Press (1989) (hereinafter referred to as *"Molecular Cloning,* Second Edition"); *Current Protocols in Molecular Biology,* John Wiley & Sons, 1987-1997 (hereinafter referred to as *"Current Protocols in Molecular Biology*"); *Nucleic Acids Research,* 10, 6487 (1982); *Proc. Natl. Acad Sci. USA,* 79, 6409 (1982); *Gene,* 34, 315 (1985); *Nucleic Acids Research,* 13, 4431 (1985); *Proc. Natl. Acad Sci., USA,* 82, 488 (1985); and the like. The number of amino acids to be deleted, substituted, inserted and/or added is one or more, and the number is not particularly limited, but is a number which can be deleted, substituted or added by a known technique such as the site-directed mutagenesis, e.g., it is 1 to several tens, preferably 1 to 20, more preferably 1 to 10, and most preferably 1 to 5.

Also, in order to maintain the CD20 activity of the protein to be used in the present invention, it has 80% or more, preferably 85% or more, more preferably 90% or more, still more preferably 95% or more, far more preferably 97% or more, and most preferably 99% or more, of homology with the amino acid sequence represented by SEQ ID NO:4 when calculated using an analyzing soft such as BLAST [*J. Mol. Biol.,* 215, 403 (1990)], FASTA [*Methods in*

*Enzymology,* 183, 63 (1990)] or the like.

In the present invention, as the sugar chain which binds to the Fc region of an antibody molecule, mentioned is an *N*-glycoside-linked sugar chain. As the *N*-glycoside-linked sugar chain, mentioned is a complex type sugar chain in which the non-reducing end side of the core structure has one or plural parallel branches of galactose-*N*-acetylglucosamine (hereinafter referred to as "Gal-GlcNAc") and the non-reducing end side of Gal-GlcNAc has a structure such as sialic acid, bisecting *N*-acetylglucosamine or the like.

In an antibody, the Fc region has positions to which each of two *N*-glycoside-linked sugar chains is bound described below. Accordingly, two sugar chains are bound per one antibody molecule. Since the *N*-glycoside-linked sugar chain which binds to an antibody includes any sugar chain represented by the following structural formula (I), there are a number of combinations of sugar chains for the two *N*-glycoside-linked sugar chains which bind to the antibody. Accordingly, identity of substances can be judged from the viewpoint of the sugar structure bound to the Fc region.

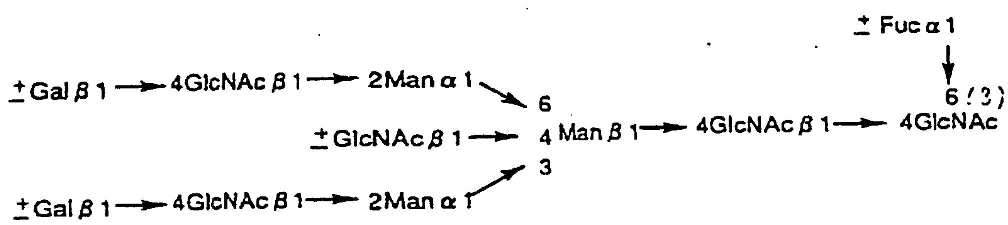

(I)

In the present invention, the composition which comprises an antibody molecule having complex *N*-glycoside-linked sugar chains in the Fc region (hereinafter referred to as "antibody composition of the present invention") may comprise an antibody having the same sugar chain structures or an antibody having different sugar chain structures, so long as the effect of the present invention is obtained from the composition.

In the present invention, "the ratio of a sugar chain in which fucose is not bound to *N*-acetylglucosamine in the reducing end in the sugar chain among the total complex *N*-glycoside-linked sugar chains bound to the Fc region contained in the antibody composition" means a ratio of the number of a sugar chain in which fucose is not bound to *N*-acetylglucosamine in the reducing end in the sugar chain to the total number of the complex *N*-glycoside-linked sugar chains bound to the Fc region contained in the composition.

In the present invention, "the sugar chain in which fucose is not bound to *N*-acetylglucosamine in the reducing end in the complex *N*-glycoside-linked sugar chain" means a sugar chain in which 1-position of the fucose is not bound to *N*-acetylglucosamine in the reducing end through α-bond in the complex *N*-glycoside-linked sugar chain. Examples include a complex *N*-glycoside-linked sugar chain in which 1-position of fucose is not bound to 6-position of *N*-acetylglucosamine through α-bond.

The ratio of a sugar chain in which fucose is not bound to *N*-acetylglucosamine in the reducing end in the sugar chain among the total complex *N*-glycoside-linked sugar chains binding to the Fc region contained in the antibody composition of the present invention is preferably 20% or more, more preferably 25% or more, still more preferably 30% or more, far preferably 40% or more, and most preferably 50% or more. The antibody composition having this ratio of a sugar chain has high ADCC activity.

As the antibody concentration is decreased, the ADCC activity is decreased accordingly. However, high ADCC activity can be obtained even though the antibody concentration is low, so long as the ratio of a sugar chain in which fucose is not bound to *N*-acetylglucosamine in the reducing end in the sugar chain is 20% or more.

The ratio of a sugar chain in which fucose is not bound to *N*-acetylglucosamine in the reducing end in the sugar chain contained in the composition which comprises an antibody molecule having complex *N*-glycoside-linked sugar chains in the Fc region can be determined by releasing the sugar chain from the antibody molecule using a known method such as hydrazinolysis, enzyme digestion or the like [*Biochemical Experimentation Methods 23 - Method for Studying Glycoprotein Sugar Chain* (Japan Scientific Societies Press), edited by Reiko Takahashi (1989)], carrying out fluorescence labeling or radioisotope labeling of the released sugar chain, and then separating the labeled sugar chain by chromatography. Alternatively, the released sugar chain can be determined by analyzing it with the HPAED-PAD method [*J. Liq. Chromatogr.*, 6, 1577 (1983)].

Furthermore, the cell of the present invention includes a cell which produces the composition of the present invention, wherein the activity of an enzyme relating to the synthesis of an intracellular sugar nucleotide, GDP-fucose and/or the activity of an enzyme relating to the modification of a sugar chain in which 1-position of fucose is bound to 6-position of *N*-acetylglucosamine in the reducing end through α-bond in the complex *N*-glycoside-linked sugar chain is decreased or deleted.

In the present invention, the enzyme relating to the synthesis of an intracellular sugar nucleotide, GDP-fucose, may be any enzyme, so long as it is an enzyme relating to the synthesis of the intracellular sugar nucleotide, GDP-fucose, as a supply source of fucose to a sugar chain. The enzyme relating to the synthesis of an intracellular sugar nucleotide, GDP-fucose, includes an enzyme which has influence on the synthesis of the intracellular sugar nucleotide, GDP-fucose.

The enzyme which has influence on the synthesis of an intracellular sugar nucleotide, GDP-fucose, includes an enzyme which has influence on the activity of the enzyme relating to the synthesis of the intracellular sugar nucleotide, GDP-fucose, and an enzyme which has influence on the structure of substance used as a substrate of the enzyme.

The intracellular sugar nucleotide, GDP-fucose, is supplied by a *de novo* synthesis pathway or a salvage synthesis pathway. Thus, all enzymes relating to the synthesis pathways are included in the enzyme relating to the synthesis of an intracellular sugar nucleotide, GDP-fucose.

The enzyme relating to the *de novo* synthesis pathway of the intracellular sugar nucleotide, GDP-fucose, include GDP-mannose 4,6-dehydratase (hereinafter referred to as "GMD"), GDP-keto-6-deoxymannose 3,5-epimerase 4,6-reductase (hereinafter referred to as "Fx") and the like.

The enzyme relating to the salvage synthesis pathway of the intracellular sugar nucleotide, GDP-fucose, include GDP-beta-L-fucose pyrophosphorylase (hereinafter referred to as "GFPP"), fucokinase and the like.

In the present invention, the GMD includes:

a protein encoded by a DNA of the following (a) or (b):

(a) a DNA comprising the nucleotide sequence represented by SEQ ID NO:41;
(b) a DNA which hybridizes with the DNA consisting of the nucleotide sequence represented by SEQ ID NO: 41 under stringent conditions and encodes a protein having GMD activity,
(c) a protein comprising the amino acid sequence represented by SEQ ID NO:61,
(d) a protein which consists of an amino acid sequence in which at least one amino acid is deleted, substituted, inserted and/or added in the amino acid sequence represented by SEQ ID NO:61 and has GMD activity, and
(e) a protein which consists of an amino acid sequence having a homology of at least 80% with the amino acid sequence represented by SEQ ID NO:61 and has GMD activity.

Also, the DNA encoding the amino acid sequence of GMD includes a DNA comprising the nucleotide sequence represented by SEQ ID NO:41 and a DNA which hybridizes with the DNA consists of the nucleotide sequence represented by SEQ ID NO:41 under stringent conditions and encodes an amino acid sequence having GMD activity.

In the present invention, the Fx includes:

a protein encoded by a DNA of the following (a) or (b):

(a) a DNA comprising the nucleotide sequence represented by SEQ ID NO:48;
(b) a DNA which hybridizes with the DNA consists of the nucleotide sequence represented by SEQ ID NO:48 under stringent conditions and encodes a protein having Fx activity,
(c) a protein comprising the amino acid sequence represented by SEQ ID NO:62,
(d) a protein which consists of an amino acid sequence in which at least one amino acid is deleted, substituted, inserted and/or added in the amino acid sequence represented by SEQ ID NO:62 and has Fx activity, and
(e) a protein which consists of an amino acid sequence having a homology of at least 80% with the amino acid sequence represented by SEQ ID NO:62 and has Fx activity.

Also, the DNA encoding the amino acid sequence of Fx includes a DNA comprising the nucleotide sequence represented by SEQ ID NO:48 and a DNA which hybridizes with the DNA consists of the nucleotide sequence represented by SEQ ID NO:48 under stringent conditions and encodes an amino acid sequence having Fx activity.

In the present invention, the GFPP includes:

a protein encoded by a DNA of the following (a) or (b):

(a) a DNA comprising the nucleotide sequence represented by SEQ ID NO:51;
(b) a DNA which hybridizes with the DNA consists of the nucleotide sequence represented by SEQ ID NO:51 under stringent conditions and encodes a protein having GFPP activity,
(c) a protein comprising the amino acid sequence represented by SEQ ID NO:63,
(d) a protein which comprises an amino acid sequence in which at least one amino acid is deleted, substituted,

inserted and/or added in the amino acid sequence represented by SEQ ID NO:63 and has GFPP activity, and
(e) a protein which consists of an amino acid sequence having a homology of at least 80% with the amino acid sequence represented by SEQ. IID NO:63 and has GFPP activity.

Also, the DNA encoding the amino acid sequence of GFPP include a DNA comprising the nucleotide sequence represented by SEQ ID NO:51 and a DNA which hybridizes with the DNA consisting of the nucleotide sequence represented by SEQ ID NO:51 under stringent conditions and encodes an amino acid sequence having Fx activity.

In the present invention, the enzyme relating to the modification of a sugar chain in which 1-position of fucose is bound to 6-position of *N*-acetylglucosamine in the reducing end through α-bond in the complex *N*-glycoside-linked sugar chain includes any enzyme, so long as it is an enzyme relating to the reaction of binding of 1-position of fucose to 6-position of *N*-acetylglucosamine in the reducing end through α-bond in the complex *N*-glycoside-linked sugar chain.

"The enzyme relating to the reaction of binding of 1-position of fucose to 6-position of N-acetylglucosamine in the reducing end through α-bond in the complex N-glycoside-linked sugar chain" means an enzyme which has influence in the reaction of binding of 1-position of fucose to 6-position of N-acetylglucosamine in the reducing end through α-bond in the complex N-glycoside-linked sugar chain.

The enzyme relating to the reaction of binding of 1-position of fucose to 6-position of *N*-acetylglucosamine in the reducing end through α-bond in the complex *N*-glycoside-linked sugar chain include α1,6-fucosyltransferase and α-L-fucosidase.

Also, examples include an enzyme which has influence on the activity the enzyme relating to the reaction of binding of 1-position of fucose to 6-position of *N*-acetylglucosamine in the reducing end through α-bond in the complex *N*-glycoside-linked sugar chain and an enzyme which has influence on the structure of substances as the substrate of the enzyme. In the present invention, the α1,6-fucosyltransferase includes:

a protein encoded by a DNA of the following (a), (b), (c) or (d):

(a) a DNA comprising the nucleotide sequence represented by SEQ ID NO:1;
(b) a DNA comprising the nucleotide sequence represented by SEQ ID NO:2;
(c) a DNA which hybridizes with the DNA consisting of the nucleotide sequence represented by SEQ ID NO: 1 under stringent conditions and encodes a protein having α1,6-fucosyltransferase activity;
(d) a DNA which hybridizes with the DNA consisting of the nucleotide sequence represented by SEQ ID NO: 2 under stringent conditions and encodes a protein having α1,6-fucosyltransferase activity;
(e) a protein comprising the amino acid sequence represented by SEQ ID NO:23,
(f) a protein comprising the amino acid sequence represented by SEQ ID NO:24,
(g) a protein which consisting of an amino acid sequence in which at least one amino acid is deleted, substituted, inserted and/or added in the amino acid sequence represented by SEQ ID NO:23 and has α1,6-fucosyltransferase activity,
(h) a protein which consisting of an amino acid sequence in which at least one amino acid is deleted, substituted, inserted and/or added in the amino acid sequence represented by SEQ ID NO:24 and has α1,6-fucosyltransferase activity,
(i) a protein which consisting of an amino acid sequence having a homology of at least 80% with the amino acid sequence represented by SEQ ID NO:23 and has α1,6-fucosyltransferase activity, and
(j) a protein which consisting of an amino acid sequence having a homology of at least 80% with the amino acid sequence represented by SEQ ID NO:24 and has α1,6-fucosyltransferase activity.

Also, the DNA encoding the amino acid sequence of α1,6-fucosyltransferase includes a DNA having the nucleotide sequence represented by SEQ ID NO:1 or 2 and a DNA which hybridizes with the DNA having the nucleotide sequence represented by SEQ ID NO:1 or 2 under stringent conditions and encodes an amino acid sequence having α1,6-fucosyltransferase activity.

In the present invention, "a DNA which hybridizes under stringent conditions" means a DNA obtained by a method such as colony hybridization, plaque hybridization or Southern blot hybridization using a DNA such as the DNA having the nucleotide sequence represented by SEQ ID NO:1, 2, 48, 51 or 41 or a partial fragment thereof as the probe. Specifically mentioned is a DNA which can be identified by carrying out hybridization at 65°C in the presence of 0.7 to 1.0 M sodium chloride using a filter to which colony- or plaque-derived DNA fragments are immobilized, and then washing the filter at 65°C using 0.1 to 2× SSC solution (composition of the 1 × SSC solution comprising 150 mM sodium chloride and 15 mM sodium citrate). The hybridization can be carried out in accordance with the methods described, e.g., in *Molecular Cloning, A Laboratoty Manual*, Second Edition., Cold Spring Harbor Laboratory Press (1989) (hereinafter referred to as *"Molecular Cloning*, Second Edition"), *Current Protocols in Molecular Biology,* John Wiley & Sons, 1987-1997 (hereinafter referred to as *"Current Protocols in Molecular Biology"*); *DNA Cloning 1: Core*

*Techniques, A Practical Approach,* Second Edition, Oxford University (1995); and the like. Examples of the hybridizable DNA include a DNA having at least 60% or more, preferably 70% or more, more preferably 80% or more, still more preferably 90% or more, far more preferably 95% or more, and most preferably 98% or more, of homology with the nucleotide sequence represented by SEQ ID NO:1, 2, 48, 51 or 41.

In the present invention, the protein which consists of an amino acid sequence in which at least one amino acid is deleted, substituted, inserted and/or added in the amino acid sequence represented by SEQ ID NO:23, 24, 61, 62 and 63 respectively and has α1,6-fucosyltransferase activity, GMD activity, Fx activity and GFPP activity can be obtained by introducing a site-directed mutation into a DNA encoding a protein having the amino acid sequence represented by SEQ ID NO:1, 2, 41, 48 and 51 respectively using the site-directed mutagenesis described, e.g., in *Molecular Cloning,* Second Edition; *Current Protocols in Molecular Biology; Nucleic Acids Research,* 10, 6487 (1982); *Proc. Natl. Acad. Sci. USA,* 79, 6409 (1982); *Gene, 34,* 315 (1985); *Nucleic Acids Research,* 13, 4431 (1985); *Proc. Natl. Acad Sci. USA*, 82, 488 (1985); and the like. The number of amino acids to be deleted, substituted; inserted and/or added is one or more, and the number is not particularly limited, but is a number which can be deleted, substituted or added by a known technique such as the site-directed mutagenesis, e.g., it is 1 to several tens, preferably 1 to 20, more preferably 1 to 10, and most preferably 1 to 5.

Also, each of proteins to be used in the present invention has at least 80% or more, preferably 85% or more, more preferably 90% or more, still more preferably 95% or more, far more preferably 97% or more, and most preferably 99% or more, of homology with the amino acid sequence represented by SEQ ID NO:23, 24, 61, 62 and 63 respectively, when calculated using an analyzing soft such as BLAST [*J. Mol. Biol.,* 215; 403 (1990)], FASTA [*Methods in Enzymology,* 183, 63 (1990)] or the like so that it can maintain the α1,6-fucosyltransferase activity, GMD activity, Fx activity and GFPP activity, respectively.

Furthermore, as the method for obtaining the cell of the present invention, that is, the cell in which the activity of an enzyme relating to the synthesis of an intracellular sugar nucleotide, GDP-fucose, and/or the activity of an enzyme relating to the modification of a sugar chain wherein 1-position of fucose is bound to 6-position of *N*-acetylglucosamine in the reducing end through α-bond in the complex *N*-glycoside-linked sugar chain is decreased or deleted, any technique can be used, so long as it can decrease the enzyme activity of interest. The technique for decreasing or deleting the enzyme activity include:

(a) a gene disruption technique targeting a gene encoding the enzyme,
(b) a technique for introducing a dominant negative mutant of a gene encoding the enzyme,
(c) a technique for introducing mutation into the enzyme,
(d) a technique for inhibiting transcription and/or translation of a gene encoding the enzyme, and
(e) a technique for selecting a cell line resistant to a lectin which recognizes a sugar chain in which 1-position of fucose is bound to 6-position of *N*-acetylglucosamine in the reducing end through α-bond in the complex *N*-glycoside-linked sugar chain.

As the lectin which recognizes a sugar chain structure in which 1-position of fucose is bound to 6-position of *N*-acetylglucosamine in the reducing end through α-bond in the *N*-glycoside-linked sugar chain, any lectin which can recognize the sugar chain structure can be used. Examples include a *Lens culinaris* lectin LCA (lentil agglutinin derived from *Lens culinaris*), a pea lectin PSA (pea lectin derived from *Pisum sativum*), a broad bean lectin VFA (agglutinin derived from *Vicia faba*), and an *Aleuria aurantia* lectin AAL (lectin derived from *Aleuria aurantia*).

The host cell for producing the antibody composition of the present invention may be any host, so long as it can express an anti-CD20 antibody molecule, i. e., a host cell transfected with a gene encoding an anti-CD20 antibody molecule. Examples include a yeast, an animal cell, an insect cell, a plant cell and the like. Examples of the cells include those described below in item 1. Among animal cells, preferred are include a CHO cell derived from a Chinese hamster ovary tissue, a rat myeloma cell line YB2/3HL.P2.G1 1.16Ag.20. cell, a mouse myeloma cell line NS0 cell, a mouse myeloma SP2/0-Ag14 cell, a BHK cell derived from a syrian hamster kidney tissue, an antibody producing-hybridoma cell, a human leukemia cell line Namalwa cell, an embryonic stem cell, a fertilized egg cell and the like. Examples include a rat myeloma cell line YB2/3HL.P2.G11.16Ag.20 cell transformed clone KM3065 (FERM BP-7834) transfected with the anti-CD20 antibody gene of the present invention.

The transgenic non-human animal or plant or the progenies thereof are not limited, so long as it is a transgenic non-human animal or plant or progeny thereof which produces an antibody composition comprising an antibody molecule which specifically binds to CD20 and has complex *N*-glycoside-linked sugar chains bound to the Fc region, wherein among the total complex *N*-glycoside-linked sugar chains bound to the Fc region in the composition, the ratio of a sugar chain in which fucose is not bound to *N*-acetylglucosamine in the reducing end in the sugar chain is 20% or more, and into which a gene encoding the antibody molecule is introduced. The antibody-producing transgenic animal can be prepared by introducing a gene encoding an antibody which specifically binds to CD20 into ES cell of a mouse, transplanting the ES cell into an early stage embryo of other mouse and then developing it. The transgenic

animal can be also prepared by introducing a gene encoding an antibody which specifically binds to CD20 into a fertilized egg and developing it.

The transgenic non-human animal include cattle, sheep, goat, pig, horse, mouse, rat, fowl, monkey, rabbit and the like.

In the present invention, the antibody molecule includes any molecule, so long as it comprises the Fc region of an antibody. Examples include an antibody, an antibody fragment, a fusion protein comprising an Fc region, and the like.

The antibody is a protein which is produced in the living body by immune response as a result of exogenous antigen stimulation and has an activity to specifically bind to the antigen. As the antibody, an antibody secreted by a hybridoma cell prepared from a spleen cell of an animal immunized with an antigen; an antibody prepared by a recombinant DNA technique, i.e., an antibody obtained by introducing an antibody gene-inserted antibody expression vector into a host cell; and the like are mentioned. Examples include an antibody produced by a hybridoma, a humanized antibody, a human antibody and the like.

As a hybridoma, a cell which is obtained by cell fusion between a B cell obtained by immunizing a mammal other than human with an antigen and a myeloma cell derived from mouse or the like and can produce a monoclonal antibody having the desired antigen specificity can be mentioned.

As the humanized antibody, a human chimeric antibody, a human complementarity determining region (hereinafter referred to as "CDR")-grafted antibody and the like can be mentioned.

A human chimeric antibody is an antibody which comprises a non-human antibody heavy chain variable region (hereinafter referred to as "HV" or "VH", the variable chain and the heavy chain being "V region" and "H chain", respectively) and a non-human antibody light chain variable region (hereinafter referred to as "LV" or "VL"), a human antibody heavy chain constant region (hereinafter also referred to as "CH") and a human antibody light chain constant region (hereinafter also referred to as "CL"). As the non-human animal, any animal such as mouse, rat, hamster, rabbit or the like can be used, so long as a hybridoma can be prepared therefrom.

The human chimeric antibody can be produced by preparing cDNAs encoding VH and VL from a monoclonal antibody-producing hybridoma, inserting them into an expression vector for host cell having genes encoding human antibody CH and human antibody CL to thereby construct a vector for expression of human chimeric antibody, and then introducing the vector into a host cell to express the antibody.

As the CH of human chimeric antibody, any CH can be used, so long as it belongs to human immunoglobulin (hereinafter referred to as "hIg"). Those belonging to the hIgG class are preferable, and any one of the subclasses belonging to the hIgG class, such as hIgG1, hIgG2, hIgG3 and hIgG4, can be used. Also, as the CL of human chimeric antibody, any CL can be used, so long as it belongs to the hIg class, and those belonging to the κ class or λ class can also be used.

A human CDR-grafted antibody is an antibody in which amino acid sequences of CDRs of VH and VL of an antibody derived from a non-human animal are grafted into appropriate positions of VH and VL of a human antibody.

The human CDR-grafted antibody can be produced by constructing cDNAs encoding V regions in which CDRs of VH and VL of an antibody derived from a non-human animal are grafted into CDRs of VH and VL of a human antibody, inserting them into an expression vector for host cell having genes encoding human antibody CH and human antibody CL to thereby construct a vector for human CDR-grafted antibody expression, and then introducing the expression vector into a host cell to express the human CDR-grafted antibody.

As the CH of human CDR-grafted antibody, any CH can be used, so long as it belongs to the hIg, but those of the hIgG class are preferable, and any one of the subclasses belonging to the hIgG class, such as hIgG1, hIgG2, hIgG3 and hIgG4, can be used. Also, as the CL of human CDR-grafted antibody, any CL can be used, so long as it belongs to the hIg class, and those belonging to the κ class or λ class can also be used.

A human antibody is originally an antibody naturally existing in the human body, but it also includes antibodies obtained from a human antibody phage library, a human antibody-producing transgenic animal and a human antibody-producing transgenic plant, which are prepared based on the recent advance in genetic engineering, cell engineering and developmental engineering techniques.

The antibody existing in the human body can be obtained by isolating a human peripheral blood lymphocyte, immortalizing it by its infection with EB virus or the like, cloning it to obtain a lymphocyte capable of producing the antibody, culturing the lymphocyte, and isolating and purifying the antibody from the culture.

The human antibody phage library is a library in which antibody fragments such as Fab (fragment of antigen binding), a single chain antibody and the like are expressed on the phage surface by inserting a gene encoding an antibody prepared from a human B cell into a phage gene. A phage expressing an antibody fragment having the desired antigen binding activity can be recovered from the library, using its activity to bind to an antigen-immobilized substrate as the marker. The antibody fragment can be converted further into a human antibody molecule comprising two full H chains and two full L chains by recombinant DNA techniques.

An antibody fragment is a fragment which comprises the Fc region of an antibody. As the antibody fragment,

an H chain monomer, an H chain dimer and the like can be mentioned.

A fusion protein comprising an Fc region includes a composition in which an antibody comprising the Fc region of an antibody or the antibody fragment is fused with a protein such as an enzyme, a cytokine or the like.

The antibody molecule of the present invention may be any antibody molecule, so long as it specifically binds to CD20. The antibody molecule is preferably an antibody molecule which specifically binds to CD20 and comprises complementarity determining regions 1, 2 and 3 of an antibody light chain variable region represented by the amino acid sequences represented by SEQ ID NOs:5, 6 and 7, respectively, and/or complementarity determining regions 1, 2 and 3 of an antibody heavy chain represented by the amino acid sequences represented by SEQ ID NOs:8, 9 and 10, respectively, and more preferably the antibody molecule which specifically binds to CD20 and comprises a light chain variable region represented by SEQ ID NO:12 and/or a heavy chain variable region represented by SEQ ID NO: 14.

The medicament of the present invention includes a medicament which comprises, as an active ingredient, the antibody composition of the present invention, i.e., the composition comprising an anti-CD20 antibody molecule.

The diseases relating to CD20 includes cancers such as B cell lymphoma, inflammatory diseases, autoimmune disease and the like.

In the present invention, the ADCC activity is a cytotoxic activity in which an antibody bound to a cell surface antigen on a tumor cell and the like in the living body activate an effector cell through an Fc receptor existing on the antibody Fc region and effector cell surface and thereby obstruct the tumor cell and the like [*Monoclonal Antibodies: Principles and Applications,* Wiley-Liss, Inc., Chapter 2.1 (1955)]. As the effector cell, a killer cell, a natural killer cell, an activated macrophage and the like can be mentioned.

The present invention is described below in detail.

1. Preparation of the cell which produces the antibody composition of the present invention

The cell of the present invention can be prepared by preparing a host cell used for producing the antibody composition of the present invention according to the following techniques and transfecting a gene encoding an anti-CD20 antibody into the host cell according to the method described in the following item 3.

(1) Gene disruption technique targeting at a gene encoding an enzyme

The host cell used for producing the cell of the present invention can be prepared using a gene disruption technique by targeting a gene encoding an enzyme relating to the synthesis of an intracellular sugar nucleotide, GDP-fucose, or targeting a gene encoding an enzyme relating to the modification of a sugar chain wherein 1-position of fucose is bound to 6-position of *N*-acetylglucosamine in the reducing end through α-bond in the complex *N*-glycoside-linked sugar chain. As the enzyme relating to the synthesis of an intracellular sugar nucleotide, GDP-fucose, GMD, Fx, GFPP, fucokinase and the like can be mentioned. As the enzyme relating to the modification of a sugar chain wherein 1-position of fucose is bound to 6-position of *N*-acetylglucosamine in the reducing end through α-bond in the complex *N*-glycoside-linked sugar chain, α1,6-fucosyltransferase, α-L-fucosidase and the like can be mentioned.

The gene as used herein includes DNA and RNA.

As the gene disruption method, any method can be include, so long as it can disrupt the gene of the target enzyme. As examples, an antisense method, a ribozyme method, a homologous recombination method, an RNA-DNA oligonucleotide method (hereinafter referred to as "RDO method"), an RNA interface method (hereinafter referred to as "RNAi method"), a method using retrovirus, a method using transposon, and the like can be mentioned. The methods are specifically described below.

(a) Preparation of the host cell for preparing the cell of the present invention by the antisense method or the ribozyme method

The host cell for preparing the cell of the present invention can be prepared by the antisense method or the ribozyme method described in *Cell Technology,* 12, 239 (1993); *BIO/TECHNOLOGY,* 17, 1097 (1999); *Hum. Mol. Genet.,* 5, 1083 (1995); *Cell Technology,* 13, 255 (1994); *Proc. Natl. Acad Sci. USA,* 96, 1886 (1999); or the like, e.g., in the following manner by targeting a gene encoding an enzyme relating to the synthesis of an intracellular sugar nucleotide, GDP-fucose, and/or a gene encoding an enzyme relating to the modification of a sugar chain wherein 1-position of fucose is bound to 6-position of *N*-acetylglucosamine in the reducing end through α-bond in the complex *N*-glycoside-linked sugar chain.

A cDNA or a genomic DNA encoding an enzyme relating to the synthesis of an intracellular sugar nucleotide, GDP-fucose, or encoding an enzyme relating to the modification of a sugar chain wherein 1-position of fucose is bound to 6-position of *N*-acetylglucosamine in the reducing end through α-bond in the complex *N*-glycoside-linked sugar chain

is prepared.

The nucleotide sequence of the prepared cDNA or genomic DNA is determined.

Based on the determined DNA sequence, an appropriate length of an antisense gene or ribozyme construct comprising a part of a DNA which encodes the enzyme relating to the synthesis of an intracellular sugar nucleotide, GDP-fucose, and/or the enzyme relating to the modification of a sugar chain wherein 1-position of fucose is bound to 6-position of *N*-acetylglucosamine in the reducing end through α-bond in the complex *N*-glycoside-linked sugar chain is designed. The designed construct can further contain a part of non-translation region or an intron.

In order to express the antisense gene or the ribozyme in a cell, a recombinant vector is prepared by inserting a fragment or total length of the prepared DNA into downstream of the promoter of an appropriate expression vector.

A transformant is obtained by introducing the recombinant vector into a host cell suitable for the expression vector.

The cell of the present invention can be obtained by selecting a transformant using, as a marker, the activity of the enzyme relating to the synthesis of an intracellular sugar nucleotide, GDP-fucose, and/or the enzyme relating to the modification of a sugar chain wherein 1-position of fucose is bound to 6-position of *N*-acetylglucosamine in the reducing end through α-bond in the complex *N*-glycoside-linked sugar chain. The host cell for preparing the cell of the present invention can also be obtained by selecting a transformant based on the sugar chain structure of a glycoprotein on the cell membrane or the sugar chain structure of the produced antibody molecule.

As the host cell for preparing the cell of the present invention, any cell such as a yeast, an animal cell, an insect cell or a plant cell can be used, so long as it has a gene encoding the target enzyme relating to the synthesis of an intracellular sugar nucleotide, GDP-fucose, or a gene encoding the target enzyme relating to the modification of a sugar chain wherein 1-position of fucose is bound to 6-position of *N*-acetylglucosamine in the reducing end through α-bond in the complex *N*-glycoside-linked sugar chain. Examples include host cells described in the following item 3.

As the expression vector, a vector which is autonomously replicable in the host cell or can be integrated into the chromosome and comprises a promoter at such a position that the designed antisense gene or ribozyme can be transferred can be used. Examples include expression vectors described in the following item 3.

Regarding the method for introducing a gene into various host cells, the methods for introducing recombinant vectors suitable for various host cells, which are described in the following item 3, can be used.

The following method can be exemplified as the method for selecting a transformant using, a marker of the activity of an enzyme relating to the synthesis of an intracellular sugar nucleotide, GDP-fucose and/or the activity of an enzyme relating to the modification of a sugar chain wherein 1-position of fucose is bound to 6-position of *N*-acetylglucosamine in the reducing end through α-bond in the complex *N*-glycoside-linked sugar chain.

Method for selecting transformant:

The method for selecting a cell in which the activity of an enzyme relating to the synthesis of an intracellular sugar nucleotide, GDP-fucose and/or the activity of an enzyme relating to the modification of a sugar chain wherein 1-position of fucose is bound to 6-position of *N*-acetylglucosamine in the reducing end through α-bond in the complex *N*-glycoside-linked sugar chain is decreased include biochemical methods or genetic engineering techniques described in *New Biochemical Experimentation Series 3-Saccharides I*, *Glycoprotein* (Tokyo Kagaku Dojin), edited by Japanese Biochemical society (1988); *Cell Engineering, Supplement, Experimental Protocol Series, Glycobiology Experimental Protocol, Glycoprotein, Glycolipid and Proteoglycan* (Shujun-sha), edited by Naoyuki Taniguchi, Akemi Suzuki, Kiyoshi Furukawa and Kazuyuki Sugawara (1996); *Molecular Cloning,* Second Edition; *Current Protocols in Molecular Biology;* and the like. The biochemical method includes a method in which the enzyme activity is evaluated using an enzyme-specific substrate and the like. The genetic engineering technique include the Northern analysis, RT-PCR and the like wherein the amount of mRNA of a gene encoding the enzyme is measured.

The method for selecting a transformant based on the sugar chain structure of a glycoprotein on the cell membrane includes methods described in the following item 1(5). The method for selecting a transformant based on the sugar chain structure of a produced antibody molecule includes methods described in the following items 4 and 5.

As the method for preparing cDNA encoding an enzyme relating to the synthesis of an intracellular sugar nucleotide, GDP-fucose, and/or an enzyme relating to the modification of a sugar chain wherein 1-position of fucose is bound to 6-position of *N*-acetylglucosamine in the reducing end through α-bond in the complex *N*-glycoside-linked sugar chain, the following method is exemplified.

Preparation method of DNA:

A total RNA or mRNA is prepared from tissues or cells of various host cells.

A cDNA library is prepared from the prepared total RNA or mRNA.

Degenerative primers are produced based on the amino acid sequence of an enzyme relating to the synthesis

of an intracellular sugar nucleotide, GDP-fucose, and/or an enzyme relating to the modification of a sugar chain wherein 1-position of fucose is bound to 6-position of *N*-acetylglucosamine in the reducing end through α-bond in the complex *N*-glycoside-linked sugar chain, and a gene fragment encoding the enzyme relating to the synthesis of an intracellular sugar nucleotide, GDP-fucose, and/or the enzyme relating to the modification of a sugar chain wherein 1-position of fucose is bound to 6-position of *N*-acetylglucosamine in the reducing end through α-bond in the complex *N*-glycoside-linked sugar chain is obtained by PCR using the prepared cDNA library as the template.

A DNA encoding the enzyme relating to the synthesis of an intracellular sugar nucleotide, GDP-fucose, and/or the enzyme relating to the modification of a sugar chain wherein 1-position of fucose is bound to 6-position of *N*-acetylglucosamine in the reducing end through α-bond in the complex *N*-glycoside-linked sugar chain can be obtained by screening the cDNA library using the obtained gene fragment as a probe.

Regarding the mRNA of a human or non-human tissue or cell, a commercially available product (e.g., manufactured by Clontech) may be used or it may be prepared from a human or non-human animal tissue or cell in the following manner. Examples of the method for preparing a total RNA from a human or non-human animal tissue or cell include the guanidine thiocyanate-cesium trifluoroacetate method [*Methods in Enzymology,* 154, 3 (1987)], the acidic guanidine thiocyanate phenol chloroform (AGPC) method [*Analytical Biochemistry,* 162, 156 (1987); *Experimental Medicine,* 9, 1937 (1991)] and the like.

Also, the method for preparing mRNA from a total RNA as poly(A)$^+$ RNA include an oligo(dT)-immobilized cellulose column method (*Molecular Cloning,* Second Edition) and the like.

In addition, mRNA can be prepared using a kit such as Fast Track mRNA Isolation Kit (manufactured by Invitrogen), Quick Prep mRNA Purification Kit (manufactured by Pharmacia) or the like.

A cDNA library is prepared from the prepared mRNA of a human or non-human animal tissue or cell. The method for preparing cDNA libraries include the methods described in *Molecular Cloning,* Second Edition; *Current Protocols in Molecular Biology; A Laboratory Manual*, Second Edition (1989); and the like, or methods using commercially available kits such as SuperScript Plasmid System for cDNA Synthesis and Plasmid Cloning (manufactured by Life Technologies), ZAP-cDNA Synthesis Kit (manufactured by STRATAGENE) and the like.

As the cloning vector for preparing the cDNA library, any vector such as a phage vector, a plasmid vector or the like can be used, so long as it is autonomously replicable in *Escherichia coli* K12. Examples include ZAP Express [manufactured by STRATAGENE, *Strategies,* 5, 58 (1992)], pBluescript II SK(+) [*Nucleic Acids Research,* 17, 9494 (1989)], Lambda ZAP II (manufactured by STRATAGENE), λgt10 and λgt11 [*DNA Cloning, A Practical Approach*, 1, 49 (1985)], λTriplEx (manufactured by Clontech), λExCell (manufactured by Pharmacia), pT7T318U (manufactured by Pharmacia), pcD2 [*Mol. Cell. Biol.*, 3, 280 (1983)], pUC18 [*Gene,* 33, 103 (1985)] and the like.

Any microorganism can be used as the host microorganism, and *Escherichia coli* is preferably used. Examples include *Escherichia coli* XL1-Blue MRF' [manufactured by STRATAGENE, *Strategies,* 5, 81 (1992)], *Escherichia coli* C600 [*Genetics*, 39, 440 (1954)], *Escherichia coli* Y1088 [*Science,* 222, 778 (1983)], *Escherichia coli* Y1090 [*Science,* 222, 778 (1983)], *Escherichia coli* NM522 [*J. Mol. Biol.*, 166, 1 (1983)], *Escherichia coli* K802 [*J. Mol. Biol.,* 16, 118 (1966)], *Escherichia coli* JM105 [*Gene,* 38, 275 (1985)] and the like

The cDNA library may be used as such in the subsequent analysis, and in order to obtain a full length cDNA as efficient as possible by decreasing the ratio of an infull length cDNA, a cDNA library prepared using the oligo cap method developed by Sugano *et al*. [*Gene,* 138, 171 (1994); *Gene,* 200, 149 (1997); *Protein*, *Nucleic Acid and Enzyme,* 41, 603 (1996); *Experimental Medicine,* 11, 2491 (1993); *cDNA Cloning* (Yodo-sha) (1996); *Methods for Preparing Gene Libraries* (Yodo-sha) (1994)] may be used in the following analysis.

Degenerative primers specific for the 5'-terminal and 3'-terminal nucleotide sequences of a nucleotide sequence presumed to encode the amino acid sequence are prepared based on the amino acid sequence of the enzyme relating to the synthesis of an intracellular sugar nucleotide, GDP-fucose, and/or the enzyme relating to the modification of a sugar chain wherein 1-position of fucose is bound to 6-position of N-acetylglucosamine in the reducing end through α-bond in the complex *N*-glycoside-linked sugar chain, and DNA is amplified by PCR [*PCR Protocols,* Academic Press (1990)] using the prepared cDNA library as the template to obtain a gene fragment encoding the enzyme relating to the synthesis of an intracellular sugar nucleotide, GDP-fucose, and/or the enzyme relating to the modification of a sugar chain wherein 1-position of fucose is bound to 6-position of *N*-acetylglucosamine in the reducing end through α-bond in the complex *N*-glycoside-linked sugar chain.

It can be confirmed that the obtained gene fragment is a DNA encoding the enzyme relating to the synthesis of an intracellular sugar nucleotide, GDP-fucose, and/or the enzyme relating to the modification of a sugar chain wherein 1-position of fucose is bound to 6-position of *N*-acetylglucosamine in the reducing end through α-bond in the complex *N*-glycoside-linked sugar chain, by a method usually used for sequencing a nucleotide, such as the dideoxy method of Sanger *et al.* [*Proc. Natl. Acad Sci. USA*, 74, 5463 (1977)], a nucleotide sequence analyzer such as ABI PRISM 377 DNA Sequencer (manufactured by Applied Biosystems) or the like.

A DNA encoding the enzyme relating to the synthesis of an intracellular sugar nucleotide, GDP-fucose, and/or the enzyme relating to the modification of a sugar chain wherein 1-position of fucose is bound to 6-position of *N*-

acetylglucosamine in the reducing end through α-bond in the complex *N*-glycoside-linked sugar chain can be obtained by carrying out colony hybridization or plaque hybridization (*Molecular Cloning,* Second Edition) for the cDNA or cDNA library synthesized from the mRNA contained in the human or non-human animal tissue or cell, using the gene fragment as a DNA probe.

Also, a DNA encoding the enzyme relating to the synthesis of an intracellular sugar nucleotide, GDP-fucose, and/or the enzyme relating to the modification of a sugar chain wherein 1-position of fucose is bound to 6-position of *N*-acetylglucosamine in the reducing end through α-bond in the complex *N*-glycoside-linked sugar chain can be obtained by carrying out screening by PCR using the primers used for obtaining the gene fragment encoding the enzyme relating to the synthesis of an intracellular sugar nucleotide, GDP-fucose; and/or the enzyme relating to the modification of a sugar chain wherein 1-position of fucose is bound to 6-position of *N*-acetylglucosamine in the reducing end through α-bond in the complex *N*-glycoside-linked sugar chain and using the cDNA or cDNA library synthesized from the mRNA contained in a human or non-human animal tissue or cell as the template.

The nucleotide sequence of the obtained DNA encoding the enzyme relating to the synthesis of an intracellular sugar nucleotide, GDP-fucose, and/or the enzyme relating to the modification of a sugar chain wherein 1-position of fucose is bound to 6-position of *N*-acetylglucosamine in the reducing end through α-bond in the complex *N*-glycoside-linked sugar chain is determined by analyzing the nucleotide sequence from its terminus by a method usually used for sequencing a nucleotide, such as the dideoxy method of Sanger *et al*. [*Proc. Natl*. *Acad*. *Sci. USA*, 74, 5463 (1977)], a nucleotide sequence analyzer such as ABI PRISM 377 DNA Sequencer (manufactured by Applied Biosystems) or the like.

A gene encoding the enzyme relating to the synthesis of an intracellular sugar nucleotide, GDP-fucose, and/or the enzyme relating to the modification of a sugar chain wherein 1-position of fucose is bound to 6-position of *N*-acetylglucosamine in the reducing end through α-bond in the complex *N*-glycoside-linked sugar chain can also be determined from genes in data bases by searching nucleotide sequence data bases such as GenBank, EMBL, DDBJ and the like using a homology searching program such as BLAST based on the determined cDNA nucleotide sequence.

The nucleotide sequence of the gene obtained by the method encoding the enzyme relating to the synthesis of an intracellular sugar nucleotide, GDP-fucose includes the nucleotide sequence represented by SEQ ID NO:48, 51 or 41. The nucleotide sequence of the gene encoding the enzyme relating to the modification of a sugar chain wherein 1-position of fucose is bound to 6-position of *N*-acetylglucosamine in the reducing end through α-bond in the complex *N*-glycoside-linked sugar chain includes the nucleotide sequence represented by SEQ ID NO:1 or 2.

The cDNA encoding the enzyme relating to the synthesis of an intracellular sugar nucleotide, GDP-fucose, and/or the enzyme relating to the modification of a sugar chain wherein 1-position of fucose is bound to 6-position of *N*-acetylglucosamine in the reducing end through α-bond in the complex *N*-glycoside-linked sugar chain can also be obtained by chemically synthesizing it with a DNA synthesizer such as DNA Synthesizer model 392 manufactured by Perkin Elmer or the like using the phosphoamidite method, based on the determined DNA nucleotide sequence.

As an example of the method for preparing a genomic DNA encoding the enzyme relating to the synthesis of an intracellular sugar nucleotide, GDP-fucose, and/or the enzyme relating to the modification of a sugar chain wherein 1-position of fucose is bound to 6-position of *N*-acetylglucosamine in the reducing end through α-bond in the complex *N*-glycoside-linked sugar chain, the method described below is exemplified.

Preparation method of genomic DNA:

As the method for preparing genomic DNA, known methods described in *Molecular Cloning,* Second Edition; *Current Protocols in Molecular Biology;* and the like can be mentioned. In addition, a genomic DNA encoding the enzyme relating to the synthesis of an intracellular sugar nucleotide, GDP-fucose, and/or the enzyme relating to the modification of a sugar chain wherein 1-position of fucose is bound to 6-position of *N*-acetylglucosamine in the reducing end through α-bond in the complex *N*-glycoside-linked sugar chain can also be isolated using a kit such as Genomic DNA Library Screening System (manufactured by Genome Systems), Universal GenomeWalker™ Kits (manufactured by CLONTECH) or the like.

The nucleotide sequence of the genomic DNA, obtained by the above method, encoding the enzyme relating to the synthesis of an intracellular sugar nucleotide, GDP-fucose, includes the nucleotide sequence represented by SEQ ID NO:57 or 60. The nucleotide sequence of the genomic DNA encoding the enzyme relating to the modification of a sugar chain wherein 1-position of fucose is bound to 6-position of *N*-acetylglucosamine in the reducing end through α-bond in the complex *N*-glycoside-linked sugar chain includes the nucleotide sequence represented by SEQ ID NO:3.

In addition, the host cell of the present invention can also be obtained without using an expression vector, by directly introducing an antisense oligonucleotide or ribozyme into a host cell, which is designed based on the nucleotide sequence encoding the enzyme relating to the synthesis of an intracellular sugar nucleotide, GDP-fucose, and/or the enzyme relating to the modification of a sugar chain wherein 1-position of fucose is bound to 6-position of *N*-acetylglucosamine in the reducing end through α-bond in the complex *N*-glycoside-linked sugar chain.

The antisense oligonucleotide or ribozyme can be prepared in the usual method or using a DNA synthesizer. Specifically, it can be prepared based on the sequence information of an oligonucleotide having a corresponding sequence of continuous 5 to 150 bases, preferably 5 to 60 bases, and more preferably 10 to 40 bases, among nucleotide sequences of a cDNA and a genomic DNA encoding the enzyme relating to the synthesis of an intracellular sugar nucleotide, GDP-fucose, and/or the enzyme relating to the modification of a sugar chain wherein 1-position of fucose is bound to 6-position of *N*-acetylglucosamine in the reducing end through α-bond in the complex *N*-glycoside-linked sugar chain, by synthesizing an oligonucleotide which corresponds to a sequence complementary to the oligonucleotide (antisense oligonucleotide) or a ribozyme comprising the oligonucleotide sequence.
The oligonucleotide includes oligo RNA and derivatives of the oligonucleotide (hereinafter referred to as "oligonucleotide derivatives").
As the oligonucleotide derivatives, oligonucleotide derivatives in which a phosphodiester bond in the oligonucleotide is converted into a phosphorothioate bond, oligonucleotide derivatives in which a phosphodiester bond in the oligonucleotide is converted into an N3'-P5' phosphoamidate bond, oligonucleotide derivatives in which ribose and a phosphodiester bond in the oligonucleotide are converted into a peptide-nucleic acid bond, oligonucleotide derivatives in which uracil in the oligonucleotide is substituted with C-5 propynyluracil, oligonucleotide derivatives in which uracil in the oligonucleotide is substituted with C-5 thiazoleuracil, oligonucleotide derivatives in which cytosine in the oligonucleotide is substituted with C-5 propynylcytosine, oligonucleotide derivatives in which cytosine in the oligonucleotide is substituted with phenoxazine-modified cytosine, oligonucleotide derivatives in which ribose in the oligonucleotide is substituted with 2'-O-propylribose, oligonucleotide derivatives in which ribose in the oligonucleotide is substituted with 2'-methoxyethoxyribose [*Cell Technology*, 16, 1463 (1997)] and the like can be mentioned.

(b) Preparation of the host cell for preparing the cell of the present invention by homologous recombination

The host cell for preparing the cell of the present invention can be produced by modifying a target gene on chromosome through a homologous recombination technique, and using a gene encoding an enzyme relating to the synthesis of an intracellular sugar nucleotide, GDP-fucose, and/or an enzyme relating to the modification of a sugar chain wherein 1-position of fucose is bound to 6-position of *N*-acetylglucosamine in the reducing end through α-bond in the complex *N*-glycoside-linked sugar chain as the target gene.
The target gene on the chromosome can be modified by using a method described in *Manipulating the Mouse Embryo, A Laboratory Manual,* Second Edition, Cold Spring Harbor Laboratory Press (1994) (hereinafter referred to as *"Manipulating the Mouse Embryo, A Laboratory Manual"*); *Gene Targeting, A Practical Approach,* IRL Press at Oxford University Press (1993); *Biomanual Series 8, Gene Targeting, Preparation of Mutant Mice using ES Cells,* Yodosha (1995) (hereinafter referred to as *"Preparation of Mutant Mice using ES Cells"*); or the like, for example, as follows.
A genomic DNA encoding an enzyme relating to the synthesis of an intracellular sugar nucleotide, GDP-fucose, and/or an enzyme relating to the modification of a sugar chain wherein 1-position of fucose is bound to 6-position of *N*-acetylglucosamine in the reducing end through α-bond in the complex *N*-glycoside-linked sugar chain is prepared.
Based on the nucleotide sequence of the genomic DNA, a target vector is prepared for homologous recombination of a target gene to be modified (e.g., structural gene of the enzyme relating to the synthesis of an intracellular sugar nucleotide, GDP-fucose, and/or the enzyme relating to the modification of a sugar chain wherein 1-position of fucose is bound to 6-position of *N*-acetylglucosamine in the reducing end through α-bond in the complex *N*-glycoside-linked sugar chain, or a promoter gene).
The host cell for preparing the cell of the present invention can be produced by introducing the prepared target vector into a host cell and selecting a cell in which homologous recombination occurred between the target gene and target vector.
As the host cell, any cell such as a yeast, an animal cell, an insect cell or a plant cell can be used, so long as it has a gene encoding the enzyme relating to the synthesis of an intracellular sugar nucleotide, GDP-fucose, and/or the enzyme relating to the modification of a sugar chain wherein 1-position of fucose is bound to 6-position of *N*-acetylglucosamine in the reducing end through α-bond in the complex *N*-glycoside-linked sugar chain. Examples include host cells described in the following item 3.
The method for preparing a genomic DNA encoding the enzyme relating to the synthesis of an intracellular sugar nucleotide, GDP-fucose, and/or the enzyme relating to the modification of a sugar chain wherein 1-position of fucose is bound to 6-position of *N*-acetylglucosamine in the reducing end through α-bond in the complex *N*-glycoside-linked sugar chain includes the methods described in the preparation of genomic DNA in item 1(1)(a) and the like.
The nucleotide sequence of genomic DNA encoding the enzyme relating to the synthesis of an intracellular sugar nucleotide, GDP-fucose, includes the nucleotide sequence represented by SEQ ID NO:57 or 60. The nucleotide sequence of genomic DNA encoding the enzyme relating to the modification of a sugar chain wherein 1-position of fucose is bound to 6-position of *N*-acetylglucosamine in the reducing end through α-bond in the complex *N*-glycoside-linked sugar chain include the nucleotide sequence represented by SEQ ID NO:3.

The target vector for the homologous recombination of the target gene can be prepared in accordance with a method described in *Gene Targeting, A Practical Approach,* IRL Press at Oxford University Press (1993); *Biomanual Series 8, Gene Targeting, Preparation of Mutant Mice using ES Cells,* Yodo-sha (1995); or the like. The target vector can be used as either a replacement type or an insertion type.
For introducing the target vector into various host cells, the methods for introducing recombinant vectors suitable for various host cells, which are described in the following item 3, can be used.
The method for efficiently selecting a homologous recombinant includes a method such as the positive selection, promoter selection, negative selection or polyA selection described in *Gene Targeting*, *A Practical Approach,* IRL Press at Oxford University Press (1993); *Biomanual Series 8, Gene Targeting*, *Preparation of Mutant Mice using ES Cells,* Yodo-sha (1995); or the like. The method for selecting the homologous recombinant of interest from the selected cell lines includes the Southern hybridization method for genomic DNA (*Molecular Cloning,* Second Edition), PCR [*PCR Protocols,* Academic Press (1990)], and the like.

(c) Preparation of the host cell for preparing the cell of the present invention by RDO method

The host cell for preparing the cell of the present invention can be prepared by an RDO (RNA-DNA oligonucleotide) method by targeting a gene encoding an enzyme relating to the synthesis of an intracellular sugar nucleotide, GDP-fucose, and/or an enzyme relating to the modification of a sugar chain wherein 1-position of fucose is bound to 6-position of *N*-acetylglucosamine in the reducing end through α-bond in the complex *N*-glycoside-linked sugar chain, for example, as follows.
A cDNA or a genomic DNA encoding an enzyme relating to the synthesis of an intracellular sugar nucleotide, GDP-fucose, and/or an enzyme relating to the modification of a sugar chain wherein 1-position of fucose is bound to 6-position of *N*-acetylglucosamine in the reducing end through α-bond in the complex *N*-glycoside-linked sugar chain is prepared.
The nucleotide sequence of the prepared cDNA or genomic DNA is determined.
Based on the determined DNA sequence, an appropriate length of an RDO construct comprising a DNA which encodes the enzyme relating to the synthesis of an intracellular sugar nucleotide, GDP-fucose, and/or the enzyme relating to the modification of a sugar chain wherein 1-position of fucose is bound to 6-position of *N*-acetylglucosamine in the reducing end through α-bond in the complex *N*-glycoside-linked sugar chain. The designed RDO construct can further comprise a part of non-translation region or a part of an intron.
The host cell of the present invention can be obtained by introducing the synthesized RDO into a host cell and then selecting a transformant in which a mutation occurred in the target enzyme, that is, the enzyme relating to the synthesis of an intracellular sugar nucleotide, GDP-fucose, and/or the enzyme relating to the modification of a sugar chain wherein 1-position of fucose is bound to 6-position of *N*-acetylglucosamine in the reducing end through α-bond in the complex *N*-glycoside-linked sugar chain.
As the host cell, any cell such as a yeast, an animal cell, an insect cell or a plant cell can be used, so long as it has a gene encoding the target enzyme relating to the synthesis of an intracellular sugar nucleotide, GDP-fucose, and/or the target enzyme relating to the modification of a sugar chain wherein 1-position of fucose is bound to 6-position of *N*-acetylglucosamine in the reducing end through α-bond in the complex *N*-glycoside-linked sugar chain. Examples include host cells described in the following item 3.
The method for introducing RDO into various host cells includes the methods for introducing recombinant vectors suitable for various host cells, which are described in the following item 3.
The method for preparing cDNA encoding the enzyme relating to the synthesis of an intracellular sugar nucleotide, GDP-fucose, and/or the enzyme relating to the modification of a sugar chain wherein 1-position of fucose is bound to 6-position of *N*-acetylglucosamine in the reducing end through α-bond in the complex *N*-glycoside-linked sugar chain include the methods described in the preparation method of DNA in item 1(1)(a) and the like.
The method for preparing a genomic DNA encoding the enzyme relating to the synthesis of an intracellular sugar nucleotide, GDP-fucose, and/or the enzyme relating to the modification of a sugar chain wherein 1-position of fucose is bound to 6-position of *N*-acetylglucosamine in the reducing end through α-bond in the complex *N*-glycoside-linked sugar chain include the methods in preparation of genomic DNA described in item 1(1)(a) and the like.
The nucleotide sequence of the DNA can be determined by digesting it with appropriate restriction enzymes, cloning the DNA fragments into a plasmid such as pBluescript SK(-) (manufactured by Stratagene) or the like, subjecting the clones to the reaction generally used as a method for analyzing a nucleotide sequence such as the dideoxy method [*Proc. Natl. Acad Sci. USA*, 74, 5463 (1977)] of Sanger *et al*. or the like, and then analyzing the clones using an automatic nucleotide sequence analyzer such as A.L.F. DNA Sequencer (manufactured by Pharmacia) or the like.
The RDO can be prepared by a usual method or using a DNA synthesizer.
The method for selecting a cell in which a mutation occurred, by introducing the RDO into the host cell, in the gene encoding the enzyme relating to the synthesis of an intracellular sugar nucleotide, GDP-fucose, and/or the enzyme

relating to the modification of a sugar chain wherein 1-position of fucose is bound to 6-position of *N*-acetylglucosamine in the reducing end through α-bond in the complex *N*-glycoside-linked sugar chain includes the methods for directly detecting mutations in chromosomal genes described in *Molecular Cloning*, Second Edition, *Current Protocols in Molecular Biology* and the like.

Also, as the method, the method described in item 1(1)(a) for selecting a transformant through the evaluation of the activity of the introduced enzyme relating to the synthesis of an intracellular sugar nucleotide, GDP-fucose, and/or the enzyme relating to the modification of a sugar chain wherein 1-position of fucose is bound to 6-position of *N*-acetylglucosamine in the reducing end through α-bond in the complex *N*-glycoside-linked sugar chain; the method for selecting a transformant based on the sugar chain structure of a glycoprotein on the cell membrane which will be described later in item 1(5); and the method for selecting a transformant based on the sugar chain structure of the produced antibody molecule which will be described later in item 4 or 5, and the like can be used.

The construct of the RDO can be designed in accordance with the methods described in *Science,* 273, 1386 (1996); *Nature Medicine,* 4, 285 (1998); *Hepatology*, 25, 1462 (1997); *Gene Therapy,* 5, 1960 (1999); *J. Mol. Med.,* 75, 829 (1997); *Proc. Natl. Acad. Sci. USA*, 96, 8774 (1999); *Proc. Natl. Acad Sci. USA*, 96, 8768 (1999); *Nuc. Acids. Res.,* 27, 1323 (1999); *Invest. Dematol.*, 111, 1172 (1998); *Nature Biotech.*, 16, 1343 (1998); *Nature Biotech.,* 18, 43 (2000); *Nature Biotech.,* 18, 555 (2000); and the like.

(d) Preparation of the host cell for preparing the cell of the present invention by the RNAi method

The host cell for preparing the cell of the present invention can be prepared by the RNAi (RNA interference) method by targeting a gene of an enzyme relating to the synthesis of an intracellular sugar nucleotide, GDP-fucose, and/or of an enzyme relating to the modification of a sugar chain wherein 1-position of fucose is bound to 6-position of *N*-acetylglucosamine in the reducing end through α-bond in the complex *N*-glycoside-linked sugar chain, for example, as follows.

A cDNA encoding an enzyme relating to the synthesis of an intracellular sugar nucleotide, GDP-fucose, and/or an enzyme relating to the modification of a sugar chain wherein 1-position of fucose is bound to 6-position of *N*-acetylglucosamine in the reducing end through α-bond in the complex *N*-glycoside-linked sugar chain is prepared.

The nucleotide sequence of the prepared cDNA is determined.

Based on the determined DNA sequence, an appropriate length of an RNAi gene construct comprising a part of DNA encoding the enzyme relating to the synthesis of an intracellular sugar nucleotide, GDP-fucose, and/or the enzyme relating to the modification of a sugar chain wherein 1-position of fucose is bound to 6-position of *N*-acetylglucosamine in the reducing end through α-bond in the complex *N*-glycoside-linked sugar chain is designed. The designed construct can further comprise a part of its non-translation region.

In order to express the RNAi gene in a cell, a recombinant vector is prepared by inserting a fragment or full length of the prepared DNA into downstream of the promoter of an appropriate expression vector.

A transformant is obtained by introducing the recombinant vector into a host cell suitable for the expression vector.

The host cell for preparing the cell of the present invention can be obtained by selecting a transformant based on the activity of the enzyme relating to the synthesis of an intracellular sugar nucleotide, GDP-fucose, and/or of the enzyme relating to the modification of a sugar chain wherein 1-position of fucose is bound to 6-position of *N*-acetylglucosamine in the reducing end through α-bond in the complex *N*-glycoside-linked sugar chain, or based on the sugar chain structure of a glycoprotein on the cell membrane or of the produced antibody molecule.

As the host cell, any cell such as a yeast, an animal cell, an insect cell or a plant cell can be used, so long as it has a gene encoding the target enzyme relating to the synthesis of an intracellular sugar nucleotide, GDP-fucose, and/or the target enzyme relating to the modification of a sugar chain wherein 1-position of fucose is bound to 6-position of *N*-acetylglucosamine in the reducing end through α-bond in the complex *N*-glycoside-linked sugar chain. Examples include host cells described in the following item 3.

As the expression vector, a vector which is autonomously replicable in the host cell or can be integrated into the chromosome and comprises a promoter at such a position that the designed RNAi gene can be transferred is used. Examples include expression vectors described in the following item 3.

As the method for introducing a gene into various host cells, the methods for introducing recombinant vectors suitable for various host cells, which are described in the following item 3, can be used.

The method for selecting a transformant based on the activity of the enzyme relating to the synthesis of an intracellular sugar nucleotide, GDP-fucose, and/or the enzyme relating to the modification of a sugar chain wherein 1-position of fucose is bound to 6-position of *N*-acetylglucosamine in the reducing end through α-bond in the complex *N*-glycoside-linked sugar chain includes the methods described in item 1(1)(a).

The method for selecting a transformant based on the sugar chain structure of a glycoprotein on the cell membrane includes the methods which will be described later in item 1(5). The method for selecting a transformant

based on the sugar chain structure of a produced antibody molecule includes the methods described in the following item 4 or 5.

The method for preparing cDNA encoding the enzyme relating to the synthesis of an intracellular sugar nucleotide, GDP-fucose, or the enzyme relating to the modification of a sugar chain wherein 1-position of fucose is bound to 6-position of *N*-acetylglucosamine in the reducing end through α-bond in the complex *N*-glycoside-linked sugar chain includes the methods described in the preparation method of DNA in item 1(1)(a) and the like.

In addition, the host cell for preparing the cell of the present invention can also-be obtained without using an expression vector, by directly introducing an RNAi gene designed based on the nucleotide sequence encoding the enzyme relating to the synthesis of an intracellular sugar nucleotide, GDP-fucose, and/or the enzyme relating to the modification of a sugar chain wherein 1-position of fucose is bound to 6-position of *N*-acetylglucosamine in the reducing end through α-bond in the complex *N*-glycoside-linked sugar chain.

The RNAi gene can be prepared in the usual method or using a DNA synthesizer.

The RNAi gene construct can be designed in accordance with the methods described in *Nature,* 391. 806 (1998); *Proc. Natl. Acad Sci. USA,* 95, 15502 (1998); *Nature,* 395, 854 (1998); *Proc. Natl. Acad. Sci. USA,* 96 5049 (1999); *Cell,* 95 1017 (1998); *Proc. Natl. Acad Sci. USA,* 96, 1451 (1999); *Proc. Natl. Acad. Sci. USA,* 95, 13959 (1998); *Nature Cell Biol.,* 2, 70 (2000); and the like.

(e) Preparation of the host cell for preparing the cell of the present invention by the method using transposon

The host cell for preparing the cell of the present invention can be prepared by inducing mutation using a transposon system described in *Nature Genet.,* 25, 35 (2000) or the like, and then by selecting a mutant based on the activity of the enzyme relating to the synthesis of an intracellular sugar nucleotide, GDP-fucose, and/or the enzyme relating to the modification of a sugar chain wherein 1-position of fucose is bound to 6-position of *N*-acetylglucosamine in the reducing end through α-bond in the complex *N*-glycoside-linked sugar chain, or based on the sugar chain structure of a glycoprotein of a produced antibody molecule or on the cell membrane.

The transposon system is a system in which a mutation is induced by randomly inserting an exogenous gene into chromosome, wherein an exogenous gene interposed between transposons is generally used as a vector for inducing a mutation, and a transposase expression vector for randomly inserting the gene into chromosome is introduced into the cell at the same time.

Any transposase can be used, so long as it is suitable for the sequence of the transposon to be used.

As the exogenous gene, any gene can be used, so long as it can induce a mutation in the DNA of a host cell.

As the host cell, any cell such as a yeast, an animal cell, an insect cell or a plant cell can be used, so long as it has a gene encoding the target enzyme relating to the synthesis of an intracellular sugar nucleotide, GDP-fucose, and/or of the target enzyme relating to the modification of a sugar chain wherein 1-position of fucose is bound to 6-position of *N*-acetylglucosamine in the reducing end through α-bond in the complex *N*-glycoside-linked sugar chain. Examples include host cells described in the following item 3. For introducing the gene into various host cells, the methods for introducing recombinant vectors suitable for various host cells, which are described in the following item 3, can be used.

The method for selecting a mutant based on the activity of the enzyme relating to the synthesis of an intracellular sugar nucleotide, GDP-fucose, and/or the enzyme relating to the modification of a sugar chain wherein 1-position of fucose is bound to 6-position of *N*-acetylglucosamine in the reducing end through α-bond in the complex *N*-glycoside-linked sugar chain includes the methods described in item 1(1)(a).

The method for selecting a mutant based on the sugar chain structure of a glycoprotein on the cell membrane includes the methods described in the following item 1(5). The method for selecting a mutant based on the sugar chain structure of a produced antibody molecule includes the methods described in the following item 4 or 5.

(2) Method for introducing a dominant negative mutant of a gene encoding an enzyme

The host cell for preparing the cell of the present invention can be prepared by targeting a gene encoding an enzyme relating to the synthesis of an intracellular sugar nucleotide, GDP-fucose, and/or an enzyme relating to the modification of a sugar chain wherein 1-position of fucose is bound to 6-position of *N*-acetylglucosamine in the reducing end through α-bond in the complex *N*-glycoside-linked sugar chain, and using a technique for introducing a dominant negative mutant of the enzyme. The enzyme relating to the synthesis of an intracellular sugar nucleotide, GDP-fucose, includes GMD, Fx, GFPP, fucokinase and the like. The enzyme relating to the modification of a sugar chain wherein 1-position of fucose is bound to 6-position of *N*-acetylglucosamine in the reducing end through α-bond in the complex *N*-glycoside-linked sugar chain includes α1,6-fucosyltransferase, α-L-fucosidase and the like.

The enzymes catalyze specific reactions having substrate specificity, and dominant negative mutants of a gene encoding the enzymes can be prepared by disrupting the active center of the enzymes which catalyze the catalytic

activity having substrate specificity. The method for preparing a dominant negative mutant is specifically described as follows with reference to GMD among the target enzymes.

As a result of the analysis of the three-dimensional structure of *E. coli*-derived GMD, it has been found that 4 amino acids (threonine at position 133, glutamic acid at position 135, tyrosine at position 157 and lysine at position 161) have an important function on the enzyme activity (*Structure,* 8, 2, 2000). That is, when mutants were prepared by substituting the 4 amino acids with other different amino acids based on the three-dimensional structure information, the enzyme activity of all of the mutants was significantly decreased. On the other hand, changes in the ability of mutant GMD to bind to GMD coenzyme NADP or its substrate GDP-mannose were hardly observed. Accordingly, a dominant negative mutant can be prepared by substituting the 4 amino acids which control the enzyme activity of GMD. For example, in GMD (SEQ ID NO:41) derived from CHO cell, a dominant negative mutant can be prepared by substituting threonine at position 155, glutamic acid at position 157, tyrosine at position 179 and lysine at position 183 with other amino acids, by comparing the homology and predicting the three-dimensional structure using the amino acid sequence information based on the results of the *E. coli*-derived GMD. Such a gene into which amino acid substitution is introduced can be prepared by the site-directed mutagenesis described in *Molecular Cloning,* Second Edition, *Current Protocols in Molecular Biology* or the like.

The host cell for preparing the cell of the present invention can be prepared in accordance with the method described in *Molecular Cloning,* Second Edition, *Current Protocols in Molecular Biology* or the like, using the prepared dominant negative mutant gene of the target enzyme, for example, as follows.

A gene encoding a dominant negative mutant (hereinafter referred to as "dominant negative mutant gene") of the enzyme relating to the synthesis of an intracellular sugar nucleotide, GDP-fucose, and/or the enzyme relating to the modification of a sugar chain wherein 1-position of fucose is bound to 6-position of *N*-acetylglucosamine in the reducing end through α-bond in the complex *N*-glycoside-linked sugar chain is prepared.

Based on the prepared full length DNA of dominant negative mutant gene, a DNA fragment of an appropriate length containing a moiety encoding the protein is prepared, if necessary.

A recombinant vector is produced by inserting the DNA fragment or full length DNA into downstream of the promoter of an appropriate expression vector.

A transformant is obtained by introducing the recombinant vector into a host cell suitable for the expression vector.

The host cell for preparing the cell of the present invention can be prepared by selecting a transformant based on the activity of the enzyme relating to the synthesis of an intracellular sugar nucleotide, GDP-fucose, and/or the activity of the enzyme relating to the modification of a sugar chain wherein 1-position of fucose is bound to 6-position of *N*-acetylglucosamine in the reducing end through α-bond in the complex *N*-glycoside-linked sugar chain, or the sugar chain structure of a glycoprotein of a produced antibody molecule or on the cell membrane.

As the host cell, any cell such as a yeast, an animal cell, an insect cell or a plant cell can be used, so long as it has a gene encoding the target enzyme relating to the synthesis of an intracellular sugar nucleotide, GDP-fucose, and/or the target enzyme relating to the modification of a sugar chain wherein 1-position of fucose is bound to 6-position of *N*-acetylglucosamine in the reducing end through α-bond in the complex *N*-glycoside-linked sugar chain. Examples include the host cells which will be described later in the following item 3.

As the expression vector, a vector which is autonomously replicable in the host cell or can be integrated into the chromosome and comprises a promoter at a position where transcription of the DNA encoding the dominant negative mutant of interest can be effected is used. Examples include expression vectors described in the following item 3.

For introducing the gene into various host cells, the methods for introducing recombinant vectors suitable for various host cells, which are described in the following item 3, can be used.

The method for selecting a transformant based on the activity of the enzyme relating to the synthesis of an intracellular sugar nucleotide, GDP-fucose, and/or the enzyme relating to the modification of a sugar chain wherein 1-position of fucose is bound to 6-position of *N*-acetylglucosamine in the reducing end through α-bond in the complex *N*-glycoside-linked sugar chain includes the methods described in item 1(1)(a).

The method for selecting a transformant based on the sugar chain structure of a glycoprotein on the cell membrane includes the methods described in item 1(5). The method for selecting a transformant based on the sugar chain structure of a produced antibody molecule includes methods described in the following item 4 or 5.

(3) Method for introducing a mutation into an enzyme

The host cell for preparing the cell of the present invention can be prepared by introducing a mutation into a gene encoding an enzyme relating to the synthesis of an intracellular sugar nucleotide, GDP-fucose, or an enzyme relating to the modification of a sugar chain wherein 1-position of fucose is bound to 6-position of *N*-acetylglucosamine in the reducing end through α-bond in the complex *N*-glycoside-linked sugar chain, and then by selecting a cell line of interest in which the mutation occurred in the enzyme.

The enzyme relating to the synthesis of an intracellular sugar nucleotide, GDP-fucose, includes GMD, Fx, GFPP, fucokinase and the like. The enzyme relating to the modification of a sugar chain wherein 1-position of fucose is bound to 6-position of *N*-acetylglucosamine in the reducing end through α-bond in the complex *N*-glycoside-linked sugar chain includes α1,6-fucosyltransferase, α-L-fucosidase and the like.
The method includes 1) a method in which a desired cell line is selected from mutants obtained by a mutation-inducing treatment of a parent cell line with a mutagen or spontaneously generated mutants, based on the activity of an enzyme relating to the synthesis of an intracellular sugar nucleotide, GDP-fucose, and/or the activity of an enzyme relating to the modification of a sugar chain wherein 1-position of fucose is bound to 6-position of *N*-acetylglucosamine in the reducing end through α-bond in the complex *N*-glycoside-linked sugar chain, 2) a method in which a desired cell line is selected from mutants obtained by a mutation-inducing treatment of a parent cell line with a mutagen or spontaneously generated mutants, based on the sugar chain structure of a produced antibody molecule, and 3) a method in which a desired cell line is selected from mutants obtained by a mutation-inducing treatment of a parent cell line with a mutagen or spontaneously generated mutants, based on the sugar chain structure of a glycoprotein on the cell membrane.
As the mutation-inducing treatment, any treatment can be used, so long as it can induce a point mutation or a deletion or frame shift mutation in the DNA of cells of the parent cell line.
Examples include treatment with ethyl nitrosourea, nitrosoguanidine, benzopyrene or an acridine pigment and treatment with radiation. Also, various alkylating agents and carcinogens can be used as mutagens. The method for allowing a mutagen to act upon cells includes the methods described in *Tissue Culture Techniques,* 3rd edition (Asakura Shoten), edited by Japanese Tissue Culture Association (1996), *Nature Genet.,* 24*,* 314 (2000) and the like.
The spontaneously generated mutant includes mutants which are spontaneously formed by continuing subculture under general cell culture conditions without applying special mutation-inducing treatment.
The method for measuring the activity of the enzyme relating to the synthesis of an intracellular sugar nucleotide, GDP-fucose, and/or the activity of the enzyme relating to the modification of a sugar chain wherein 1-position of fucose is bound to 6-position of *N*-acetylglucosamine in the reducing end through α-bond in the complex N-glycoside-linked sugar chain includes the methods described in item 1(1)(a). The method for discriminating the sugar chain structure of a prepared antibody molecule includes methods described in the following item 4 or 5. The method for discriminating the sugar chain structure of a glycoprotein on the cell membrane includes the methods described in item 1(5).

(4) Method for inhibiting transcription and/or translation of a gene encoding an enzyme

The host cell of the present invention can be prepared by inhibiting transcription and/or translation of a target gene through a method such as the antisense RNA/DNA technique [*Bioscience and Industry*, 50, 322 (1992); *Chemistry*, 46 681 (1991); *Biotechnology,* 9, 358 (1992); *Trends in Biotechnology,* 10, 87 (1992); *Trends in Biotechnology,* 10, 152 (1992); *Cell Engineering,* 16, 1463 (1997)], the triple helix technique [*Trends in Biotechnology,* 10, 132 (1992)] or the like, using a gene encoding an enzyme relating to the synthesis of an intracellular sugar nucleotide, GDP-fucose, and/or an enzyme relating to the modification of a sugar chain wherein 1-position of fucose is bound to 6-position of *N*-acetylglucosamine in the reducing end through α-bond in the complex *N*-glycoside-linked sugar chain, as the target.
The enzyme relating to the synthesis of an intracellular sugar nucleotide, GDP-fucose includes GMD, Fx, GFPP, fucokinase and the like. The enzyme relating to the modification of a sugar chain wherein 1-position of fucose is bound to 6-position of *N*-acetylglucosamine in the reducing end through α-bond in the complex *N*-glycoside-linked sugar chain includes α1,6-fucosyltransferase, α-L-fucosidase and the like.

(5) Method for selecting a cell line resistant to a lectin which recognizes a sugar chain structure in which 1-position of fucose is bound to 6-position of *N*-acetylglucosamine in the reducing end through α-bond in the *N*-glycoside-linked sugar chain

The host cell for preparing the cell of the present invention can be prepared by using a method for selecting a cell line resistant to a lectin which recognizes a sugar chain structure in which 1-position of fucose is bound to 6-position of N-acetylglucosamine in the reducing end through α-bond in the *N*-glycoside-linked sugar chain.
The method for selecting a cell line resistant to a lectin which recognizes a sugar chain structure in which 1-position of fucose is bound to 6-position of *N*-acetylglucosamine in the reducing end through α-bond in the *N*-glycoside-linked sugar chain includes the methods using lectin described in *Somatic Cell Mol. Genet.,* 12, 51 (1986) and the like.
As the lectin, any lectin can be used, so long as it is a lectin which recognizes a sugar chain structure in which 1-position of fucose is bound to 6-position of *N*-acetylglucosamine in the reducing end through α-bond in the *N*-glycoside-linked sugar chain. Examples include a *Lens culinaris* lectin LCA (lentil agglutinin derived from *Lens culinaris*), a

pea lectin PSA (pea lectin derived from *Pisum sativum*), a broad bean lectin VFA (agglutinin derived from *Vicia faba*), an *Aleuria aurantia* lectin AAL (lectin derived from *Aleuria aurantia*) and the like.

Specifically, the cell line of the present invention resistant to a lectin which recognizes a sugar chain structure in which 1-position of fucose is bound to 6-position of *N*-acetylglucosamine in the reducing end through α-bond in the *N*-glycoside-linked sugar chain can be selected by culturing cells for 1 day to 2 weeks, preferably from 1 day to 1 week, using a medium comprising the lectin at a concentration of 1 μg/ml to 1 mg/ml, subculturing surviving cells or picking up a colony and transferring it into a culture vessel, and subsequently continuing the culturing using the lectin-containing medium.

2. Preparation of a transgenic non-human animal or plant or the progenies thereof of the present invention

The transgenic non-human animal or plant or the progenies thereof in which a genome gene is modified in such a manner that the activity of an enzyme relating to the modification of a sugar chain of an antibody molecule can be controlled can be prepared from the embryonic stem cell, the fertilized egg cell or the plant callus cell of the present invention prepared by the above item 1 using a gene encoding an enzyme relating to the synthesis of an intracellular sugar nucleotide, GDP-fucose, and/or an enzyme relating to the modification of a sugar chain wherein 1-position of fucose is bound to 6-position of *N*-acetylglucosamine in the reducing end through α-bond in the complex *N*-glycoside-linked sugar chain, as the target, for example, as follows.

In a transgenic non-human animal, the embryonic stem cell of the present invention in which the activity of the enzyme relating to the synthesis of an intracellular sugar nucleotide, GDP-fucose, and/or the activity of the enzyme relating to the modification of a sugar chain wherein 1-position of fucose is bound to 6-position of *N*-acetylglucosamine in the reducing end through α-bond in the complex *N*-glycoside-linked sugar chain is controlled can be prepared by the method described in item 1 to an embryonic stem cell of the intended non-human animal such as cattle, sheep, goat, pig, horse, mouse, rat, fowl, monkey, rabbit or the like.

Specifically, a mutant clone is prepared in which a gene encoding the enzyme relating to the synthesis of an intracellular sugar nucleotide, GDP-fucose, and/or the enzyme relating to the modification of a sugar chain wherein 1-position of fucose is bound to 6-position of *N*-acetylglucosamine in the reducing end through α-bond in the complex *N*-glycoside-linked sugar chain is inactivated or substituted with any sequence, by a known homologous recombination technique [e.g., *Nature*, 326, 6110, 295 (1987); *Cell,* 51, 3, 503 (1987); or the like]. Using the prepared embryonic stem cell (e.g., the mutant clone), a chimeric individual comprising the embryonic stem cell clone and a normal cell can be prepared by an injection chimera method into blastocyst of fertilized egg of an animal or by an aggregation chimera method. The chimeric individual is crossed with a normal individual, so that a transgenic non-human animal in which the activity of the enzyme relating to the synthesis of an intracellular sugar nucleotide, GDP-fucose, and/or the activity of the enzyme relating to the modification of a sugar chain wherein 1-position of fucose is bound to 6-position of *N*-acetylglucosamine in the reducing end through α-bond in the complex *N*-glycoside-linked sugar chain is decreased or deleted in the whole body cells can be obtained.

Also, a fertilized egg cell of the present invention in which the activity of an enzyme relating to the synthesis of an intracellular sugar nucleotide, GDP-fucose, and/or the activity of an enzyme relating to the modification of a sugar chain wherein 1-position of fucose is bound to 6-position of *N*-acetylglucosamine in the reducing end through α-bond in the complex *N*-clycoside-linked sugar chain is decreased or deleted can be prepared by applying the method similar to that in item 1 to fertilized egg of a non-human animal of interest such as cattle, sheep, goat, pig, horse, mouse, rat, fowl, monkey, rabbit or the like.

A transgenic non-human animal in which the activity of an enzyme relating to the synthesis of an intracellular sugar nucleotide, GDP-fucose, and/or the activity of an enzyme relating to the modification of a sugar chain wherein 1-position of fucose is bound to 6-position of *N*-acetylglucosamine in the reducing end through α-bond in the complex *N*-glycoside-linked sugar chain is decreased can be prepared by transplanting the prepared fertilized egg cell into the oviduct or uterus of a pseudopregnant female using the embryo transplantation method described in *Manipulating Mouse Embryo,* Second Edition or the like, followed by childbirth by the animal.

In a transgenic plant, the callus of the present invention in which the activity of an enzyme relating to the synthesis of an intracellular sugar nucleotide, GDP-fucose, and/or the activity of an enzyme relating to the modification of a sugar chain wherein 1-position of fucose is bound to 6-position of *N*-acetylglucosamine in the reducing end through α-bond in the complex *N*-glycoside-linked sugar chain is decreased or deleted can be prepared by applying the method similar to that in item 1 to a callus or cell of the plant of interest.

A transgenic plant in which the activity of an enzyme relating to the synthesis of an intracellular sugar nucleotide, GDP-fucose, and/or the activity of an enzyme relating to the modification of a sugar chain wherein 1-position of fucose is bound to 6-position of *N*-acetylglucosamine in the reducing end through α-bond in the complex *N*-glycoside-linked sugar chain is decreased can be prepared by culturing the prepared callus using a medium comprising auxin and cytokinin to redifferentiate it in accordance with a known method [*Tissue Culture*, 20 (1994); *Tissue Culture,* 21

(1995); *Trends in Biotechnology*, 15, 45 (1997)].

3. Process for producing the antibody composition

The antibody composition can be obtained by expressing it in a host cell using the methods described in *Molecular Cloning,* Second Edition; *Current Protocols in Molecular Biology; Antibodies, A Laboratory Manual,* Cold Spring Harbor Laboratory, 1988 (hereinafter referred also to as *"Antibodies"); Monoclonal Antibodies: Principles and Practice,* Third Edition, Acad. Press, 1993 (hereinafter referred also to as *"Monoclonal Antibodies");* and *Antibody Engineering, A Practical Approach,* IRL Press at Oxford University Press (hereinafter referred also to as *"Antibody Engineering");* for example, as follows.

A full length cDNA encoding the anti-CD20 antibody molecule of the present invention is prepared, and an appropriate length of a DNA fragment comprising a region encoding the antibody molecule is prepared.

A recombinant vector is prepared by inserting the DNA fragment or the full length cDNA into downstream of the promoter of an appropriate expression vector.

A transformant which produces the antibody molecule can be obtained by introducing the recombinant vector into a host cell suitable for the expression vector.

As the host cell, any of a yeast, an animal cell, an insect cell, a plant cell or the like can be used, so long as it can express the gene of interest.

As the host cell, a cell into which an enzyme relating to the modification of an *N*-glycoside-linked sugar chain which binds to the Fc region of the antibody molecule, i.e., an enzyme relating to the synthesis of an intracellular sugar nucleotide, GDP-fucose and/or the activity of an enzyme relating to the modification of a sugar chain in which 1-position of fucose is bound to 6-position of *N*-acetylglucosamine in the reducing end through α-bond in the complex *N*-glycoside-linked sugar chain is decreased or deleted or a cell obtained by various artificial techniques described in item 1 can also be used.

As the expression vector, a vector which is autonomously replicable in the host cell or can be integrated into the chromosome and comprises a promoter at such a position that the DNA encoding the antibody molecule of interest can be transferred is used.

The cDNA can be prepared from a human or non-human tissue or cell using, e.g., a probe primer specific for the antibody molecule of interest, in accordance with the methods described in the preparation method of DNA in item 1(1)(a).

When a yeast is used as the host cell, the expression vector includes YEP13 (ATCC 37115); YEp24 (ATCC 37051), YCp50 (ATCC 37419) and the like.

Any promoter can be used, so long as it can function in yeast. Examples include a promoter of a gene of the glycolytic pathway such as a hexose kinase gene, PH05 promoter, PGK promoter, GAP promoter, ADH promoter, gal 1 promoter, gal 10 promoter, heat shock protein promoter, MF α1 promoter, CUP 1 promoter and the like.

The host cell includes microorganisms belonging to the genus *Saccharomyces*, the genus *Schizosaccharomyces*, the genus *Kluyveromyces*, the genus *Trichosporon*, the genus *Schwanniomyces* and the like, such as *Saccharomyces cerevisiae*, *Schizosaccharomyces pombe*, *Kluyveromyces lactis*, *Trichosporon pullulans* and *Schwanniomyces alluvius.*

As the method for introducing the recombinant vector, any method can be used, so long as it can introduce DNA into yeast. Examples include electroporation [*Methods in Enzymology,* 194, 182 (1990)], the spheroplast method [*Proc. Natl. Acad Sci. USA,* 84, 1929 (1978)], the lithium acetate method [*J. Bacteriol.,* 153, 163 (1983)], the method described in *Proc. Natl. Acad Sci. USA, 75*, 1929 (1978) and the like.

When an animal cell is used as the host, the expression vector includes pcDNAI, pcDM8 (available from Funakoshi), pAGE107 [Japanese Published Examined Patent Application No. 22979/91; *Cytotechnology,* 3, 133 (1990)], pAS3-3 (Japanese Published Examined Patent Application No. 227075/90), pCDM8 [*Nature,* 329, 840 (1987)], pcDNAI/Amp (manufactured by Invitrogen), pREP4 (manufactured by Invitrogen), pAGE103 [*J. Biochemistry,* 101, 1307 (1987)], pAGE210 and the like.

Any promoter can be used, so long as it can function in an animal cell. Examples include a promoter of IE (immediate early) gene of cytomegalovirus (CMV), an early promoter of SV40, a promoter of retrovirus, a promoter of metallothionein, a heat shock promoter, an SRα promoter and the like. Also, an enhancer of the IE gene of human CMV may be used together with the promoter.

The host cell includes a human cell such as Namalwa cell, a monkey cell such as COS cell, a Chinese hamster cell such as CHO cell or HBT5637 (Japanese Published Examined Patent Application No. 299/88), a rat myeloma cell, a mouse myeloma cell, a cell derived from syrian hamster kidney, an embryonic stem cell, a fertilized egg cell and the like.

As the method for introducing the recombinant vector, any method can be used, so long as it can introduce DNA into an animal cell. Examples include electroporation [*Cytotechnology,* 3, 133 (1990)], the calcium phosphate

method (Japanese Published Examined Patent Application No. 227075/90), the lipofection method [*Proc. Natl. Acad Sci. USA,* 84, 7413 (1987)], the injection method [*Manipulating the Mouse Embryo, A Laboratory Manual*], the method using particle gun (gene gun) (Japanese Patent No. 2606856, Japanese Patent No. 2517813), the DEAE-dextran method [*Biomanual Series 4-Gene Transfer and Expression Analysis* (Yodo-sha), edited by Takashi Yokota and Kenichi Arai (1994)], the virus vector method [*Manipulating Mouse Embryo,* Second Edition] and the like.

When an insect cell is used as the host, the protein can be expressed by the method described in *Current Protocols in Molecular Biology, Baculovirus Expression Vectors, A Laboratory Manual,* W.H. Freeman and Company, New York (1992), *Bio/Technology,* 6, 47 (1988) or the like.

That is, the protein can be expressed by co-introducing a recombinant gene-introducing vector and a baculovirus into an insect cell to obtain a recombinant virus in an insect cell culture supernatant and then infecting the insect cell with the recombinant virus.

The gene introducing vector used in the method includes pVL1392, pVL1393, pBlueBacIII (all manufactured by Invitrogen) and the like.

The baculovirus includes *Autographa californica* nuclear polyhedrosis virus infected with an insect of the family *Barathra*.

The insect cell includes *Spodoptera frugiperda* ovarian Sf9 and Sf21 [*Current Protocols in Molecular Biology, Baculovirus Expression Vectors, A Laboratory Manual,* W.H. Freeman and Company, New York (1992)], a *Trichoplusia ni* ovarian High 5 (manufactured by Invitrogen) and the like.

The method for the simultaneously introducing the recombinant gene-introducing.vector and the baculovirus for preparing the recombinant virus includes the calcium phosphate method (Japanese Published Examined Patent Application No. 227075/90), the lipofection method [*Proc. Natl. Acad. Sci. USA,* 84, 7413 (1987)] and the like.

When a plant cell is used as the host cell, the expression vector include Ti plasmid, tobacco mosaic virus and the like.

As the promoter, any promoter can be used, so long as it can function in a plant cell. Examples include cauliflower mosaic virus (CaMV) 35S promoter, rice actin 1 promoter and the like.

The host cell includes plant cells of tobacco, potato, tomato, carrot, soybean, rape, alfalfa, rice, wheat, barley, and the like.

As the method for introducing the recombinant vector, any method can be used, so long as it can introduce DNA into a plant cell. Examples include a method using *Agrobacterium* (Japanese Published Examined Patent Application No. 140885/84, Japanese Published Examined Patent Application No. 70080/85, WO 94/00977), electroporation (Japanese Published Examined Patent Application No. 251887/85), a method using a particle gun (gene gun) (Japanese Patent No. 2606856, Japanese Patent No. 2517813) and the like.

As the method for expressing a gene, secretion production, expression of a fusion protein of the Fc region with other protein and the like can be carried out in accordance with the method described in *Molecular Cloning,* Second Edition or the like, in addition to the direct expression.

When a gene is expressed by a bacterium, a yeast, an animal cell, an insect cell or a plant cell into which a gene relating to the synthesis of a sugar chain is introduced, an antibody molecule to which a sugar or a sugar chain is added by the introduced gene can be obtained.

An antibody composition can be obtained by culturing the obtained transformant in a medium to form and accumulate the antibody molecule in the culture and then recovering it from the culture. The method for culturing the transformant using a medium can be carried out in accordance with a general method which is used for the culturing of host cells.

As the medium for culturing a transformant obtained by using a prokaryote such as *Escherichia coli* or a eukaryote such as yeast as the host, the medium may be either a natural medium or a synthetic medium, so long as it comprises materials such as a carbon source, a nitrogen source, an inorganic salt and the like which can be assimilated by the organism and culturing of the transformant can be efficiently carried out.

As the carbon source, those which can be assimilated by the organism can be used. Examples include carbohydrates such as glucose, fructose, sucrose, molasses containing them, starch, and starch hydrolysate; organic acids such as acetic acid and propionic acid; alcohols such as ethanol and propanol; and the like.

The nitrogen source includes ammonia; ammonium salts of inorganic acid or organic acid such as ammonium chloride, ammonium sulfate, ammonium acetate and ammonium phosphate; other nitrogen-containing compounds; peptone; meat extract; yeast extract; corn steep liquor; casein hydrolysate; soybean meal; soybean meal hydrolysate; various fermented cells and hydrolysates thereof; and the like.

The inorganic material includes potassium dihydrogen phosphate, dipotassium hydrogen phosphate, magnesium phosphate, magnesium sulfate, sodium chloride, ferrous sulfate, manganese sulfate, copper sulfate, calcium carbonate, and the like. The culturing is carried out generally under aerobic conditions such as shaking culture or submerged-aeration stirring culture. The culturing temperature is preferably 15 to 40°C, and the culturing time is generally 16 hours to 7 days. During the culturing, the pH is maintained at 3.0 to 9.0. The pH is adjusted using inorganic

or organic acid, an alkali solution, urea, calcium carbonate, ammonia or the like.

Also, if necessary, an antibiotic such as ampicillin or tetracycline may be added to the medium during the culturing.

When a microorganism transformed with a recombinant vector obtained by using an inducible promoter as the promoter is cultured, an inducer may be added to the medium, if necessary. For example, when a microorganism transformed with a recombinant vector obtained by using *lac* promoter is cultured, isopropyl-β-D-thiogalactopyranoside may be added to the medium, and when a microorganism transformed with a recombinant vector obtained by using *trp* promoter is cultured, indoleacrylic acid may be added to the medium.

When a transformant obtained by using an animal cell as the host is cultured, examples of the medium include generally used RPMI 1640 medium [*The Journal of the American Medical Association,* 199, 519 (1967)], Eagle's MEM medium [*Science,* 122, 501 (1952)], Dulbecco's modified MEM medium [*Virology*, 8, 396 (1959)], 199 medium [*Proceeding of the Society for the Biological Medicine,* 73, 1 (1950)] and Whitten's medium [*Developmental Engineering Experimentation Manual-Preparation of Transgenic Mice* (Kodan-sha), edited by M. Katshuki (1987)], wherein the media are added to fetal calf serum.

The culturing is carried out generally at a pH of 6 to 8 and 30 to 40°C for 1 to 7 days in the presence of 5% $CO_2$.

If necessary, an antibiotic such as kanamycin or penicillin may be added to the medium during the culturing.

The medium for culturing of a transformant obtained by using an insect cell as the host includes usually used TNM-FH medium (manufactured by Pharmingen), Sf-900 II SFM medium (manufactured by Life Technologies), ExCell 400 and ExCell 405 (both manufactured by JRH Biosciences), Grace's Insect Medium [*Nature*, 195, 788 (1962)] and the like.

The culturing is carried out generally at a medium pH of 6 to 7 and 25 to 30°C for 1 to 5 days.

If necessary, antibiotics such as gentamicin may be added to the medium during the culturing.

A transformant obtained by using a plant cell as the host can be cultured as a cell or by differentiating it into a plant cell or organ. The medium for culturing the transformant includes generally used Murashige and Skoog (MS) medium and White medium, wherein the media are added to a plant hormone such as auxin or cytokinin.

The culturing is carried out generally at a pH of 5 to 9 and 20 to 40°C for 3 to 60 days.

If necessary, an antibiotic such as kanamycin, hygromycin or the like may be added to the medium during the culturing.

Thus, an antibody composition can be produced by culturing a transformant derived from a microorganism, an animal cell or a plant cell, which comprises a recombinant vector into which a DNA encoding an antibody molecule is inserted, in accordance with a general culturing method, to thereby form and accumulate the antibody composition, and then recovering the antibody composition from the culture.

As the method for expressing the gene, secretion production, expression of a fusion protein and the like can be carried out in accordance with the method described in *Molecular Cloning,* Second Edition, in addition to the direct expression.

The method for producing an antibody composition includes a method of intracellular expression in a host cell, a method of extracellular secretion from a host cell, and a method of production on a host cell membrane outer envelope. The method can be selected by changing the host cell used or the structure of the antibody composition produced.

When the antibody composition of the present invention is produced in a host cell or on a host cell membrane outer envelope, it can be positively secreted extracellularly in accordance with the method of Paulson *et al.* [*J. Biol. Chem.,* 264, 17619 (1989)], the method of Lowe *et al.* [*Proc. Natl. Acad Sci. USA,* 86, 8227 (1989), *Genes Develop.,* 4, 1288 (1990)], the methods described in Japanese Published Examined Patent Application No. 336963/93 and Japanese Published Examined Patent Application No. 823021/94 and the like.

That is, an antibody molecule of interest can be positively secreted extracellularly from a host cell by inserting a DNA encoding the antibody molecule and a DNA encoding a signal peptide suitable for the expression of the antibody molecule into an expression vector using a recombinant DNA technique, introducing the expression vector into the host cell and then expressing the antibody molecule.

Also, its production amount can be increased in accordance with the method described in Japanese Published Examined Patent Application No. 227075/90 using a gene amplification system using a dihydrofolate reductase gene.

In addition, the antibody composition can also be produced by using a gene-introduced animal individual (transgenic non-human animal) or a plant individual (transgenic plant) which is constructed by the redifferentiation of an animal or plant cell into which the gene is introduced.

When the transformant is an animal individual or a plant individual, an antibody composition can be produced in accordance with a general method by rearing or cultivating it to thereby form and accumulate the antibody composition and then recovering the antibody composition from the animal or plant individual.

The method for producing an antibody composition using an animal individual includes a method in which the antibody composition of interest is produced in an animal constructed by introducing a gene in accordance with a

known method [*American Journal of Clinical Nutrition*, 63, 639S (1996); *American Journal of Clinical Nutrition*, 63, 627S (1996); *Bio/Technology*, 9, 830 (1991)].

In the case of an animal individual, an antibody composition can be produced by rearing a transgenic non-human animal into which a DNA encoding an antibody molecule is introduced to thereby form and accumulate the antibody composition in the animal, and then recovering the antibody composition from the animal. The place of the animal where the composition is produced and accumulated includes milk (Japanese Published Examined Patent Application No. 309192/88) and an egg of the animal. As the promoter used in this case, any promoter can be used, so long as it can function in an animal. Preferred examples include mammary gland cell-specific promoters such as α casein promoter, β casein promoter, β lactoglobulin promoter and whey acidic protein promoter.

The method for producing an antibody composition using a plant individual includes a method in which an antibody composition is produced by cultivating a transgenic plant into which a DNA encoding an antibody molecule is introduced by a known method [*Tissue Culture,* 20 (1994); *Tissue Culture,* 21 (1995); *Trends in Biotechnology*, 15, 45 (1997)] to form and accumulate the antibody composition in the plant, and then recovering the antibody composition from the plant.

Regarding purification of an antibody composition produced by a transformant into which a gene encoding an antibody molecule is introduced, for example, when the antibody composition is intracellularly expressed in a dissolved state, the cells after culturing are recovered by centrifugation, suspended in an aqueous buffer and then disrupted using a sonicator, French press, Manton Gaulin homogenizer, dynomill or the like to obtain a cell-free extract. A purified product of the antibody composition can be obtained from a supernatant obtained by centrifuging the cell-free extract, by using a general enzyme isolation purification techniques such as solvent extraction; salting out with ammonium sulfate, *etc.;* desalting; precipitation with an organic solvent; anion exchange chromatography using a resin such as diethylaminoethyl (DEAE)-Sepharose or DIAION HPA-75 (manufactured by Mitsubishi Chemical); cation exchange chromatography using a resin such as S-Sepharose FF (manufactured by Pharmacia).; hydrophobic chromatography using a resin such as butyl-Sepharose or phenyl-Sepharose; gel filtration using a molecular sieve; affinity chromatography; chromatofocusing; electrophoresis such as isoelectric focusing; and the like which may be used alone or in combination.

Also, when the antibody composition is expressed intracellularly by forming an inclusion body, the cells are recovered, disrupted and centrifuged in the same manner, and the inclusion body of the antibody composition is recovered as a precipitation fraction. The recovered inclusion body of the antibody composition is solubilized by using a protein denaturing agent. The antibody composition is made into a normal three-dimensional structure by diluting or dialyzing the solubilized solution, and then a purified product of the antibody composition is obtained by the same isolation purification method.

When the antibody composition is secreted extracellularly, the antibody composition or derivatives thereof can be recovered from the culture supernatant. That is, the culture is treated by a technique such as centrifugation to obtain a soluble fraction, and a purified preparation of the antibody composition can be obtained from the soluble fraction by the same isolation purification method.

The thus obtained antibody composition includes an antibody, the fragment of the antibody, and a fusion protein comprising the Fc region of the antibody.

As an example for obtaining the antibody composition, a method for producing a humanized antibody composition is described below in detail, but other antibody compositions can also be obtained in a manner similar to the method.

(1) Construction of vector for expression of humanized antibody

A vector for expression of humanized antibody is an expression vector for animal cell into which genes encoding the C regions of heavy chain (H chain) and light chain (L chain) of a human antibody are inserted, which can be constructed by cloning each of genes encoding the C regions of H chain and L chain of a human antibody into an expression vector for animal cell.

The C regions of a human antibody can be the C regions of H chain and L chain of any human antibody. Examples include the C region belonging to IgGl subclass in the H chain of a human antibody (hereinafter referred to as "hCγl"), the C region belonging to κ class in the L chain of a human antibody (hereinafter referred to as "hCκ"), and the like.

As the genes encoding the C regions of H chain and L chain of a human antibody, a genomic DNA comprising an exon and an intron can be used and a cDNA can also be used.

As the expression vector for animal cell, any vector can be used, so long as a gene encoding the C region of a human antibody can be inserted thereinto and expressed therein. Examples include pAGE107 [*Cytotechnology*, 3, 133 (1990)], pAGE103 [*J. Biochem*., 101, 1307 (1987)], pHSG274 [*Gene,* 27 223 (1984)], pKCR [*Proc. Natl. Acad. Sci. USA*, 78, 1527 (1981), pSG1 β d2-4 [*Cytotechnology*, 4, 173 (1990)] and the like. Examples of the promoter and

enhancer in the expression vector for animal cell include SV40 early promoter and enhancer [*J. Biochem.*, 101, 1307 (1987)], Moloney mouse leukemia virus LTR promoter [*Biochem. Biophys. Res. Commun.*, 149, 960 (1987)], immunoglobulin H chain promoter [*Cell*, 41, 479 (1985)] and enhancer [*Cell*, 33, 717 (1983)], and the like.

The vector for expression of humanized antibody can be any type; wherein genes encoding the H chain and L chain of an antibody exist on separate vectors or genes exist on the same vector (hereinafter referred to as "tandem type"). In respect of easiness of construction of a vector for expression of humanized antibody, easiness of introduction into animal cells, and balance between the expression amounts of the H and L chains of an antibody in animal cells, a tandem type of the vector for expression of humanized antibody is preferred [*J. Immunol. Methods,* 167, 271 (1994)]. The tandem type of the vector for expression of humanized antibody includes pKANTEX93 [*Mol. Immunol., 37.* 1035 (2000)], pEE18 [*Hybridoma,* 17, 559 (1998)] and the like.

The constructed vector for expression of humanized antibody can be used for expression of a human chimeric antibody and a human CDR-grafted antibody in animal cells.

(2) Preparation of a cDNA encoding the V region of an antibody derived from a non-human animal

cDNAs encoding the V regions of H chain and L chain of an antibody derived from a non-human animal, such as a mouse antibody, can be obtained in the following manner.

A cDNA is synthesized by extracting mRNA from a hybridoma cell which produces the mouse antibody of interest. The synthesized cDNA is cloned into a vector such as a phage or a plasmid to obtain a cDNA library. Each of a recombinant phage or recombinant plasmid comprising a cDNA encoding the V region of H chain and a recombinant phage or recombinant plasmid comprising a cDNA encoding the V region ofL chain is isolated from the library by using a C region part or a V region part of an existing mouse antibody as the probe. The full nucleotide sequences of the V regions of H chain and L chain of the mouse antibody of interest on the recombinant phage or recombinant plasmid are determined, and the full amino acid sequences of the V regions ofH chain and L chain are deduced from the nucleotide sequences.

As the non-human animal, any animal such as mouse, rat, hamster, rabbit or the like can be used so long as a hybridoma cell can be produced therefrom.

The method for preparing total RNA from a hybridoma cell includes the guanidine thiocyanate-cesium trifluoroacetate method [*Methods in Enzymology*, 154, 3 (1987)] and the like. The method for preparing mRNA from total RNA includes an oligo(dT)-immobilized cellulose column method (*Molecular Cloning: A Laboratory Manual*, Cold Spring Harbor Lab. Press New York, 1989) and the like. In addition, a kit for preparing mRNA from a hybridoma cell includes Fast Track mRNA Isolation Kit (manufactured by Invitrogen), Quick Prep mRNA Purification Kit (manufactured by Pharmacia) and the like.

The method for synthesizing cDNA and preparing a cDNA library includes the usual methods *(Molecular Cloning: A Laboratory Manual,* Cold Spring Harbor Lab. Press New York, 1989, *Current Protocols in Molecular Biology,* Supplement 1-34); methods using a commercially available kit such as SuperScript™ Plasmid System for cDNA Synthesis and Plasmid Cloning (manufactured by GIBCO BRL) and ZAP-cDNA Synthesis Kit (manufactured by Stratagene); and the like.

In preparing the cDNA library, the vector into which a cDNA synthesized using mRNA extracted from a hybridoma cell as the template is inserted can be any vector so long as the cDNA can be inserted. Examples include ZAP Express [*Strategies*, *5*, 58 (1992)], pBluescript II SK(+) [*Nucleic Acids Research*, 17, 9494 (1989)], λzapII (manufactured by Stratagene), λgt10 and λgt11 [*DNA Cloning, A Practical Approach,* I, 49 (1985)], Lambda BlueMid (manufactured by Clontech), λExCell and pT7T3 18U (manufactured by Pharmacia), pcD2 [*Mol. Cell. Biol.*, 3, 280 (1983)], pUC18 [*Gene,* 33, 103 (1985)] and the like.

As *Escherichia coli* into which the cDNA library constructed from a phage or plasmid vector is introduced, any *Escherichia coli* can be used, so long as the cDNA library can be introduced, expressed and maintained. Examples include XL1-Blue MRF' [*Strategies,* 5, 81 (1992)], C600 [*Genetics,* 39, 440 (1954)], Y1088 and Y1090 [*Science,* 222, 778 (1983)], NM522 [*J. Mol. Biol.,* 166, 1 (1983)], K802 [*J. Mol. Biol.,* 16, 118 (1966)], JM105 [*Gene,* 38, 275 (1985)] and the like.

As the method for selecting a cDNA clone encoding the V regions of H chain and L chain of an antibody derived from a non-human animal from the cDNA library, a colony hybridization or a plaque hybridization using an isotope- or fluorescence-labeled probe can be used *(Molecular Cloning,* Second Edition, Cold Spring Harbor Lab. Press New York, 1989). The cDNA encoding the V regions of H chain and L chain can also be prepared by preparing primers and carrying out polymerase chain reaction (hereinafter referred to as "PCR"; *Molecular Cloning,* Second Edition, Cold Spring Harbor Lab. Press New York, 1989; *Current Protocols in Molecular Biology,* Supplement 1-34) using a cDNA synthesized from mRNA or a cDNA library as the template.

The nucleotide sequences of the cDNAs can be determined by digesting the selected cDNAs with appropriate restriction enzymes, cloning the fragments into a plasmid such as pBluescript SK(-) (manufactured by Stratagene),

carrying out the reaction of a generally used nucleotide sequence analyzing method such as the dideoxy method [*Proc. Natl. Acad. Sci. USA,* 74, 5463 (1977)] of Sanger *et al*., and then analyzing the clones using an automatic nucleotide sequence analyzer such as A.L.F. DNA Sequencer (manufactured by Pharmacia).

Whether or not the obtained cDNAs are encoding the full length of amino acid sequences of the V regions of H chain and L chain of the antibody containing a secretory signal sequence can be confirmed by deducing the full amino acid sequences of the V regions of H chain and L chain from the determined nucleotide sequence and comparing them with the full length amino acid sequences of the V regions of H chain and L chain of known antibodies [*Sequences of Proteins of Immunological Interest*, US Dep. Health and Human Services (1991)].

Furthermore, when the amino acid sequence of an antibody variable region or the nucleotide sequence of a DNA encoding the variable region is known, the cDNA can be prepared according to the following method.

When the amino acid sequence is known, the amino acid sequence is converted to a DNA sequence based on frequency of codon usage [*Sequences of Proteins of Immunological Interest*, US Dep. Health and Human Services (1991)], several synthetic DNAs having a length of about 100 bases are synthesized based on the designed DNA sequence, and PCR is carried out by using the DNAs to prepare the cDNA. When the nucleotide sequence is known, several synthetic DNAs having a length of about 100 bases are synthesized based on the designed DNA sequence, and PCR is carried out by using the DNAs to prepare the cDNA.

(3) Analysis of the amino acid sequence of the V region of an antibody derived from a non-human animal

Regarding the full length of the amino acid sequences of the V regions of H chain and L chain of an antibody comprising a secretory signal sequence, the length of the secretory signal sequence and the *N*-terminal amino acid sequences can be deduced and subgroups to which they belong can also be found, by comparing them with the full length of the amino acid sequences of the V regions of H chain and L chain of known antibodies [*Sequences of Proteins of Immunological Interest,* US Dep. Health and Human Services, (1991)]. In addition, the amino acid sequences of each CDR of the V regions of H chain and L chain can also be found by comparing them with the amino acid sequences of the V regions ofH chain and L chain of known antibodies [*Sequences of Proteins of Immunological Interest,* US Dep. Health and Human Services, (1991)].

(4) Construction of vector for expression of human chimeric antibody

A vector for expression of human chimeric antibody can be constructed by cloning cDNAs encoding the V regions of H chain and L chain of an antibody derived from a non-human animal into upstream of genes encoding the C regions of H chain and L chain of a human antibody in the vector for humanized antibody expression described in item 3(1). For example, a vector for expression of human chimeric antibody can be constructed by linking each of cDNAs encoding the V regions of H chain and L chain of an antibody derived from a non-human animal to a synthetic DNA comprising nucleotide sequences at the 3'-terminals of the V regions of H chain and L chain of an antibody derived from a non-human animal and nucleotide sequences at the 5'-terminals of the C regions of H chain and L chain of a human antibody and also having a recognition sequence of an appropriate restriction enzyme at both terminals, and by cloning them into upstream of genes encoding the C regions ofH chain and L chain of a human antibody contained in the vector for humanized antibody expression constructed as described in item 3(1) in the form suitable for expression.

(5) Construction of cDNA encoding the V region of a human CDR-grafted antibody

cDNAs encoding the V regions of H chain and L chain of a human CDR-grafted antibody can be obtained as follows. First, amino acid sequences of the frameworks (hereinafter referred to as "FR") of the V regions of H chain and L chain of a human antibody for grafting CDR of the V regions of H chain and L chain of an antibody derived from a non-human animal is selected. As the amino acid sequences of FRs of the V regions of H chain and L chain of a human antibody, any amino acid sequences can be used so long as they are derived from a human antibody. Examples include amino acid sequences of FRs of the V regions ofH chain and L chain of human antibodies registered at databases such as Protein Data Bank; amino acid sequences common in each subgroup of FRs of the V regions of H chain and L chain of human antibodies [*Sequences of Proteins of Immunological Interest,* US Dep. Health and Human Services (1991)]; and the like. In order to produce a human CDR-grafted antibody having potent activity, it is preferable to select an amino acid sequence having a homology as high as possible (at least 60% or more) with amino acid sequences of the V regions of H chain and L chain of an antibody of interest derived from a non-human animal.

Next, the amino acid sequences of CDRs of the V regions of H chain and L chain of the antibody of interest derived from a non-human animal are grafted to the selected amino acid sequences of FRs of the V regions of H chain and L chain of a human antibody to design amino acid sequences of the V regions of H chain and L chain of the human

CDR-grafted antibody. The designed amino acid sequences are converted into DNA sequences by considering the frequency of codon usage found in nucleotide sequences of antibody genes [*Sequences of Proteins of Immunological Interest,* US Dep. Health and Human Services (1991)], and the DNA sequences encoding the amino acid sequences of the V regions of H chain and L chain of the human CDR-grafted antibody are designed. Based on the designed DNA sequences, several synthetic DNAs having a length of about 100 bases are synthesized, and PCR is carried out by using them. In this case, it is preferable in each of the H chain and the L chain that 4 to 6 synthetic DNAs are designed in view of the reaction efficiency of PCR and the lengths of DNAs which can be synthesized.

Also, they can be easily cloned into the vector for humanized antibody expression constructed in item 3(1) by introducing recognition sequences of an appropriate restriction enzyme into the 5'-terminals of the synthetic DNA present on both terminals. After the PCR, the amplified product is cloned into a plasmid such as pBluescript SK(-) (manufactured by Stratagene) or the like, and the nucleotide sequences are determined by the method in item 3(2) to thereby obtain a plasmid having DNA sequences encoding the amino acid sequences of the V regions of H chain and L chain of the desired human CDR-grafted antibody.

(6) Construction of vector for human CDR-grafted antibody expression

A vector for human CDR-grafted antibody expression can be constructed by cloning the cDNAs encoding the V regions of H chain and L chain of the human CDR-grafted antibody constructed in item 3(5) into upstream of the gene encoding C regions of H chain and L chain of a human antibody in the vector for humanized antibody expression described in item 3(1). For example, the vector for human CDR-grafted antibody expression can be constructed by introducing recognizing sequences of an appropriate restriction enzyme into the 5'-terminals of both terminals of a synthetic DNA fragment, among the synthetic DNA fragments which are used when PCR is carried out in item 3(5) for constructing the V regions of H chain and L chain of the human CDR-grafted antibody, so that they are cloned into upstream of the genes encoding the C regions of H chain and L chain of a human antibody in the vector for humanized antibody expression described in item 3(1) in such a manner that they can be expressed in a suitable form.

(7) Stable production of a humanized antibody

A transformant capable of stably producing a human chimeric antibody and a human CDR-grafted antibody (both hereinafter referred to as "humanized antibody") can be obtained by introducing the vector for expression of humanized antibody described in items 3(4) or (6) into an appropriate animal cell.

The method for introducing a vector for expression of humanized antibody into an animal cell includes electroporation [Japanese Published Examined Patent Application No. 257891/90, *Cytotechnology,* 3, 133 (1990)] and the like.

As the animal cell into which a vector for expression of humanized antibody is introduced, any cell can be used so long as it is an animal cell which can produce the humanized antibody.

Examples include mouse myeloma cells such as NS0 cell and SP2/0 cell; Chinese hamster ovary cells such as *CHO*/*dhfr*$^-$ cell and CHO/DG44 cell; rat myeloma such as YB2/0 cell and IR983F cell; BHK cell derived from a syrian hamster kidney; a human myeloma cell such as Namalwa cell; and the like. A Chinese hamster ovary cell CHO/DG44 cell and a rat myeloma YB2/0 cell are preferred.

After introduction of the vector for expression of humanized antibody, a transformant capable of stably producing the humanized antibody can be selected, in accordance with the method disclosed in Japanese Published Examined Patent Application No. 257891/90 using a medium for animal cell culture comprising an agent such as G418 sulfate (hereinafter referred to as "G418"; manufactured by SIGMA). As the medium for animal cell culture, RPMI 1640 medium (manufactured by Nissui Pharmaceutical), GIT medium (manufactured by Nihon Pharmaceutical), EX-CELL 302 medium (manufactured by JRH), IMDM medium (manufactured by GIBCO BRL), Hybridoma-SFM medium (manufactured by GIBCO BRL), media obtained by adding various additives such as fetal bovine serum (hereinafter referred to as "FCS") to these media, and the like can be mentioned. The humanized antibody can be produced and accumulated in the culture medium by culturing the obtained transformant in a medium. The production and antigen binding activity of the humanized antibody in the culture medium can be measured by a method such as enzyme-linked immunosorbent assay [hereinafter referred to as "ELISA"; *Antibodies: A Laboratory Manual*, Cold Spring Harbor Laboratory, Chapter 14 (1998), *Monoclonal Antibodies: Principles and Practice*, Academic Press Limited (1996)] or the like. Also, the production of the humanized antibody by the transformant can be increased by using a DHFR gene amplification system in accordance with the method disclosed in Japanese Published Examined Patent Application No. 257891/90.

The humanized antibody can be purified from a culture supernatant of the transformant by using a protein A column [*Antibodies*: *A Laboratory Manual,* Cold Spring Harbor Laboratory, Chapter 8 (1988), *Monoclonal Antibodies: Principles and Practice,* Academic Press Limited (1996)]. In addition, purification methods generally used for the purification of proteins can also be used. For example, the purification can be carried out through the combination of gel

filtration, ion exchange chromatography and ultrafiltration. The molecular weight of the H chain, L chain and antibody molecule as a whole of the purified humanized antibody, respectively, can be measured, e.g., by polyacrylamide gel electrophoresis [hereinafter referred to as "SDS-PAGE"; *Nature*, 227, 680 (1970)], Western blotting [*Antibodies: A Laboratory Manual*, Cold Spring Harbor Laboratory, Chapter 12 (1988), *Monoclonal Antibodies: Principles and Practice*, Academic Press Limited (1996)] or the like.

Thus, methods for producing an antibody composition using an animal cell as the host have been described, but, as described above, the antibody composition can also be produced by a yeast, an insect cell, a plant cell, an animal individual or a plant individual by the same methods as the animal cell.

When a host cell has the ability to express an antibody molecule, the antibody composition of the present invention can be produced by preparing a cell expressing an antibody molecule by using the method described in item 1, culturing the cell and then purifying the antibody composition of interest from the resulting culture.

4. Activity evaluation of the antibody composition

As the method for measuring the amount of the protein of the purified antibody composition, the activity to bind to an antigen and the effector function of the purified antibody composition, the known methods described in *Monoclonal Antibodies, Anlibody Engineering* and the like can be used.

For example, when the antibody composition is a humanized antibody, the binding activity with an antigen and the binding activity with an antigen-positive cultured cell line can be measured by methods such as ELISA and an immunofluorescent method [*Cancer-Immunol. Immunother.,* 36, 373 (1993)]. The cytotoxic activity against an antigen-positive cultured cell line can be evaluated by measuring CDC activity, ADCC activity [*Cancer Immunol. Immunother.*, 36, 373 (1993)] and the like.

Also, safety and therapeutic effect of the antibody composition in human can be evaluated by using an appropriate model of animal species relatively close to human, such as *Macaca fascicularis.*

5. Analysis of sugar chains in the antibody composition

The sugar chain structure of the antibody molecule expressed in various cells can be analyzed in accordance with the general analysis of the sugar chain structure of a glycoprotein. For example, the sugar chain which is bound to IgG molecule comprises a neutral sugar such as galactose, mannose or fucose, an amino sugar such as *N*-acetylglucosamine, and an acidic sugar such as sialic acid, and can be analyzed by a method such as a sugar chain structure analysis using sugar composition analysis, two dimensional sugar chain mapping or the like.

(1) Analysis of neutral sugar and amino sugar compositions

The sugar chain of the antibody composition can be analyzed by carrying out acid hydrolysis of sugar chains with an acid such as trifluoroacetic acid to release a neutral sugar or an amino sugar and measuring the composition ratio.

Examples include a method using a sugar composition analyzer (BioLC) manufactured by Dionex. The BioLC is an apparatus which analyzes a sugar composition by HPAEC-PAD (high performance anion-exchange chromatography-pulsed amperometric detection) [*J. Liq. Chromatogr*., 6, 1577 (1983)].

The composition ratio can also be analyzed by a fluorescence labeling method using 2-aminopyridine. Specifically, the compositional ratio can be calculated in accordance with a known method [*Agric*. *Biol*. *Chem*., 55(1), 283-284 (1991)], by labeling an acid-hydrolyzed sample with a fluorescence with 2-aminopyridylation and then analyzing the composition by HPLC.

(2) Analysis of sugar chain structure

The sugar chain structure of the antibody molecule can be analyzed by the two dimensional sugar chain mapping method [*Anal. Biochem.,* 171, 73 (1988), *Biochemical Experimentation Methods 23 - Methods for Studying Glycoprotein Sugar Chains* (Japan Scientific Societies Press) edited by Reiko Takahashi (1989)]. The two dimensional sugar chain mapping method is a method for deducing a sugar chain structure by, e.g., plotting the retention time or elution position of a sugar chain by reverse phase chromatography as the X axis and the retention time or elution position of the sugar chain by normal phase chromatography as the Y axis, respectively, and comparing them with such results of known sugar chains.

Specifically, sugar chains are released from an antibody by subjecting the antibody to hydrazinolysis, and the released sugar chain is subjected to fluorescence labeling with 2-aminopyridine (hereinafter referred to as "PA") [*J. Biochem.,* 95, 197 (1984)], and then the sugar chains are separated from an excess PA-treating reagent by gel filtration,

and subjected to reverse phase chromatography. Thereafter, each peak of the separated sugar chains are subjected to normal phase chromatography. The sugar chain structure can be deduced by plotting the results on a two dimensional sugar chain map and comparing them with the spots of a sugar chain standard (manufactured by Takara Shuzo) or a literature [*Anal. Biochem.,* 171, 73 (1988)].

The structure deduced by the two dimensional sugar chain mapping method can be confirmed by further carrying out mass spectrometry such as MALDI-TOF-MS of each sugar chain.

6. Immunological determination method for discriminating the sugar chain structure of an antibody molecule

An antibody composition comprises various antibody molecules in which sugar chains binding to the Fc region of the antibody are different in structure. In the antibody composition of the present invention, the ratio of a sugar chain in which fucose is not bound to *N*-acetylglucosamine in the reducing end in the sugar chain is 20% or more among the total complex *N*-glycoside-linked sugar chains binding to the Fc region in the antibody composition, and the antibody composition has potent ADCC activity. The antibody composition can be identified by using the method for analyzing the sugar chain structure of an antibody molecule described in item 5. Also, it can be identified by an immunological determination method using a lectin.

The sugar chain structure of an antibody molecule can be identified by the immunological determination method using a lectin in accordance with the known immunological determination method such as Western staining, IRA (radioimmunoassay), VIA (viroimmunoassay), EIA (enzymoimmunoassay), FIA (fluoroimmunoassay) and MIA (metalloimmunoassay) described in *Monoclonal Antibodies: Principles and Applications,* Wiley-Liss, Inc. (1995); *Immunoassay,* 3rd Ed., Igakushoin (1987); *Enzyme Antibody Method,* Revised Edition, Gakusai Kikaku (1985); and the like.

A lectin which recognizes the sugar chain structure of an antibody molecule comprised in an antibody composition is labeled, and the labeled lectin is allowed to react with an antibody composition as a sample. Then, the amount of the complex of the labeled lectin with the antibody molecule is measured.

The lectin for identifying the sugar chain structure of an antibody molecule includes WGA (wheat-germ agglutinin derived from *T. vulgaris),* ConA (cocanavalin A derived from *C. ensiformis),* RIC (a toxin derived from *R. communis),* L-PHA (leucoagglutinin derived from *P. vulgaris),* LCA (lentil agglutinin derived from *L. culinaris),* PSA (pea lectin derived from *P. sativum),* AAL *(Aleuria aurantia* lectin), ACL (*Amaranthus caudatus* lectin), BPL *(Bauhinia purpurea* lectin), DSL *(Datura stramonium* lectin), DBA *(Dolichos biflorus* agglutinin), EBL (elderberry balk lectin), ECL *(Erythrina cristagalli* lectin), EEL *(Euonymus eoropaeus* lectin), GNL *(Galanthus nivalis* lectin), GSL *(Griffonia simplicifolia* lectin), HPA *(Helix pomatia* agglutinin), HHL *(Hippeastrum* hybrid lectin), Jacalin, LTL *(Lotus tetragonolobus* lectin), LEL *(Lycopersicon esculentum* lectin), MAL *(Maackia amurensis* lectin), MPL *(Maclura pomifera* lectin), NPL *(Narcissus pseudonarcissus* lectin), PNA (peanut agglutinin), E-PHA *(Phaseolus vulgaris* erythroagglutinin), PTL *(Psophocarpus tetragonolobus* lectin), RCA *(Ricinus communis* agglutinin), STL *(Solanum tuberosum* lectin), SJA *(Sophora japonica* agglutinin), SBA (soybean agglutinin), UEA *(Ulex europaeus* agglutinin), VVL *(Vicia villosa* lectin) and WFA *(Wisteria floribunda* agglutinin).

It is preferable to use a lectin which specifically recognizes a sugar chain structure wherein fucose binds to the *N*-acetylglucosamine in the reducing end in the complex *N*-glycoside-linked sugar chain. Examples include *Lens culinaris* lectin LCA (lentil agglutinin derived from *Lens culinaris*), pea lectin PSA (pea lectin derived from *Pisum sativum*), broad bean lectin VFA (agglutinin derived from *Vicia faba*) and *Aleuria aurantia* lectin AAL (lectin derived from *Aleuria aurantia*).

7. Application of the antibody molecule of the present invention

Since the antibody composition of the present invention specifically binds to CD20 and has potent antibody-dependent cell-mediated cytotoxic activity, it is useful for preventing and treating various diseases relating to CD20-expressing cells such as cancers.

In the case of cancers, namely malignant tumors, cancer cells grow, and, for example, particular B cells abnormally grow in B cell lymphoma. General anti-tumor agents inhibit the growth of cancer cells. In contrast, an antibody having potent antibody-dependent cell-mediated cytotoxic activity can cure cancers by injuring cancer cells through its cell killing effect, and therefore, it is more effective as a therapeutic agent which express the antingen than the general anti-tumor agents. Particularly, in the therapeutic agent for cancers, an anti-tumor effect of an antibody medicament alone is insufficient at the present so that combination therapy with chemotherapy has been carried out [*Science,* 280, 1197 (1998)]. If more potent anti-tumor effect is found by the antibody composition of the present invention alone, the dependency on chemotherapy will be decreased and side effects will be reduced.

The antibody composition of the present invention can be administered as a therapeutic agent alone. Generally, it is preferable to mix the antibody composition with at least one pharmaceutical acceptable carrier and provide it as a pharmaceutical formulation produced by an appropriate method well known in the technical field of manufacturing

pharmacy.

It is preferable to select a route of administration which is the most effective in treatment. Examples include oral administration and parenteral administration such as buccal, tracheal, rectal, subcutaneous, intramuscular and intravenous. In an antibody preparation, intravenous administration is preferable.

The dosage form includes sprays, capsules, tablets, granules, syrups, emulsions, suppositories, injections, ointments, tapes and the like.

Examples of the pharmaceutical preparation suitable for oral administration include emulsions, syrups, capsules, tablets, powders, granules and the like.

Liquid preparations, such as emulsions and syrups, can be produced using, as additives, water; saccharides such as sucrose, sorbitol and fructose; glycols such as polyethylene glycol and propylene glycol; oils such as sesame oil, olive oil and soybean oil; antiseptics such as p-hydroxybenzoic acid esters; flavors such as strawberry flavor and peppermint; and the like.

Capsules, tablets, powders, granules and the like can be produced using, as additive, excipients such as lactose, glucose, sucrose and mannitol; disintegrating agents such as starch and sodium arginate; lubricants such as magnesium stearate and talc; binders such as polyvinyl alcohol, hydroxypropylcellulose and gelatin; surfactants such as fatty acid ester; plasticizers such as glycerine; and the like.

The pharmaceutical preparation suitable for parenteral administration includes injections, suppositories, sprays and the like.

Injections may be prepared using a carrier such as a salt solution, a glucose solution or a mixture of both thereof, or the like. Also, powdered injections can be prepared by freeze-drying the antibody composition in the usual way and adding sodium chloride thereto.

Suppositories may be prepared using a carrier such as cacao butter, hydrogenated fat or carboxylic acid.

Also, sprays may be prepared using the antibody composition as such or using a carrier which does not stimulate the buccal or airway mucous membrane of the patient and can facilitate absorption of the antibody composition by dispersing it as fine particles.

The carrier includes lactose, glycerine and the like. Depending on the properties of the antibody composition and the carrier, it is possible to produce pharmaceutical preparations such as aerosols and dry powders. In addition, the components exemplified as additives for oral preparations can also be added to the parenteral preparations.

Although the clinical dose or the frequency of administration varies depending on the objective therapeutic effect, administration method, treating period, age, body weight and the like, it is usually 10 μg/kg to 20 mg/kg per day and per adult.

Also, as the method for examining antitumor effect of the antibody composition against various tumor cells, *in vitro* tests include CDC activity measuring method, ADCC activity measuring method, and the like; and *in vivo* tests include antitumor experiments using a tumor system in an experimental animal such as a mouse, and the like.

CDC activity and ADCC activity measurements and antitumor experiments can be carried out in accordance with the methods described in *Cancer Immunology Immunotherapy*, 36, 373 (1993); *Cancer Research,* 54, 1511 (1994) and the like.

The present invention will be described below in detail based on Examples; however, Examples are only simple illustrations of the present invention, and the scope of the present invention is not limited thereto.

## BRIEF DESCRIPTION OF THE DRAWINGS

Fig. 1 shows a construction step of plasmid pBS-2B8L.

Fig. 2 shows a construction step of plasmid pBS-2B8Hm.

Fig. 3 shows a construction step of plasmid pKANTEX2B8P.

Fig. 4 shows a result of measurement of the activity of purified anti-CD20 chimeric antibody KM3065 and Rituxan™ to bind to a human CD20-expressing cell, Raji cell while changing the concentration of the antibodies by using the immunofluorescent method. The ordinate and the abscissa show the relative fluorescence intensity at each concentration and the antibody concentration, respectively. "■" and "○" show the activities of Rituxan™ and KM3065, respectively.

Fig. 5 shows a result of measurement of the activity of purified anti-CD20 chimeric antibody KM3065 and Rituxan™ to bind to a human CD20-negative cell, CCRF-CEM cell, using the immunofluorescent method.

Fig. 6 shows ADCC activity of purified anti-CD20 chimeric antibody KM3065 and Rituxan™ to a human CD20-expressing cell. In Figs. 6A, 6B and 6C, Raji cell, Ramos cell and WIL2-S were used as the target cell. The ordinate and the abscissa show the cytotoxic activity and the antibody concentration. "■" and "○" show the activities of Rituxan™ and KM3065, respectively.

Fig. 7 shows elution patterns obtained by preparing PA-modified sugar chains from purified anti-CD20 chimeric antibody KM3065 and Rituxan™ and analyzing them by reverse phase HPLC. The ordinate and the abscissa show the relative fluorescence intensity and the elution time, respectively.

Fig. 8 shows construction of a plasmid CHfFUT8-pCR2.1.

Fig. 9 shows construction of a plasmid ploxPPuro.

Fig. 10 shows construction of a plasmid pKOFUT8gE2-1.

Fig. 11 shows construction of a plasmid pKOFUT8gE2-2.

Fig. 12 shows construction of a plasmid pscFUT8gE2-3.

Fig. 13 shows construction of a plasmid pKOFUT8gE2-3.

Fig. 14 shows construction of a plasmid pKOFUT8gE2-4.

Fig. 15 shows construction of a plasmid pKOFUT8gE2-5.

Fig. 16 shows construction of a plasmid pKOFUT8Puro.

Fig. 17 shows a result of measurement of the binding activity of an anti-CD20 chimeric antibody R92-3-1 produced by lectin-resistant CHO/DG44 cell while changing the concentration of the antibody using the immunofluorescent method. The ordinate and the abscissa show the relative fluorescence intensity at each concentration and the antibody concentration, respectively. "■" and "○" show the activities of Rituxan™ and R92-3-1, respectively.

Fig: 18 shows a result of the evaluation of ADCC activity of the anti-CD20 chimeric antibody R92-3-1 produced by lectin-resistant CHO/DG44 cell, using Raji cell as the target cell. The ordinate and the abscissa show the cytotoxic activity on the target cell and the antibody concentration, respectively. "■" and "○" show the activities of Rituxan™ and R92-3-1, respectively.

Fig. 19 shows an elution pattern obtained by reverse phase HPLC analysis of a PA-modified sugar chain prepared from the anti-CD20 chimeric antibody R92-3-1 produced by lectin-resistant CHO/DG44 cell. The ordinate and the abscissa show the relative fluorescence intensity and the elution time, respectively. Analytical conditions of the reverse phase HPLC, identification of the sugar chain structure and calculation of the ratio of sugar chains to which $\alpha$1,6-fucose was not bound were carried out in the same manner as in Example 3.

Fig. 20 shows a construction step of a plasmid CHO-GMD prepared by introducing 5'-terminal of a clone 34-2 into 5'-terminal of a CHO cell-derived GMD cDNA clone 22-8.

Fig. 21 shows elution patterns obtained by reverse phase HPLC analysis of PA-modified sugar chains prepared from three anti-CD20 chimeric antibodies. The ordinate and abscissa show the relative fluorescence intensity and the elution time, respectively. Analytical conditions of the reverse phase HPLC, identification of the sugar chain structure and calculation of the ratio of sugar chains to which $\alpha$1,6-fucose was not bound were carried out in the same manner as in Example 3.

Fig. 22 shows a result of the measurement of the CD20-expressing cell-binding activity against five anti-CD20 chimeric antibodies having a different ratio of antibody molecules to which an $\alpha$1,6-fucose-free sugar chain bound while changing the concentration of the antibodies using the immunofluorescent method. The ordinate and the abscissa show the binding activity to CD20 and the antibody concentration, respectively. "□", "■", "Δ", "▲" and "○" show the activities of an anti-CD20 chimeric antibody (96%), an anti-CD20 chimeric antibody (44%), an anti-CD20 chimeric antibody (35%), an anti-CD20 chimeric antibody (26%) and an anti-CD20 chimeric antibody (6%), respectively.

Fig. 23 shows a result of the measurement of ADCC activity of anti-CD20 chimeric antibodies having a different ratio of antibody molecules to which an $\alpha$1,6-fucose-free sugar chain is bound against WIL2-S cell. It shows a result measured by the $^{51}$Cr method using effector cells of donor A. The ordinate and the abscissa show the cytotoxic activity and the antibody concentration, respectively. "□", "■", "Δ", "▲" and "○" show the activities of an anti-CD20 chimeric antibody (96%), an anti-CD20 chimeric antibody (44%), an anti-CD20 chimeric antibody (35%), an anti-CD20 chimeric antibody (26%) and an anti-CD20 chimeric antibody (6%), respectively.

Fig. 24 shows a result of the measurement of ADCC activity of anti-CD20 chimeric antibodies having a different ratio of antibody molecules to which a $\alpha$1,6-fucose-free sugar chain is bound against Raji cell. It shows a result measured by the LDH method using effector cells of donor B. The ordinate and the abscissa show the cytotoxic activity and the antibody concentration. "□", "■", "Δ", "▲" and "○" show the activities of an anti-CD20 chimeric antibody (96%), an anti-CD20 chimeric antibody (44%), an anti-CD20 chimeric antibody (35%), an anti-CD20 chimeric antibody (26%) and an anti-CD20 chimeric antibody (6%), respectively.

Fig. 25 shows an elution pattern obtained by separating anti-CD20 chimeric antibody KM3065 using a column immobilized with lectin having affinity for sugar chains containing bisecting GlcNAc. The ordinate and the abscissa show absorbance at 280 nm and the elution time, respectively.① to④ show elution positions of fractions① to④ .

Fig. 26 shows elution patterns of fractions① to④ separated using a column immobilized with lectin having affinity for sugar chains containing bisecting GlcNAc and the PA-modified sugar chains prepared from anti-CD20 chimeric antibody KM3065 before the separation, each obtained by reverse phase HPLC analysis. The upper and left drawing, the upper and right drawing, the middle and left drawing, the middle and right drawing and the lower and left

drawing show the elution patterns of KM3065 before the separation, fraction ①, fraction ②, fraction ③ and fraction ④, respectively. The ordinate and the abscissa show the relative fluorescence intensity and the elution time, respectively. In the drawing, the peak painted out in black shows antibody-derived PA-modified sugar chains, and " *" shows PA-modified sugar chains having bisecting GlcNAc.

Fig. 27 shows ADCC activity of fractions ① to ④ separated using a column immobilized with lectin having affinity for sugar chains containing bisecting GlcNAc and the anti-CD20 chimeric antibody KM3065 before the separation, against Raji cell. It shows a result of the measurement by the LDH method using effector cells derived from a healthy donor. The ordinate and the abscissa show the cytotoxicity and the antibody concentration, respectively. "●", "○", "Δ", "◊", "♦", "□" and × show the activities of KM3065 before separation, fraction ①, fraction ②, fraction ③ and fraction ④, Rituxan™ and no antibody-added case.

EMBODIMENT FOR CARRYING OUT THE INVENTION

Example 1

Preparation of an anti-CD20 human chimeric antibody:

1. Preparation of anti-CD20 vector for expression of human chimeric antibody

(1) Construction of a cDNA encoding the V region of L chain of an anti-CD20 mouse monoclonal antibody

A cDNA (represented by SEQ ID NO:11) encoding the amino acid sequence of the V region of L chain (hereinafter referred to as "VL") of an anti-CD20 mouse monoclonal antibody 2B8 described in WO 94/11026 was constructed using PCR as follows.

First, binding nucleotide sequences of primers for amplification at the time of the PCR and restriction enzyme recognizing sequences for cloning into a vector for humanized antibody expression were added to the 5'-terminal and 3'-terminal of the nucleotide sequence of the VL described in WO 94/11026. A designed nucleotide sequence was divided from the 5'-terminal side into a total of 6 nucleotide sequences each having about 100 bases (adjacent nucleotide sequences are designed such that their termini have an overlapping sequence of about 20 bases), and 6 synthetic DNA fragments, actually those represented by SEQ ID NOs:15, 16, 17, 18, 19 and 20, were prepared from them in alternate order of a sense chain and an antisense chain (consigned to GENSET).

Each oligonucleotide was added to 50 μl of a reaction mixture [KOD DNA polymerase-attached PCR Buffer #1 (manufactured by TOYOBO), 0.2 mM dNTPs, 1 mM magnesium chloride, 0.5 μM M13 primer M4 (manufactured by Takara Shuzo) and 0.5 μM M13 primer RV (manufactured by Takara Shuzo)] to give a final concentration of 0.1 μM, and using a DNA thermal cycler GeneAmp PCR System 9600 (manufactured by Perkin Elmer), the reaction was carried out by heating at 94°C for 3 minutes, adding 2.5 units of KOD DNA Polymerase (manufactured by TOYOBO) thereto, subsequent 25 cycles of heating at 94°C for 30 seconds, 55°C for 30 seconds and 74°C for 1 minute as one cycle and then further heating at 72°C for 10 minutes. After 25 μl of the reaction mixture was subjected to agarose gel electrophoresis, a VL PCR product of about 0.44 kb was recovered by using QIAquick Gel Extraction Kit (manufactured by QIAGEN).

Next, 0.1 μg of a DNA fragment obtained by digesting a plasmid pBluescript II SK(-) (manufactured by Stratagene) with a restriction enzyme *Sma*I (manufactured by Takara Shuzo) and about 0.1 μg of the PCR product obtained in the above were added to sterile water to adjust the total volume to 7.5 μl, and then 7.5 μl of solution I of TAKARA ligation kit ver. 2 (manufactured by Takara Shuzo) and 0.3 μl of the restriction enzyme *Sma*I (manufactured by Takara Shuzo) were added thereto to carry out the reaction at 22°C for 2 hours. Using the recombinant plasmid DNA solution obtained in this manner, *E. coli* DH5α (manufactured by TOYOBO) was transformed. Each plasmid DNA was prepared from the transformant clones and allowed to react using BigDye Terminator Cycle Sequencing Ready Reaction Kit v2.0 (manufactured by Applied Biosystems) in accordance with the instructions attached thereto, and then the nucleotide sequence was analyzed by a DNA sequencer ABI PRISM 377 manufactured by the same company. In this manner, the plasmid pBS-2B8L shown in Fig. 1 having the objective nucleotide sequence was obtained.

(2) Construction of a cDNA encoding the V region of H chain of an anti-CD20 mouse monoclonal antibody

A cDNA (represented by SEQ ID NO:13) encoding the amino acid sequence of the V region of H chain (hereinafter referred to as "VH") of the anti-CD20 mouse monoclonal antibody 2B8 described in WO 94/11026 was constructed using PCR as follows.

First, binding nucleotide sequences of primers for amplification at the time of the PCR and a restriction enzyme recognizing sequence for cloning into a vector for humanized antibody expression were added to the 5'-terminal and

3'-terminal of the nucleotide sequence of the VH described in WO 94/11026. A designed nucleotide sequence was divided from the 5'-terminal side into a total of 6 nucleotide sequences each having about 100 bases (adjacent nucleotide sequences are designed such that their termini have an overlapping sequence of about 20 bases), and 6 synthetic DNA fragments, actually those represented by SEQ ID NOs:25, 26, 27, 28, 29 and 30, were prepared from them in alternate order of a sense chain and an antisense chain (consigned to GENSET).

Each oligonucleotide was added to 50 μl of a reaction mixture [KOD DNA polymerase-PCR Buffer #1 (manufactured by TOYOBO), 0.2 mM dNTPs, 1 mM magnesium chloride, 0.5 μM M13 primer M4 (manufactured by Takara Shuzo) and 0.5 μM M13 primer RV (manufactured by Takara Shuzo)] to give a final concentration of 0.1 μM, and using a DNA thermal cycler GeneAmp PCR System 9600 (manufactured by Perkin Elmer), the reaction was carried out by heating at 94°C for 3 minutes, adding 2.5 units of KOD DNA Polymerase (manufactured by TOYOBO), subsequent 25 cycles of heating at 94°C for 30 seconds, 55°C for 30 seconds and 74°C for 1 minute as one cycle and then heating at 72°C for 10 minutes. After 25 μl of the reaction mixture was subjected to agarose gel electrophoresis, a VH PCR product of about 0.49 kb was recovered by using QIAquick Gel Extraction Kit (manufactured by QIAGEN).

Next, 0.1 μ g of a DNA fragment obtained by digesting the plasmid pBluescript II SK(-) (manufactured by Stratagene) with the restriction enzyme *Sma*I (manufactured by Takara Shuzo) and about 0.1 μg of the PCR product obtained in the above were added to sterile water to adjust the total volume to 7.5 μl, and then 7.5 μl of solution I of TAKARA ligation kit ver. 2 (manufactured by Takara Shuzo) and 0.3 μl of the restriction enzyme *Sma*I (manufactured by Takara Shuzo) were added thereto to carry out the reaction at 22°C overnight.

Using the recombinant plasmid DNA solution obtained in this manner, *E. coli* DH5α (manufactured by TOYOBO) was transformed. Each plasmid DNA was prepared from the transformant clones and allowed to react using BigDye Terminator Cycle Sequencing Ready Reaction Kit v2.0 (manufactured by Applied Biosystems) in accordance with the manufacture's instructions attached thereto, and then the nucleotide sequence was analyzed by the DNA sequencer ABI PRISM 377 manufactured by the same company. In this manner, the plasmid pBS-2B8H shown in Fig. 2 comprising the objective nucleotide sequence was obtained.

Next, in order to substitute the amino acid residue at position 14 from Ala to Pro, the synthetic DNA shown in SEQ ID NO:31 was designed, and base substitution was carried out by PCR using LA PCR *in vitro* Mutagenesis Primer Set for pBluescript II (manufactured by Takara Shuzo) as follows. After 50 μl of a reaction mixture [LA PCR Buffer II (manufactured by Takara Shuzo), 2.5 units of TaKaRa LA Taq, 0.4 mM dNTPs, 2.5 mM magnesium chloride, 50 nM T3 BcaBEST Sequencing primer (manufactured by Takara Shuzo) and 50 nM of the primer for mutagenesis (SEQ ID NO:31, manufactured by GENSET)] containing 1 ng of the plasmid pBS-2B8H was prepared, the PCR was carried out by using a DNA thermal cycler GeneAmp PCR System 9600 (manufactured by Perkin Elmer) by 25 cycles of heating at 94°C for 30 seconds, 55°C for 2 minutes and 72°C for 1.5 minutes as one cycle. After 30 μl of the reaction mixture was subjected to agarose gel electrophoresis, a PCR product of about 0.44 kb was recovered by using QIAquick Gel Extraction Kit (manufactured by QIAGEN) and made into 30 μl of an aqueous mixture. In the same manner, PCR was carried out by using 50 μl of a reaction mixture [LA PCR Buffer II (manufactured by Takara Shuzo), 2.5 units of TaKaRa LA Taq, 0.4 mM dNTPs, 2.5 mM magnesium chloride, 50 nM T7 BcaBEST Sequencing primer (manufactured by Takara Shuzo) and 50 nM MUT B1 primer (manufactured by Takara Shuzo)] containing 1 ng of the plasmid pBS-2B8H. After 30 μl of the reaction mixture was subjected to agarose gel electrophoresis, a PCR product of about 0.63 kb was recovered by using QIAquick Gel Extraction Kit (manufactured by QIAGEN) and made into 30 μl of aqueous solution. Next, 0.5 μl of each of 0.44 kb PCR product and 0.63 kb PCR product thus obtained were added to 47.5 μl of a reaction mixture [LA PCR Buffer II (manufactured by Takara Shuzo), 0.4 mM dNTPs, and 2.5 mM magnesium chloride], and using a DNA thermal cycler GeneAmp PCR System 9600 (manufactured by Perkin Elmer), annealing of the DNA was carried out by heating the reaction mixture at 90°C for 10 minutes, cooling it to 37°C over 60 minutes and then keeping it at 37°C for 15 minutes. After carrying out the reaction at 72°C for 3 minutes by adding 2.5 units of TaKaRa LA Taq (manufactured by Takara Shuzo), 10 pmol of each of T3 BcaBEST Sequencing primer (manufactured by Takara Shuzo) and T7 BcaBEST Sequencing primer (manufactured by Takara Shuzo) were added thereto to make the volume of the reaction mixture to 50 μl, which was subjected to 10 cycles of heating 94°C for 30 seconds, 55°C for 2 minutes and 72°C for 1.5 minutes as one cycle. After 25 μl of the reaction mixture was purified using QIA quick PCR purification kit (manufactured by QIAGEN), a half volume thereof was allowed to react at 37°C for 1 hour using 10 units of a restriction enzyme *Kpn*I (manufactured by Takara Shuzo) and 10 units of a restriction enzyme *Sac*I (manufactured by Takara Shuzo). The reaction mixture was fractionated by using agarose gel electrophoresis to recover a *Kpn*I-*Sac*I fragment of about 0.59 kb.

Next, 1 μ g of pBluescript II SK(-) (manufactured by Stratagene) was allowed to react at 37°C for 1 hour using 10 units of the restriction enzyme *Kpn*I (manufactured by Takara Shuzo) and 10 units of the restriction enzyme *Sac*I (manufactured by Takara Shuzo), and then the reaction mixture was subjected to agarose gel electrophoresis to recover a *Kpn*I-*Sac*I fragment of about 2.9 kb.

The PCR product-derived *Kpn*I-*Sac*I fragment and plasmid pBluescript II SK(-)-derived *Kpn*I-*Sac*I fragment thus obtained were ligated by using Solution I of DNA Ligation Kit Ver. 2 (manufactured by Takara Shuzo) in accordance

with the manufacture's instructions attached thereto. Using the recombinant plasmid DNA solution obtained in this manner, *E. coli* DH5α (manufactured by TOYOBO) was transformed. Each plasmid DNA was prepared from the transformant clones, and allowed to react by using BigDye Terminator Cycle Sequencing Ready Reaction Kit v2.0 (manufactured by Applied Biosystems) in accordance with the manufacture's instructions attached thereto, and then the nucleotide sequence was analyzed by the DNA sequencer ABI PRISM 377 manufactured by the same company.

In this manner, the plasmid pBS-2B8Hm shown in Fig. 2 comprising the objective nucleotide sequence was obtained.

(3) Construction of an anti-CD20 vector for expression of human chimeric antibody

By using pKANTEX93, a vector for expression of humanized antibody, *(Mol. Immunol.,* 37, 1035, 2000) and the plasmids pBS-2B8L and pBS-2B8Hm obtained in items 1(1) and (2) of Example 1, an anti-CD20 human chimeric antibody (hereinafter referred to as "anti-CD20 chimeric antibody") expression vector pKANTEX2B8P was constructed as follows.

After 2 μg of the plasmid pBS-2B8L obtained in item 1(1) in Example 1 was allowed to react at 55°C for 1 hour using 10 units of a restriction enzyme *Bsi*WI (manufactured by New England Biolabs), followed by reaction at 37°C for 1 hour using 10 units of a restriction enzyme *Eco*RI (manufactured by Takara Shuzo). The reaction mixture was fractionated by agarose gel electrophoresis to recover a *Bsi*WI-*Eco*RI fragment of about 0.41 kb.

Next, 2 μg of pKANTEX93, a vector for expression of humanized antibody, was allowed to react at 55°C for 1 hour using 10 units of the restriction enzyme *Bsi*WI (manufactured by New England Biolabs), followed by reaction at 37°C for 1 hour using 10 units of the restriction enzyme *Eco*RI (manufactured by Takara Shuzo). The reaction mixture was fractionated by agarose gel electrophoresis to recover a *Bsi*WI-*Eco*RI fragment of about 12.75 kb.

Next, the plasmid pBS-2B8L-derived *Bsi*WI-*Eco*RI fragment and plasmid pKANTEX93-derived *Bsi*WI-*Eco*RI fragment thus obtained were ligated by using Solution I of DNA Ligation Kit Ver. 2 (manufactured by Takara Shuzo) in accordance with the manufacture's instructions attached thereto. By using the recombinant plasmid DNA solution obtained in this manner, *E. coli* DH5α (manufactured by TOYOBO) was transformed to obtain the plasmid pKANTEX2B8-L shown in Fig. 3.

Next, 2 μg of the plasmid pBS-2B8Hm obtained in item 1(2) of Example I was allowed to react at 37°C for 1 hour by using 10 units of a restriction enzyme *Apa*I (manufactured by Takara Shuzo), followed by reaction at 37°C for 1 hour using 10 units of a restriction enzyme *Not*I (manufactured by Takara Shuzo). The reaction mixture was fractionated by agarose gel electrophoresis to recover an *Apa*I-*Not*I fragment of about 0.45 kb.

Next, 3 μg of the plasmid pKANTEX2B8-L was allowed to react at 37°C for 1 hour by using 10 units of the restriction enzyme *Apa*I (manufactured by Takara Shuzo), followed by reaction at 37°C for 1 hour using 10 units of the restriction enzyme *Not*I (manufactured by Takara Shuzo). The reaction mixture was fractionated by agarose gel electrophoresis to recover an *Apa*I-*Not*I fragment of about 13.16 kb.

Next, the plasmid pBS-2B8Hm-derived *Apa*I-*Not*I fragment and plasmid pKANTEX2B8-L-derived *Apa*I-*Not*I fragment thus obtained were ligated by using Solution I of DNA Ligation Kit Ver. 2 (manufactured by Takara Shuzo) in accordance with the manufacture's instructions attached thereto. By using the recombinant plasmid DNA solution obtained in this manner, *E. coli* DH5α (manufactured by TOYOBO) was transformed, and each plasmid DNA was prepared from the transformant clones.

The nucleotide sequence of the thus obtained plasmid was analyzed by using BigDye Terminator Cycle Sequencing Ready Reaction Kit v 2.0 (manufactured by Applied Biosystems) and the DNA sequencer 377 of the same company, and it was confirmed that the plasmid pKANTEX2B8P shown in Fig. 3 into which the objective DNA had been cloned was obtained.

2. Stable expression of an anti-CD20 chimeric antibody by using animal cells

(1) Preparation of a production cell by using rat myeloma YB2/0 cell

By using the anti-CD20 chimeric antibody expression vector, pKANTEX2B8P, obtained in item 1 (3) of Example 1, the anti-CD20 chimeric antibody was expressed in animal cells as follows. After 10 μg of the plasmid pKANTEX2B8P was introduced into $4\times 10^6$ cells of a rat myeloma cell line YB2/0 cell (ATCC CRL 1662) by electroporation [*Cytotechnology*, 3, 133 (1990)], the cells were suspended in 40 ml of H-SFM medium (manufactured by GIBCO-BRL supplemented with 5% fetal calf serum (FCS)) and dispensed at 200 μl/well into a 96 well microtiter plate (manufactured by Sumitomo Bakelite). After culturing at 37°C for 24 hours in a 5% $CO_2$ incubator, G418 was added thereto to give a concentration of 1 mg/ml, followed by culturing for 1 to 2 weeks. Culture supernatants were recovered from wells where colonies of transformants showing G418 resistance were formed and transformants became confluent, and the produced amount of the human IgG antibody in the culture supernatant was measured by ELISA described in item 2(2)

of Example 1.

Regarding a transformant in a well where expression of human IgG antibody was found in the culture supernatant, in order to increase the antibody expression level using a *dhfr* gene amplification system, it was suspended in H-SFM medium containing 1 mg/ml G418 and 50 nM methotrexate (hereinafter referred to as "MTX", manufactured by SIGMA) as an inhibitor of the *dhfr* gene product dihydrofolate reductase (hereinafter referred to as "DHFR") to give a density of 1 to $2\times10^5$ cells/ml, and the suspension was dispensed at 1 ml into each well of a 24 well plate (manufactured by Greiner). Culturing was carried out at 37°C for 1 to 2 weeks in a 5% $CO_2$ incubator to induce transformants showing 50 nM MTX resistance. When a transformant became confluent in a well, the produced amount of the human IgG antibody in the culture supernatant was measured by ELISA described in item 2(2) of Example 1. Regarding a transformant in well where expression of human IgG antibody was found in the culture supernatant, the MTX concentration was increased to 100 nM and then to 200 nM by the same method to finally obtain a transformant which can grow in H-SFM containing 1 mg/ml of G418 and 200 nM of MTX and also can perform high expression of the anti-CD20 chimeric antibody. The obtained transformant was cloned by limiting dilution, whereby a clone KM3065 which expresses an anti-CD20 chimeric antibody was obtained. Also, using the determination method of transcription product of α1,6-fucosyltransferase gene described in Example 8 of WO 00/61739, a cell line producing a relatively low level of the transcription product was selected and used as a suitable cell line.

The obtained transformant clone KM3065 which produces the anti-CD20 chimeric antibody has been deposited on December 21, 2001, as FERM 7834 in International Patent Organism Depositary, National Institute of Advanced Industrial Science and Technology (AIST Tsukuba Central 6, 1-1, Higashi 1-Chome Tsukuba-shi, Ibaraki-ken, Japan).

(2) Measurement of a human IgG antibody concentration in culture supernatant (ELISA)

A goat anti-human IgG (H & L) antibody (manufactured by American Qualex) was diluted with a phosphate buffered saline (hereinafter referred to as "PBS") to give a concentration of 1 μg/ml, dispensed at 50 μl/well into a 96 well ELISA plate (manufactured by Greiner) and then allowed to stand at 4°C overnight for adhesion. After washing with PBS, 1% bovine serum albumin (hereinafter referred to as "BSA"; manufactured by AMPC)-containing PBS (hereinafter referred to as "1% BSA-PBS") was added thereto at 100 μl/well and allowed to react at room temperature for 1 hour to block the remaining active groups. After discarding 1% BSA-PBS, culture supernatant of a transformant and variously diluted solutions of a purified human chimeric antibody were added thereto at 50 μl/well and allowed to react at room temperature for 2 hours. After the reaction, each well was washed with 0.05% Tween 20-containing PBS (hereinafter referred to as "Tween-PBS"), and then, as a secondary antibody solution, a peroxidase-labeled goat anti-human IgG (H & L) antibody solution (manufactured by American Qualex) 3,000 folds-diluted with 1% BSA-PBS was added thereto at 50 μl/well and allowed to react at room temperature for 1 hour. After the reaction and subsequent washing with Tween-PBS, an ABTS substrate solution (a solution prepared by dissolving 0.55 g of 2,2'-azino-bis (3-ethylbenzothiazoline-6-sulfonic acid)ammonium in 1 liter of 0.1 M citrate buffer (pH 4.2), and adding 1 μl/ml hydrogen peroxide just before use) was dispensed at 50 μl/well for coloration, and the absorbance at 415 nm (hereinafter referred to as "OD415") was measured.

3. Purification of anti-CD20 chimeric antibody from culture supernatant

The transformant cell clone KM3065 capable of expressing the anti-CD20 chimeric antibody, obtained in item 2(1) of Example 1, was suspended in H-SFM (manufactured by GIBCO-BRL) containing 200 nM MTX and 5% of Daigo's GF21 (manufactured by Wako Pure Chemical Industries), to give a density of $1\times10^5$ cells/ml, and dispensed at 50 ml into 182 $cm^2$ flasks (manufactured by Greiner). The cells were cultured at 37°C for 7 days in a 5% $CO_2$ incubator, and the culture supernatant was recovered when they became confluent. The anti-CD20 chimeric antibody KM3065 was purified from the culture supernatant using a Prosep-A (manufactured by Millipore) column in accordance with the manufacture's instructions attached thereto. About 3 μg of the obtained anti-CD20 chimeric antibody KM3065 was subjected to electrophoresis in accordance with the known method [*Nature,* 227, 680 (1970)] to examine its molecular weight and purification degree. As a result, the purified anti-CD20 chimeric antibody KM3065 was about 150 kilodaltons (hereinafter referred to as "Kd") under non-reducing condition, and two bands of about 50 Kd and about 25 Kd were observed under reducing conditions. These sizes of protein coincided with reports stating that an IgG type antibody has a molecular weight of about 150 Kd under non-reducing condition and is degraded into H chain having a molecular weight of about 50 Kd and L chain having a molecular weight of about 25 Kd under reducing condition due to cutting of the intramolecular disulfide bond (hereinafter referred to as "S-S bond") [*Antibodies: A Laboratory Manual,* Cold Spring Harbor Laboratory, Chapter 14 (1988), *Monoclonal Antibodies: Principles and Practice,* Academic Press Limited (1996)] and also almost coincided with the electrophoresis pattern of Rituxan™, and accordingly, it was confirmed that the anti-CD20 chimeric antibody KM3065 is expressed as the antibody molecule of a correct structure.

Example 2

Activity evaluation of an anti-CD20 chimeric antibody:

1. Binding activity of an anti-CD20 chimeric antibody to CD20-expressing cells (immunofluorescent method)

Binding activity of the purified CD20 chimeric antibody obtained in item 3. of Example 1 was evaluated by an immunofluorescent method using a flow cytometry. A human lymphoma cell line, Raji cell (JCRB 9012), as a CD20-positive cell was dispensed at of $2\times10^5$ cells into each well of a 96 well U-shape plate (manufactured by Falcon). An antibody solution (a concentration of 0.039 to 40 μg/ml) prepared by diluting the anti-CD20 chimeric antibody with an FACS buffer (1% BSA-PBS, 0.02% EDTA, 0.05% $NaN_3$) was added thereto at 50 μl/well and allowed to react on ice for 30 minutes. After washing twice with 200 μl/well of the FACS buffer, a solution prepared by diluting a PE-labeled anti-human IgG antibody (manufactured by Coulter) 100 folds with FACS buffer was added thereto at 50 μl/well. After 30 minutes of the reaction on ice under a shade and subsequent three times of washing at 200 μl/well, the cells were finally suspended at 500 μl of the mixture to measure the fluorescence intensity by a flow cytometer. The results are shown in Fig. 4. Antibody concentration-dependent increase in the fluorescence intensity was observed in both of KM3065 and Rituxan™, and it was confirmed that they show almost the same binding activity. Also, their activity to bind to a CD20-negative cell, human CCRF-CEM cell (ATCC CCL 119), was examined in the same manner by adjusting the antibody concentration to 40 μg/ml. The results are shown in Fig. 5. Since neither KM3065 nor Rituxan™ bound thereto, it was suggested that KM3065 specifically binds to CD20.

2. *In vitro* cytotoxic activity (ADCC activity) of an anti-CD20 chimeric antibody

In order to evaluate *in vitro* cytotoxic activity of the purified anti-CD20 chimeric antibodies obtained in item 3 of Example 1, the ADCC activity was measured in accordance with the following method.

(1) Preparation of a target cell solution

A human B lymphocyte cultured cell line WIL2-S cell (ATCC CRL8885), Ramos cell (ATCC CRL1596) or Raji cell (JCRB9012) cultured in RPMI1640-FCS(10) medium (RPMI1640 medium (manufactured by GIBCO BRL) containing 10% FCS) were washed with RPMI1640-FCS(5) medium (RPMI1640 medium (manufactured by GIBCO BRL) containing 5% FCS) by centrifugation and suspension, and then adjusted to $2\times10^5$ cells/ml by adding the RPMI1640-FCS(5) medium as the target cell solution.

(2) Preparation of an effector cell solution

From a healthy person, 50 ml of venous blood was collected, and 0.5 ml of heparin sodium (manufactured by Shimizu Pharmaceutical) was added thereto and gently mixed. The mixture was centrifuged to isolate a mononuclear cell layer using Lymphoprep (manufactured by AXIS SHIELD) in accordance with the manufacture's instructions (800×g, 20 minutes). After washing with the RPMI1640-FCS(5) medium by centrifugation three times, the resulting precipitate was re-suspended to give a density of $4\times 10^6$ cells/ml using the same medium and used as an effector cell solution.

(3) Measurement of ADCC activity

Into each well of a 96 well U-shaped bottom plate (manufactured by Falcon), 50 μl of the target cell solution prepared in the above (1) ($1\times10^4$ cells/well) was dispensed. Next, 50 μl of the effector cell solution prepared in the above (2) was added thereto ($2\times10^5$ cells/well, the ratio of effector cells to target cells became 20:1). Subsequently, each of the anti-CD20 chimeric antibodies was added thereto to give a final concentration from 0.3 to 3000 ng/ml, and the total volume was made up to 150 μl, followed by reaction at 37°C for 4 hours. After the reaction, the plate was centrifuged, and the lactate dehydrogenase (LDH) activity in the supernatant was measured by obtaining the absorbance data using CytoTox96 Non-Radioactive Cytotoxicity Assay (manufactured by Promega) according to the manufacture's instructions. The absorbance data of spontaneously released target cells and the absorbance data of spontaneously released effector cells were obtained in the same manner as the above, except that the medium alone was used instead of the effector cell solution and the antibody solution, and that the medium alone was used instead of the target cell solution and the antibody solution, respectively. The absorbance data of the total released target cells was obtained by measuring the LDH activity in the supernatant in the same manner as the above, by using the medium instead of the antibody solution and the effector cell solution and adding 15 μl of a 9% Triton X-100 solution to the

medium 45 minutes before the reaction termination. The ADCC activity was measured by the following equation.

$$\text{Cytotoxic activity (\%)} = \frac{\left(\text{Absorbance of sample}\right) - \left(\text{Absorbance of spontaneously released effector cells}\right) - \left(\text{Absorbance of spontaneously released target cells}\right)}{\left(\text{Absorbance of total released target cells}\right) - \left(\text{Absorbance of spontaneously released target cells}\right)} \times 100$$

Fig. 6 shows results of using 3 cell lines as the target. Fig. 6A, 6B and 6C show results of using Raji cell (JCRB9012), Ramos cell (ATCC CRL1596) and WIL2-S cell (ATCC CRL8885), respectively. As shown in Fig. 6, KM3065 show higher ADCC activity at all antibody concentrations and higher maximum cytotoxic activity than Rituxan™.

Example 3

Sugar chain analysis of anti-CD20 chimeric antibodies:

Sugar chains of the anti-CD20 antibodies purified in item 3 of Example 1 were analyzed. The sugar chains were cleaved from proteins by subjecting KM3065 and Rituxan™ to hydrazinolysis [*Method of Enzymology*, 83, 263 (1982)]. After removing hydrazine by evaporation under a reduced pressure, *N*-acetylation was carried out by adding an aqueous ammonium acetate solution and acetic anhydride. After freeze-drying, fluorescence labeling by 2-aminopyridine was carried out [*Journal of Biochemistry,* 95, 197 (1984)]. A fluorescence-labeled sugar chain group (hereinafter "PA-treated sugar chain group") was separated from excess reagents using Superdex Peptide HR 10/30 column (manufactured by Pharmacia). The sugar chain fractions were dried using a centrifugation concentrator and used as a purified PA-treated sugar chain group. Next, the purified PA-treated sugar chain group was subjected to reverse phase HPLC analysis using a CLC-ODS column (manufactured by Shimadzu).

Fig. 7 shows elution patterns obtained by carrying out reverse phase HPLC analysis of each of PA-treated sugar chains prepared from the anti-CD20 chimeric antibodies. Figs. 7A and 7B show elution patterns of KM3065 and Rituxan™, respectively. The ordinate and the abscissa show the relative fluorescence intensity and the elution time, respectively. Using a 10 mM sodium phosphate buffer (pH 3.8) as buffer A and a 10 mM sodium phosphate buffer (pH 3.8) + 0.5% 1-butanol as buffer B, the analysis was carried out by the following gradient.

Table 1

| Time (minute) | 0 | 80 | 90 | 90.1 | 120 |
|---|---|---|---|---|---|
| Buffer B (%) | 0 | 60 | 60 | 0 | 0 |

Peaks ① to ⑩ in Fig. 7 show the following structures (1) to (10), respectively.

(1)

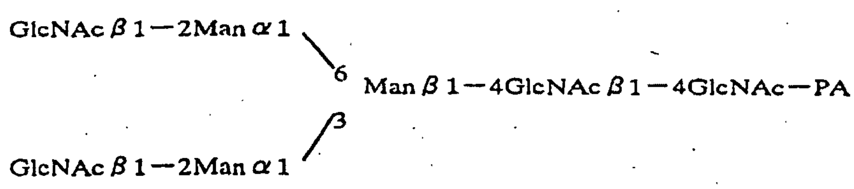

(2)

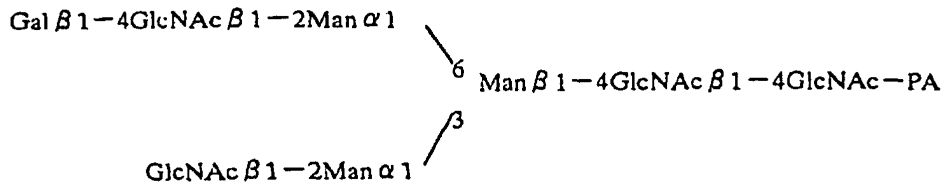

(3)

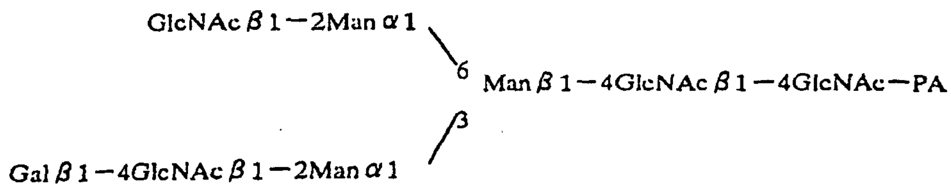

(4)

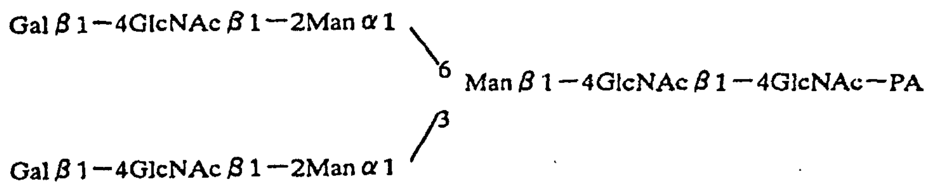

(5)

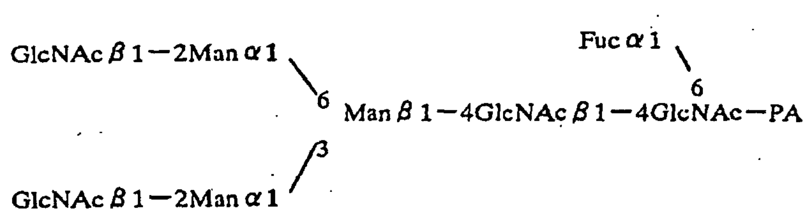

(6)

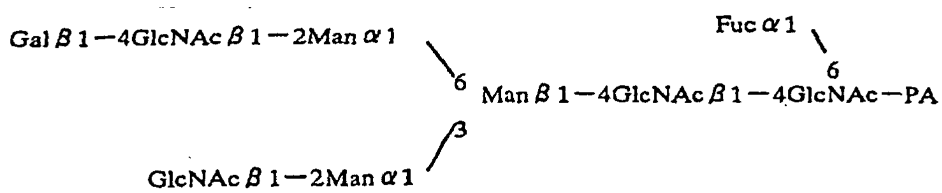

(7)

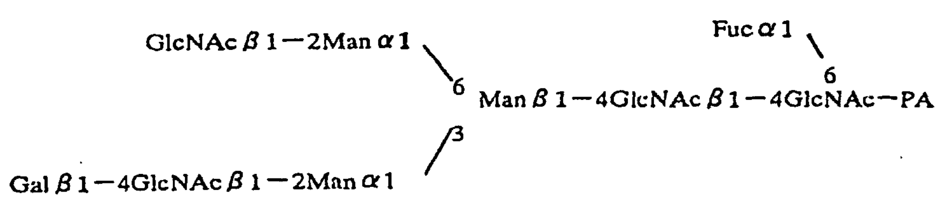

(8) 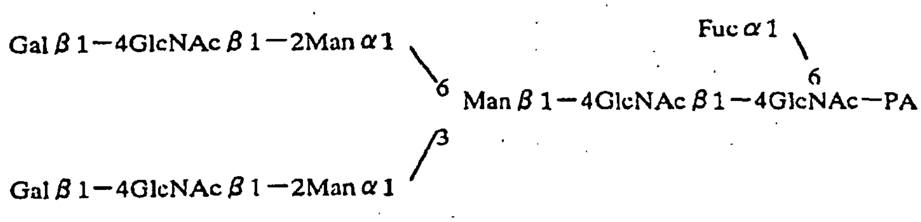

(9) 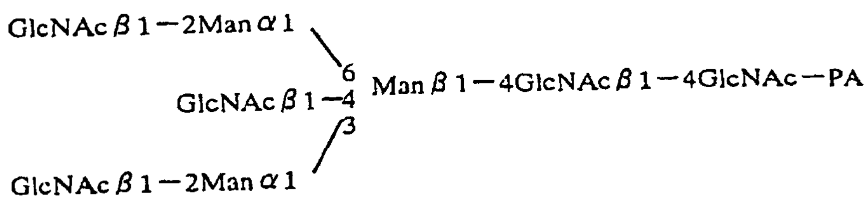

(10) 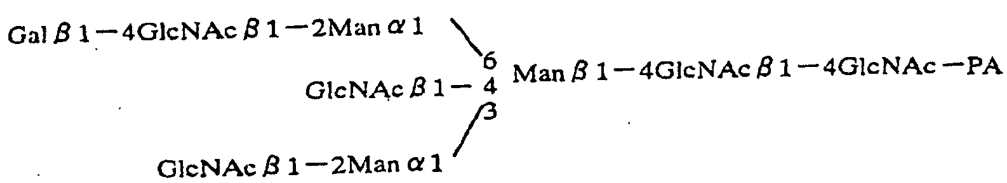

GlcNAc, Gal, Man, Fuc and PA represent *N*-acetylglucosamine, galactose, mannose, fucose and a pyridylamino group, respectively. In Fig. 7, the ratio of the sugar chain group in which 1-position of fucose is not bound to 6-position of *N*-acetylglucosamine in the complex *N*-glycoside-linked reducing end through α-bond (hereinafter referred to as "α1,6-fucose-free sugar chain group" or "α1,6-fucose-not-bound sugar chain group") was calculated from the area occupied by the peaks ① to ④, ⑨ and ⑩ among the areas occupied by the peaks ① to ⑩. Also, the ratio of the sugar chain group in which 1-position of fucose is bound to 6-position of *N*-acetylglucosamine in the complex *N*-glycoside-linked reducing end through α-bond (hereinafter referred to as "α1,6-fucose-bound sugar chain group") was calculated from the area occupied by the peaks ⑤ to ⑧ among the areas occupied by the peaks of ① to ⑩.

As a result, in Rituxan™, the ratio of the α1,6-fucose-not-bound sugar chains was 6%, whereas the ratio of the α1,6-fucose-bound sugar chains was 94%. In KM3065, the ratio of the α1,6-fucose-not-bound sugar chains was 96%, whereas the ratio of the α1,6-fucose-bound sugar chains was 4%. The results show that KM3065 has a much higher ratio of the α1,6-fucose-not-bound sugar chains than Rituxan™.

Example 4

Preparation of α1,6-fucosyltransferase (FUT8) gene derived from CHO cell:

(1) Preparation of α1,6-fucosyltransferase (FUT8) cDNA sequence from CHO cell

From a single-stranded cDNA prepared from CHO/DG44 cells on the 2nd day of culturing in Example 8(1) of WO00/61739, Chinese hamster FUT8 cDNA was obtained by the following procedure (Fig. 8).

First, a forward primer specific for a 5'-terminal non-translation region (shown in SEQ ID NO:21) and a reverse primer specific for a 3'-terminal non-translation region (shown in SEQ ID NO:22) were designed from a mouse FUT8 cDNA sequence (GenBank, AB025198).

Next, 25 μl of a reaction mixture [ExTaq buffer (manufactured by Takara Shuzo), 0.2 mmol/l dNTPs, 4% DMSO and 0.5 μmol/l specific primers (SEQ ID NOs:21 and 22)] containing 1 μl of the CHO/DG44 cell-derived cDNA was prepared and PCR was carried out by using a DNA polymerase ExTaq (manufactured by Takara Shuzo). The PCR was carried out by heating at 94°C for 1 minute, subsequent 30 cycles of heating at 94°C for 30 seconds, 55°C for 30 seconds and 72°C for 2 minutes as one cycle, and final heating at 72°C for 10 minutes.

After the PCR, the reaction mixture was subjected to 0.8% agarose gel electrophoresis, and a specific amplified fragment of about 2 Kb was purified. Into a plasmid pCR2.1, 4 μl of the DNA fragment was introduced in accordance with the manufacture's instructions attached to TOPO TA Cloning Kit (manufactured by Invitrogen), and *E. coli* DH5α was transformed with the reaction mixture. Plasmid DNAs were isolated from cDNA-inserted 8 clones among the obtained kanamycin-resistant colonies in accordance with a known method.

The nucleotide sequence of each cDNA inserted into the plasmid was determined using DNA Sequencer 377 (manufactured by Applied Biosystems) and BigDye Terminator Cycle Sequencing FS Ready Reaction Kit (manufactured by Applied Biosystems) in accordance with the method of the manufacture's instructions. It was confirmed by the method that all of the inserted cDNAs encode a sequence containing the full ORF of CHO cell FUT8. Among these, a plasmid DNA containing absolutely no reading error of bases by the PCR in the sequences was selected. Herein, the plasmid is referred to as CHfFUT8-pCR2.1. The determined nucleotide sequence of the cDNA of CHO FUT8 is represented by SEQ ID NO:1. The translation region (open reading frame: ORF) in SEQ ID NO:1 is nucleotides at position 100-1827, and the amino acid sequence corresponding to nucleotides at positions 100 to 1824 excluding the termination codon is represented by SEQ ID NO:23.

(2) Preparation of α1,6-fucosyltransferase (FUT8) genomic sequence from CHO cell

Using the ORF full length cDNA fragment of CHO cell FUT8 obtained in item (1) as a probe, a CHO cell FUT8 genomic clone was obtained from CHO-K1 cell-derived λ-phage genome library (manufactured by Strategene) in accordance with a known genome screening method described, e.g., in *Molecular Cloning*, Second Edition, *Current Protocols in Molecular Biology, A Laboratory Manual*, Second Edition (1989). Next, after digesting the obtained genomic clone using various restriction enzymes, the Southern hybridization was carried out by using an *Afa*I-*Sau*3AI fragment (about 280 bp) containing initiation codon of the CHO cell FUT8 cDNA as a probe, and then a *Xba*I-*Xba*I fragment (about 2.5 Kb) and a *Sac*I-*Sac*I fragment (about 6.5 Kb) were selected from restriction enzyme fragments showing positive reaction, inserted into pBluescript II KS(+) (manufactured by Stratagene), respectively.

The nucleotide sequence of each of the obtained genomic fragments was determined by using DNA Sequencer 377 (manufactured by Applied Biosystems) and BigDye Terminator Cycle Sequencing FS Ready Reaction Kit (manufactured by Parkin Elmer) in accordance with the method of the manufacture's instructions. Thereby, it was confirmed that the *Xba*I-*Xba*I fragment encodes a sequence of an upstream intron of about 2.5 Kb containing exon 2 of the CHO cell FUT8, and the *Sac*I-*Sac*I fragment encodes a sequence of a downstream intron of about 6.5 Kb containing exon 2 of the CHO cell FUT8. Herein, the plasmid containing *Xba*I-*Xba*I fragment and the plasmid containing *Sac*I-*Sac*I fragment are referred to as pFUT8fgE2-2 and pFUT8fgE2-4, respectively. The determined nucleotide sequence (about 9.0 Kb) of the genome region containing exon 2 of the CHO cell FUT8 is shown in SEQ ID NO:3.

Example 5

Preparation of CHO cell in which α1,6-fucosyltransferase gene is disrupted:

A CHO cell from which the genomic region comprising exon 2 of α1,6-fucosyltransferase (FUT8) gene derived from the CHO cell was deleted was prepared and the ADCC activity of an antibody produced by the cell was evaluated.

1. Construction of Chinese hamster α1,6-fucosyltransferase (FUT8) gene exon 2 targeting vector plasmid pKOFUT8Puro

(1) Construction of plasmid ploxPPuro

A plasmid ploxPPuro was constructed by the following procedure (Fig. 9).

In 35 μl of NEBuffer 4 (manufactured by New England Biolabs), 1.0 μg of a plasmid pKOSelectPuro (manufactured by Lexicon) was dissolved, and 20 units of a restriction enzyme AscI (manufactured by New England Biolabs) were added thereto, followed by digestion reaction at 37°C for 2 hours. After the digestion reaction, the mixture was subjected to 0.8% (w/v) agarose gel electrophoresis to purify a DNA fragment of about 1.5 Kb containing a puromycin resistance gene expression unit.

Separately, 1.0 μg of a plasmid ploxP described in Japanese Published Examined Patent Application No. 314512/99 was dissolved in 35 μl of NEBuffer 4 (manufactured by New England Biolabs), and 20 units of a restriction enzyme *Asc*I (manufactured by New England Biolabs) were added thereto, followed by digestion reaction at 37°C for 2 hours. After the digestion reaction, the mixture was subjected to 0.8% (w/v) agarose gel electrophoresis to purify a DNA fragment of about 2.0 Kb.

The obtained *Asc*I-*Asc*I fragment (4.5 μl, about 1.5 Kb) derived from the plasmid pKOSelectPuro, 0.5 μl of the *Asc*I-*Asc*I fragment (about 2.0 Kb) derived from the plasmid ploxP and 5.0 μl of Ligation High (manufactured by

Toyobo) were mixed, followed by ligation reaction at 16°C for 30 minutes. *E. coli* DH5α was transformed by using the reaction mixture, and a plasmid DNA was isolated in accordance with a known method from the obtained ampicillin-resistant clones. Herein, the plasmid is referred to as ploxPPuro.

(2) Construction of plasmid pKOFUT8gE2-1

A plasmid pKOFUT8gE2-1 was constructed by the following procedure, by using the plasmid pFUT8fgE2-2 having a genome region comprising exon 2 of Chinese hamster FUT8 obtained in Example 4(2) (Fig. 10).

In 35 μl of NEBuffer 1 (manufactured by New England Biolabs) containing 100 μg/ml of BSA (manufactured by New England Biolabs), 2:0 μg of the plasmid pFUT8fgE2-2 was dissolved, and 20 units of a restriction enzyme *Sac*I (manufactured by New England Biolabs) were added thereto, followed by digestion reaction at 37°C for 2 hours. A DNA fragment was recovered from the reaction mixture by ethanol precipitation and dissolved in 35 μl of NEBuffer 2 (manufactured by New England Biolabs) containing 100 μg/ml BSA (manufactured by New England Biolabs), and 20 units of a restriction enzyme *Eco*RV (manufactured by New England Biolabs) were added thereto, followed by digestion reaction at 37°C for 2 hours. After the digestion reaction, the mixture was subjected to 0.8% (w/v) agarose gel electrophoresis to purify a DNA fragment of about 1.5 Kb.

Separately, 1.0 μg of a plasmid LITMUS28 (manufactured by New England Biolabs) was dissolved in 35 μl of NEBuffer 1 (manufactured by New England Biolabs) containing 100 μg/ml of BSA (manufactured by New England Biolabs), and 20 units of a restriction enzyme *Sac*I (manufactured by New England Biolabs) were added thereto, followed by digestion reaction at 37°C for 2 hours. A DNA fragment was recovered from the reaction mixture by ethanol precipitation and dissolved in 35 μl of NEBuffer 2 (manufactured by New England Biolabs) containing 100 μg/ml BSA (manufactured by New England Biolabs), and 20 units of a restriction enzyme *Eco*RV (manufactured by New England Biolabs) were added thereto, followed by digestion reaction at 37°C for 2 hours. After the digestion reaction, the mixture was subjected to 0.8% (w/v) agarose gel electrophoresis to purify a DNA fragment of about 2.8 Kb.

The obtained *Eco*RV-*Sac*I fragment (4.5 μl, about 1.5 Kb) derived from the plasmid pFUT8fgE2-2, 0.5 μl of the *Eco*RV-*Sac*I fragment (about 2.8 Kb) derived from the plasmid LITMUS28 and 5.0 μl of Ligation High (manufactured by Toyobo) were mixed, followed by ligation reaction at 16°C for 30 minutes. *E. coli* DH5α was transformed by using the reaction mixture, and a plasmid DNA was isolated from the obtained ampicillin-resistant clones in accordance with a known method. Herein, the plasmid is referred to as pKOFUT8gE2-1.

(3) Construction of plasmid pKOFUT8gE2-2

A plasmid pKOFUT8gE2-2 was constructed by the following procedure, by using the plasmid pKOFUT8gE2-1 obtained in item (2) (Fig. 11).

In 30 μl of NEBuffer 2 (manufactured by New England Biolabs) containing 100 μg/ml of BSA (manufactured by New England Biolabs), 2.0 μg of the plasmid pKOFUT8gE2-1 was dissolved, and 20 units of a restriction enzyme *Eco*RV (manufactured by New England Biolabs) were added thereto, followed by digestion reaction at 37°C for 2 hours. A DNA fragment was recovered from the reaction mixture by ethanol precipitation and dissolved in 30 μl of NEBuffer 1 (manufactured by New England Biolabs) containing 100 μg/ml BSA (manufactured by New England Biolabs), and 20 units of a restriction enzyme *Kpn*I (manufactured by New England Biolabs) were added thereto, followed by digestion reaction at 37°C for 2 hours. After the digestion reaction, the mixture was subjected to 0.8% (w/v) agarose gel electrophoresis to purify a DNA fragment of about 1.5 Kb.

Separately, 1.0 μg of the plasmid ploxPPuro was dissolved in 30 μl of NEBuffer 4 (manufactured by New England Biolabs), and 20 units of a restriction enzyme *Hpa*I (manufactured by New England Biolabs) were added thereto, followed by digestion reaction at 37°C for 2 hours. A DNA fragment was recovered from the reaction mixture by ethanol precipitation and dissolved in 30 μl of NEBuffer 1 (manufactured by New England Biolabs) containing 100 μg/ml BSA (manufactured by New England Biolabs), and 20 units of a restriction enzyme *Kpn*I (manufactured by New England Biolabs) were added thereto, followed by digestion reaction at 37°C for 2 hours. After the digestion reaction, the mixture was subjected to 0.8% (w/v) agarose gel electrophoresis to purify a DNA fragment of about 3.5 Kb.

The obtained *Eco*RV-*Kpn*I fragment (4.0 μl, about 1.5 Kb) derived from the plasmid pKOFUT8gE2-1, 1.0 μl of the *Hpa*I-*Kpn*I fragment (about 3.5 Kb) derived from the plasmid ploxPPuro. and 5.0 μl of Ligation High (manufactured by Toyobo) were mixed, followed by ligation reaction at 16°C for 30 minutes. *E. coli* DH5α was transformed by using the reaction mixture, and a plasmid DNA was isolated in accordance with a known method from the obtained ampicillin-resistant clones. Herein, the plasmid is referred to as pKOFUT8gE2-2.

(4) Construction of plasmid pscFUT8gE2-3

A plasmid pscFUT8gE2-3 was constructed by the following procedure, by using the plasmid pFUT8fgE2-4

having a genome region comprising exon 2 of Chinese hamster FUT8 obtained in Example 4(2) (Fig. 12).

In 35 µl of NEBuffer 1 (manufactured by New England Biolabs), 2.0 µg of the plasmid pFUT8fgE2-4 was dissolved, and 20 units of a restriction enzyme *Hpa*II (manufactured by New England Biolabs) were added thereto, followed by digestion reaction at 37°C for 2 hours. A DNA fragment was recovered from the reaction mixture by ethanol precipitation, and then the DNA termini were changed to blunt ends by using Blunting High (manufactured by Toyobo) in accordance with the manufacture's instructions. The DNA fragment was recovered by carrying out phenol/chloroform extraction and ethanol precipitation and dissolved in 35 µl of NEBuffer 2 (manufactured by New England Biolabs), and 20 units of a restriction enzyme *Hind*III (manufactured by New England Biolabs) were added thereto, followed by digestion reaction at 37°C for 2 hours. After the digestion reaction, the mixture was subjected to 0.8% (w/v) agarose gel electrophoresis to purify a DNA fragment of about 3.5 Kb.

Separately, 1.0 µg of a plasmid LITMUS39 (manufactured by New England Biolabs) was dissolved in 35 µl of NEBuffer 2 (manufactured by New England Biolabs), and the mixture was mixed with 20 units of a restriction enzyme *Eco*RV (manufactured by New England Biolabs) and 20 units of a restriction enzyme *Hind*III (manufactured by New England Biolabs) and subjected to the digestion reaction at 37°C for 2 hours. After the digestion reaction, the mixture was subjected to 0.8% (w/v) agarose gel electrophoresis to purify a DNA fragment of about 2.8 Kb.

The obtained *Hpa*II-*Hind*III fragment (4.0 µl, about 3.5 Kb) derived from the plasmid pFUT8fgE2-4, 1.0 µl of the *Eco*RV-*Hind*III fragment (about 2.8 Kb) derived from the plasmid LITMUS39 and 5.0 µl of Ligation High (manufactured by Toyobo) were mixed, followed by ligation reaction at 16°C for 30 minutes. *E. coli* DH5α was transformed by using the reaction mixture, and a plasmid DNA was isolated in accordance with a known method from the obtained ampicillin-resistant clones. Herein, the plasmid is referred to as pscFUT8gE2-3.

(5) Construction of plasmid pKOFUT8gE2-3

A plasmid pKOFUT8gE2-3 was constructed by the following procedure, by using the plasmid pFUT8fgE2-4 obtained in Example 4(2) having a genome region comprising exon 2 of Chinese hamster FUT8 (Fig. 13).

In 35 µl of NEBuffer for *Eco*RI (manufactured by New England Biolabs), 2.0 µg of the plasmid pFUT8fgE2-4 was dissolved, and 20 units of a restriction enzyme *Eco*RI (manufactured by New England Biolabs) and 20 units of a restriction enzyme *Hind*III (manufactured by New England Biolabs) were added thereto, followed by digestion reaction at 37°C for 2 hours. After the digestion reaction, the mixture was subjected to 0.8% (w/v) agarose gel electrophoresis to purify a DNA fragment of about 1.8 Kb.

Separately, 1.0 µg of a plasmid pBluescript II KS(+) (manufactured by Stratagene) was dissolved in 35 µl of NEBuffer for *Eco*RI (manufactured by New England Biolabs). Then 20 units of a restriction enzyme *Eco*RI (manufactured by New England Biolabs) and 20 units of a restriction enzyme *Hind*III (manufactured by New England Biolabs) were added thereto, followed by digestion reaction at 37°C for 2 hours. After the digestion reaction, the mixture was subjected to 0.8% (w/v) agarose gel electrophoresis to purify a DNA fragment of about 3.0 Kb.

The obtained *Hind*III-*Eco*RI fragment (4.0 µl, about 1.8 Kb) derived from the plasmid pFUT8fgE2-4, 1.0 µl of the *Hind*III-*Eco*RI fragment (about 3.0 Kb) derived from the plasmid pBluescript II KS(+) and 5.0 µl of Ligation High (manufactured by Toyobo) were mixed, followed by ligation reaction at 16°C for 30 minutes. *E. coli* DH5α was transformed by using the reaction mixture, and a plasmid DNA was isolated in accordance with a known method from the obtained ampicillin-resistant clones. Herein, the plasmid is referred to as pKOFUT8gE2-3.

(6) Construction of plasmid pKOFUT8gE2-4

A plasmid pKOFUT8gE2-4 was constructed by the following procedure, by using the plasmids pscFUT8gE2-3 and pKOFUT8gE2-3 obtained in items (4) and (5) (Fig. 14).

In 35 µl of NEBuffer for *Sal*I (manufactured by New England Biolabs) containing 100 µg/ml of BSA (manufactured by New England Biolabs), 1.0 µg of the plasmid pscFUT8gE2-3 was dissolved, and 20 units of a restriction enzyme *Sal*I (manufactured by New England Biolabs) were added thereto, followed by digestion reaction at 37°C for 2 hours. A DNA fragment was recovered from the reaction mixture by ethanol precipitation and dissolved in 30 µl ofNEBuffer 2 (manufactured by New England Biolabs), containing 100 µg/ml BSA (manufactured by New England Biolabs), and 20 units of a restriction enzyme *Hind*III (manufactured by New England Biolabs) were added thereto, followed by digestion reaction at 37°C for 2 hours. After the digestion reaction, the mixture was subjected to 0.8% (w/v) agarose gel electrophoresis to purify a DNA fragment of about 3.6 Kb.

Separately, 1.0 µg of the plasmid pKOFUT8gE2-3 was dissolved in 35 µl of NEBuffer for *Sal*I (manufactured by New England Biolabs), containing 100 µg/ml BSA (manufactured by New England Biolabs), and 20 units of a restriction enzyme *Sal*I (manufactured by New England Biolabs) were added thereto, followed by digestion reaction at 37°C for 2 hours. A DNA fragment was recovered from the reaction mixture by ethanol precipitation and dissolved in 35 µl ofNEBuffer 2 (manufactured by New England Biolabs), and 20 units of a restriction enzyme *Hin*dIII (manufactured

by New England Biolabs) were added thereto, followed by digestion reaction at 37°C for 2 hours. After the digestion reaction, 35 µl of 1 mol/l Tris-HCl buffer (pH 8.0) and 3.5 µl of *E. coli* C15-derived alkaline phosphatase (manufactured by Takara Shuzo) were added thereto, followed by reaction at 65°C for 30 minutes to dephosphorylate the DNA termini. After the dephosphorylation treatment, a DNA fragment was recovered by carrying out phenol/chloroform extraction and ethanol precipitation, and dissolved in 10 µl of sterile water.

The obtained *Sal*I-*Hind*III fragment (4.0 µl, about 3.1 Kb) derived from the plasmid pscFUT8gE2-3, 1.0 µl of the *Sal*I-*Hind*III fragment (about 4.8 Kb) derived from the plasmid pKOFUT8gE2-3 and 5.0 µl of Ligation High (manufactured by Toyobo) were mixed, followed by ligation reaction at 16°C for 30 minutes. *E. coli* DH5α was transformed by using the reaction mixture, and a plasmid DNA was isolated in accordance with a known method from the obtained ampicillin-resistant clones. Herein, the plasmid is referred to as pKOFUT8gE2-4.

(7) Construction of plasmid pKOFUT8gE2-5

A plasmid pKOFUT8gE2-5 was constructed by the following procedure, by using the plasmids pKOFUT8gE2-2 and pKOFUT8gE2-4 obtained in items (3) and (6) (Fig. 15).

In 30 µl of NEBuffer 4 (manufactured by New England Biolabs), 1.0 µg of the plasmid pKOFUT8gE2-2 was dissolved, and 20 units of a restriction enzyme *Sma*I (manufactured by New England Biolabs) were added thereto, followed by digestion reaction at 25°C for 2 hours. A DNA fragment was recovered from the reaction mixture by ethanol precipitation and dissolved in 30 µl of NEBuffer 2 (manufactured by New England Biolabs), and 20 units of a restriction enzyme *Bam*HI (manufactured by New England Biolabs) were added thereto, followed by digestion reaction at 37°C for 2 hours. After the digestion reaction, 30 µl of 1 mol/l Tris-HCl buffer (pH 8.0) and 3.0 µl of *E. coli* C15-derived alkaline phosphatase (manufactured by Takara Shuzo) were added thereto, followed by reaction at 65°C for 1 hour to dephosphorylate the DNA termini. After the dephosphorylation treatment, the DNA fragment was recovered by carrying out phenol/chloroform extraction and ethanol precipitation, and dissolved in 10 µl of sterile water.

Separately, 1.0 µg of the plasmid pKOFUT8gE2-4 was dissolved in 30 µl of NEBuffer 4 (manufactured by New England Biolabs), and 20 units of a restriction enzyme *Sma*I (manufactured by New England Biolabs) were added thereto, followed by digestion reaction at 25°C for 2 hours. A DNA fragment was recovered from the reaction mixture by ethanol precipitation and dissolved in 30 µl of NEBuffer 2 (manufactured by New England Biolabs), and 20 units of a restriction enzyme *Bam*HI (manufactured by New England Biolabs) were added thereto, followed by digestion reaction at 37°C for 2 hours. After the digestion reaction, the mixture was subjected to 0.8% (w/v) agarose gel electrophoresis to purify a DNA fragment of about 5.2 Kb.

The obtained *Sma*I-*Bam*HI fragment (0.5 µl, about 5.0 Kb) derived from the plasmid pKOFUT8gE2-2, 4.5 µl of the *Sma*I-*Bam*HI fragment (about 5.2 Kb) derived from the plasmid pKOFUT8gE2-4 and 5.0 µl of Ligation High (manufactured by Toyobo) were mixed, followed by ligation reaction at 16°C for 15 hours. *E. coli* DH5α was transformed by using the reaction mixture, and a plasmid DNA was isolated in accordance with a known method from the obtained ampicillin-resistant clones. Herein, the plasmid is referred to as pKOFUT8gE2-5.

(8) Construction of plasmid pKOFUT8Puro

A plasmid pKOFUT8Puro was constructed by the following procedure, by using the plasmid pKOFUT8gE2-5 obtained in item (7) (Fig. 16).

In 50 µl of NEBuffer 4 (manufactured by New England Biolabs), 1.0 µg of a plasmid pKOSelectDT (manufactured by Lexicon) was dissolved, and 16 units of a restriction enzyme *Rsr*II (manufactured by New England Biolabs) were added thereto, followed by digestion reaction at 37°C for 2 hours. After the digestion reaction, the mixture was subjected to 0.8% (w/v) agarose gel electrophoresis to purify a DNA fragment of about 1.2 Kb comprising a diphtheria toxin expression unit.

Separately, 1.0 µg of the plasmid pKOFUT8gE2-5 was dissolved in 50 µl of NEBuffer 4 (manufactured by New England Biolabs), and 16 units of a restriction enzyme *Rsr*II (manufactured by New England Biolabs) were added thereto, followed by digestion reaction at 37°C for 2 hours. After the digestion reaction, 30 µl of 1 mol/l Tris-HCl buffer (pH 8.0) and 3.0 µl of *E. coli* C15-derived alkaline phosphatase (manufactured by Takara Shuzo) were added thereto, followed by reaction at 65°C for 1 hour to dephosphorylate the DNA termini. After the dephosphorylation treatment, the DNA fragment was recovered by carrying out phenol/chloroform extraction and ethanol precipitation, and dissolved in 10 µl of sterile water.

1.0 µg of the obtained *Rsr*II-*Rsr*II fragment (about 1.2 Kb) derived from the plasmid pKOSelectDT, 1.0 µl of the *Rsr*II-*Rsr*II fragment (about 10.4 Kb) derived from the plasmid pKOFUT8gE2-5, 3.0 µl of sterile water and 5.0 µl of Ligation High (manufactured by Toyobo) were mixed, followed by ligation reaction at 16°C for 30 minutes. *E. coli* DH5α was transformed by using the reaction mixture, and a plasmid DNA was isolated in accordance with a known method from the obtained ampicillin-resistant clones. Herein, the plasmid is referred to as pKOFUT8Puro. The plasmid is used

as a targeting vector for constructing CHO cell-derived FUT8 gene knock out cell.

Example 6

Preparation of lectin-resistant CHO/DG44 cell and production of antibody using the cell:

1. Preparation of lectin-resistant CHO/DG44

CHO/DG44 cells were grown until they reached a stage of just before confluent, by culturing in a 75 $cm^2$ flask for adhesion culture (manufactured by Greiner) using IMDM-FBS(10) medium [IMDM medium comprising 10% of fetal bovine serum (FBS) and 1 × concentration of HT supplement (manufactured by GIBCO BRL)]. After washing the cells with 5 ml of Dulbecco's PBS (manufactured by Invitrogen), 1.5 ml of 0.05% trypsin (manufactured by Invitrogen) diluted with Dulbecco's PBS was added thereto and allowed to stand at 37°C for 5 minutes to dissociate the cells from the flask bottom. The disociated cells were recovered by a centrifugation operation generally used in cell culture and suspended in IMDM-FBS(10) medium at a density of $1\times10^5$ cells/ml. To the cell suspension, and then 0.1 µg/ml of an alkylating agent *N*-methyl-N'-nitro-*N*-nitrosoguanidine (hereinafter referred to as "MNNG", manufactured by Sigma) may be added, if necessary. After incubating them at 37°C for 3 days in a $CO_2$ incubator (manufactured by TABAI), the culture supernatant was discarded, and the cells were again washed, dissociated and recovered by the same operations, suspended in IMDM-FBS(10) medium and then inoculated into a tissue culture 96 well plate (manufactured by IWAKI Glass) at a density of 1,000 cells/well. To each well, as the final concentration in medium, 1 mg/ml *Lens culinaris* agglutinin (hereinafter referred to as "LCA", manufactured by Vector), 1 mg/ml *Aleuria aurantia* agglutinin (*Aleuria aurantia* lectin; hereinafter referred to as "AAL", manufactured by Vector) or 1 mg/ml kidney bean agglutinin (*Phaseolus vulgaris* leucoagglutinin; hereinafter referred to as "L-PHA", manufactured by Vector) was added. After culturing them at 37°C for 2 weeks in a $CO_2$ incubator, the appeared colonies were obtained as lectin-resistant CHO/DG44. Regarding the obtained lectin-resistant CHO/DG44, an LCA-resistant cell line, an AAL-resistant cell line and an L-PHA-resistant cell line were named CHO-LCA, CHO-AAL and CHO-PHA, respectively. When the resistance of these cell lines to various kinds of lectin was examined, it was found that the CHO-LCA was also resistant to AAL, and the CHO-AAL was also resistant LCA. In addition, the CHO-LCA and CHO-AAL also showed a resistance to a lectin which recognizes a sugar chain structure identical to the sugar chain structure recognized by LCA and AAL, namely a lectin which recognizes a sugar chain structure in which 1-position of fucose is bound to 6-position of *N*-acetylglucosamine residue in the reducing end through α-bond in the *N*-glycoside-linked sugar chain. Specifically, it was found that the CHO-LCA and CHO-AAL can show resistance and survive even in a medium supplemented with a pea agglutinin (*Pisum sativum* agglutinin; hereinafter referred to as "PSA", manufactured by Vector) at a final concentration of 1 mg/ml. In addition, even when the alkylating agent MNNG was not added, it was able to obtain lectin-resistant cell. lines by increasing the number of cells to be treated. Hereinafter, these cell lines were used in analyses.

2. Preparation of anti-CD20 human chimeric antibody-producing cells

Into $1.6\times 10^6$ cells of the CHO/DG44 cell which was the lectin-resistant cell line obtained in the above item 1, 4 µg of an anti-CD20 vector for expression of human chimeric antibody pKANTEX2B8P was introduced by electroporation [*Cytotechnology,* 3, 133 (1990)], the cells were suspended in 10 ml of IMDM-dFBS(10)-HT(1) [IMDM medium (manufactured by Invitrogen) containing 10% dFBS (manufactured by Invitrogen) and HT supplement (manufactured by Invitrogen) at 1× concentration] and the suspension was dispensed into a 96-well culture plate (manufactured by Iwaki Glass) at 100 µl/well. The cells were cultured in a 5% $CO_2$ incubator at 37°C for 24 hours, and then its medium was changed to IMDM-dFBS(10) (IMDM medium containing 10% dialyzed FBS), followed by culturing for 1 to 2 weeks. Since colonies of transformants showing HT-independent growth were observed, the transformants in the wells in which growth was observed were subjected to a DHFR gene amplification, and the amount of the antibody production was increased. Specifically, the cells were suspended in IMDM-dFBS(10) medium containing 50 nM MTX at a density of 1 to $2\times10^5$ cells/ml, and the suspension was dispensed to a 24-well plate (manufactured by Iwaki Glass) at 0.5 ml/well. The cells were cultured in a 5% $CO_2$ incubator at 37°C for 1 to 2 weeks to induce transformants showing 50 nM MTX resistance. Regarding the transformants in wells in which growth was observed, the MTX concentration of the medium was increased to 200 nM, and then a transformant capable of growing in the IMDM-dFBS(10) medium containing 200 nM MTX and of producing the anti-CD20 human chimeric antibody in a large amount was finally obtained in the same manner as described above.

3. Culturing of an antibody-expressing cell line and purification of an antibody

The LCA lectin-resistant CHO/DG44 transformant cells capable of producing the anti-CD20 human chimeric

antibody in a large amount obtained in the above item 2 was named R92-3-1. R92-3-1 has been deposited on March 26, 2002, as FERM BP-7976 in International Patent Organism Depositary, National Institute of Advanced Industrial Science and Technology (AIST Tsukuba Central 6, 1-1, Higashi 1-Chome Tsukuba-shi, Ibaraki-ken, Japan).

R92-3-1 was cultured in IMDM-dFBS(10) containing 200 nM MTX until the cells became confluent and was washed with Dulbecco's PBS (manufactured by Invitrogen), and then the medium was changed to EX-CELL301 (manufactured by JRH). The cells were cultured in a 5% $CO_2$ incubator at 37°C for 7 days and the culture supernatant was collected. An anti-CD20 chimeric antibody was purified by using Prosep-A column (manufactured by Millipore) from the culture supernatant, and was named R92-3-1 antibody.

Example 7

Purification of an anti-CD20 chimeric antibody produced by lectin-resistant CHO/DG44 cell and evaluation of its activity

1. Evaluation of binding activity of the antibody derived from lectin-resistant CHO/DG44 cell (immunofluorescent method)

Binding activity of R92-3-1 antibody obtained in above item 3 of Example 6 to Raji cell line, in which CD20 is expressed, was examined according to the immunofluorescent method described in the above item 1 of Example 2 and compared with that of commercially available antibody Rituxan™ derived from ordinary CHO cell. As shown in Fig. 17, the fluorescent intensity was increased in dependence on antibody concentration in both R92-3-1 antibody and Rituxan™, and it was confirmed that they have almost similar binding activity.

2. Evaluation of *in vitro* cytotoxic activity of the antibody derived from lectin-resistant CHO/DG44 cell (ADCC activity)

In order to evaluate *in vitro* ADCC activity of R92-3-1 antibody obtained in item 3 of Example 6, the ADCC activity was measured according to the method described in the above item 2 of Example 2. The ratio of the effector cell and the target cell, Raji cell, was 25:1, the final antibody concentration was 0.001 to 10 μg/mL, and the reaction was carried out at a total volume of 200 μL. The results are shown in Fig. 18.

The results show that R92-3-1 antibody derived from LCA lectin-resistant CHO/DG44 cell has higher ADCC activity than Rituxan™.

3. Sugar chain analysis of the antibody derived from lectin-resistant CHO/DG44 cell

Sugar chain analysis of R92-3-1 antibody obtained in the above item 3 of Example 6 was carried out according to the method described in Example 3. The results are shown in Fig. 19. The sugar chain structures of peaks① to ⑧ in Fig. 19 are the same as those of peaks ① to ⑧ in Fig. 7, respectively.

In Fig. 19, the ratio of the α1,6-fucose-free sugar chain group was calculated from the area occupied by the peaks ① to ④ , ⑨ and ⑩ among ① to ⑩ . Also, the ratio of the α1,6-fucose-bound sugar chain group was calculated from the area occupied by the peaks ⑤ to ⑧ among ① to ⑩ .

As a result, in R92-3-1 antibody, the ratio of the α1,6-fucose-not-bound sugar chain group was 33%, whereas the ratio of the α1,6-fucose-bound sugar chains was 67%. When compared with the sugar chain analysis of Rituxan™ carried out in Example 3, the antibody produced by LCA lectin-resistant CHO/DG44 cells has a higher ratio of α1,6-fucose-not bound sugar chains.

Example 8

Preparation of CHO cell-derived GMD gene:

1. Determination of cDNA sequence of CHO cell-derived GMD gene

(1) Preparation of cDNA of CHO cell-derived GMD gene (preparation of partial cDNA excluding 5'- and 3'-terminal sequences)

cDNA of rodents-derived GMD was searched in a public data base (BLAST) by using cDNA sequence of a human-derived GMD (GenBank Accession No. AF042377) registered at GenBank as a query, and three kinds of mouse EST sequences were obtained (GenBank Accession Nos. BE986856, BF158988 and BE284785). By ligating these EST sequences, a deduced cDNA sequence of mouse GMD was determined.

On the base of cDNA sequence of the mouse-derived GMD, a 28 mer primer having the sequence represented

by SEQ ID NO:32, a 27 mer primer having the sequence represented by SEQ ID NO:33, a 25 mer primer having the sequence represented by SEQ ID NO:34, a 24 mer primer having the sequence represented by SEQ ID NO:35 and a 25 mer primer having the sequence represented by SEQ ID NO:36 were prepared.

Next, CHO/DG44 cell was subcultured in a 5% $CO_2$ incubator at 37°C, followed by culturing. After culturing, a total RNA was prepared from $1 \times 10^7$ cells of each cell line by using RNeasy Protect Mini Kit (manufactured by QIAGEN) according to the manufacture's instructions, and a single-stranded cDNA was synthesized from 5 μg of each RNA in a 20 μl of a reaction mixture using RT-PCR (manufactured by GIBCO BRL) according to the manufacture's instructions.

Next, in order to amplify the CHO cell-derived cDNA, PCR was carried out by the following method. Specifically, 20 μl of a reaction mixture [1 × Ex Taq buffer (manufactured by Takara Shuzo), 0.2 mM dNTPs, 0.5 unit of Ex Taq polymerase (manufactured by Takara Shuzo) and 0.5 μM of two synthetic DNA primers] containing 0.5 μl of the CHO cell-derived single-stranded cDNA as the template was prepared. In this case, combinations of SEQ ID NO:32 with SEQ ID NO:33, SEQ ID NO:34 with SEQ ID NO:33, SEQ ID NO:32 with SEQ ID NO:35 and SEQ ID NO:32 with SEQ ID NO:36 were used as the synthetic DNA primers. The reaction was carried out by using DNA Thermal Cycler 480 (manufactured by Perkin Elmer) by heating at 94°C for 5 minutes and subsequent 30 cycles of heating at 94°C for 1 minute and 68°C for 2 minutes as one cycle.

The PCR reaction mixture was subjected to agarose electrophoresis for fractionation to find that a DNA fragment of about 1.2 kbp was amplified in the PCR product when synthetic DNA primers of SEQ ID NOs:32 and 33 were used, a fragment of about 1.1 kbp was amplified in the PCR product when synthetic DNA primers of SEQ ID NOs:33 and 34 were used, a fragment of about 350 bp was amplified in the PCR product when synthetic DNA primers of SEQ ID NOs:32 and 35 were used and a fragment of about 1 kbp was amplified in the PCR product when synthetic DNA primers of SEQ ID NOs:32 and 36 were used. The DNA fragments were recovered by using Gene Clean II Kit (manufactured by BIO101) in accordance with the manufacture's instructions. The recovered DNA fragments were ligated to a pT7Blue(R) vector (manufactured by Novagen) using DNA Ligation Kit (manufactured by Takara Shuzo), and *E. coli* DH (manufactured by Toyobo) was transformed by using the obtained recombinant plasmid DNA samples to thereby obtain plasmids 22-8 (having a DNA fragment of about 1.2 kbp amplified from synthetic DNA primers of SEQ ID NO: 32 and SEQ ID NO:33), 23-3 (having a DNA fragment of about 1.1 kbp amplified from synthetic DNA primers of SEQ ID NO:34 and SEQ ID NO:33), 31-5 (a DNA fragment of about 350 bp amplified from synthetic DNA primers of SEQ ID NO:32 and SEQ ID NO:35) and 34-2 (having a DNA fragment of about I kbp amplified from synthetic DNA primers of SEQ ID NO:32 and SEQ ID NO:36). The cDNA sequence of CHO cell-derived GMD contained in these plasmids was determined by using a DNA sequencer ABI PRISM 377 (manufactured by Perkin Elmer) (since a sequence of 28 bases in downstream of the initiation codon methionine in the 5'-terminal side and a sequence of 27 bases in upstream of the termination codon in the 3'-terminal side are originated from synthetic oligo DNA sequences, they are mouse GMD cDNA sequences) in the usual method.

In addition, the following steps were carried out in order to prepare a plasmid in which cDNA fragments of the CHO cell-derived GMD contained in the plasmids 22-8 and 34-2 are combined. After 1 μg of the plasmid 22-8 was allowed to react with a restriction enzyme *Eco*RI (manufactured by Takara Shuzo) at 37°C for 16 hours, the digest was subjected to agarose electrophoresis, and then a DNA fragment of about 4 kbp was recovered by using Gene Clean II Kit (manufactured by BIO101) in accordance with the manufacture's instructions. After 2 μg of the plasmid 34-2 was allowed to react with a restriction enzyme *Eco*RI at 37°C for 16 hours, the digest was subjected to agarose electrophoresis and then a DNA fragment of about 150 bp was recovered by using Gene Clean II Kit (manufactured by BIO101) in accordance with the manufacture's instructions. The recovered DNA fragments were respectively subjected to terminal dephosphorylation by using Calf Intestine Alkaline Phosphatase (manufactured by Takara Shuzo) and then ligated by using DNA Ligation Kit (manufactured by Takara Shuzo), and *E. coli* DH5α (manufactured by Toyobo) was transformed by using the obtained recombinant plasmid DNA to obtain a plasmid CHO-GMD (Fig. 20).

(2) Determination of the 5'-terminal sequence of CHO cell-derived GMD cDNA

A 24 mer primer having the nucleotide sequence represented by SEQ ID NO:37 was prepared from 5'-terminal side non-coding region nucleotide sequences of CHO cell-derived GMD cDNA, and a 32 mer primer having the nucleotide sequence represented by SEQ ID NO:38 from CHO cell-derived GMD cDNA sequence was prepared, and PCR was carried out by the following method to amplify cDNA. Then, 20 μl of a reaction mixture [1 × Ex Taq buffer (manufactured by Takara Shuzo), 0.2 mM dNTPs, 0.5 unit of Ex Taq polymerase (manufactured by Takara Shuzo) and 0.5 μM of the synthetic DNA primers of SEQ ID NO:37 and SEQ ID NO:38] containing 0.5 μl of the single-stranded cDNA as the template derived from CHO cell was prepared, and the reaction was carried out therein by using DNA Thermal Cycler 480 (manufactured by Perkin Elmer) by heating at 94°C for 5 minutes, subsequent 20 cycles of heating at 94°C for I minute, 55°C for 1 minute and 72°C for 2 minutes as one cycle and further 18 cycles of heating at 94°C for 1 minute and 68°C for 2 minutes as one cycle. After fractionation of the PCR reaction mixture by agarose electrophoresis,

a DNA fragment of about 300 bp was recovered by using Gene Clean II Kit (manufactured by BIO101) in accordance with the manufacture's instructions. The recovered DNA fragment was ligated to a pT7Blue(R) vector (manufactured by Novagen) using DNA Ligation Kit (manufactured by Takara Shuzo), and *E. coli* DH5α (manufactured by Toyobo) was transformed by using the obtained recombinant plasmid DNA samples to thereby obtain a plasmid 5'GMD. By using DNA Sequencer 377 (manufactured by Applied Biosystems), the nucleotide sequence of 28 bases in downstream of the initiation methionine of CHO cell-derived GMD cDNA contained in the plasmid was determined.

(3) Determination of the 3'-terminal sequence of CHO cell-derived GMD cDNA

In order to obtain the 3'-terminal cDNA sequence of a CHO cell-derived GMD, RACE method was carried out by the following method. A single-stranded cDNA for 3' RACE was prepared from the CHO cell-derived RNA by using SMART™ RACE cDNA Amplification Kit (manufactured by CLONTECH) in accordance with the manufacture's instructions. In this case, PowerScript™ Reverse Transcriptase (manufactured by CLONTECH) was used as the reverse transcriptase. The single-stranded cDNA after the preparation was diluted 10 folds with the Tricin-EDTA buffer attached to the kit and used as the template of PCR.

Next, 20 μl of a reaction mixture [ExTaq buffer (manufactured by Takara Shuzo), 0.2 mM dNTPs, 0.5 unit of EX Taq polymerase (manufactured by Takara Shuzo), 0.5 μM of the 24 mer synthetic DNA primer shown in SEQ ID NO:39 [prepared on the base of cDNA sequence of the CHO cell-derived GMD determined in item (1)] and 1 × concentration of Universal Primer Mix (attached to SMART™ RACE cDNA Amplification Kit; manufactured by CLONTECH] containing 1 μl of the cDNA for 3' RACE as the template was prepared, and PCR was carried out by using DNA Thermal Cycler 480 (manufactured by Perkin Elmer) by heating at 94°C for 5 minutes and subsequent 30 cycles of heating at 94°C for 1 minute and 68°C for 2 minutes as one cycle.

After completion of the reaction, 1 μl of the PCR reaction mixture was diluted 20 folds with Tricin-EDTA buffer (manufactured by CLONTECH). Then, 20 μl of a reaction mixture [ExTaq buffer (manufactured by Takara Shuzo), 0.2 mM dNTPs, 0.5 unit of EX Taq polymerase (manufactured by Takara Shuzo), 0.5 μM of the 25 mer synthetic DNA primer shown in SEQ ID NO:40 [prepared on the base of the cDNA sequence of CHO cell-derived GMD determined in item (1)] and 0.5 μM of Nested Universal Primer (attached to SMART™ RACE cDNA Amplification Kit; manufactured by CLONTECH) containing 1 μl of the 20 folds-diluted aqueous solution as the template] was prepared, and the reaction was carried out by using DNA Thermal Cycler 480 (manufactured by Perkin Elmer) by heating at 94°C for 5 minutes and subsequent 30 cycles at 94°C for 1 minute and 68°C for 2 minutes as one cycle.

After completion of the reaction, the PCR reaction mixture was subjected to agarose electrophoresis for fractionation and then a DNA fragment of about 700 bp was recovered by using Gene Clean II Kit (manufactured by BI0101) in accordance with the manufacture's instructions. The recovered DNA fragment was ligated to a pT7Blue(R) vector (manufactured by Novagen) by using DNA Ligation Kit (manufactured by Takara Shuzo), and *E. coli* DH5α (manufactured by Toyobo) was transformed by using the obtained recombinant plasmid DNA to thereby obtain a plasmid 3'GMD. By using DNA Sequencer 377 (manufactured by Applied Biosystems), the nucleotide sequences of 27 bases in upstream of the termination codon and 415 bases in the non-coding region in the 3'-terminal of CHO cell-derived GMD cDNA contained in the plasmid were determined.

The full length cDNA sequence of the CHO-derived GMD gene determined in items (1), (2) and (3) and the corresponding amino acid sequence are shown in SEQ ID NOs:41 and 61, respectively.

2. Determination of a genomic sequence containing CHO/DG44-derived cell GMD gene

A 25 mer primer having the nucleotide sequence represented by SEQ ID NO:56 was prepared from the cDNA sequence of mouse GMD determined in item 1 of Example 8. Next, a CHO cell-derived genomic DNA was obtained by the following method. CHO/DG44 cell was suspended in IMDM-dFBS(10)-HT(1) medium [IMDM-dFBS(10) medium comprising 1 × concentration of HT supplement (manufactured by Invitrogen)) at a density of 3 × $10^5$ cells/ml, and the suspension was dispensed into a 6 well flat bottom tissue culture plate for adhesion cell (manufactured by Greiner) at 2 ml/well. After culturing them at 37°C in a 5% $CO_2$ incubator until the cells became confluent on the plate, genomic DNA was prepared from the cells on the plate by a known method [*Nucleic Acids Research,* 3, 2303 (1976)] and dissolved overnight in 150 μl of TE-RNase buffer (pH 8.0) (10 mmol/l Tris-HCl, 1 mmol/l EDTA, 200 μg/ml RNase A).

Next, 100 ng of the obtained CHO/DG44 cell-derived genomic DNA and 20 μl of a reaction mixture [1 × Ex Taq buffer (manufactured by Takara Shuzo), 0.2 mM dNTPs, 0.5 unit of EX Taq polymerase (manufactured by Takara Shuzo) and 0.5 μM synthetic DNA primers of SEQ ID NO:35 and SEQ ID NO:56] were prepared, and PCR was carried out by using DNA Thermal Cycler 480 (manufactured by Perkin Elmer) by heating at 94°C for 5 minutes and subsequent 30 cycles of heating at 94°C for 1 minute and 68°C for 2 minutes as one cycle. After completion of the reaction, the PCR reaction mixture was subjected to agarose electrophoresis for fractionation and then a DNA fragment of about 100 bp was recovered by using Gene Clean II Kit (manufactured by BIO101) in accordance with the manufacture's

instructions. The recovered DNA fragment was ligated to a pT7Blue(R) vector (manufactured by Novagen) by using DNA Ligation Kit (manufactured by Takara Shuzo), and *E. coli* DH5α (manufactured by Toyobo) was transformed by using the obtained recombinant plasmid DNA, thereby obtaining a plasmid ex3. By using DNA Sequencer 377 (manufactured by Applied Biosystems), the nucleotide sequence of CHO cell-derived genomic DNA contained in the plasmid was determined. The determined nucleotide sequence is shown in SEQ ID NO:57.

Next, a 25 mer primer having the nucleotide sequence represented by SEQ ID NO:58 and a 25 mer primer having the nucleotide sequence represented by SEQ ID NO:59 were prepared on the base of the cDNA sequence of CHO cell-derived GMD determined in item 1 of Example 8. Next, 100 ng of the CHO/DG44-derived genomic DNA and 20 μl of a reaction mixture [1× Ex Taq buffer (manufactured by Takara Shuzo), 0.2 mM dNTPs, 0.5 unit of EX Taq polymerase (manufactured by Takara Shuzo) and 0.5 μM synthetic DNA primers of SEQ ID NO:58 and SEQ ID NO: 59] were prepared, and PCR was carried out by using DNA Thermal Cycler 480 (manufactured by Perkin Elmer) by heating at 94°C for 5 minutes and subsequent 30 cycles of heating at 94°C for 1 minute and 68°C for 2 minutes as one cycle.

After completion of the reaction, the PCR reaction mixture was subjected to agarose electrophoresis for fractionation and then a DNA fragment of about 200 bp was recovered by using Gene Clean II Kit (manufactured by BIO101) in accordance with the manufacture's instructions. The recovered DNA fragment was ligated to a pT7Blue(R) vector (manufactured by Novagen) by using DNA Ligation Kit (manufactured by Takara Shuzo), and *E. coli* DH5α (manufactured by Toyobo) was transformed by using the obtained recombinant plasmid DNA, thereby obtaining a plasmid ex4. By using DNA Sequencer 377 (manufactured by Applied Biosystems), the nucleotide sequence of CHO cell-derived genomic DNA contained in the obtained plasmid was determined. The determined nucleotide sequence is shown in SEQ ID NO:60.

Example 9

Preparation of various CHO cell-derived genes encoding enzymes relating to the sugar chain synthesis:

1. Determination of CHO cell-derived FX cDNA sequence

(1) Extraction of total RNA derived from CHO/DG44 cell

CHO/DG44 cells were suspended in IMDM medium containing 10% fetal bovine serum (manufactured by Life Technologies) and 1× concentration HT supplement (manufactured by Life Technologies), and 15 ml of the suspension was inoculated into a T75 tissue culture flask for adhesion cell culture (manufactured by Greiner) at a density of 2 × $10^5$ cells/ml. On the second day after culturing at 37°C in a 5% $CO_2$ incubator, 1 × $10^7$ cells were recovered and a total RNA was extracted therefrom by using RNAeasy (manufactured by QIAGEN) in accordance with the manufacture's instructions.

(2) Preparation of CHO-DG44 cell-derived single-stranded cDNA

The total RNA prepared in item (1) was dissolved in 45 μl of sterile water, and 1 μl of RQ1 RNase-Free DNase (manufactured by Promega), 5 μl of the attached 10×DNase buffer and 0.5 μl of RNasin Ribonuclease Inhibitor (manufactured by Promega) were added thereto, followed by reaction at 37°C for 30 minutes to degrade genomic DNA contaminated in the sample. After the reaction, the total RNA was purified again by using RNAeasy (manufactured by QIAGEN) and dissolved in 50 μl of sterile water.

In a 20 μl of reaction mixture using oligo(dT) as a primer, single-stranded cDNA was synthesized from 3 μg of the obtained total RNA samples by carrying out reverse transcription reaction using SUPERSCRIPT™ Preamplification System for First Strand cDNA Synthesis (manufactured by Life Technologies) in accordance with the manufacture's instructions. A 50 folds-diluted aqueous solution of the reaction mixture was used in the cloning of GFPP and FX. This was stored at -80°C until use.

(3) Preparation of a cDNA partial fragment of Chinese hamster-derived FX

A cDNA partial fragment derived from Chinese hamster-derived FX was prepared by the following procedure. First, primers (shown in SEQ ID NOs:42 and 43) specific for nucleotide sequences common to a human FX cDNA (Genebank Accession No. U58766) and a mouse Fx cDNA (Genebank Accession No. M30127), were designed.

Next, 25 μl of a reaction mixture [ExTaq buffer (manufactured by Takara Shuzo), 0.2 mM dNTPs and 0.5 μmol/l, gene-specific primers (SEQ ID NOs:42 and 43)] containing 1 μl of the CHO/DG44-derived single-stranded cDNA prepared in item (2) was prepared, and polymerase chain reaction (PCR) was carried out by using a DNA polymerase

ExTaq (manufactured by Takara Shuzo). The PCR was carried out by heating at 94°C for 5 minutes, subsequent 30 cycles of heating at 94°C for 1 minute, 58°C for 2 minutes and 72°C for 3 minutes as one cycle, and final heating at 72°C for 10 minutes.

After the PCR, the reaction mixture was subjected to 2% agarose gel electrophoresis, and a specific amplified fragment of 301 bp was purified by using QiaexII Gel Extraction Kit (manufactured by QIAGEN) and eluted with 20 μl of sterile water (hereinafter, the method was used for the purification of DNA fragments from agarose gel). Into a plasmid pCR2.1, 4 μl of the amplified fragment was inserted by TOPO TA Cloning Kit (manufactured by Invitrogen) in accordance with the manufacture's instructions attached thereto, and *E. coli* DH5α was transformed with the reaction mixture by the method of Cohen *et al*. [*Proc. Natl. Acad. Sci. USA,* 69, 2110 (1972)] (hereinafter, the method was used for the transformation of *E. coli).* Plasmid DNA was isolated in accordance with a known method *[Nucleic Acids Research*, 7, 1513 (1979)] (hereinafter, the method was used for the isolation of plasmid) from the obtained several kanamycin-resistant colonies to obtain 2 clones into which cDNA partial fragments of Fx were respectively inserted. They are referred to as pCRFX clone 8 and pCRFX clone 12.

The nucleotide sequence of the cDNA inserted into each of the FX clone 8 and FX clone 12 was determined by using DNA Sequencer 377 (manufactured by Applied Biosystems) and BigDye Terminator Cycle Sequencing FS Ready Reaction kit (manufactured by Applied Biosystems) in accordance with the method of the manufacture's instructions. It was confirmed that each of the inserted cDNA whose sequence was determined encodes open reading frame (ORF) partial sequence of the Chinese hamster-derived FX.

(4) Synthesis of a single-stranded cDNA for RACE

Single-stranded cDNA samples for 5' and 3' RACE were prepared from the CHO/DG44 total RNA extracted in item (1) by using SMART™ RACE cDNA Amplification Kit (manufactured by CLONTECH) in accordance with the manufacture's instructions. As the reverse transcriptase, PowerScript™ Reverse Transcriptase (manufactured by CLONTECH) was used. Each of the prepared single-stranded cDNA was diluted 10 folds with the Tricin-EDTA buffer attached to the kit and used as the template of PCR.

Based on the partial sequence of Chinese hamster-derived Fx determined in item (3), primers FXGSP1-1 (SEQ ID NO:44) and FXGSP1-2 (SEQ ID NO:45) for the Chinese hamster FX-specific 5' RACE and primers FXGSP2-1 (SEQ ID NO:46) and FXGSP2-2 (SEQ ID NO:47) for the Chinese hamster FX-specific 3' RACE were designed.

Next, polymerase chain reaction (PCR) was carried out by using Advantage2 PCR Kit (manufactured by CLONTECH), by preparing 50 μl of a reaction mixture [Advantage2 PCR buffer (manufactured by CLONTECH), 0.2 mM dNTPs, 0.2 μmol/l Chinese hamster FX-specific primers for RACE and 1× concentration of common primers (manufactured by CLONTECH)] containing 1 μl of the CHO/DG44-derived single-stranded cDNA for RACE prepared in item (4).

The PCR was carried out by repeating 20 cycles of heating at 94°C for 5 seconds, 68°C for 10 seconds and 72°C for 2 minutes as one cycle.

After completion of the reaction, 1 μl of the reaction mixture was diluted 50-folds with the Tricin-EDTA buffer, and 1 μl of the diluted solution was used as a template, the reaction mixture was again prepared, and the PCR was carried out under the same conditions. The combination of primers used in the first and second PCRs and the length of amplified DNA fragments by the PCRs are shown in Table 2.

Table 2

| Combination of primers used in Chinese hamster-derived FX cDNA RACE PCR and the size of PCR products | | | |
|---|---|---|---|
| 5' RACE | FX-specific primers | Common primers | PCR-amplified product size |
| First<br>Second | FXGSP1-1<br>FXGSP1-2 | UPM (Universal primer mix)<br>NUP (Nested Universal primer) | <br>300 bp |
| | | | |
| 3' RACE | FX-specific primers | Common primers | PCR-amplified product size |
| First<br>Second | FXGSP2-1<br>FXGSP2-2 | UPM (Universal primer mix)<br>NUP (Nested Universal primer) | <br>1,100 bp |

After the PCR, the reaction mixture was subjected to 1% agarose gel electrophoresis, and the specific amplified fragment of interest was purified by using QiaexII Gel Extraction Kit (manufactured by QIAGEN) and eluted with 20 μl of sterile water. Into a plasmid pCR2.1, 4 μl of the amplified fragment was inserted, and *E. coli* DH5α was transformed by using the reaction mixture in accordance with the manufacture's instructions attached to TOPO TA Cloning

Kit (manufactured by Invitrogen).

Plasmid DNAs were isolated from the appeared several kanamycin-resistant colonies, and 6 cDNA clones containing Chinese hamster FX 5' region were obtained. They are referred to as FX5' clone 25, FX5' clone 26, FX5' clone 27, FX5' clone 28, FX5' clone 31 and FX5' clone 32.

In the same manner, 5 cDNA clones containing Chinese hamster FX 3' region were obtained. These FX3' clones are referred to as FX3' clone 1, FX3' clone 3, FX3' clone 6, FX3' clone 8 and FX3' clone 9.

The nucleotide sequence constituting the cDNA of each of the clones obtained by the 5' and 3' RACE was determined by using DNA Sequencer 377 (manufactured by Applied Biosystems) in accordance with the method described in the manufacture's instructions. By comparing the cDNA nucleotide sequences determined by the method, reading errors of nucleotide in PCR were excluded, and the full length nucleotide sequence of Chinese hamster-derived FX cDNA was determined. The determined sequence is represented by SEQ ID NO:48. ORF of SEQ ID NO:48 corresponds to nucleotides at positions 95 to 1060, and the amino acid sequence corresponding to nucleotides at positions 95 to 1057 excluding the termination codon is represented by SEQ ID NO:62.

2. Determination of a GFPP cDNA sequence of CHO cell

(1) Preparation of a cDNA partial fragment of Chinese hamster-derived GFPP

A cDNA partial fragment of Chinese hamster GFPP was prepared by the following procedure.

First, a nucleotide sequence of a human-derived GFPP cDNA (Genebank Accession No. AF017445), mouse EST sequences having high homology with the nucleotide sequence (Genebank Accession Nos. AI467195, AA422658, BE304325 and AI466474) and rat EST sequences (Genebank Accession Nos. BF546372, AI058400 and AW 144783), registered at public data bases, were compared, and primers of GFPP FW9 and GFPP RV9 (SEQ ID NOs:49 and 50), specific for rat GFPP were designed on a highly preserved region among these three species.

Next, polymerase chain reaction (PCR) was carried out by using a DNA polymerase ExTaq (manufactured by Takara Shuzo), by preparing 25 µl of a reaction mixture [ExTaq buffer (manufactured by Takara Shuzo), 0.2 mM dNTPs and 0.5 µmol/l GFPP-specific primers GFPP FW9 and GFPP RV9 (SEQ ID NOs:49 and 50)] containing 1 µl of the CHO/DG44-derived single-stranded cDNA prepared in item 1(2). The PCR was carried out by heating at 94°C for 5 minutes, subsequent 30 cycles of heating at 94°C for 1 minute, 58°C for 2 minutes and 72°C for 3 minutes as one cycle, and final heating at 72°C for 10 minutes.

After the PCR, the reaction mixture was subjected to 2% agarose gel electrophoresis, and a specific amplified fragment of 1.4 Kbp was purified using QuiaexII Gel Extraction Kit (manufactured by QIAGEN) and eluted with 20 µl of sterile water. Into a plasmid pCR2.1, 4 µl of the amplified fragment was inserted to insert in accordance with the manufacture's instructions attached to TOPO TA Cloning Kit (manufactured by Invitrogen), and *E. coli* DH5α was transformed by using the reaction mixture.

Plasmid DNAs were isolated from the appeared several kanamycin-resistant colonies, and 3 clones transfected with GFPP cDNA partial fragments were obtained. They are referred to as GFPP clone 8, GFPP clone 11 and GFPP clone 12.

The nucleotide sequence of the cDNA inserted into each of the GFPP clone 8, GFPP clone 11 and GFPP clone 12 was determined by using DNA Sequencer 377 (manufactured by Applied Biosystems) and BigDye Terminator Cycle Sequencing FS Ready Reaction kit (manufactured by Applied Biosystems) in accordance with the method described in the manufacture's instructions. It was confirmed that the inserted cDNA whose sequence was determined encodes a partial sequence of the open reading frame (ORF) of the Chinese hamster-derived GFPP.

(2) Determination of full length cDNA of Chinese hamster-derived GFPP by RACE method

Based on the Chinese hamster FX partial sequence determined in item 2(1), primers GFPP GSP1-1 (SEQ ID NO:52) and GFPP GSP1-2 (SEQ ID NO:53) for the Chinese hamster FX-specific 5' RACE and primers GFPP GSP2-1 (SEQ ID NO:54) and GFPP GSP2-2 (SEQ ID NO:55) for the Chinese hamster GFPP-specific 3' RACE were designed.

Next, polymerase chain reaction (PCR) was carried out by using Advantage2 PCR Kit (manufactured by CLONTECH), by preparing 50 µl of a reaction mixture [Advantage2 PCR buffer (manufactured by CLONTECH), 0.2 mM dNTPs, 0.2 µmol/l Chinese hamster GFPP-specific primers for RACE and 1× concentration of common primers (manufactured by CLONTECH)] containing 1 µl of the CHO/DG44-derived single-stranded cDNA for RACE prepared in item (4).

The PCR was carried out by repeating 20 cycles of heating at 94°C for 5 seconds, 68°C for 10 seconds and 72°C for 2 minutes as one cycle.

After completion of the reaction, 1 µl of the reaction mixture was diluted 50 folds with the Tricin-EDTA buffer.

By using 1 µl of the diluted solution as a template, the reaction mixture was again prepared and the PCR was carried out under the same conditions. The combination of primers used in the first and second PCRs and the size of amplified DNA fragments by the PCRs are shown in Table 3.

Table 3

| Combination of primers used in Chinese hamster-derived GFPP cDNA RACE PCR and the size of PCR products | | | |
|---|---|---|---|
| 5'RACE | GFPP-specific primers | Common primers | PCR-amplified product size |
| First<br>Second | GFPPGSP1-1<br>GFPPGSP1-2 | UPM (Universal primer mix)<br>NUP (Nested Universal primer) | 1,100 bp |
| | | | |
| 3'RACE | GFPP-specific primers | Common primers Common primers | PCR-amplified product size |
| First<br>Second | GFPPGSP2-1<br>GFPPGSP2-2 | UPM (Universal primer mix)<br>NUP (Nested Universal primer) | 1,400 bp |

After the PCR, the reaction mixture was subjected to 1% agarose gel electrophoresis, and the specific amplified fragment of interest was purified using QiaexII Gel Extraction Kit (manufactured by QIAGEN) and eluted with 20 µl of sterile water. Into a plasmid pCR2.1, 4 µl of the amplified fragment was inserted and *E. coli* DH5α was transformed with the reaction mixture in accordance with the manufacture's instructions attached to TOPO TA Cloning Kit (manufactured by Invitrogen).

Plasmid DNAs were isolated from the appeared several kanamycin-resistant colonies to obtain 4 cDNA clones containing Chinese hamster GFPP 5' region. They are referred to as GFPP5' clone 1, GFPP5' clone 2, GFPP5' clone 3 and GFPP5' clone 4.

In the same manner, 3 cDNA clones containing Chinese hamster GFPP 3' region were obtained. They are referred to as GFPP3' clone 10, GFPP3' clone 16 and GFPP3' clone 20.

The nucleotide sequence of the cDNA of each of the clones obtained by the 5' and 3' RACE was determined by using DNA Sequencer 377 (manufactured by Applied Biosystems) in accordance with the method described in the manufacture's instructions. By comparing the cDNA nucleotide sequences after the nucleotide sequence determination, reading errors of bases in PCR were excluded and the full length nucleotide sequence of Chinese hamster GFPP cDNA was determined. The determined sequence is shown in SEQ ID NO: 51. ORF of SEQ ID NO:51 corresponds to nucleotides at positions 27 to 1799, and the amino acid sequence corresponding to nucleotides at positions 27 to 1796 excluding the termination codon is represented by SEQ ID NO:63.

## Example 10

### Evaluation of activity of anti-CD20 chimeric antibodies having a different ratio of antibody molecules to which an α1,6-fucose-free sugar chain is bound

### 1. Preparation of anti-CD20 chimeric antibodies having a different ratio of antibody molecules to which an α1,6-fucose-free sugar chain is bound

KM3065 purified in item 3 of Example 1 was mixed with CHO produced-Rituxan™ at a ratio of KM3065 : Rituxan™ =24 : 66, 34 : 56 or 44 : 46. Sugar chain analysis of these samples was carried out in accordance with the method of Example 3. Ratios of the antibody molecules to which an α1,6-fucose-free sugar chain was bound were 26%, 35% and 44%, respectively. Hereinafter, these samples are called anti-CD20 chimeric antibody (26%), anti-CD20 chimeric antibody (35%) and anti-CD20 chimeric antibody (44%). Results of the sugar chain analysis of each sample are shown in Fig. 21.

### 2. Evaluation of binding activity to CD20-expressing cell line (immunofluorescent method)

Binding activities of a total of five antibodies, including the 3 anti-CD20 chimeric antibodies having a different ratio of sugar chain of antibody molecules to which an α1,6-fucose-free sugar chain is bound, prepared in item 1 of Example 10, and KM3065 and Rituxan™ whose sugar chain analysis was carried out in Example 3 (referred to as "anti-CD20 chimeric antibody (96%)" and "anti-CD20 chimeric antibody (6%)", respectively), were measured by the immunofluorescent method described in item 1 of Example 2. As shown in Fig. 22, all of these antibodies showed almost the same binding activity to the CD20-positive Raji cell (JCRB 9012) at an antibody concentration of 0.016 to

2 μg/ml, and it was found that the ratio of sugar chain of antibody molecules to which an α1,6-fucose-free sugar chain is bound does not have influence on the antigen-binding activity of antibodies.

3. Evaluation of cytotoxic activity to CD20-expressing cell line ($^{51}Cr$ release method)

The ADCC activity against a CD20-positive human B lymphoid cell line WIL2-S (ATCC CRL 8885) was measured as follows using effector cells collected from a healthy donor A.

(1) Preparation of target cell suspension

After $2\times10^6$ cells of the WIL2-S cell were prepared, the cells were isotope-labeled by adding 3.7 MBq equivalents of a radioactive substance $Na_2{}^{51}CrO_4$ and carrying out the reaction at 37°C for 1 hour. After the reaction, the cells were washed three times by repeating their suspension in PRMI 1640-FCS(10) medium and subsequent centrifugation, re-suspended in the medium and then allowed to stand at 4°C for 30 minutes in ice for spontaneous dissociation of the radioactive substance. After centrifugation, the cells were adjusted to a density of $2\times10^5$ cells/ml by adding 10 ml of the medium and used as the target cell suspension.

(2) Preparation of human effector cell suspension

After 50 ml of peripheral blood was collected from a health person, 0.5 ml of heparin sodium (manufactured by Shimizu Pharmaceutical) was added thereto, followed by gently mixing. The mixture was centrifuged (800 × g, 20 minutes) using Lymphoprep (manufactured by AXIS SHIELD) in accordance with the manufacture's instructions attached thereto to separate a mononuclear leukocyte layer. After washing with a medium three times by centrifugation (1,400 rpm, 5 minutes), the cells were re-suspended by using the medium to a density of $2\times10^6$ cells/ml and used as the human effector cell suspension.

(3) Measurement of ADCC activity

The target cell suspension prepared in (1) (50 μl) was dispensed into wells of a 96-well U-bottom plate (manufactured by Falcon) ($1\times10^4$ cells/well). Next, 100 μl of the human effector cell suspension prepared in (2) was dispensed ($2\times10^5$ cells/well, the ratio of human effector cells to target cells becomes 20 : 1). Subsequently, various anti-CD20 chimeric antibodies having a different ratio of α1,6-fucose-free sugar chain group was bound were added thereto to give a respective final concentration of 0.001 to 1 μg/ml and then allowed to react at 37°C for 4 hours. After the reaction, the plate was subjected to centrifugation and the amount of $^{51}Cr$ in the supernatant was measured using a γ-counter. The amount of the spontaneously released $^{51}Cr$ was calculated by carrying out the same procedure using the medium alone instead of the human effector cell suspension and antibody solution and measuring amount of $^{51}Cr$ in the supernatant. The amount of the total released $^{51}Cr$ was calculated by carrying out the same procedure by using 1 mol/l hydrochloric acid solution instead of the antibody solution and human effector cell suspension and measuring amount of $^{51}Cr$ in the supernatant. The cytotoxic activity (%) was calculated based on the following equation.

$$\text{Cytotoxic activity (\%)} = \frac{{}^{51}\text{Cr in sample supernatant - spontaneously released } {}^{51}\text{Cr}}{\text{total released } {}^{51}\text{Cr - spontaneously released } {}^{51}\text{Cr}} \times 10C$$

Fig. 23 shows a result of the measurement of ADCC activity by using effector cells of a healthy donor A at various concentrations (from 0.001 to 1 μg/ml) of the anti-CD20 chimeric antibodies having a different ratio of antibody molecules α1,6-fucose-free sugar chain group. As shown in Fig. 23, the ADCC activity of anti-CD20 chimeric antibodies showed a tendency to increase at each antibody concentration, as the ratio of antibody molecules to which an α1,6-fucose-free sugar chain is bound increased. When the antibody concentration is low, the ADCC activity is decreased. At an antibody concentration of 0.01 μg/ml, antibodies having 26%, 35%, 44% and 96% of the α1,6-fucose-not-bound sugar chain showed almost the same high ADCC activity, but the antibody having 6% of the α1,6-fucose-not-bound sugar chain showed low ADCC activity.

4. Evaluation of ADCC activity to CD20-expressing cell line (LDH method)

The ADCC activity to Raji cell was evaluated by the LDH (lactate dehydrogenase) activity measuring method described in item 2 of Example 2 using effector cells collected from a healthy donor B. The ratio of the effector cell to the target cell was 20 : 1, the final antibody concentration was 0.0001 to 1 μg/ml, the reaction was carried out at a total

volume of 200 μL at 37°C for 4 hours, and then the measurement was carried out in accordance with the item 2 of Example 2. Fig. 24 shows a result of the measurement of ADCC activity using effector cells of a healthy donor B at various concentrations (from 0.0001 to 1 μg/ml) of the anti-CD20 chimeric antibodies having a different ratio of antibody molecules to which an α1,6-fucose-free sugar chain is bound. As shown in Fig. 24, the ADCC activity of anti-CD20 chimeric antibodies showed a tendency to increase at each antibody concentration, as the ratio of antibody molecules to which an α1,6-fucose-free sugar chain is bound increased. When the antibody concentration is low, the ADCC activity is decreased. At an antibody concentration of 0.01 μg/ml, antibodies having 26%, 35%, 44% and 96% of the α1,6-fucose-not-bound sugar chain showed high ADCC activity, but the antibody having 6% of the α1,6-fucose-not-bound sugar chain showed low ADCC activity.

The results of Fig. 23 and Fig. 24 show that the ADCC activity increases in response to the ratio of antibody molecules to which an α1,6-fucose-free sugar chain is bound and that an antibody composition having about 20% or more of the ratio of antibody molecules to which an α1,6-fucose-free sugar chain is bound has sufficiently high ADCC activity, and the same results were obtained when the donor of human effector cells and the target cell was changed.

Example 11

Activity evaluation of anti-CD20 chimeric antibodies having a different ratio of antibody molecules to which a sugar chain having bisecting GlcNAc is bound:

(1) Separation of an anti-CD20 chimeric antibody by lectin chromatography

By using a column immobilized with lectin which has the affinity for sugar chains having bisecting GlcNAc, the anti-CD20 chimeric antibody KM3065 purified in item 3 of Example 1 was separated.

A solution containing the purified anti-CD20 chimeric antibody KM3065 was applied to a lectin column (LA-PHA-$E_4$, 4.6 × 150 mm, manufactured by Hohnen Corp.). By using LC-6A manufactured by Shimadzu as the HPLC system, the lectin chromatography was carried out at a flow rate of 0.5 ml/min and at room temperature as the column temperature. The column was equilibrated with 50 mM Tris-sulfuric acid buffer (pH 8.0), and then a solution containing the purified KM3065 was injected and eluted by a linear gradient (35 minutes) of 0 M to 58 mM of potassium tetraborate ($K_2B_4O_7$, manufactured by Nakalai Tesque) in 50 mM Tris-sulfuric acid buffer (pH 8.0). Thereafter, the potassium tetraborate concentration was kept at 100 mM for 5 minutes, and then 50 mM Tris-sulfuric acid buffer (pH 8.0) was further passed through the column for 20 minutes to thereby separate the anti-CD20 chimeric antibody KM3065 into 4 fractions (fractions ① to ④ ) eluted during a period of 9 to 14 minutes, of 14 to 17 minutes, 17 to 22 minutes and 22 to 34 minutes (Fig. 25).

(2) Sugar chain analysis

Sugar chain analysis of the thus separated 4 fractions (fractions ① to ④ ) and the anti-CD20 chimeric antibody KM3065 before separation was carried out by the method described in Example 3. The PA-modified sugar chains were eluted during a period of 15 minutes to 45 minutes. When the ratio of the sugar chain having bisecting GlcNAc based on the total of peak area of each PA-modified sugar chain was calculated, the ratio of the sugar chain in the anti-CD20 chimeric antibody KM3065 before separation was 20%, whereas it was 0% in the fraction ① , 8% in the fraction ② , 33% in the fraction ③ and 45% in the fraction ④ (Fig. 26). The ratio of the antibody molecule to which an α1,6-fucose-free sugar chain was bound was the anti-CD20 chimeric antibody KM3065 before separation: 96%, fraction ① : 93%, fraction ② : 94%, fraction ③ : 92% and fraction ④ : 90%. Based on these results, it was confirmed that the ratio of antibody molecules to which an α1,6-fucose-free sugar chain was bound was almost uniform when calculated using a column immobilized with lectin which has the affinity for sugar chains having bisecting GlcNAc, and that anti-CD20 chimeric antibodies having different ratio of antibody molecules to which a sugar chain having bisecting GlcNAc is bound were prepared.

(3) Measurement of *in vitro* cytotoxic activity (ADCC activity)

Measurement of *in vitro* cytotoxic activity (ADCC activity) of the 4 fractions (fractions ① to ④ separated by lectin chromatography and the anti-CD20 chimeric antibody KM3065 before separation was carried out by the method described in item 2 of Example 2 (Fig. 27). As a result, the 4 fractions separated by lectin chromatography showed almost the same strength of ADCC activity as that of the anti-CD20 chimeric antibody KM3065 before separation. Since antibody molecules to which an α1,6-fucose-free sugar chain is bound have almost the same ratio of 90% to 96% according to the results of the above item, it was considered that influences of the antibody molecules to which an α1,6-fucose-free sugar chain is bound on the ADCC activity are almost the same. Strength of the ADCC activity

was not increased when the bisecting GlcNAc was further added to the antibody which has a high ratio of antibody molecule to which an $\alpha$1,6-fucose-free sugar chain is bound and also has high ADCC activity. That is, it was found that the antibody having a high binding ratio of $\alpha$1,6-fucose-free sugar chain has higher ADCC activity than the antibody having a high binding ratio of a sugar chain having $\alpha$1,6-fucose, independent of the presence or absence of bisecting GlcNAc.

## INDUSTRIAL APPLICABILITY

The present invention provides an antibody composition comprising an antibody molecule which specifically binds to CD20 and has complex N-glycoside-linked sugar chains bound to the Fc region, a cell or a transgenic non-human animal or plant which produces the antibody composition, a production process of the antibody composition, and a medicament comprising the antibody composition.

**Claims**

1. A cell which produces an antibody composition comprising an antibody molecule which specifically binds to CD20 and has complex *N*-glycoside-linked sugar chains bound to the Fc region, wherein among the total complex *N*-glycoside-linked sugar chains bound to the Fc region in the composition, the ratio of a sugar chain in which fucose is not bound to *N*-acetylglucosamine in the reducing end in the sugar chain is 20% or more.

2. The cell according to claim 1, wherein the sugar chain to which fucose is not bound is a complex *N*-glycoside-linked sugar chain in which 1-position of fucose is not bound to 6-position of *N*-acetylglucosamine in the reducing end through $\alpha$-bond.

3. The cell according to claim 1 or 2, wherein the activity of an enzyme relating to the synthesis of an intracellular sugar nucleotide, GDP-fucose, and/or the activity of an enzyme relating to the modification of a sugar chain in which 1-position of fucose is bound to 6-position of *N*-acetylglucosamine in the reducing end through $\alpha$-bond in the complex *N*-glycoside-linked sugar chain is decreased or deleted.

4. The cell according to claim 3, wherein the enzyme relating to the synthesis of an intracellular sugar nucleotide, GDP-fucose is an enzyme selected from the group consisting of the following (a), (b) and (c):

   (a) GMD (GDP-mannose 4,6-dehydratase);
   (b) Fx (GDP-keto-6-deoxymannose 3,5-epimerase, 4-reductase);
   (c) GFPP (GDP-beta-L-fucose pyrophosphorylase).

5. The cell according to claim 4, wherein the GMD is a protein encoded by a DNA of the following (a) or (b):

   (a) a DNA comprising the nucleotide sequence represented by SEQ ID NO:41;
   (b) a DNA which hybridizes with the DNA consisting of the nucleotide sequence represented by SEQ ID NO: 41 under stringent conditions and encodes a protein having GMD activity.

6. The cell according to claim 4, wherein the GMD is a protein selected from the group consisting of the following (a), (b) and (c):

   (a) a protein comprising the amino acid sequence represented by SEQ ID NO:61;
   (b) a protein which consists of an amino acid sequence in which at least one amino acid is deleted, substituted, inserted and/or added in the amino acid sequence represented by SEQ ID NO:61 and has GMD activity;
   (c) a protein which consists of an amino acid sequence having a homology of at least 80% with the amino acid sequence represented by SEQ ID NO:61 and has GMD activity.

7. The cell according to claim 4, wherein the Fx is a protein encoded by a DNA of the following (a) or (b):

   (a) a DNA comprising the nucleotide sequence represented by SEQ ID NO:48;
   (b) a DNA which hybridizes with the DNA consisting of the nucleotide sequence represented by SEQ ID NO: 48 under stringent conditions and encodes a protein having Fx activity.

8. The cell according to claim 4, wherein the Fx is a protein selected from the group consisting of the following (a), (b) and (c):

   (a) a protein comprising the amino acid sequence represented by SEQ ID NO:62;

(b) a protein which consists of an amino acid sequence in which at least one amino acid is deleted, substituted, inserted and/or added in the amino acid sequence represented by SEQ ID NO:62 and has Fx activity;
(c) a protein which consists of an amino acid sequence having a homology of at least 80% with the amino acid sequence represented by SEQ ID NO:62 and has Fx activity.

**9.** The cell according to claim 4, wherein the GFPP is a protein encoded by a DNA of the following (a) or (b):

(a) a DNA comprising the nucleotide sequence represented by SEQ ID NO:51;
(b) a DNA which hybridizes with the DNA consisting of the nucleotide sequence represented by SEQ ID NO: 51 under stringent conditions and encodes a protein having GFPP activity.

**10.** The cell according to claim 4, wherein the GFPP is a protein selected from the group consisting of the following (a), (b) and (c):

(a) a protein comprising the amino acid sequence represented by SEQ ID NO:63;
(b) a protein which consists of an amino acid sequence in which at least one amino acid is deleted, substituted, inserted and/or added in the amino acid sequence represented by SEQ ID NO:63 and has GFPP activity;
(c) a protein which consists of an amino acid sequence having a homology of at least 80% with the amino acid sequence represented by SEQ ID NO:63 and has GFPP activity.

**11.** The cell according to claim 3, wherein the enzyme relating to the modification of a sugar chain in which 1-position of fucose is bound to 6-position of the *N*-acetylglucosamine in the reducing end through α-bond in the complex *N*-glycoside-linked sugar chain is α1,6-fucosyltransferase.

**12.** The cell according to claim 11, wherein the α1,6-fucosyltransferase is a protein encoded by a DNA of the following (a), (b), (c) and (d):

(a) a DNA comprising the nucleotide sequence represented by SEQ ID NO:1;
(b) a DNA comprising the nucleotide sequence represented by SEQ ID NO:2;
(c) a DNA which hybridizes with the DNA consisting of the nucleotide sequence represented by SEQ ID NO: 1 under stringent conditions and encodes a protein having α1,6-fucosyltransferase activity;
(d) a DNA which hybridizes with the DNA consisting of the nucleotide sequence represented by SEQ ID NO: 2 under stringent conditions and encodes a protein having α1,6-fucosyltransferase activity.

**13.** The cell according to claim 11, wherein the α1,6-fucosyltransferase is a protein selected from the group consisting of the following (a), (b), (c), (d), (e) and (f):

(a) a protein comprising the amino acid sequence represented by SEQ ID NO:23;
(b) a protein comprising the amino acid sequence represented by SEQ ID NO:24;
(c) a protein which consists of an amino acid sequence in which at least one amino acid is deleted, substituted, inserted and/or added in the amino acid sequence represented by SEQ ID NO:23 and has α1,6-fucosyltransferase activity;
(d) a protein which consists of an amino acid sequence in which at least one amino acid is deleted, substituted, inserted and/or added in the amino acid sequence represented by SEQ ID NO:24 and has α1,6-fucosyltransferase activity;
(e) a protein which consists of an amino acid sequence having a homology of at least 80% with the amino acid sequence represented by SEQ ID NO:23 and has α1,6-fucosyltransferase activity.
(f) a protein which consists of an amino acid sequence having a homology of at least 80% with the amino acid sequence represented by SEQ ID NO:24 and has α1,6-fucosyltransferase activity.

**14.** The cell according to any one of claims 3 to 13, wherein the enzyme activity is decreased or deleted by a technique selected from the group consisting of the following (a), (b), (c), (d) and (e):

(a) a gene disruption technique targeting a gene encoding the enzyme;
(b) a technique for introducing a dominant negative mutant of a gene encoding the enzyme;
(c) a technique for introducing mutation into the enzyme;
(d) a technique for inhibiting transcription or translation of a gene encoding the enzyme;
(e) a technique for selecting a cell line resistant to a lectin which recognizes a sugar chain in which 1-position

of fucose is bound to 6-position of *N*-acetylglucosamine in the reducing end through α-bond in the complex *N*-glycoside-linked sugar chain.

**15.** The cell according to any one of claims 1 to 14, which is resistant to at least a lectin which recognizes a sugar chain in which 1-position of fucose is bound to 6-position of *N*-acetylglucosamine, in the reducing end through α-bond in the complex *N*-glycoside-linked sugar chain.

**16.** The cell according to any one of claims 1 to 15, which is a cell selected from the group consisting of the following (a) to (j):

(a) a CHO cell derived from a Chinese hamster ovary tissue;
(b) a rat myeloma cell line, YB2/3HL.P2.G11.16Ag.20 cell;
(c) a mouse myeloma cell line, NS0 cell;
(d) a mouse myeloma cell line, SP2/0-Agl4 cell;
(e) a BHK cell derived from a syrian hamster kidney tissue;
(f) a monkey COS cell;
(g) an antibody-producing hybridoma cell;
(h) a human leukemia cell line, Namalwa cell;
(i) an embryonic stem cell;
(j) a fertilized egg cell.

**17.** A transgenic non-human animal or plant or the progenies thereof into which an antibody molecule which specifically binds, to CD20 and has complex *N*-glycoside-linked sugar chains bound to the Fc region is introduced, which produces an antibody composition comprising the antibody molecule, wherein among the total complex *N*-glycoside-linked sugar chains bound to the Fc region in the composition, the ratio of a sugar chain in which fucose is not bound to *N*-acetylglucosamine in the reducing end in the sugar chain is 20% or more.

**18.** The transgenic non-human animal or plant or the progenies thereof according to claim 17, wherein the sugar chain in which fucose is not bound to *N*-acetylglucosamine is a sugar chain in which 1-position of the fucose is not bound to 6-position of *N*-acetylglucosamine in the reducing end through α-bond in the *N*-glycoside-linked sugar chain.

**19.** The transgenic non-human animal or plant or the progenies thereof according to claim 17 or 18, wherein a genome is modified such that the activity of an enzyme relating to the synthesis of an intracellular sugar nucleotide, GDP-fucose and/or the activity of an enzyme relating to the modification of a sugar chain in which 1-position of fucose is bound to 6-position of *N*-acetylglucosamine in the reducing end through α-bond in the *N*-glycoside-linked sugar chain is decreased.

**20.** The transgenic non-human animal or plant or the progenies thereof according to claim 17 or 18, wherein a gene encoding the enzyme relating to the synthesis of an intracellular sugar nucleotide, GDP-fucose, and/or a gene encoding the enzyme relating to the modification of a sugar chain in which 1-position of fucose is bound to 6-position of *N*-acetylglucosamine in the reducing end through α-bond in the *N*-glycoside-linked sugar chain is knocked out.

**21.** The transgenic non-human animal or plant or the progenies thereof according to claim 19 or 20, wherein the enzyme relating to the synthesis of an intracellular sugar nucleotide, GDP-fucose is an enzyme selected from the group consisting of the following (a), (b) and (c):

(a) GMD (GDP-mannose 4,6-dehydratase);
(b) Fx (GDP-keto-6-deoxymannose 3,5-epimerase, 4-reductase);
(c) GFPP (GDP-beta-L-fucose pyrophosphorylase).

**22.** The transgenic non-human animal or plant or the progenies thereof according to claim 21, wherein the GMD is a protein encoded by a DNA of the following (a) or (b):

(a) a DNA comprising the nucleotide sequence represented by SEQ ID NO:41;
(b) a DNA which hybridizes with the DNA consisting of the nucleotide sequence represented by SEQ ID NO: 41 under stringent conditions and encodes a protein having GMD activity.

**23.** The transgenic non-human animal or plant or the progenies thereof according to claim 2I, wherein the Fx is a

protein encoded by a DNA of the following (a) or (b):

(a) a DNA comprising the nucleotide sequence represented by SEQ ID NO:48;
(b) a DNA which hybridizes with the DNA consisting of the nucleotide sequence represented by SEQ ID NO: 48 under stringent conditions and encodes a protein having Fx activity.

**24.** The transgenic non-human animal or plant or the progenies thereof according to claim 21, wherein the GFPP is a protein encoded by a DNA of the following (a) or (b):

(a) a DNA comprising the nucleotide sequence represented by SEQ ID NO:51;
(b) a DNA which hybridizes with the DNA consisting of the nucleotide sequence represented by SEQ ID NO: 51 under stringent conditions and encodes a protein having GFPP activity.

**25.** The transgenic non-human animal or plant or the progenies thereof according to claim 19 or 20, wherein the enzyme relating to the modification of a sugar chain in which 1-position of fucose is bound to 6-position of *N*-acetylglucosamine in the reducing end through $\alpha$-bond in the *N*-glycoside-linked sugar chain is $\alpha$1,6-fucosyltransferase.

**26.** The transgenic non-human animal or plant or the progenies thereof according to claim 25, wherein the $\alpha$1,6-fucosyltransferase is a protein encoded by a DNA selected from the group consisting of the following (a), (b), (c) and (d):

(a) a DNA comprising the nucleotide sequence represented by SEQ ID NO:1;
(b) a DNA comprising the nucleotide sequence represented by SEQ ID NO:2;
(c) a DNA which hybridizes with the DNA consisting of the nucleotide sequence represented by SEQ ID NO: 1 under stringent conditions and encodes a protein having $\alpha$1,6-fucosyltransferase activity;
(d) a DNA which hybridizes with the DNA consisting of the nucleotide sequence represented by SEQ ID NO: 2 under stringent conditions and encodes a protein having $\alpha$1,6-fucosyltransferase activity.

**27.** The transgenic non-human animal or plant or the progenies thereof according to any one of claims 17 to 26, wherein the transgenic non-human animal is an animal selected from the group consisting of cattle, sheep, goat, pig, horse, mouse, rat, fowl, monkey and rabbit.

**28.** The cell according to any one of claims 1 to 16, wherein the antibody molecule is a molecule selected from the group consisting of (a), (b), (c) and (d):

(a) a human antibody;
(b) a humanized antibody;
(c) an antibody fragment comprising an Fc region of (a) or (b);
(d) a fusion protein comprising an Fc region of (a) or (b).

**29.** The cell according to any one of claims 1 to 16 and 28, wherein the antibody molecule belongs to an IgG class.

**30.** The cell according to any one of claims 1 to 16, 28 and 29, wherein the antibody molecule comprises complementarity determining regions 1, 2 and 3 of an antibody light chain variable region comprising the amino acid sequences represented by SEQ ID NOs:5, 6 and 7, respectively, and/or complementarity determining regions 1, 2 and 3 of an antibody heavy chain comprising the amino acid sequences represented by SEQ ID NOs:8, 9 and 10, respectively.

**31.** The cell according to any one of claims 1 to 16, 28, 29 and 30, wherein the antibody molecule comprises a light chain variable region comprising the amino acid sequence represented by SEQ ID NO:12 and/or a heavy chain variable region comprising the amino acid sequence represented by SEQ ID NO:14,

**32.** The transgenic non-human animal or plant or the progenies thereof according to any one of claims 17 to 27, wherein the antibody molecule is a molecule selected from the group consisting of (a), (b), (c) and (d):

(a) a human antibody;
(b) a humanized antibody;

(c) an antibody fragment comprising an Fc region of (a) or (b);
(d) a fusion protein comprising an Fc region of (a) or (b).

**33.** The transgenic non-human animal or plant or the progenies thereof according to any one of claims 17 to 27 and 32, wherein the antibody molecule belongs to an IgG class.

**34.** The transgenic non-human animal or plant or the progenies thereof according to any one of claims 17 to 27, 32 and 33, wherein the antibody molecule comprises complementarity determining regions 1, 2 and 3 of an antibody light chain variable region comprising the amino acid sequences represented by SEQ ID NOs:5, 6 and 7, respectively, and/or complementarity determining regions 1, 2 and 3 of an antibody heavy chain comprising the amino acid sequences represented by SEQ ID NOs:8, 9 and 10, respectively.

**35.** The transgenic non-human animal or plant or the progenies thereof according to any one of claims 17 to 27, 32, 33 and 34, wherein the antibody molecule comprises a light chain variable region comprising the amino acid sequence represented by SEQ ID NO:12 and/or a heavy chain variable region comprising the amino acid sequence represented by SEQ ID NO:14.

**36.** An antibody composition which is produced by the cell according to any one of claims 1 to 16 and 28 to 31.

**37.** An antibody composition which is obtainable by rearing the transgenic non-human animal or plant or the progenies thereof according to any one of claims 17 to 27 and 32 to 35.

**38.** An antibody composition comprising an antibody molecule which specifically binds to CD20 and has complex *N*-glycoside-linked sugar chains bound to the Fc region, wherein among the total complex *N*-glycoside-linked sugar chains bound to the Fc region in the composition, the ratio of a sugar chain in which fucose is not bound to *N*-acetylglucosamine in the reducing end in the sugar chain is 20% or more.

**39.** The antibody composition according to claim 38, wherein the sugar chain to which fucose is not bound is a complex *N*-glycoside-linked sugar chain in which 1-position of fucose is not bound to 6-position of *N*-acetylglucosamine in the reducing end through $\alpha$-bond.

**40.** The antibody composition according to claim 38, wherein the antibody molecule is a molecule selected from the group consisting of (a), (b), (c) and (d):

(a) a human antibody;
(b) a humanized antibody;
(c) an antibody fragment comprising an Fc region of (a) or (b);
(d) a fusion protein comprising an Fc region of (a) or (b).

**41.** The antibody composition according to any one of claims 38 to 40, wherein the antibody molecule belongs to an IgG class.

**42.** The antibody composition according to any one of claims 38 to 41, wherein the antibody molecule comprises complementarity determining regions 1, 2 and 3 of an antibody light chain variable region comprising the amino acid sequences represented by SEQ ID NOs:5, 6 and 7, respectively, and/or complementarity determining regions 1, 2 and 3 of an antibody heavy chain comprising the amino acid sequences represented by SEQ ID NOs:8, 9 and 10, respectively.

**43.** The antibody composition according to any one of claims 38 to 42, wherein the antibody molecule comprises a light chain variable region comprising the amino acid sequence represented by SEQ ID NO:12 and/or a heavy chain variable region comprising the amino acid sequence represented by SEQ ID NO: 14.

**44.** A process for producing the antibody composition according to any one of claims 36 and 38 to 43, which comprises culturing the cell according to any one of claims 1 to 16 and 28 to 31 to form and accumulate the antibody composition in the culture; and recovering the antibody composition from the culture.

**45.** A process for producing the antibody composition according to any one of claims 36 and 38 to 43, which comprises rearing the transgenic non-human animal or plant or the progenies thereof according to any one of claims 17 to

27 and 32 to 35; isolating tissue or body fluid from the reared animal or plant; and recovering the antibody composition from the isolated tissue or body fluid.

**46.** A medicament which comprises the antibody composition according to any one of claims 36 to 43 as an active ingredient.

**47.** An agent for treating diseases relating to CD20, which comprises the antibody composition according to any one of claims 36 to 43 as an active ingredient.

**48.** The agent according to claim 47, wherein the disease relating to CD20 is a cancer or an immunological disease.

FIG. 1

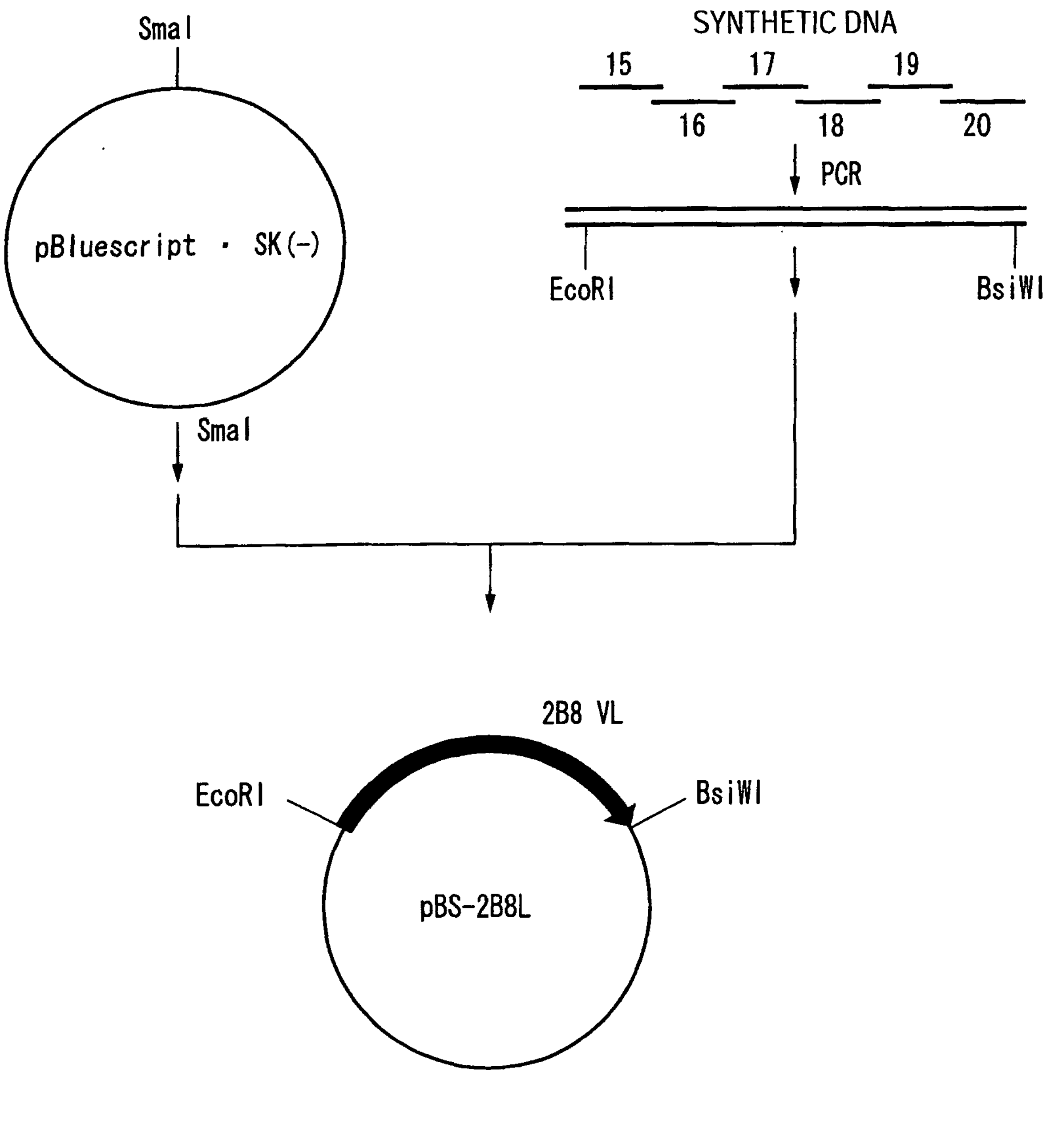
SmaI
pBluescript · SK(-)
SmaI
SYNTHETIC DNA
15
17
19
16
18
20
PCR
EcoRI
BsiWI
2B8 VL
EcoRI
BsiWI
pBS-2B8L

FIG. 2

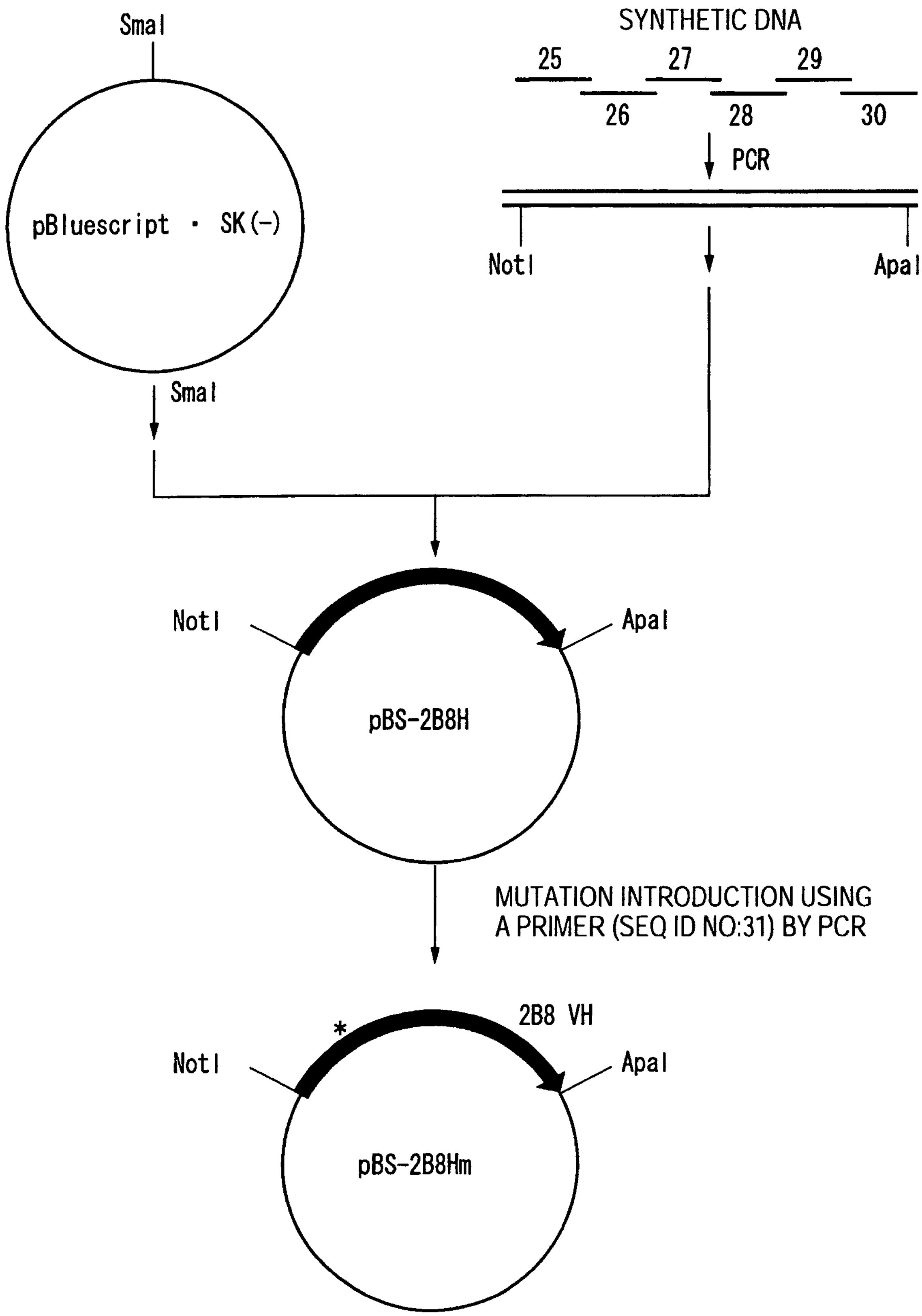
SmaI
pBluescript · SK(-)
SYNTHETIC DNA
25
27
29
26
28
30
PCR
NotI
ApaI
SmaI
NotI
ApaI
pBS-2B8H
MUTATION INTRODUCTION USING A PRIMER (SEQ ID NO:31) BY PCR
*
2B8 VH
NotI
ApaI
pBS-2B8Hm

FIG. 3

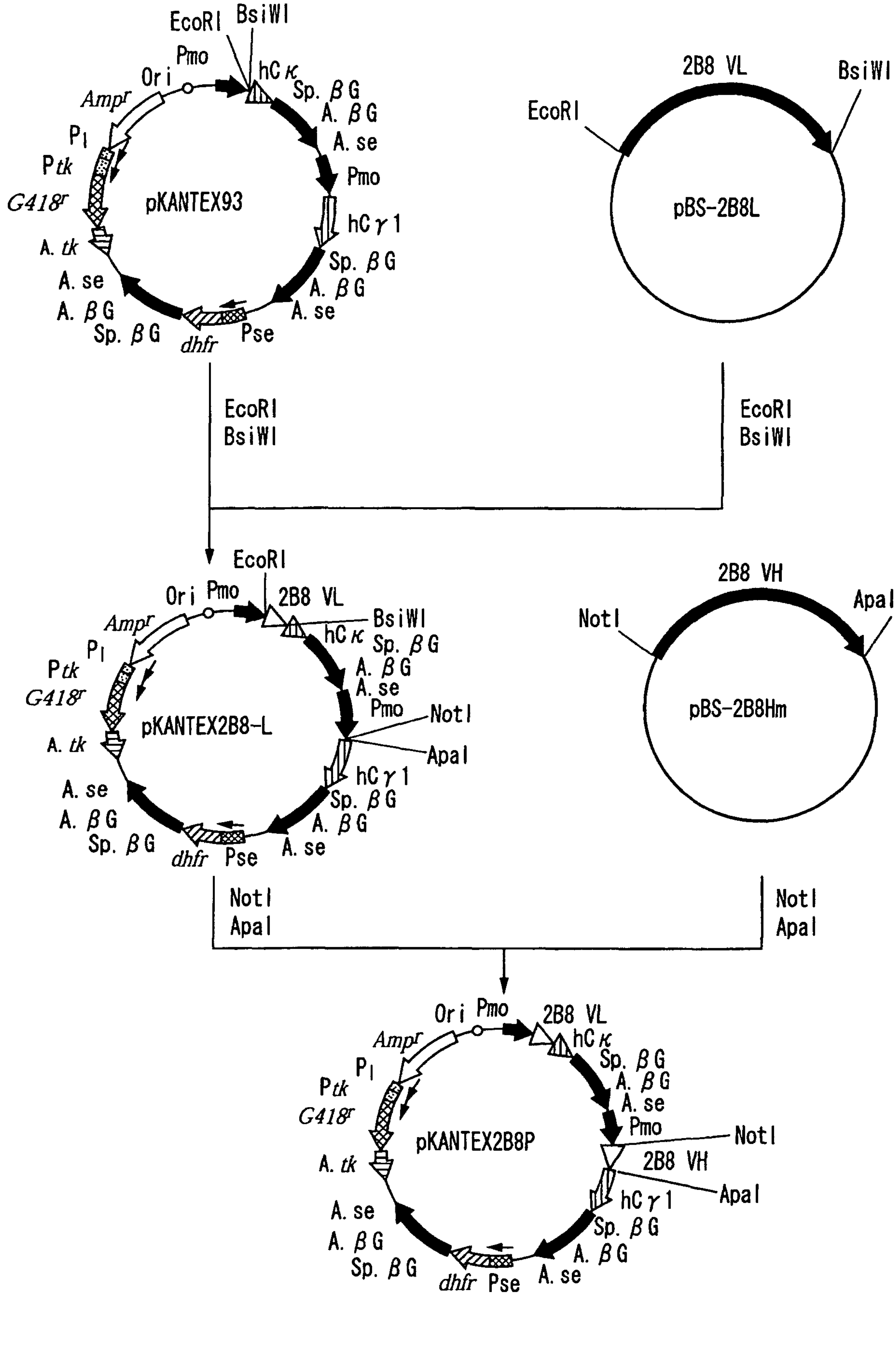
EcoRI
BsiWI
Pmo
Ori
hCκ
Sp. βG
A. βG
A. se
Pmo
hCγ1
Sp. βG
A. βG
A. se
Pse
dhfr
Sp. βG
A. βG
A. se
A. tk
G418r
Ptk
P1
Ampr
pKANTEX93
2B8 VL
EcoRI
BsiWI
pBS-2B8L
EcoRI
BsiWI
EcoRI
BsiWI
EcoRI
Ori
Pmo
2B8 VL
BsiWI
hCκ
Sp. βG
A. βG
A. se
Pmo
NotI
ApaI
hCγ1
Sp. βG
A. βG
A. se
Pse
dhfr
Sp. βG
A. βG
A. se
A. tk
G418r
Ptk
P1
Ampr
pKANTEX2B8-L
2B8 VH
NotI
ApaI
pBS-2B8Hm
NotI
ApaI
NotI
ApaI
Ori
Pmo
2B8 VL
hCκ
Sp. βG
A. βG
A. se
Pmo
NotI
2B8 VH
ApaI
hCγ1
Sp. βG
A. βG
A. se
Pse
dhfr
Sp. βG
A. βG
A. se
A. tk
G418r
Ptk
P1
Ampr
pKANTEX2B8P

FIG. 4

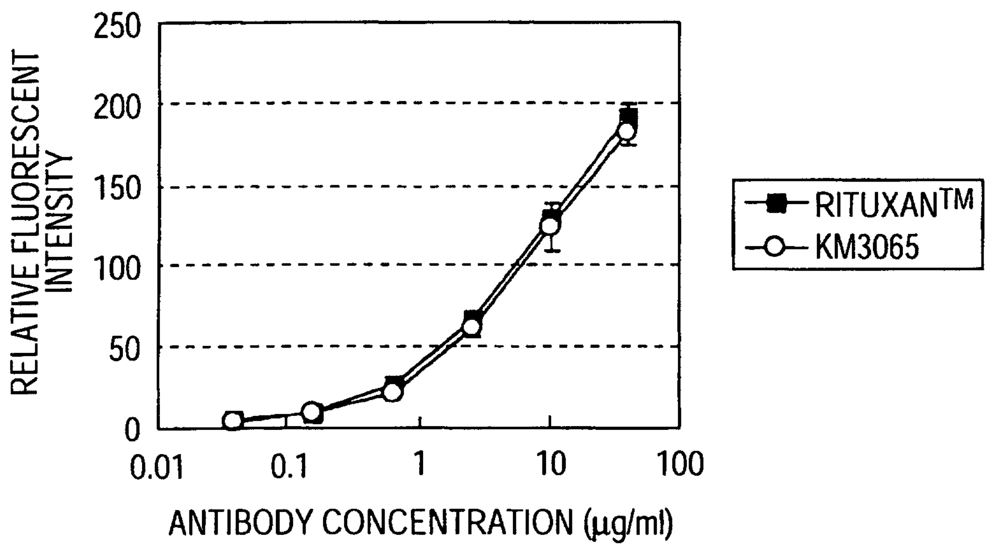
250
200
150
100
50
0
RELATIVE FLUORESCENT INTENSITY
0.01
0.1
1
10
100
ANTIBODY CONCENTRATION (μg/ml)
RITUXANTM
KM3065

FIG. 5

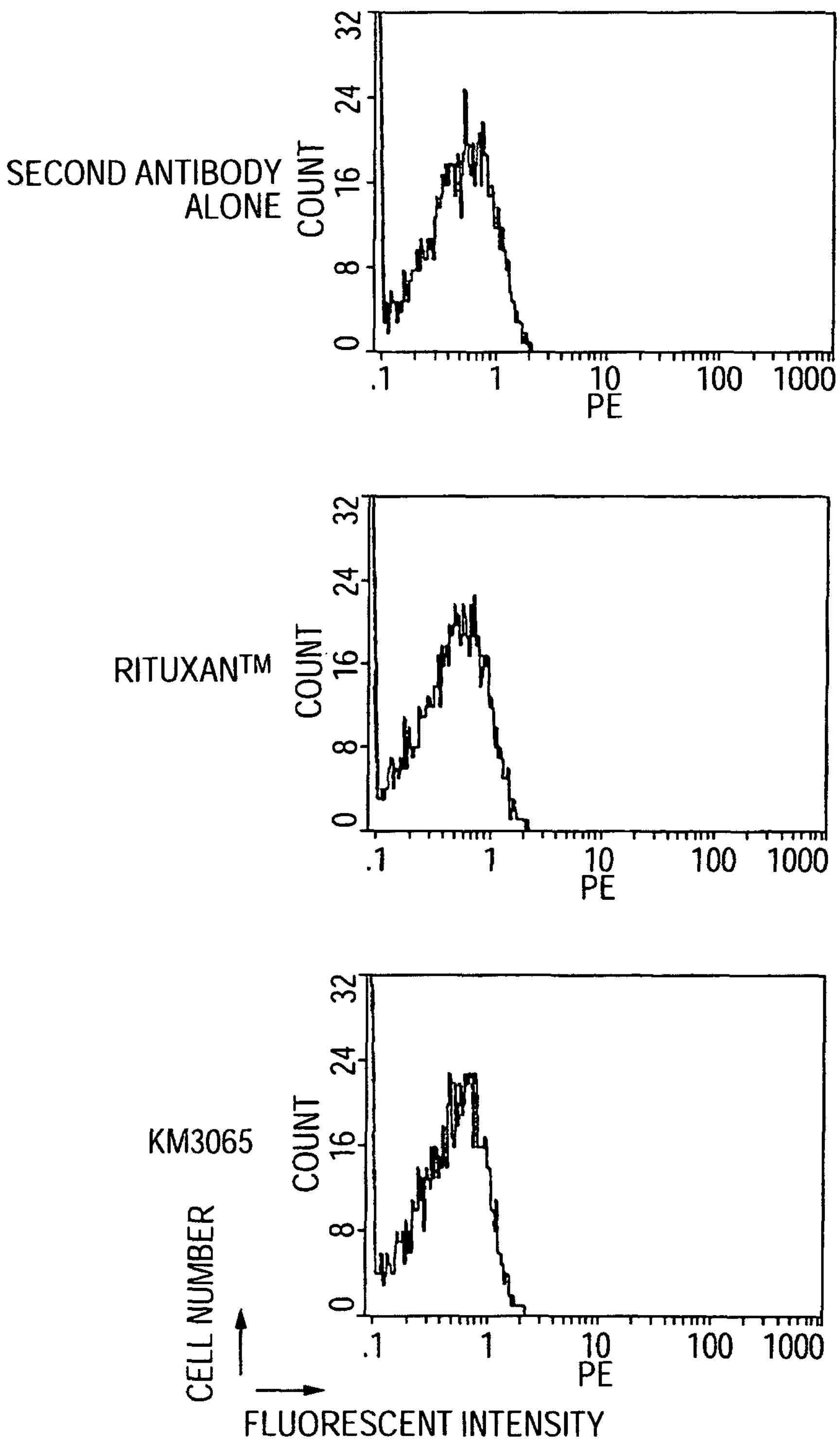
SECOND ANTIBODY ALONE
COUNT
0
8
16
24
32
.1
1
10
100
1000
PE
RITUXAN™
COUNT
0
8
16
24
32
.1
1
10
100
1000
PE
KM3065
COUNT
0
8
16
24
32
.1
1
10
100
1000
PE
CELL NUMBER
FLUORESCENT INTENSITY

FIG. 6

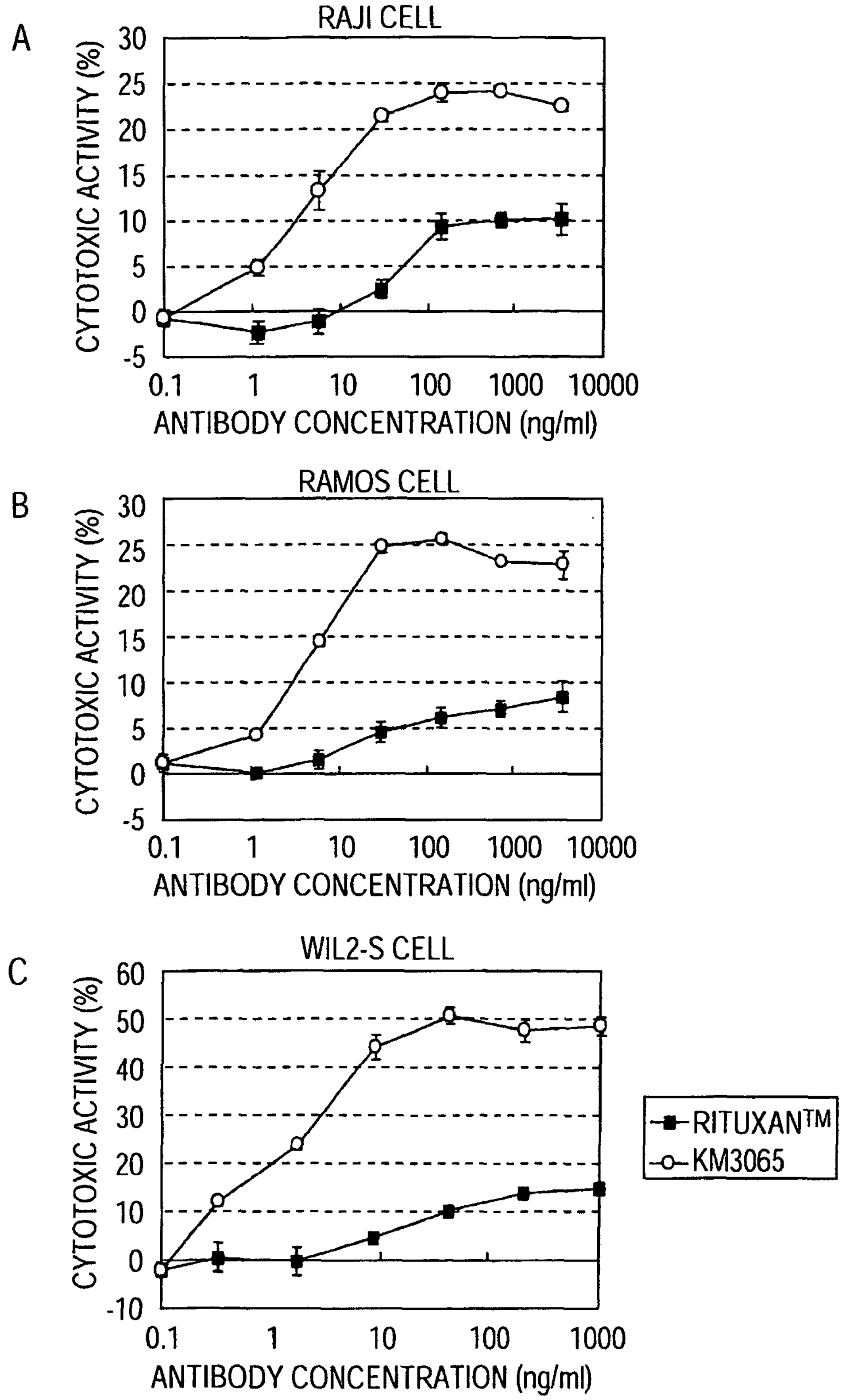
A
RAJI CELL
CYTOTOXIC ACTIVITY (%)
30
25
20
15
10
5
0
-5
0.1
1
10
100
1000
10000
ANTIBODY CONCENTRATION (ng/ml)
B
RAMOS CELL
CYTOTOXIC ACTIVITY (%)
30
25
20
15
10
5
0
-5
0.1
1
10
100
1000
10000
ANTIBODY CONCENTRATION (ng/ml)
C
WIL2-S CELL
CYTOTOXIC ACTIVITY (%)
60
50
40
30
20
10
0
-10
0.1
1
10
100
1000
ANTIBODY CONCENTRATION (ng/ml)
RITUXAN™
KM3065

FIG. 7

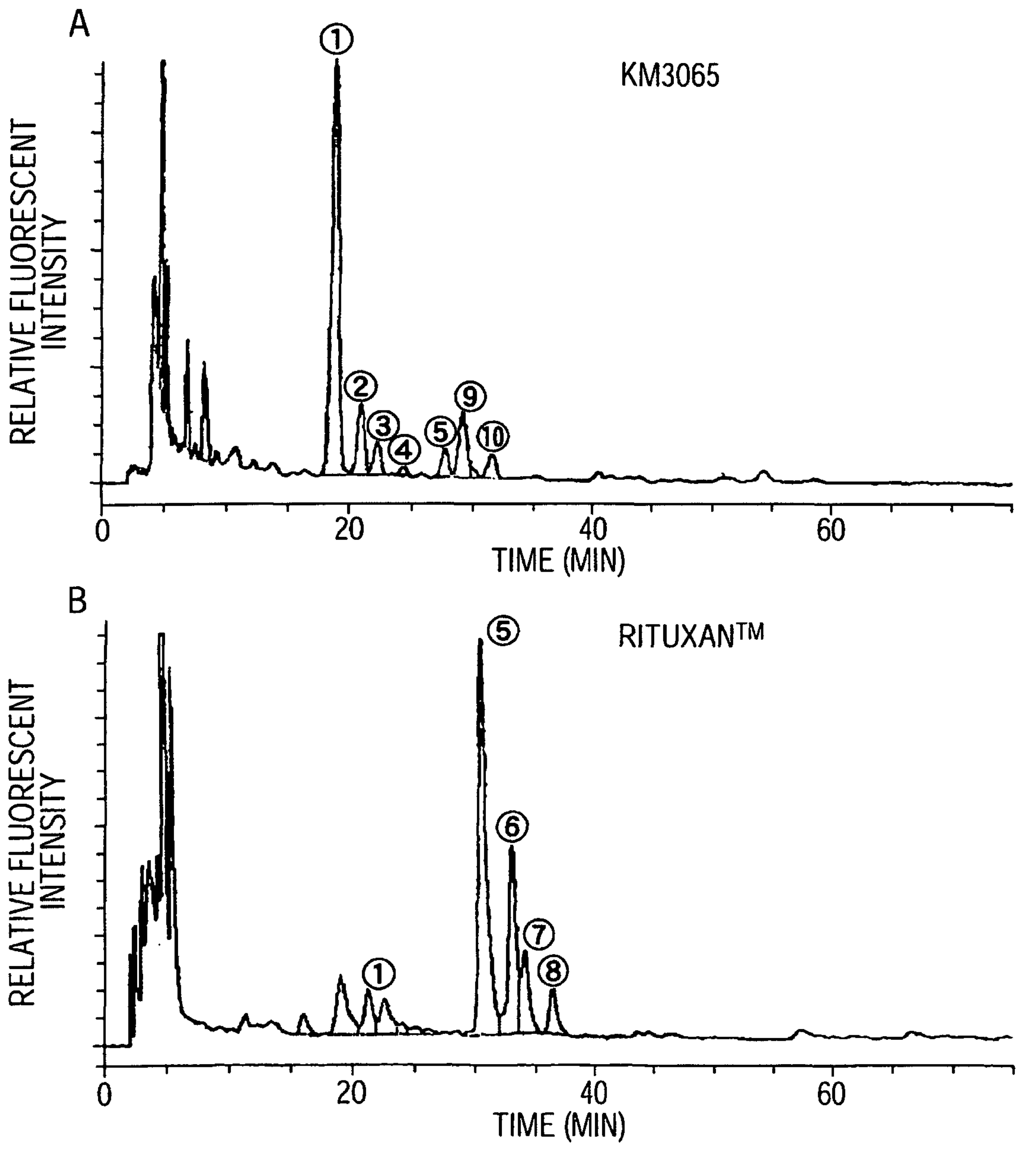
A
KM3065
RELATIVE FLUORESCENT INTENSITY
1
2
3
4
5
9
10
0
20
40
60
TIME (MIN)
B
RITUXAN™
RELATIVE FLUORESCENT INTENSITY
5
6
7
8
1
0
20
40
60
TIME (MIN)

FIG. 8

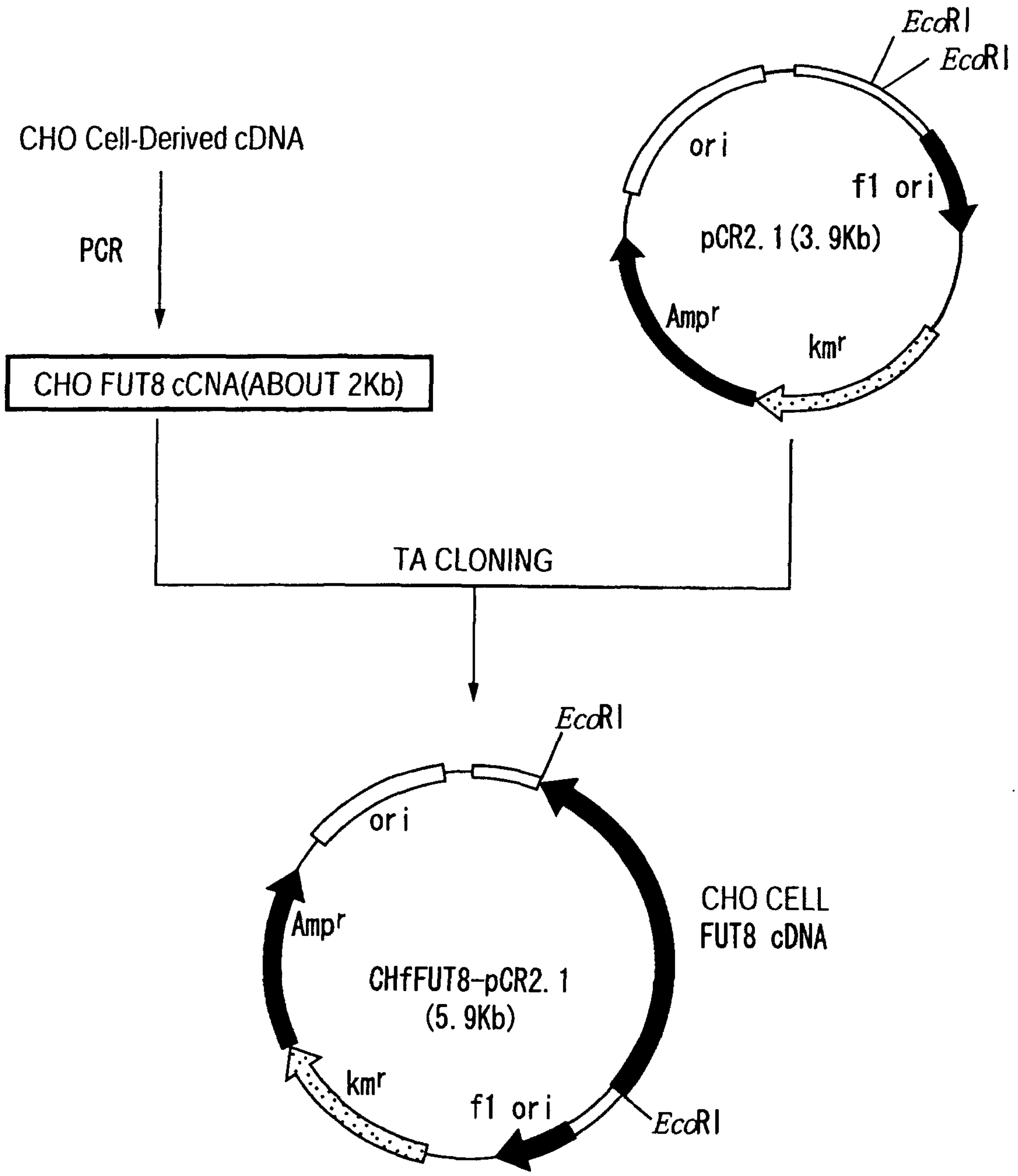
CHO Cell-Derived cDNA
PCR
CHO FUT8 cCNA(ABOUT 2Kb)
EcoRI
EcoRI
ori
f1 ori
pCR2. 1 (3. 9Kb)
Ampr
kmr
TA CLONING
EcoRI
ori
Ampr
CHfFUT8-pCR2. 1
(5. 9Kb)
CHO CELL
FUT8 cDNA
kmr
f1 ori
EcoRI

FIG. 9

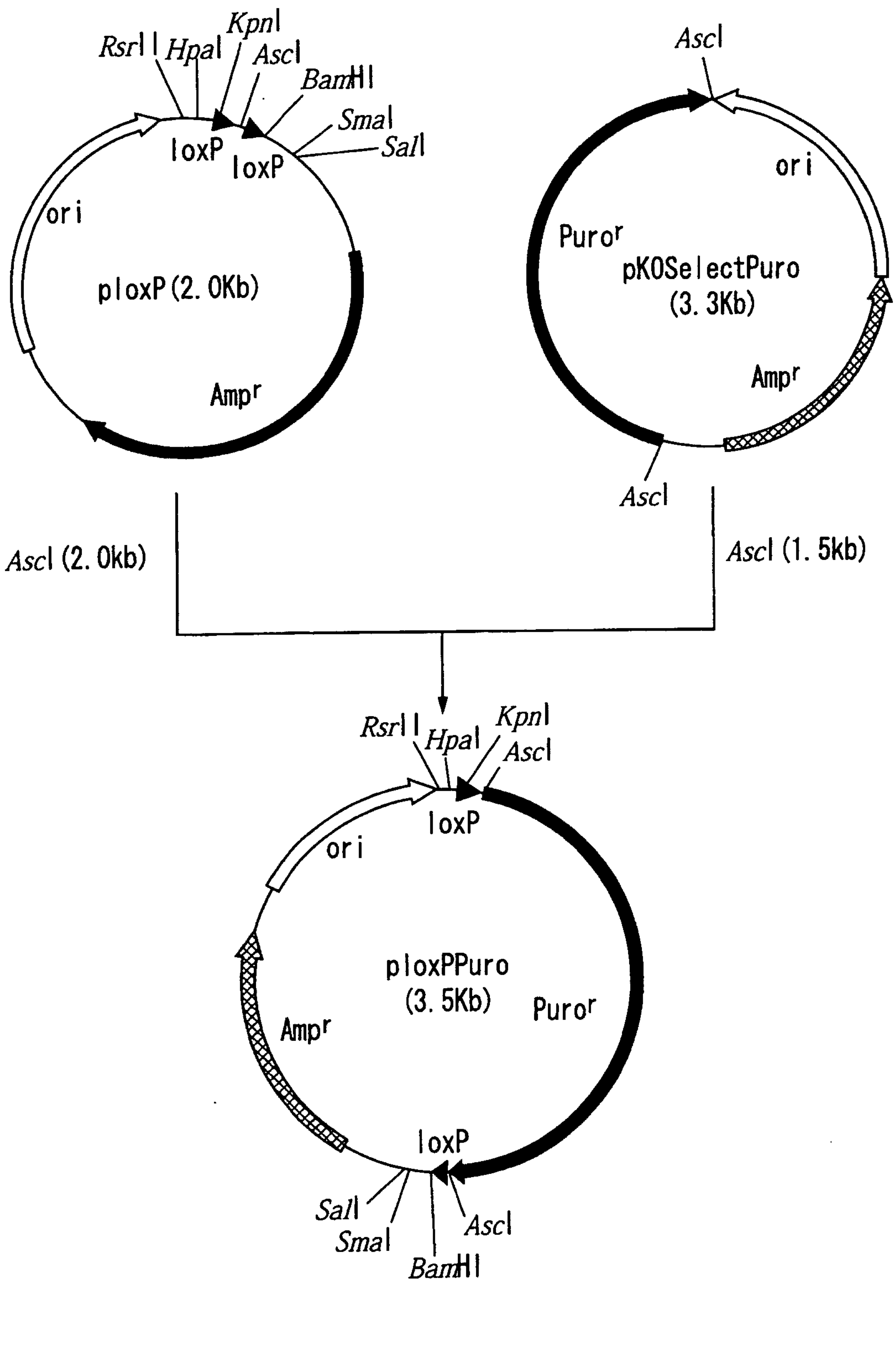

RsrII
HpaI
KpnI
AscI
BamHI
SmaI
SalI
loxP
loxP
ori
ploxP (2.0Kb)
Ampr
AscI
ori
Puror
pKOSelectPuro
(3.3Kb)
Ampr
AscI
AscI (2.0kb)
AscI (1.5kb)
RsrII
HpaI
KpnI
AscI
loxP
ori
ploxPPuro
(3.5Kb)
Puror
Ampr
loxP
SalI
SmaI
BamHI
AscI

FIG. 10

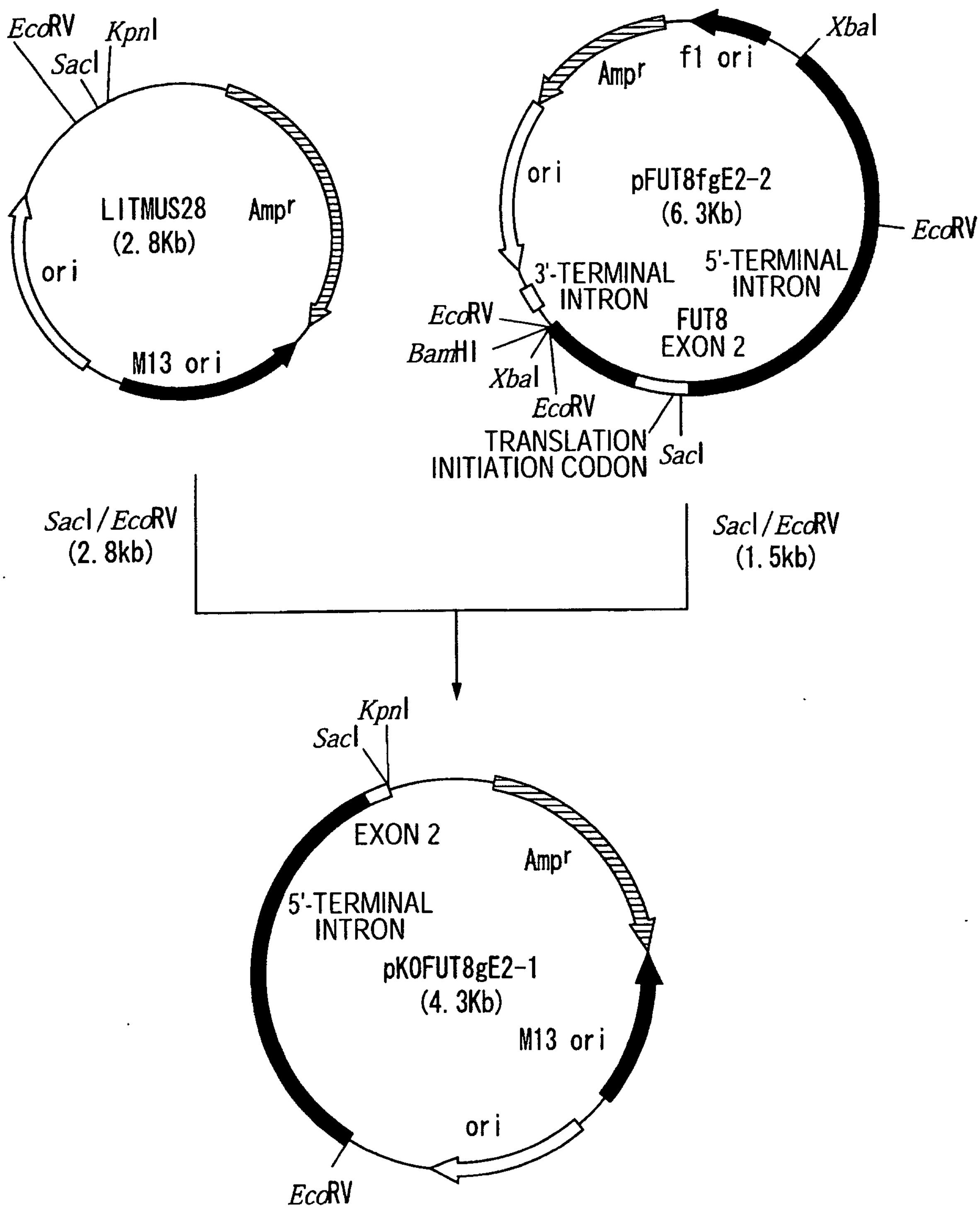

EcoRV
KpnI
SacI
LITMUS28
(2. 8Kb)
Ampr
ori
M13 ori
XbaI
f1 ori
Ampr
ori
pFUT8fgE2-2
(6. 3Kb)
EcoRV
3'-TERMINAL INTRON
5'-TERMINAL INTRON
FUT8 EXON 2
EcoRV
BamHI
XbaI
EcoRV
TRANSLATION INITIATION CODON
SacI
SacI/EcoRV
(2. 8kb)
SacI/EcoRV
(1. 5kb)
KpnI
SacI
EXON 2
Ampr
5'-TERMINAL INTRON
pKOFUT8gE2-1
(4. 3Kb)
M13 ori
ori
EcoRV

FIG. 11

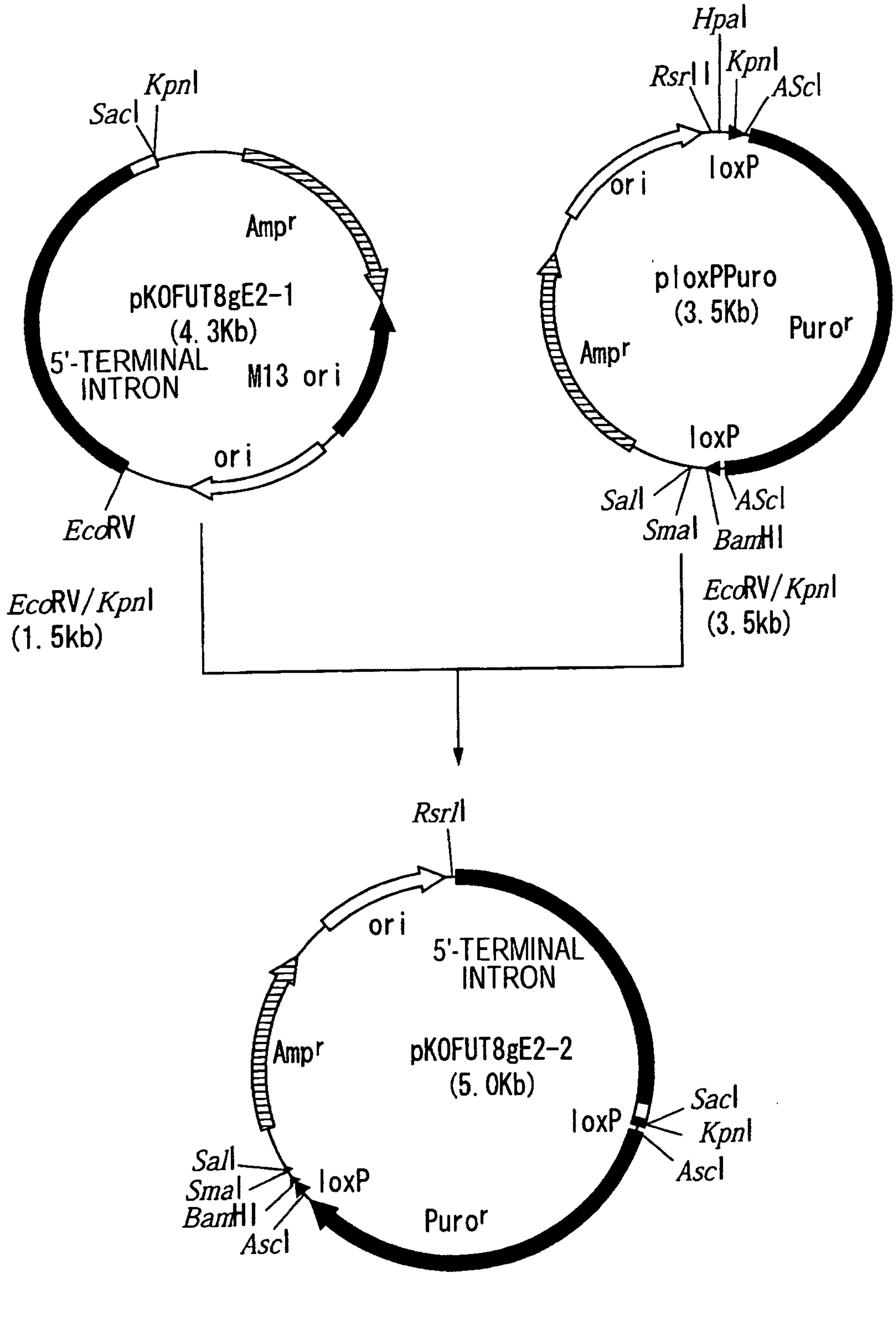
SacI
KpnI
Ampr
pKOFUT8gE2-1
(4.3Kb)
5'-TERMINAL INTRON
M13 ori
ori
EcoRV
HpaI
RsrII
KpnI
AScI
loxP
ori
ploxPPuro
(3.5Kb)
Puror
Ampr
loxP
SalI
SmaI
AScI
BamHI
EcoRV/KpnI
(1.5kb)
EcoRV/KpnI
(3.5kb)
RsrII
ori
5'-TERMINAL INTRON
Ampr
pKOFUT8gE2-2
(5.0Kb)
SacI
KpnI
loxP
AscI
SalI
SmaI
BamHI
AscI
loxP
Puror

# FIG. 12

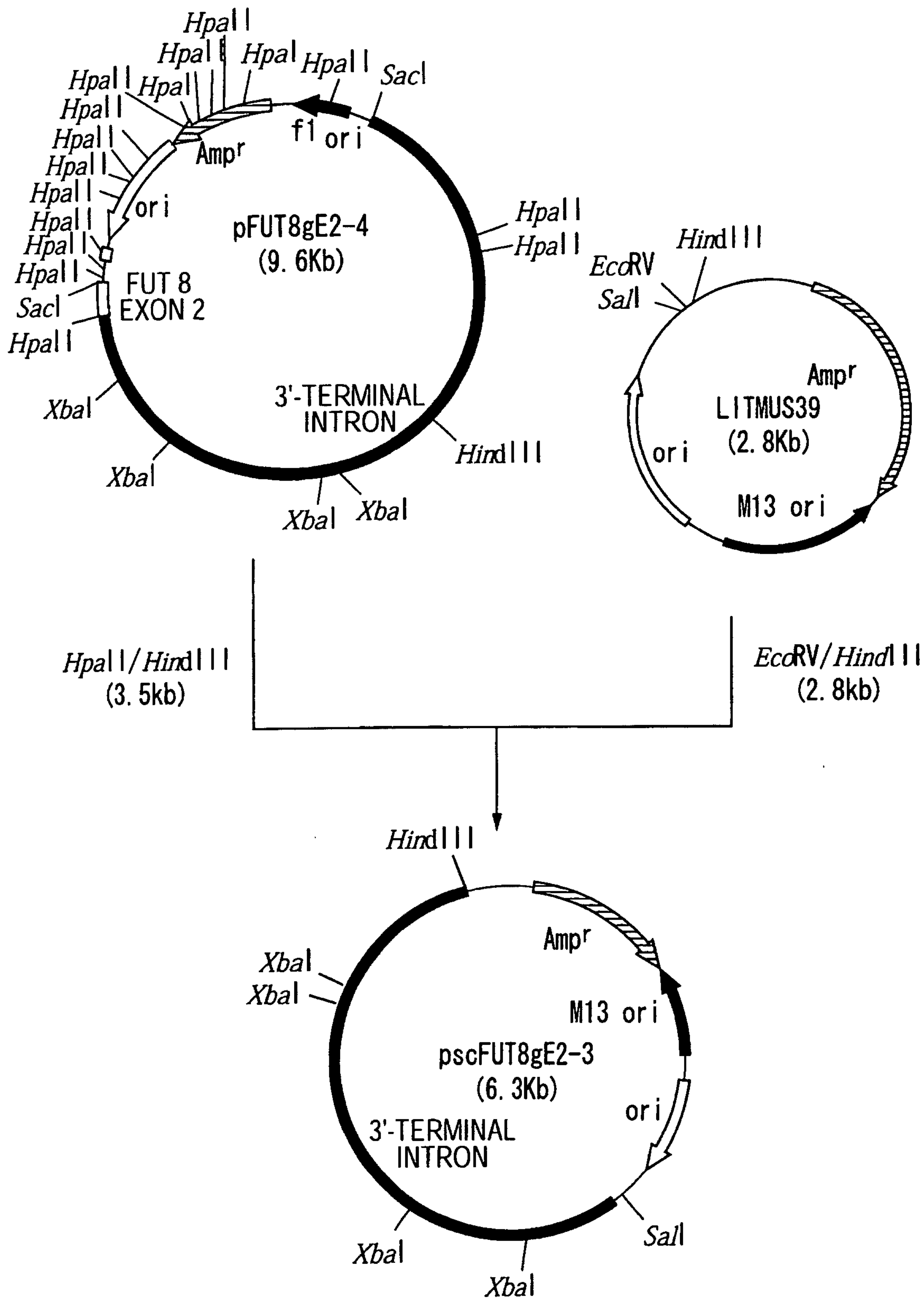

HpaII
HpaII
HpaI
HpaII
SacI
HpaII
HpaI
HpaII
HpaII
HpaII
HpaII
HpaII
HpaII
HpaII
SacI
HpaII
f1 ori
Ampr
ori
pFUT8gE2-4
(9. 6Kb)
FUT 8
EXON 2
HpaII
HpaII
3'-TERMINAL
INTRON
HindIII
XbaI
XbaI
XbaI
XbaI
HindIII
EcoRV
SalI
Ampr
LITMUS39
(2. 8Kb)
ori
M13 ori
HpaII/HindIII
(3. 5kb)
EcoRV/HindIII
(2. 8kb)
HindIII
XbaI
XbaI
Ampr
M13 ori
pscFUT8gE2-3
(6. 3Kb)
ori
3'-TERMINAL
INTRON
SalI
XbaI
XbaI

FIG. 13

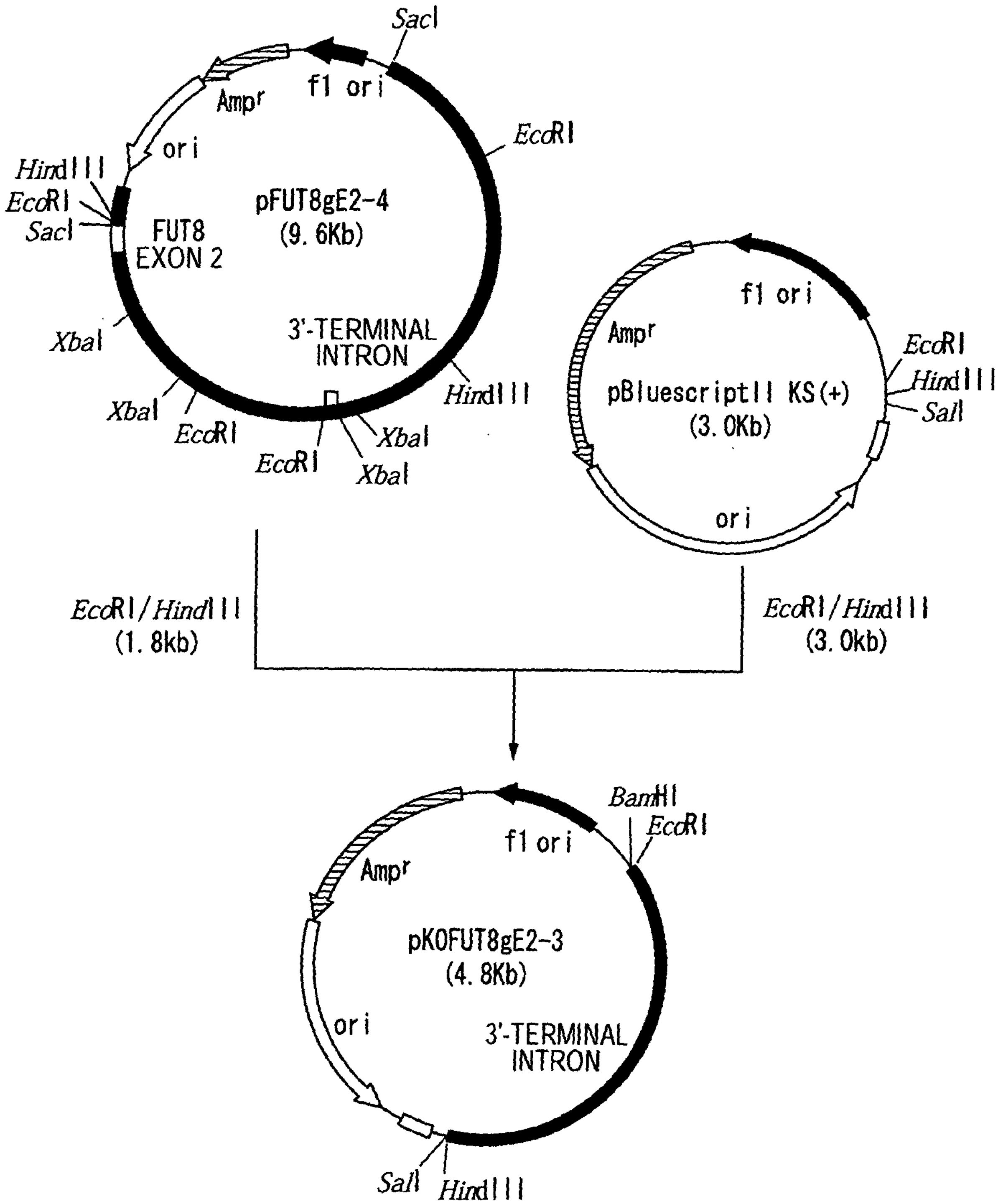
SacI
f1 ori
Amp^r
EcoRI
ori
HindIII
EcoRI
SacI
pFUT8gE2-4
(9. 6Kb)
FUT8
EXON 2
XbaI
3'-TERMINAL
INTRON
HindIII
XbaI
EcoRI
EcoRI
XbaI
XbaI
f1 ori
Amp^r
EcoRI
HindIII
SalI
pBluescriptII KS(+)
(3. 0Kb)
ori
EcoRI/HindIII
(1. 8kb)
EcoRI/HindIII
(3. 0kb)
BamHI
EcoRI
f1 ori
Amp^r
pKOFUT8gE2-3
(4. 8Kb)
ori
3'-TERMINAL
INTRON
SalI
HindIII

FIG. 14

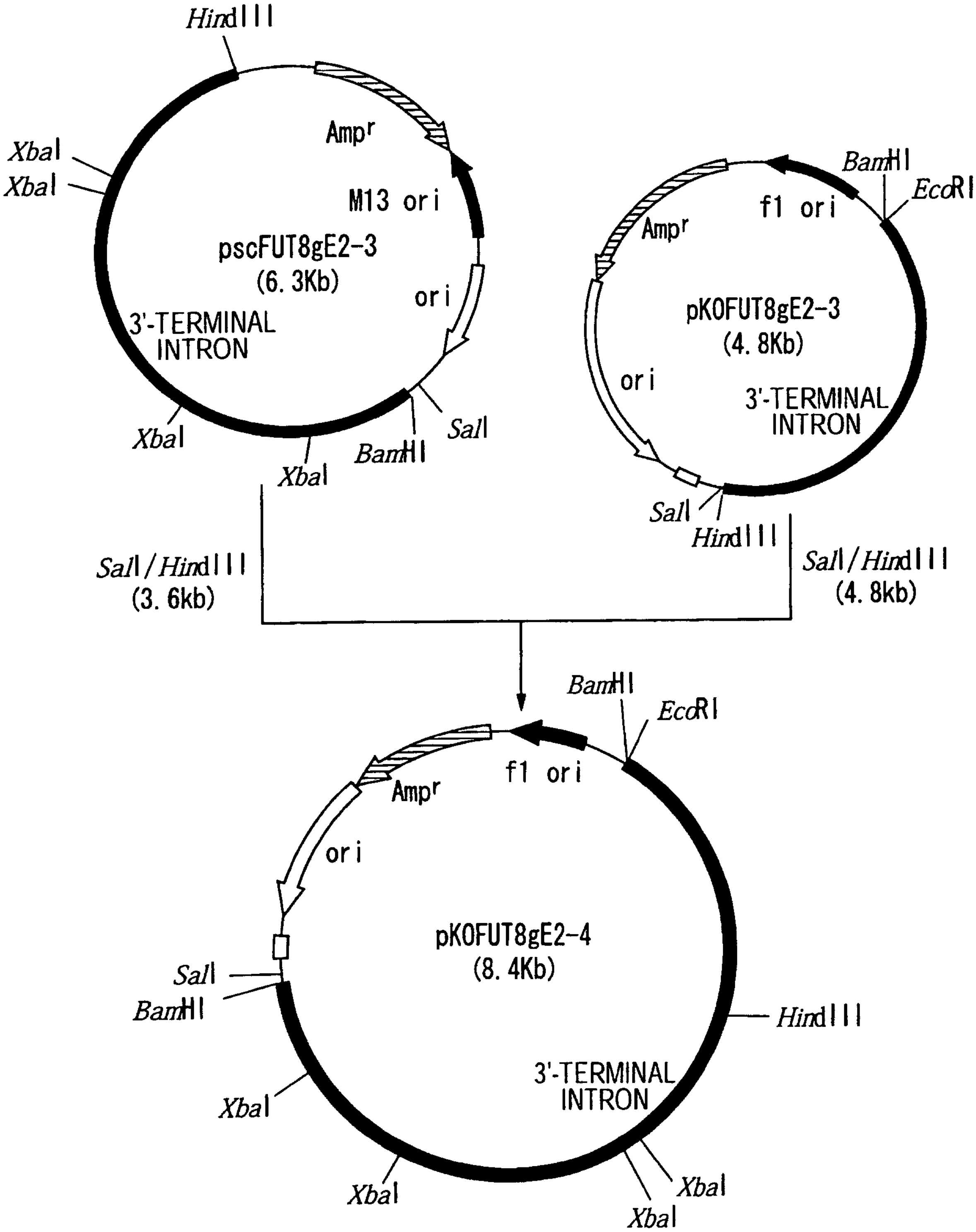
HindIII
XbaI
XbaI
Amp^r
M13 ori
pscFUT8gE2-3
(6. 3Kb)
ori
3'-TERMINAL INTRON
XbaI
XbaI
BamHI
SalI
BamHI
EcoRI
f1 ori
Amp^r
pKOFUT8gE2-3
(4. 8Kb)
ori
3'-TERMINAL INTRON
SalI
HindIII
SalI/HindIII
(3. 6kb)
SalI/HindIII
(4. 8kb)
BamHI
EcoRI
f1 ori
Amp^r
ori
pKOFUT8gE2-4
(8. 4Kb)
SalI
BamHI
HindIII
3'-TERMINAL INTRON
XbaI
XbaI
XbaI
XbaI

FIG. 15

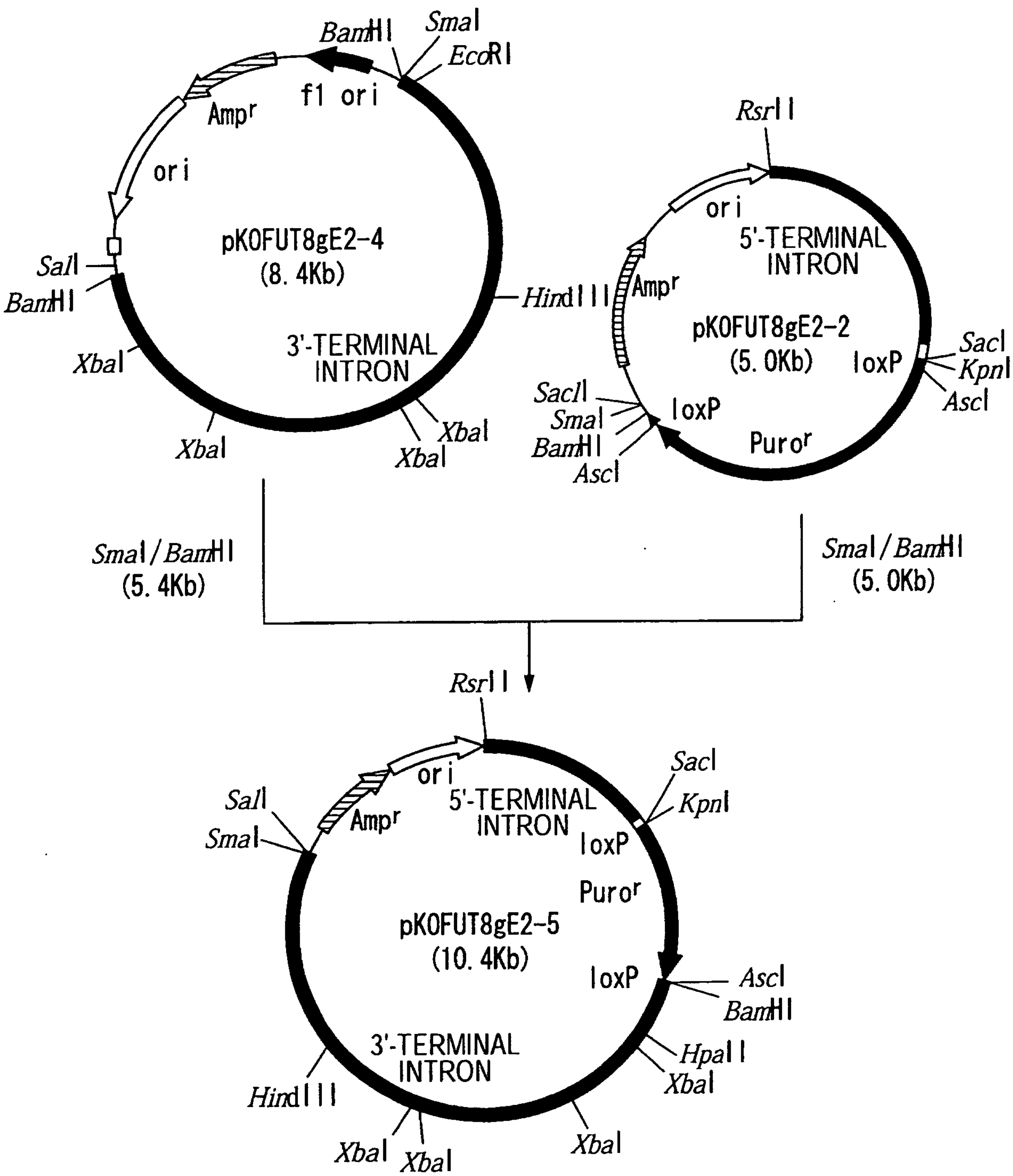
BamHI
SmaI
EcoRI
f1 ori
Amp^r
ori
pKOFUT8gE2-4
(8. 4Kb)
SalI
BamHI
HindIII
3'-TERMINAL INTRON
XbaI
XbaI
XbaI
XbaI
RsrII
ori
5'-TERMINAL INTRON
Amp^r
pKOFUT8gE2-2
(5. 0Kb)
loxP
SacI
KpnI
AscI
SacII
SmaI
BamHI
AscI
loxP
Puro^r
SmaI/BamHI
(5. 4Kb)
SmaI/BamHI
(5. 0Kb)
RsrII
SacI
KpnI
SalI
SmaI
ori
Amp^r
5'-TERMINAL INTRON
loxP
Puro^r
pKOFUT8gE2-5
(10. 4Kb)
loxP
AscI
BamHI
HpaII
XbaI
3'-TERMINAL INTRON
HindIII
XbaI
XbaI
XbaI

# FIG. 16

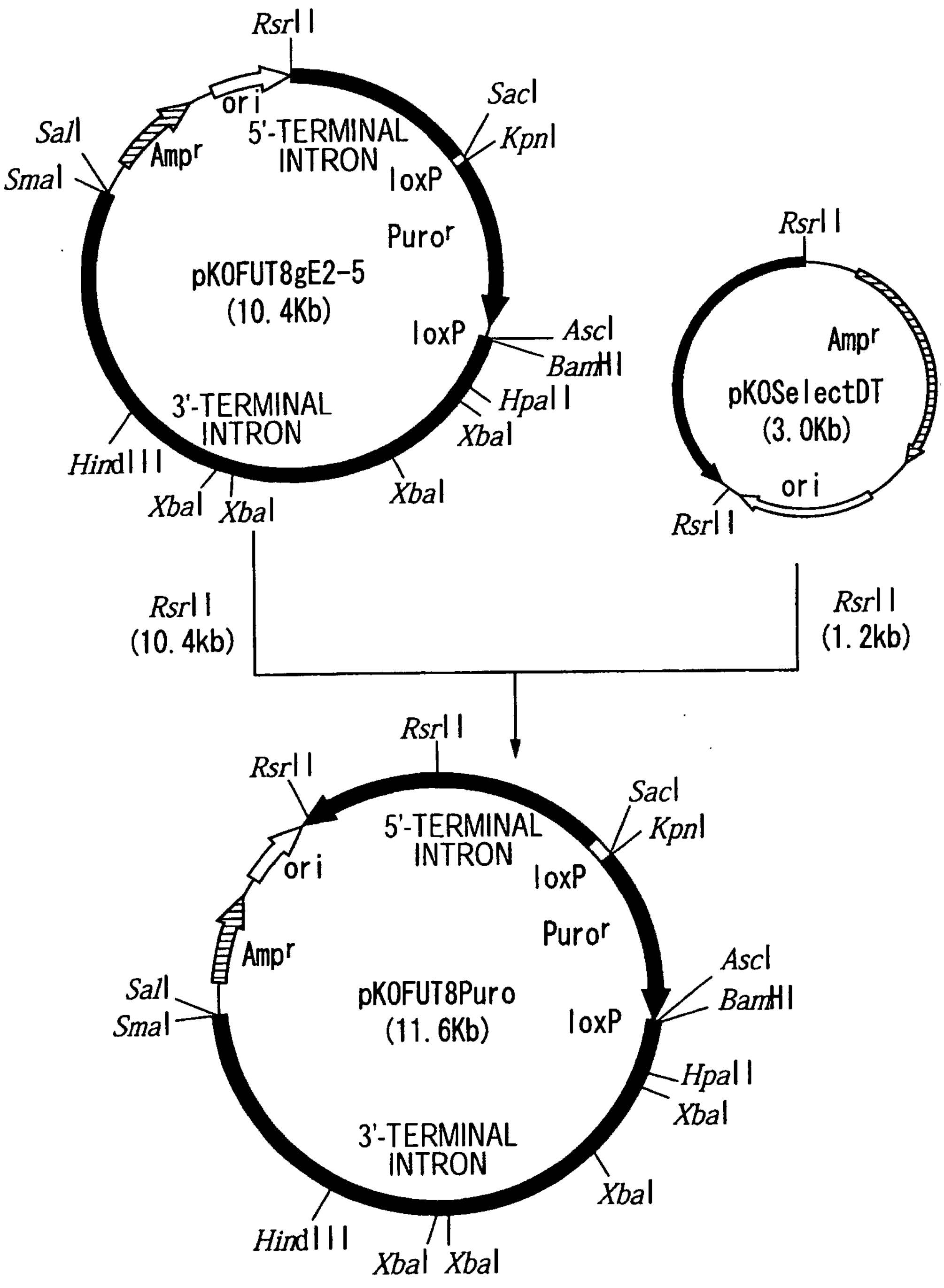
RsrII
SacI
KpnI
SalI
SmaI
ori
5'-TERMINAL INTRON
Ampr
loxP
Puror
pKOFUT8gE2-5
(10.4Kb)
loxP
AscI
BamHI
HpaII
XbaI
3'-TERMINAL INTRON
HindIII
XbaI
XbaI
XbaI
RsrII
Ampr
pKOSelectDT
(3.0Kb)
ori
RsrII
RsrII
(10.4kb)
RsrII
(1.2kb)
RsrII
RsrII
SacI
KpnI
5'-TERMINAL INTRON
ori
loxP
Ampr
Puror
SalI
SmaI
pKOFUT8Puro
(11.6Kb)
loxP
AscI
BamHI
HpaII
XbaI
3'-TERMINAL INTRON
XbaI
HindIII
XbaI
XbaI

FIG. 17

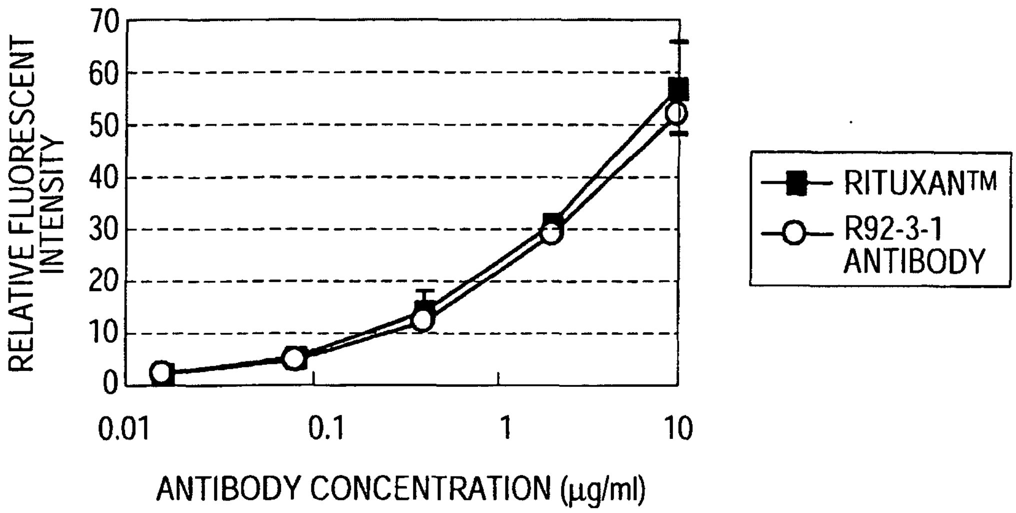
70
60
50
40
30
20
10
0
RELATIVE FLUORESCENT INTENSITY
0.01
0.1
1
10
ANTIBODY CONCENTRATION (μg/ml)
RITUXAN™
R92-3-1 ANTIBODY

FIG. 18

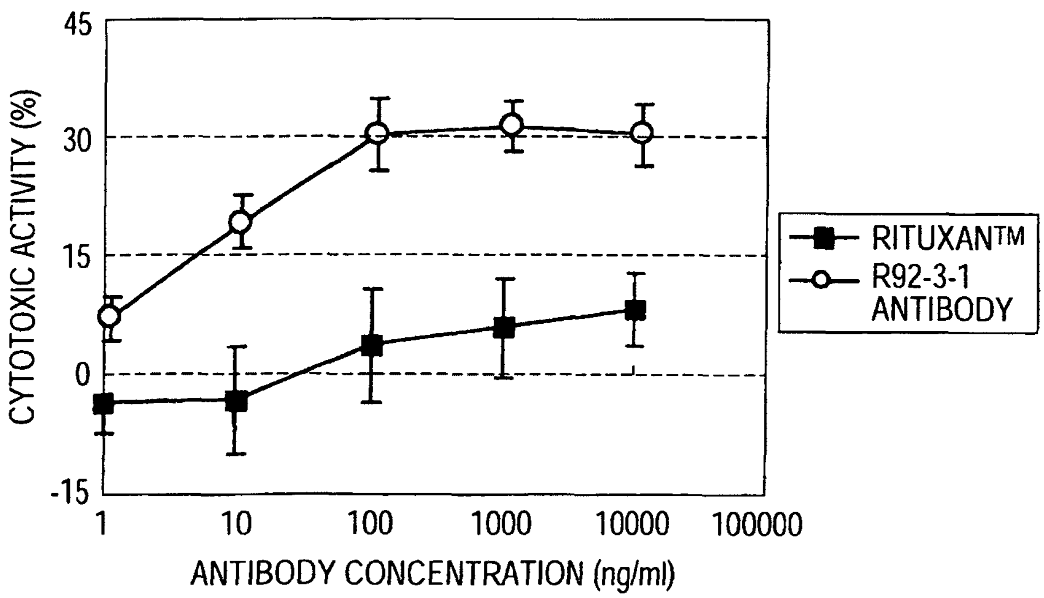
45
30
15
0
-15
CYTOTOXIC ACTIVITY (%)
1
10
100
1000
10000
100000
ANTIBODY CONCENTRATION (ng/ml)
RITUXAN™
R92-3-1 ANTIBODY

FIG. 19

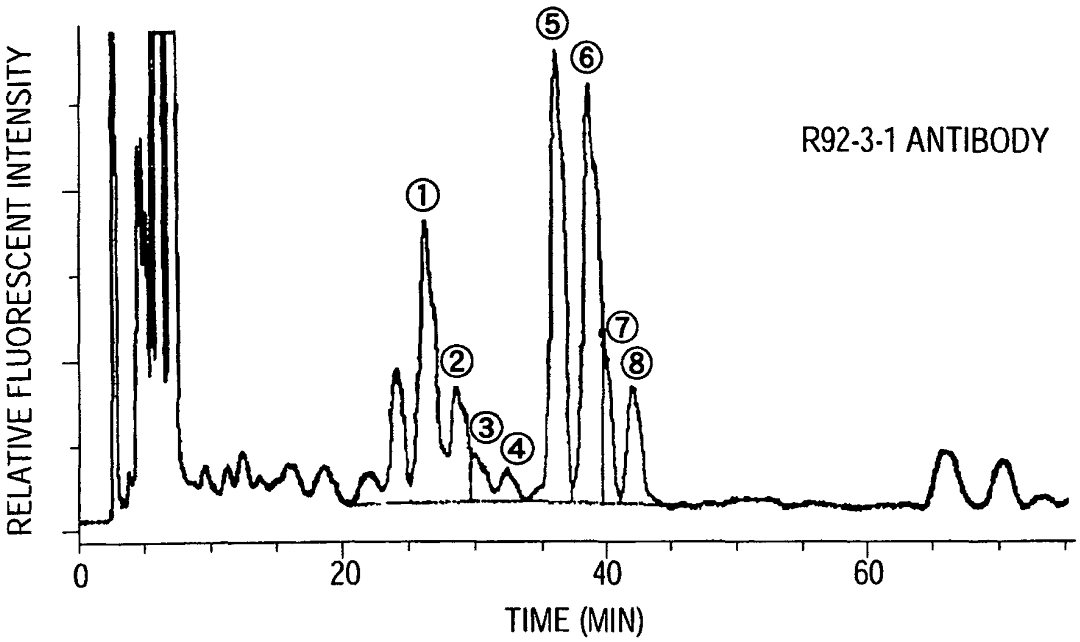
RELATIVE FLUORESCENT INTENSITY
R92-3-1 ANTIBODY
①
②
③
④
⑤
⑥
⑦
⑧
0
20
40
60
TIME (MIN)

FIG. 20

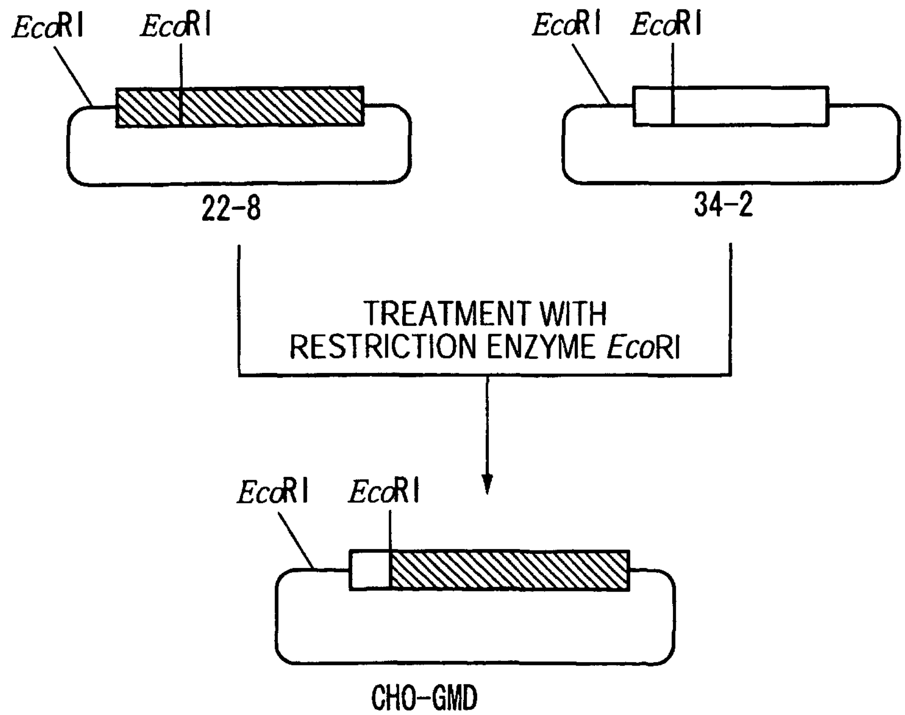
EcoRI
EcoRI
EcoRI
EcoRI
22-8
34-2
TREATMENT WITH
RESTRICTION ENZYME EcoRI
EcoRI
EcoRI
CHO-GMD

FIG. 21

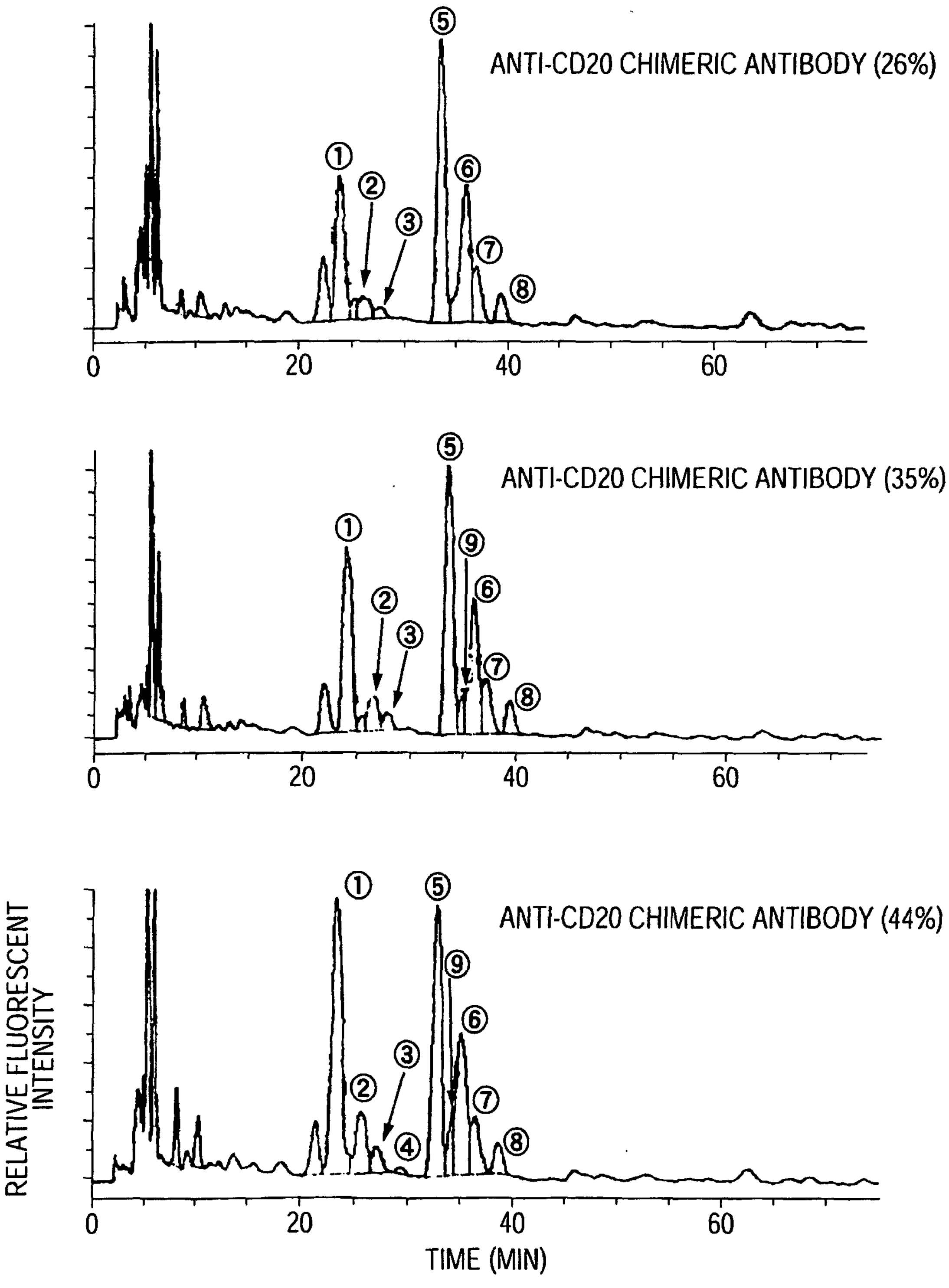
ANTI-CD20 CHIMERIC ANTIBODY (26%)
ANTI-CD20 CHIMERIC ANTIBODY (35%)
ANTI-CD20 CHIMERIC ANTIBODY (44%)
RELATIVE FLUORESCENT INTENSITY
TIME (MIN)
0
20
40
60

FIG. 22

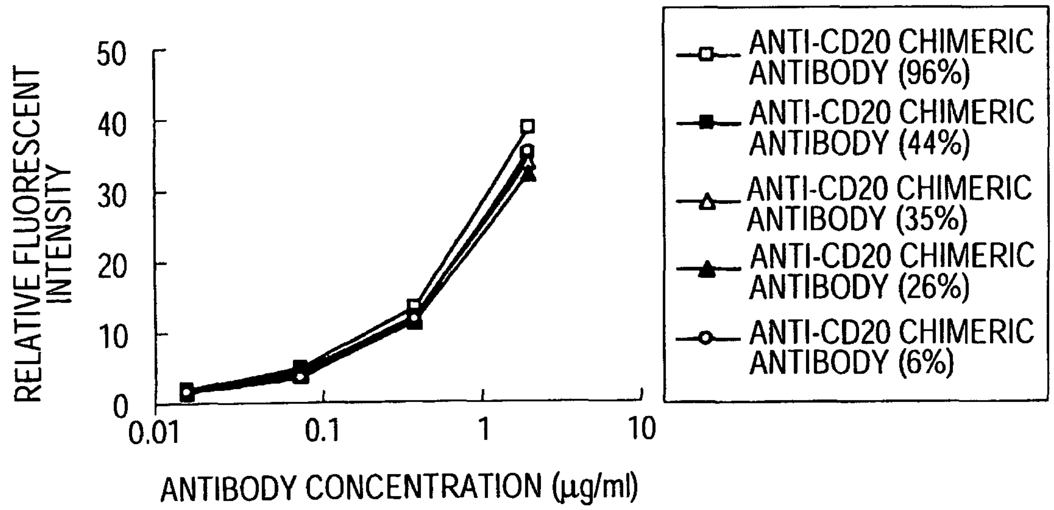

RELATIVE FLUORESCENT INTENSITY
50
40
30
20
10
0
0.01
0.1
1
10
ANTIBODY CONCENTRATION (μg/ml)
ANTI-CD20 CHIMERIC ANTIBODY (96%)
ANTI-CD20 CHIMERIC ANTIBODY (44%)
ANTI-CD20 CHIMERIC ANTIBODY (35%)
ANTI-CD20 CHIMERIC ANTIBODY (26%)
ANTI-CD20 CHIMERIC ANTIBODY (6%)


FIG. 23

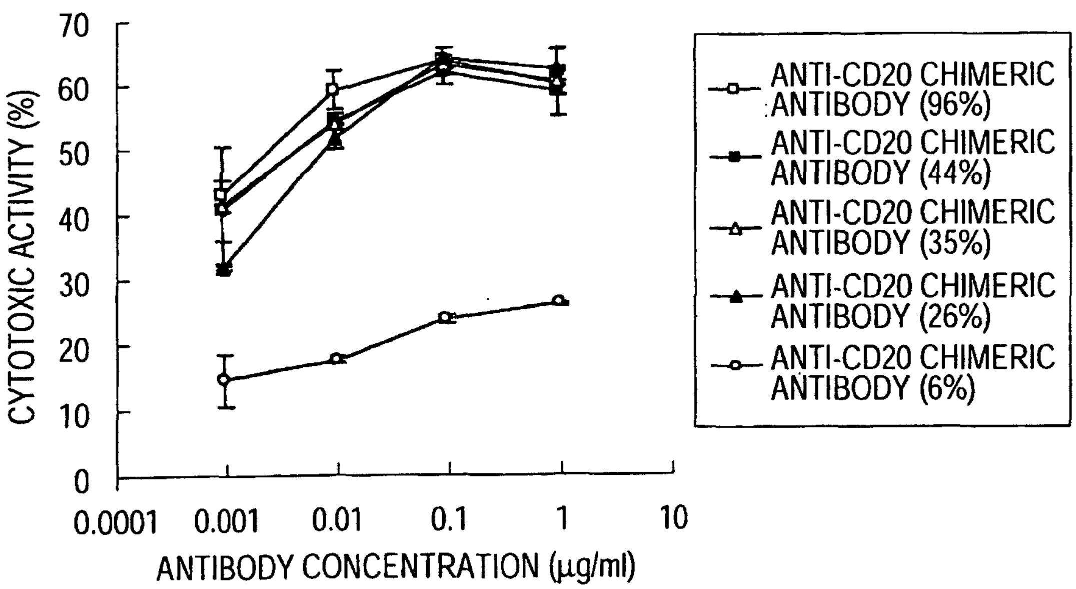

CYTOTOXIC ACTIVITY (%)
70
60
50
40
30
20
10
0
0.0001
0.001
0.01
0.1
1
10
ANTIBODY CONCENTRATION (μg/ml)
ANTI-CD20 CHIMERIC ANTIBODY (96%)
ANTI-CD20 CHIMERIC ANTIBODY (44%)
ANTI-CD20 CHIMERIC ANTIBODY (35%)
ANTI-CD20 CHIMERIC ANTIBODY (26%)
ANTI-CD20 CHIMERIC ANTIBODY (6%)

FIG. 24

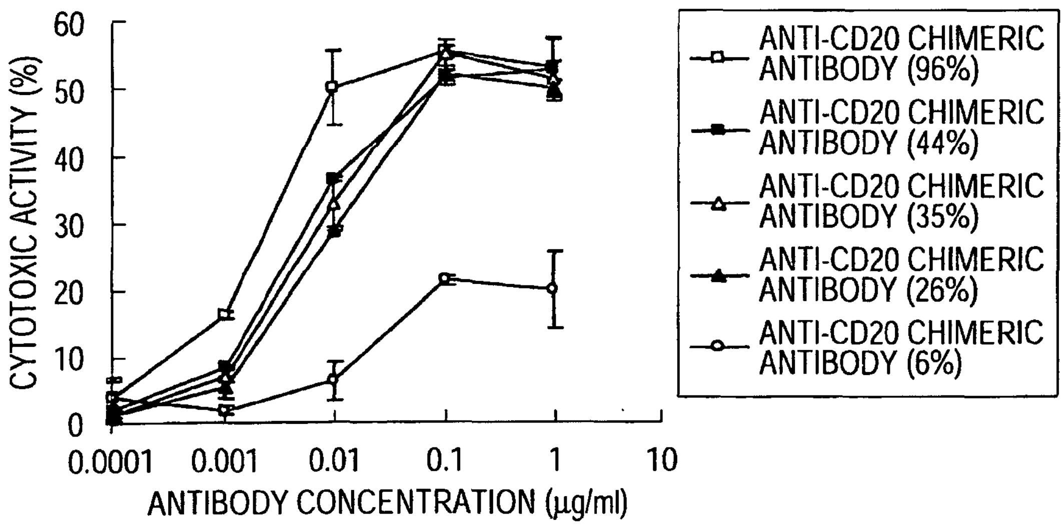
CYTOTOXIC ACTIVITY (%)
60
50
40
30
20
10
0
0.0001
0.001
0.01
0.1
1
10
ANTIBODY CONCENTRATION (µg/ml)
ANTI-CD20 CHIMERIC ANTIBODY (96%)
ANTI-CD20 CHIMERIC ANTIBODY (44%)
ANTI-CD20 CHIMERIC ANTIBODY (35%)
ANTI-CD20 CHIMERIC ANTIBODY (26%)
ANTI-CD20 CHIMERIC ANTIBODY (6%)

FIG. 25

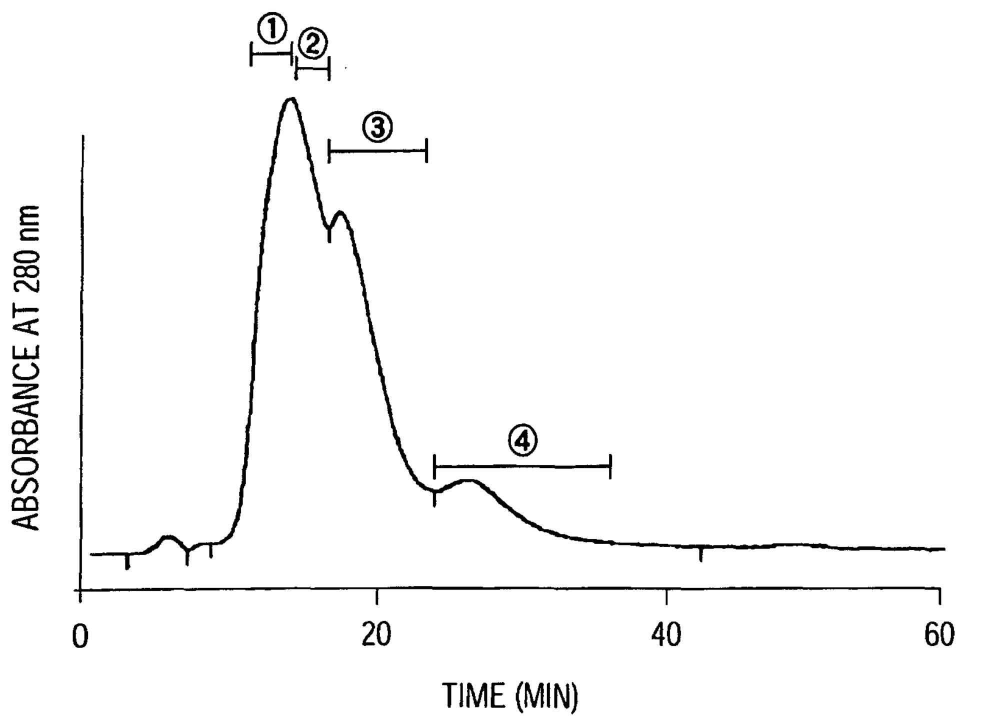
①
②
③
④
ABSORBANCE AT 280 nm
0
20
40
60
TIME (MIN)

FIG. 26

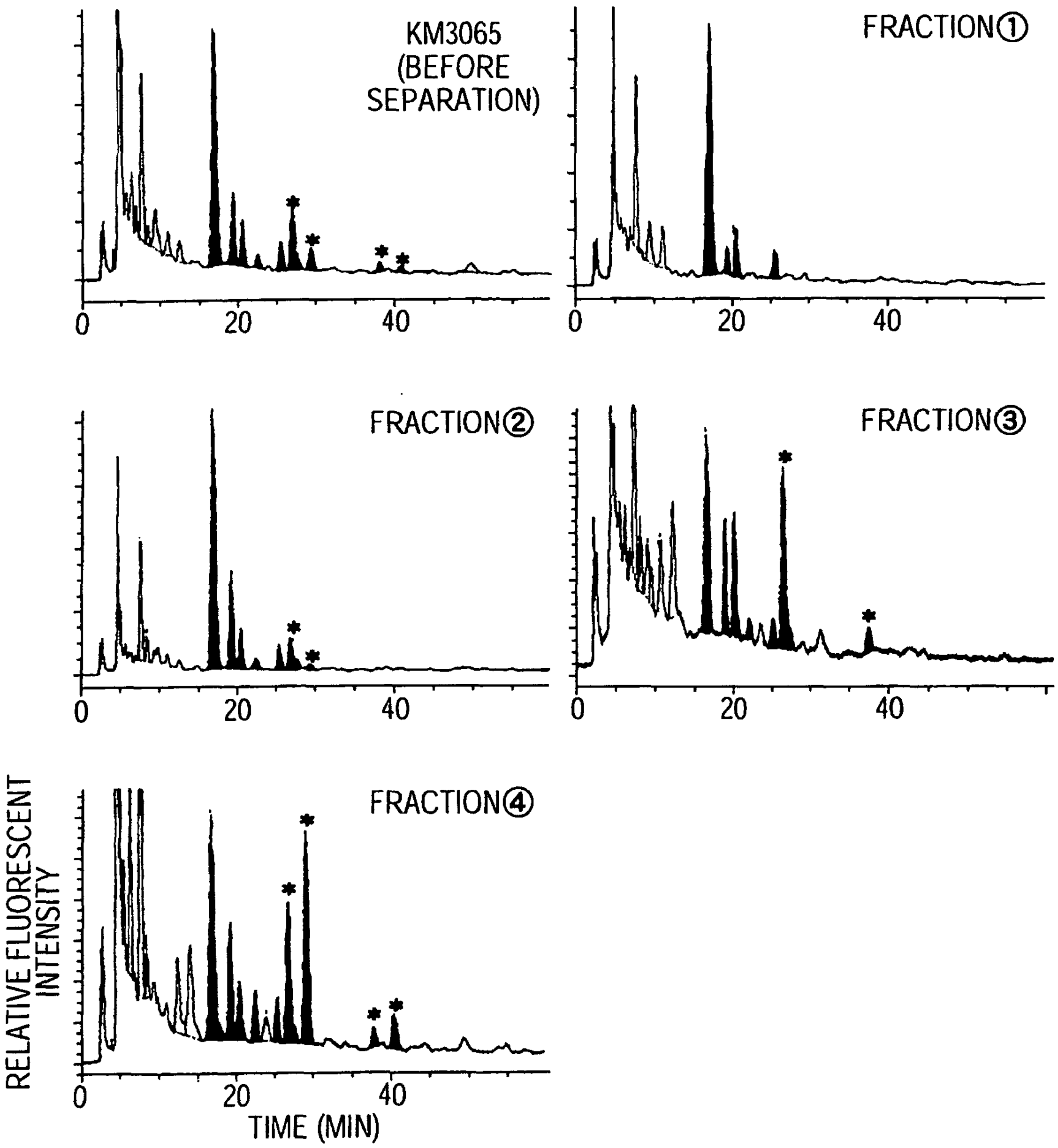
KM3065
(BEFORE
SEPARATION)
FRACTION①
FRACTION②
FRACTION③
FRACTION④
RELATIVE FLUORESCENT
INTENSITY
0
20
40
TIME (MIN)

FIG. 27

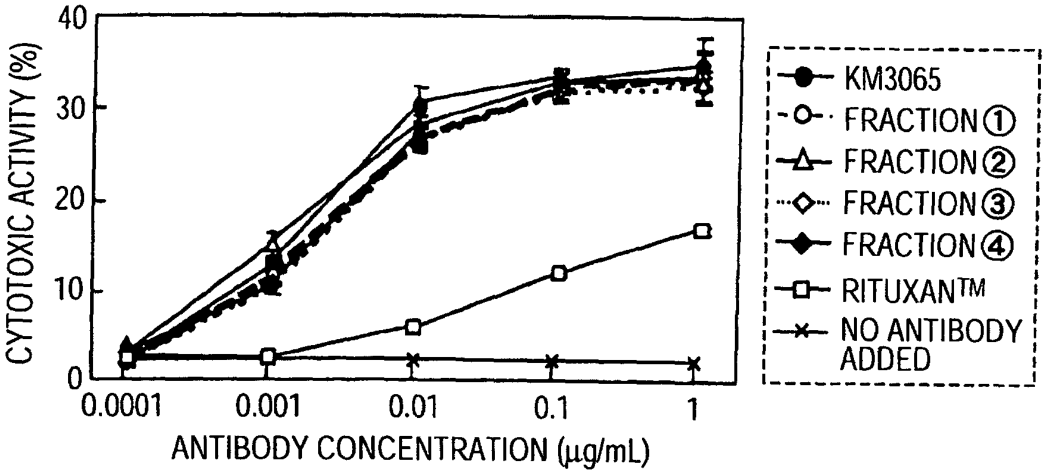
40
30
20
10
0
CYTOTOXIC ACTIVITY (%)
0.0001
0.001
0.01
0.1
1
ANTIBODY CONCENTRATION (μg/mL)
KM3065
FRACTION ①
FRACTION ②
FRACTION ③
FRACTION ④
RITUXAN™
NO ANTIBODY ADDED

**INTERNATIONAL SEARCH REPORT**

International application No.
PCT/JP02/13534

A. CLASSIFICATION OF SUBJECT MATTER
Int.Cl[7] C12N5/10, C12N15/00, A01K67/027, A01H5/00, C07K16/28, A61K39/395, A61P35/00, A61P37/00

According to International Patent Classification (IPC) or to both national classification and IPC

B. FIELDS SEARCHED

Minimum documentation searched (classification system followed by classification symbols)
Int.Cl[7] C12N5/10, C12N15/00, A01K67/027, A01H5/00, C07K16/28, A61K39/395, A61P35/00, A61P37/00

Documentation searched other than minimum documentation to the extent that such documents are included in the fields searched

Electronic data base consulted during the international search (name of data base and, where practicable, search terms used)
WPI, BIOSIS, PIR/SWISSPROT/GeneSeq

C. DOCUMENTS CONSIDERED TO BE RELEVANT

| Category* | Citation of document, with indication, where appropriate, of the relevant passages | Relevant to claim No. |
|---|---|---|
| T | SHINKAWA T. et al., The Absence of Fucose but Not the Presence of Galactose or Bisecting N-Acetylglucosamine of Human IgG1 Complex-type Oligosaccharides Shows the Critical Role of Enhancing Antibody-dependent Cellular Cytotoxicity. J.Biol.Chem., Jan.2003, Vol.278, No.5, pages 3466 to 3473 | 1-48 |
| A | Esohe E. Idusogie et al., Mapping of the C1q Binding Site on Rituxan, a Chimeric Antibody with a Human IgG1 Fc. J.Immunol., 2000, Vol.164, pages 4178 to 4184 | 1-48 |

[X] Further documents are listed in the continuation of Box C. [ ] See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 04 March, 2003 (04.03.03) | 18 March, 2003 (18.03.03) |
| Name and mailing address of the ISA/ Japanese Patent Office | Authorized officer |
| Facsimile No. | Telephone No. |

Form PCT/ISA/210 (second sheet) (July 1998)

**INTERNATIONAL SEARCH REPORT**

International application No.
PCT/JP02/13534

C (Continuation). DOCUMENTS CONSIDERED TO BE RELEVANT

| Category* | Citation of document, with indication, where appropriate, of the relevant passages | Relevant to claim No. |
|---|---|---|
| A | Davies J. et al., Expression of GnTIII in a recombinant anti-CD20 CHO production cell line: Expression of antibodies with altered glycoforms leads to an increase in ADCC through higher affinity for FC gamma RIII. Biotechnol.Bioeng., Aug.2001, Vol.74, No.4, pages 288 to 294 | 1-48 |
| P,A | WO 02/31140 A1 (Kyowa Hakko Kogyo Co., Ltd.), 18 April, 2002 (18.04.02), (Family: none) | 1-48 |
| A | US 5869307 A (Genetics Inst. Inc.), 09 February, 1999 (09.02.99), (Family: none) | 4-6 |
| A | WO 99/64618 A1 (DCV INC. DBA BIO-TECH RESOURSES), 16 December, 1999 (16.12.99), & EP 1084267 A & JP 2002-517256 A | 4,7,8 |
| A | WO 97/37683 A1 (CYTEL CORP.), 16 October, 1997 (16.10.97), & EP 904101 A1 & JP 2002-503082 A | 4,9,10 |
| A | Hayashi H. et al., Molecular cloning of mouse alpha-1,6-fucosyltransferase and expression of its mRNA in the developing cerebrum. DNA Seq., 2000, Vol.11, No.1-2, pages 91 to 96 | 11-13 |

Form PCT/ISA/210 (continuation of second sheet) (July 1998)